# EUROPEAN PATENT APPLICATION

(11) **EP 4 534 524 A1**
(43) Date of publication of application: **09.04.2025**
(21) Application number: 23816181.4
(22) Date of filing: 02.06.2023
(51) Int. Cl.: C07C 229/08, C07C 227/18, C07C 231/02, C07C 233/47, C07D 209/18, C07D 213/55, C07D 213/61, C07D 213/62, C07D 213/74, C07D 213/79, C07D 215/12, C07D 217/26, C07D 231/56, C07D 401/12, C07D 405/12, C07D 409/12, C07D 413/12, C07D 471/04, C07D 495/04, C12N 15/09, C40B 40/10

(54) **AMINO ACID ACTIVE ESTER AND SALT THEREOF**

(30) Priority: 03.06.2022 JP 2022091201
(71) Applicant: PeptiDream Inc., Kawasaki-shi, Kanagawa 210-0821 (JP)
(72) Inventor: MATSUDA, Ayumu, Kawasaki-shi, Kanagawa 210-0821 (JP); CARY, Douglas Robert, Kawasaki-shi, Kanagawa 210-0821 (JP); MATSUI, Katsuma, Kawasaki-shi, Kanagawa 210-0821 (JP); BASHIRUDDIN, Nasir Kato, Kawasaki-shi, Kanagawa 210-0821 (JP); MASUYA, Keiichi, Kawasaki-shi, Kanagawa 210-0821 (JP)
(74) Representative: Mewburn Ellis LLP
(86) International application number: PCT/JP2023/021439
(87) International publication number: WO 2023/234425

(57) **Abstract**

The present invention provides a novel amino acid active ester for use in the aminoacylation of a tRNA, said ester being capable of reducing safety and waste risks. The present invention provides a compound represented by formula (I): 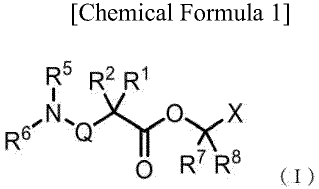 or a salt thereof, in particular dichloropyridinyl methyl ester and 2,2,2-trifluoroethyl ester or a salt thereof.

## Description

### TECHNICAL FIELD

The present disclosure relates to an amino acid active ester and a method for producing an aminoacylated tRNA using the ester.

### BACKGROUND ART

In recent years, among peptide therapeutics, special peptides containing unnatural amino acids are expected not only to exhibit a high affinity and specificity for target molecules but also to be excellent in in vivo stability and membrane permeability, making them important candidates for drug discovery. The methods for synthetizing special peptides reported to date include a method for synthetizing a special peptide from various amino acids or amino acid derivatives including unnatural amino acids with a flexizyme that catalyzes the aminoacylation of a tRNA (PTLs 1 to 6 and NPLs 4 and 5). In this method, dinitrobenzyl esters (DBEs) and cyanomethyl esters (CMEs) have conventionally been used as amino acid active esters for use in the aminoacylation of a tRNA. It is also disclosed that various esters, including picolyl esters, pyridoxy esters and 2,2,2-trifluoroethyl esters, are used in the synthesis of amino acid esters and the synthesis of peptides (PTLs 7 to 13 and NPLs 1 to 3). PTL 14 discloses the use of 2,6-dichloro-4-pyridinemethanol derivatives as an agricultural chemical.

### CITATION LIST

### PATENT LITERATURE

PTL 1: International Publication No. WO 2007/066627 A1
PTL 2: International Publication No. WO 2008/059823 A1
PTL 3: International Publication No. WO 2011/049157 A1
PTL 4: International Publication No. WO 2015/030014 A1
PTL 5: Japanese Patent Laid-Open No. 2008-125396
PTL 6: International Publication No. WO 2020/040840 A2
PTL 7: British Patent No. 1212533
PTL 8: European Patent Publication No. EP 0 450 356 A1
PTL 9: International Publication No. WO 2010/057961 A1
PTL 10: International Publication No. WO 2013/100132 A1
PTL 11: International Publication No. WO 2016/118877 A1
PTL 12: International Publication No. WO 2018/174078 A1
PTL 13: International Publication No. WO 2018/225864 A1
PTL 14: International Publication No. WO 1999/012907 A1

### NON PATENT LITERATURE

NPL 1: J.A.Maclaren, Aust.J.Chem. 1972, 25, 1293-1299.
NPL 2: J.A.Maclaren, Aust.J.Chem. 1978, 31, 1865-1868.
NPL 3: Sklyarov et al., Russian Journal of Bioorganic Chemistry 2000, 26, 245-256.
NPL 4: Murakami et al., 2003, Chemistry & Biology, 10, 655-662.
NPL 5: Journal of Bioscience and Bioengineering, 93 (12), 744.

### SUMMARY OF INVENTION

### TECHNICAL PROBLEM

DBE and CME, which have been conventionally used as amino acid active esters for use in the aminoacylation of tRNAs, are explosive nitro compounds or highly toxic cyanogen compounds, and therefore require careful and complicated handling and associated costs to ensure safety during use, storage and disposal thereof.

The present invention provides a novel amino acid active ester for use in the aminoacylation of a tRNA, which is easy to handle during use, storage and disposal, and can also reduce handling costs.

### SOLUTION TO PROBLEM

After diligent research to obtain an active ester alternative for DBE and CME, the present inventors have found dichloropyridinyl methyl ester and 2,2,2-trifluoroethyl ester. They have confirmed that the aminoacylation of a tRNA with a flexizyme proceeds by using these amino acid active esters, and they have found that the esters are easy to handle during use, storage and disposal and can also reduce handling costs.

The present specification includes the disclosure of the following inventions.
[1-1] A compound represented by formula (I): 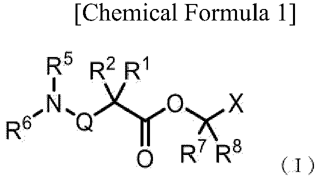 wherein:
   X is pyridyl substituted with two or more halogen atoms, or a C₁₋₃ alkyl substituted with three or more fluorine atoms;
   Q is a group represented by 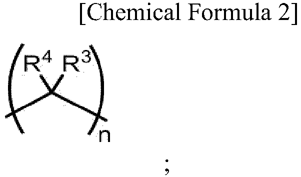
   n is an integer from 0 to 3, and preferably an integer from 0 to 2;
   R⁷ and R⁸ are each independently selected from a hydrogen atom or a C₁₋₃ alkyl;
   R¹ and R² are each independently selected from a hydrogen atom, a halogen atom, cyano, a C₁₋₁₀ alkyl optionally substituted with one or more substituents selected from Y¹, a C₂₋₁₀ alkenyl optionally substituted with one or more substituents selected from Y¹, a C₂₋₁₀ alkynyl optionally substituted with one or more substituents selected from Y¹, a C₃₋₁₀ cycloalkyl optionally substituted with one or more substituents selected from Y², a C₃₋₁₀ cycloalkenyl optionally substituted with one or more substituents selected from Y², a 3- to 14-membered non-aromatic heterocyclyl optionally substituted with one or more substituents selected from Y², a C₆₋₁₄ aryl optionally substituted with one or more substituents selected from Y³, a 5- to 14-membered heteroaryl optionally substituted with one or more substituents selected from Y³, -COOR¹¹, and -CONR¹²R¹³; or
   R¹ and R⁵, together with the carbon atom and the nitrogen atom, respectively, to which they are attached, form a 3- to 10-membered heterocyclic ring optionally substituted with one or more substituents selected from Y²;
   R¹ and R², together with the carbon atom to which they are attached, form a C₃₋₁₀ carbocyclic ring optionally substituted with one or more substituents selected from Y², or a 3- to 10-membered heterocyclic ring optionally substituted with one or more substituents selected from Y²;
   R³ and R⁴ are each independently selected from a hydrogen atom, a halogen atom, cyano, a C₁₋₁₀ alkyl optionally substituted with one or more substituents selected from Y¹, a C₂₋₁₀ alkenyl optionally substituted with one or more substituents selected from Y¹, a C₂₋₁₀ alkynyl optionally substituted with one or more substituents selected from Y¹, a C₃₋₁₀ cycloalkyl optionally substituted with one or more substituents selected from Y², a C₃₋₁₀ cycloalkenyl optionally substituted with one or more substituents selected from Y², a 3- to 14-membered non-aromatic heterocyclyl optionally substituted with one or more substituents selected from Y², a C₆₋₁₄ aryl optionally substituted with one or more substituents selected from Y³, and a 5- to 14-membered heteroaryl optionally substituted with one or more substituents selected from Y³, -COOR¹¹, and -CONR¹²R¹³; or
   R³ and R⁴, together with the carbon atom to which they are attached, form a C₃₋₁₀ carbocyclic ring optionally substituted with one or more substituents selected from Y², or a 3- to 10-membered heterocyclic ring optionally substituted with one or more substituents selected from Y²;
   R¹¹, R¹² and R¹³ are each independently selected from a C₁₋₁₀ alkyl optionally substituted with one or more substituents selected from Y¹, a C₂₋₁₀ alkenyl optionally substituted with one or more substituents selected from Y¹, a C₂₋₁₀ alkynyl optionally substituted with one or more substituents selected from Y¹, a C₃₋₁₀ cycloalkyl optionally substituted with one or more substituents selected from Y², a C₃₋₁₀ cycloalkenyl optionally substituted with one or more substituents selected from Y², a 3- to 14-membered non-aromatic heterocyclyl optionally substituted with one or more substituents selected from Y², a C₆₋₁₄ aryl optionally substituted with one or more substituents selected from Y³, and a 5- to 14-membered heteroaryl optionally substituted with one or more substituents selected from Y³;
   each Y¹ is independently selected from a halogen atom, nitro, cyano, a C₃₋₁₀ cycloalkyl optionally substituted with one or more substituents selected from Y⁵, a C₃₋₁₀ cycloalkenyl optionally substituted with one or more substituents selected from Y⁵, a 3- to 14-membered non-aromatic heterocyclyl optionally substituted with one or more substituents selected from Y⁵, a C₆₋₁₄ aryl optionally substituted with one or more substituents selected from Y⁶, a 5- to 14-membered heteroaryl optionally substituted with one or more substituents selected from Y⁶, -SH, -COOR²¹, -NR²²R²³, -S(O)ₚR²⁴, -OR²⁵, -SO₂NR²⁶R²⁷ and - CONR²⁸R²⁹, and oxo;
   each Y² is independently selected from a halogen atom, nitro, cyano, a C₁₋₁₀ alkyl optionally substituted with one or more substituents selected from Y⁴, a C₂₋₁₀ alkenyl optionally substituted with one or more substituents selected from Y⁴, a C₂₋₁₀ alkynyl optionally substituted with one or more substituents selected from Y⁴, a C₃₋₁₀ cycloalkyl optionally substituted with one or more substituents selected from Y⁵, a C₃₋₁₀ cycloalkenyl optionally substituted with one or more substituents selected from Y⁵, a 3- to 14-membered non-aromatic heterocyclyl optionally substituted with one or more substituents selected from Y⁵, a C₆₋₁₄ aryl optionally substituted with one or more substituents selected from Y⁶, a 5- to 14-membered heteroaryl optionally substituted with one or more substituents selected from Y⁶, -SH, -COOR²¹, -NR²²R²³, -S(O)ₚR²⁴, -OR²⁵, -SO₂NR²⁶R²⁷ and -CONR²⁸R²⁹, and oxo;
   each Y³ is independently selected from a halogen atom, nitro, cyano, a C₁₋₁₀ alkyl optionally substituted with one or more substituents selected from Y⁴, a C₂₋₁₀ alkenyl optionally substituted with one or more substituents selected from Y⁴, a C₂₋₁₀ alkynyl optionally substituted with one or more substituents selected from Y⁴, a C₃₋₁₀ cycloalkyl optionally substituted with one or more substituents selected from Y⁵, a C₃₋₁₀ cycloalkenyl optionally substituted with one or more substituents selected from Y⁵, a 3- to 14-membered non-aromatic heterocyclyl optionally substituted with one or more substituents selected from Y⁵, a C₆₋₁₄ aryl optionally substituted with one or more substituents selected from Y⁶, a 5- to 14-membered heteroaryl optionally substituted with one or more substituents selected from Y⁶, -SH, -COOR²¹, -NR²²R²³, -S(O)ₚR²⁴, -OR²⁵, -SO₂NR²⁶R²⁷ and -CONR²⁸R²⁹;
   R²¹, R²⁶, R²⁷, R²⁸ and R²⁹ are each independently selected from a hydrogen atom, a C₁₋₁₀ alkyl optionally substituted with one or more substituents selected from Y⁴, a C₂₋₁₀ alkenyl optionally substituted with one or more substituents selected from Y⁴, a C₂₋₁₀ alkynyl optionally substituted with one or more substituents selected from Y⁴, a C₃₋₁₀ cycloalkyl optionally substituted with one or more substituents selected from Y⁵, a C₃₋₁₀ cycloalkenyl optionally substituted with one or more substituents selected from Y⁵, a 3- to 14-membered non-aromatic heterocyclyl optionally substituted with one or more substituents selected from Y⁵, a C₆₋₁₄ aryl optionally substituted with one or more substituents selected from Y⁶, and a 5- to 14-membered heteroaryl optionally substituted with one or more substituents selected from Y⁶;
   R²² and R²⁵ are each independently selected from a hydrogen atom, a C₁₋₁₀ alkyl optionally substituted with one or more substituents selected from Y⁴, a C₂₋₁₀ alkenyl optionally substituted with one or more substituents selected from Y⁴, a C₂₋₁₀ alkynyl optionally substituted with one or more substituents selected from Y⁴, a C₃₋₁₀ cycloalkyl optionally substituted with one or more substituents selected from Y⁵, a C₃₋₁₀ cycloalkenyl optionally substituted with one or more substituents selected from Y⁵, a 3- to 14-membered non-aromatic heterocyclyl optionally substituted with one or more substituents selected from Y⁵, a C₆₋₁₄ aryl optionally substituted with one or more substituents selected from Y⁶, a 5- to 14-membered heteroaryl optionally substituted with one or more substituents selected from Y⁶, -COR^{a1}, -CONR^{a2}R^{a3}, -COOR^{a4}, -C(=NR^{a5})NR^{a6}R^{a7}, and -SO₂NR^{a8}R^{a9};
   R²³ is selected from a hydrogen atom, a C₁₋₁₀ alkyl optionally substituted with one or more substituents selected from Y⁴, a C₂₋₁₀ alkenyl optionally substituted with one or more substituents selected from Y⁴, a C₂₋₁₀ alkynyl optionally substituted with one or more substituents selected from Y⁴, a C₃₋₁₀ cycloalkyl optionally substituted with one or more substituents selected from Y⁵, a C₃₋₁₀ cycloalkenyl optionally substituted with one or more substituents selected from Y⁵, a 3- to 14-membered non-aromatic heterocyclyl optionally substituted with one or more substituents selected from Y⁵, a C₆₋₁₄ aryl optionally substituted with one or more substituents selected from Y⁶, and a 5- to 14-membered heteroaryl optionally substituted with one or more substituents selected from Y⁶;
   R²⁴ is selected from a C₁₋₁₀ alkyl optionally substituted with one or more substituents selected from Y⁴, a C₂₋₁₀ alkenyl optionally substituted with one or more substituents selected from Y⁴, a C₂₋₁₀ alkynyl optionally substituted with one or more substituents selected from Y⁴, a C₃₋₁₀ cycloalkyl optionally substituted with one or more substituents selected from Y⁵, a C₃₋₁₀ cycloalkenyl optionally substituted with one or more substituents selected from Y⁵, a 3-to 14-membered non-aromatic heterocyclyl optionally substituted with one or more substituents selected from Y⁵, a C₆₋₁₄ aryl optionally substituted with one or more substituents selected from Y⁶, and a 5- to 14-membered heteroaryl optionally substituted with one or more substituents selected from Y⁶;
   R^{a1}, R^{a2}, R^{a3}, R^{a4}, R^{a5}, R^{a6}, R^{a7}, R^{a8} and R^{a9} are each independently selected from a hydrogen atom, a C₁₋₁₀ alkyl optionally substituted with one or more substituents selected from Y⁴, a C₂₋₁₀ alkenyl optionally substituted with one or more substituents selected from Y⁴, a C₂₋₁₀ alkynyl optionally substituted with one or more substituents selected from Y⁴, a C₃₋₁₀ cycloalkyl optionally substituted with one or more substituents selected from Y⁵, a C₃₋₁₀ cycloalkenyl optionally substituted with one or more substituents selected from Y⁵, a 3- to 14-membered non-aromatic heterocyclyl optionally substituted with one or more substituents selected from Y⁵, a C₆₋₁₄ aryl optionally substituted with one or more substituents selected from Y⁶, and a 5- to 14-membered heteroaryl optionally substituted with one or more substituents selected from Y⁶;
   each Y⁴ is independently selected from a halogen atom, nitro, cyano, a C₃₋₁₀ cycloalkyl optionally substituted with one or more substituents selected from Y⁵, a C₃₋₁₀ cycloalkenyl optionally substituted with one or more substituents selected from Y⁵, a 3- to 14-membered non-aromatic heterocyclyl optionally substituted with one or more substituents selected from Y⁵, a C₆₋₁₄ aryl optionally substituted with one or more substituents selected from Y⁶, a 5- to 14-membered heteroaryl optionally substituted with one or more substituents selected from Y⁶, -SH, -COOR³¹, -NR³²R³³, -S(O)ₚR¹⁴, -OR³⁵, -SO₂NR³⁶R³⁷ and - CONR³⁸R³⁹, and oxo;
   each Y⁵ is independently selected from a halogen atom, nitro, cyano, a C₁₋₁₀ alkyl optionally substituted with one or more substituents selected from Y⁷, a C₂₋₁₀ alkenyl optionally substituted with one or more substituents selected from Y⁷, a C₂₋₁₀ alkynyl optionally substituted with one or more substituents selected from Y⁷, a C₃₋₁₀ cycloalkyl optionally substituted with one or more substituents selected from Y⁸, a C₃₋₁₀ cycloalkenyl optionally substituted with one or more substituents selected from Y⁸, a 3- to 14-membered non-aromatic heterocyclyl optionally substituted with one or more substituents selected from Y⁸, a C₆₋₁₄ aryl optionally substituted with one or more substituents selected from Y⁹, a 5- to 14-membered heteroaryl optionally substituted with one or more substituents selected from Y⁹, -SH, -COOR³¹, -NR³²R³³, -S(O)ₚR³⁴, -OR³⁵, -SO₂NR³⁶R³⁷, -CONR³⁸R³⁹, and oxo;
   each Y⁶ is independently selected from a halogen atom, nitro, cyano, a C₁₋₁₀ alkyl optionally substituted with one or more substituents selected from Y⁷, a C₂₋₁₀ alkenyl optionally substituted with one or more substituents selected from Y⁷, a C₂₋₁₀ alkynyl optionally substituted with one or more substituents selected from Y⁷, a C₃₋₁₀ cycloalkyl optionally substituted with one or more substituents selected from Y⁸, a C₃₋₁₀ cycloalkenyl optionally substituted with one or more substituents selected from Y⁸, a 3- to 14-membered non-aromatic heterocyclyl optionally substituted with one or more substituents selected from Y⁸, a C₆₋₁₄ aryl optionally substituted with one or more substituents selected from Y⁹, a 5- to 14-membered heteroaryl optionally substituted with one or more substituents selected from Y⁹, -SH, -COOR³¹, -NR³²R³³, -S(O)ₚR³⁴, -OR³⁵, -SO₂NR³⁶R³⁷, and -CONR³⁸R³⁹;
   R³¹, R³⁶, R³⁷, R³⁸ and R³⁹ are each independently selected from a hydrogen atom, a C₁₋₁₀ alkyl optionally substituted with one or more substituents selected from Y⁷, a C₂₋₁₀ alkenyl optionally substituted with one or more substituents selected from Y⁷, a C₂₋₁₀ alkynyl optionally substituted with one or more substituents selected from Y⁷, a C₃₋₁₀ cycloalkyl optionally substituted with one or more substituents selected from Y⁸, a C₃₋₁₀ cycloalkenyl optionally substituted with one or more substituents selected from Y⁸, a 3- to 14-membered non-aromatic heterocyclyl optionally substituted with one or more substituents selected from Y⁸, a C₆₋₁₄ aryl optionally substituted with one or more substituents selected from Y⁹, and a 5- to 14-membered heteroaryl optionally substituted with one or more substituents selected from Y⁹;
   R³² and R³⁵ are each independently selected from a hydrogen atom, a C₁₋₁₀ alkyl optionally substituted with one or more substituents selected from Y⁷, a C₂₋₁₀ alkenyl optionally substituted with one or more substituents selected from Y⁷, a C₂₋₁₀ alkynyl optionally substituted with one or more substituents selected from Y⁷, a C₃₋₁₀ cycloalkyl optionally substituted with one or more substituents selected from Y⁸, a C₃₋₁₀ cycloalkenyl optionally substituted with one or more substituents selected from Y⁸, a 3- to 14-membered non-aromatic heterocyclyl optionally substituted with one or more substituents selected from Y⁸, a C₆₋₁₄ aryl optionally substituted with one or more substituents selected from Y⁹, a 5- to 14-membered heteroaryl optionally substituted with one or more substituents selected from Y⁹, -COR^{b1}, -CONR^{b2}R^{b3}, -CO₂R^{b4}, -C(=NR^{b5})NR^{b6}R^{b7} and -SO₂NR^{b8}R^{b9};
   R³³ is selected from a hydrogen atom, a C₁₋₁₀ alkyl optionally substituted with one or more substituents selected from Y⁷, a C₂₋₁₀ alkenyl optionally substituted with one or more substituents selected from Y⁷, a C₂₋₁₀ alkynyl optionally substituted with one or more substituents selected from Y⁷, a C₃₋₁₀ cycloalkyl optionally substituted with one or more substituents selected from Y⁸, a C₃₋₁₀ cycloalkenyl optionally substituted with one or more substituents selected from Y⁸, a 3- to 14-membered non-aromatic heterocyclyl optionally substituted with one or more substituents selected from Y⁸, a C₆₋₁₄ aryl optionally substituted with one or more substituents selected from Y⁹, and a 5- to 14-membered heteroaryl optionally substituted with one or more substituents selected from Y⁹;
   R³⁴ is selected from a C₁₋₁₀ alkyl optionally substituted with one or more substituents selected from Y⁷, a C₂₋₁₀ alkenyl optionally substituted with one or more substituents selected from Y⁷, a C₂₋₁₀ alkynyl optionally substituted with one or more substituents selected from Y⁷, a C₃₋₁₀ cycloalkyl optionally substituted with one or more substituents selected from Y⁸, a C₃₋₁₀ cycloalkenyl optionally substituted with one or more substituents selected from Y⁸, a 3-to 14-membered non-aromatic heterocyclyl optionally substituted with one or more substituents selected from Y⁸, a C₆₋₁₄ aryl optionally substituted with one or more substituents selected from Y⁹, and a 5- to 14-membered heteroaryl optionally substituted with one or more substituents selected from Y⁹;
   R^{b1}, R^{b2}, R^{b3}, R^{b4}, R^{b5}, R^{b6}, R^{b7}, R^{b8} and R^{b9} are each independently selected from a hydrogen atom, a C₁₋₁₀ alkyl optionally substituted with one or more substituents selected from Y⁷, a C₂₋₁₀ alkenyl optionally substituted with one or more substituents selected from Y⁷, a C₂₋₁₀ alkynyl optionally substituted with one or more substituents selected from Y⁷, a C₃₋₁₀ cycloalkyl optionally substituted with one or more substituents selected from Y⁸, a C₃₋₁₀ cycloalkenyl optionally substituted with one or more substituents selected from Y⁸, a 3- to 14-membered non-aromatic heterocyclyl optionally substituted with one or more substituents selected from Y⁸, a C₆₋₁₄ aryl optionally substituted with one or more substituents selected from Y⁹, and a 5- to 14-membered heteroaryl optionally substituted with one or more substituents selected from Y⁹;
   each Y⁷ is independently selected from a halogen atom, nitro, cyano, a C₁₋₁₀ alkyl optionally substituted with one or more substituents selected from Y¹⁰, a C₂₋₁₀ alkenyl optionally substituted with one or more substituents selected from Y¹⁰, a C₂₋₁₀ alkynyl optionally substituted with one or more substituents selected from Y¹⁰, a C₃₋₁₀ cycloalkyl optionally substituted with one or more substituents selected from Y¹¹, a C₃₋₁₀ cycloalkenyl optionally substituted with one or more substituents selected from Y¹¹, a 3- to 14-membered non-aromatic heterocyclyl optionally substituted with one or more substituents selected from Y¹¹, a C₆₋₁₄ aryl optionally substituted with one or more substituents selected from Y¹², a 5- to 14-membered heteroaryl optionally substituted with one or more substituents selected from Y¹², -SH, -COOR⁴⁰, -NR⁴¹R⁴², -S(O)ₚR⁴³, -OR⁴⁴, -SO₂NR⁴⁵R⁴⁶ and -CONR⁴⁷R⁴⁸, and oxo;
   each Y⁸ is independently selected from a halogen atom, nitro, cyano, a C₁₋₁₀ alkyl optionally substituted with one or more substituents selected from Y¹⁰, a C₂₋₁₀ alkenyl optionally substituted with one or more substituents selected from Y¹⁰, a C₂₋₁₀ alkynyl optionally substituted with one or more substituents selected from Y¹⁰, a C₃₋₁₀ cycloalkyl optionally substituted with one or more substituents selected from Y¹¹, a C₃₋₁₀ cycloalkenyl optionally substituted with one or more substituents selected from Y¹¹, a 3- to 14-membered non-aromatic heterocyclyl optionally substituted with one or more substituents selected from Y¹¹, a C₆₋₁₄ aryl optionally substituted with one or more substituents selected from Y¹², a 5- to 14-membered heteroaryl optionally substituted with one or more substituents selected from Y¹², -SH, -COOR⁴⁰, -NR⁴¹R⁴², -S(O)ₚR⁴³, -OR⁴⁴, -SO₂NR⁴⁵R⁴⁶, -CONR⁴⁷R⁴⁸, and oxo;
   each Y⁹ is independently selected from a halogen atom, nitro, cyano, a C₁₋₁₀ alkyl optionally substituted with one or more substituents selected from Y¹⁰, a C₂₋₁₀ alkenyl optionally substituted with one or more substituents selected from Y¹⁰, a C₂₋₁₀ alkynyl optionally substituted with one or more substituents selected from Y¹⁰, a C₃₋₁₀ cycloalkyl optionally substituted with one or more substituents selected from Y¹¹, a C₃₋₁₀ cycloalkenyl optionally substituted with one or more substituents selected from Y¹¹, a 3- to 14-membered non-aromatic heterocyclyl optionally substituted with one or more substituents selected from Y¹¹, a C₆₋₁₄ aryl optionally substituted with one or more substituents selected from Y¹², a 5- to 14-membered heteroaryl optionally substituted with one or more substituents selected from Y¹², -SH, -COOR⁴⁰, -NR⁴¹R⁴², -S(O)ₚR⁴³, -OR⁴⁴, -SO₂NR⁴⁵R⁴⁶ and -CONR⁴⁷R⁴⁸;
   R⁴⁰, R⁴⁵, R⁴⁶, R⁴⁷ and R⁴⁸ are each independently selected from a hydrogen atom, a C₁₋₁₀ alkyl optionally substituted with one or more substituents selected from Y¹⁰, a C₂₋₁₀ alkenyl optionally substituted with one or more substituents selected from Y¹⁰, a C₂₋₁₀ alkynyl optionally substituted with one or more substituents selected from Y¹⁰, a C₃₋₁₀ cycloalkyl optionally substituted with one or more substituents selected from Y¹¹, a C₃₋₁₀ cycloalkenyl optionally substituted with one or more substituents selected from Y¹¹, a 3- to 14-membered non-aromatic heterocyclyl optionally substituted with one or more substituents selected from Y¹¹, a C₆₋₁₄ aryl optionally substituted with one or more substituents selected from Y¹², and a 5- to 14-membered heteroaryl optionally substituted with one or more substituents selected from Y¹²,
   R⁴¹ and R⁴⁴ are each independently selected from a hydrogen atom, a C₁₋₁₀ alkyl optionally substituted with one or more substituents selected from Y¹⁰, a C₂₋₁₀ alkenyl optionally substituted with one or more substituents selected from Y¹⁰, a C₂₋₁₀ alkynyl optionally substituted with one or more substituents selected from Y¹⁰, a C₃₋₁₀ cycloalkyl optionally substituted with one or more substituents selected from Y¹¹, a C₃₋₁₀ cycloalkenyl optionally substituted with one or more substituents selected from Y¹¹, a 3- to 14-membered non-aromatic heterocyclyl optionally substituted with one or more substituents selected from Y¹¹, a C₆₋₁₄ aryl optionally substituted with one or more substituents selected from Y¹², a 5- to 14-membered heteroaryl optionally substituted with one or more substituents selected from Y¹², -COR^{c1}, -CONR^{c2}R^{c3}, -COOR^{c4}, -C(=NR^{c5})NR^{c6}R^{c7}, and -SO₂NR^{c8}R^{c9};
   R⁴² is selected from a hydrogen atom, a C₁₋₁₀ alkyl optionally substituted with one or more substituents selected from Y¹⁰, a C₂₋₁₀ alkenyl optionally substituted with one or more substituents selected from Y¹⁰, a C₂₋₁₀ alkynyl optionally substituted with one or more substituents selected from Y¹⁰, a C₃₋₁₀ cycloalkyl optionally substituted with one or more substituents selected from Y¹¹, a C₃₋₁₀ cycloalkenyl optionally substituted with one or more substituents selected from Y¹¹, a 3- to 14-membered non-aromatic heterocyclyl optionally substituted with one or more substituents selected from Y¹¹, a C₆₋₁₄ aryl optionally substituted with one or more substituents selected from Y¹², and a 5- to 14-membered heteroaryl optionally substituted with one or more substituents selected from Y¹²,
   R⁴³ is selected from a C₁₋₁₀ alkyl optionally substituted with one or more substituents selected from Y¹⁰, a C₂₋₁₀ alkenyl optionally substituted with one or more substituents selected from Y¹⁰, a C₂₋₁₀ alkynyl optionally substituted with one or more substituents selected from Y¹⁰, a C₃₋₁₀ cycloalkyl optionally substituted with one or more substituents selected from Y¹¹, a C₃₋₁₀ cycloalkenyl optionally substituted with one or more substituents selected from Y¹¹, a 3- to 14-membered non-aromatic heterocyclyl optionally substituted with one or more substituents selected from Y¹¹, a C₆₋₁₄ aryl optionally substituted with one or more substituents selected from Y¹², and a 5- to 14-membered heteroaryl optionally substituted with one or more substituents selected from Y¹²,
   R^{c1}, R^{c2}, R^{c3}, R^{c4}, R^{c5}, R^{c6}, R^{c7}, R^{c8} and R^{c9} are each independently selected from a hydrogen atom, a C₁₋₁₀ alkyl optionally substituted with one or more substituents selected from Y¹⁰, a C₂₋₁₀ alkenyl optionally substituted with one or more substituents selected from Y¹⁰, a C₂₋₁₀ alkynyl optionally substituted with one or more substituents selected from Y¹⁰, a C₃₋₁₀ cycloalkyl optionally substituted with one or more substituents selected from Y¹¹, a C₃₋₁₀ cycloalkenyl optionally substituted with one or more substituents selected from Y¹¹, a 3- to 14-membered non-aromatic heterocyclyl optionally substituted with one or more substituents selected from Y¹¹, a C₆₋₁₄ aryl optionally substituted with one or more substituents selected from Y¹², and a 5- to 14-membered heteroaryl optionally substituted with one or more substituents selected from Y¹²,
   each Y¹⁰ is independently selected from a halogen atom, nitro, cyano, a C₁₋₁₀ alkoxy selected from a halogen atom and an alkoxy, a C₃₋₁₀ cycloalkyl optionally substituted with one or more substituents selected from a halogen atom and a C₁₋₆ alkoxy, a C₃₋₁₀ cycloalkenyl optionally substituted with one or more substituents selected from a halogen atom and a C₁₋₆ alkoxy, a 3- to 14-membered non-aromatic heterocyclyl optionally substituted with one or more substituents selected from a halogen atom and a C₁₋₆ alkoxy, a C₆₋₁₄ aryl optionally substituted with one or more substituents selected from a halogen atom and a C₁₋₆ alkoxy, a 5-to 14-membered heteroaryl optionally substituted with one or more substituents selected from a halogen atom and a C₁₋₆ alkoxy, a (C₁₋₁₀ alkoxy)carbonyl optionally substituted with one or more substituents selected from a halogen atom and a C₁₋₆ alkoxy, a di(C₁₋₆ alkyl)aminocarbonyl optionally substituted with one or more substituents selected from a halogen atom and a C₁₋₆ alkoxy, a C₁₋₆ alkoxy optionally substituted with one or more substituents selected from a halogen atom and a C₁₋₆ alkoxy, a C₁₋₆ alkylthio optionally substituted with one or more substituents selected from a halogen atom and a C₁₋₆ alkoxy, a C₁₋₆ alkylsulfanyl optionally substituted with one or more substituents selected from a halogen atom and a C₁₋₆ alkoxy, a C₁₋₆ alkylsulfinyl optionally substituted with one or more substituents selected from a halogen atom and a C₁₋₆ alkoxy, a C₁₋₆ alkylsulfonyl optionally substituted with one or more substituents selected from a halogen atom and a C₁₋₆ alkoxy, and oxo;
   each Y¹¹ is independently selected from a halogen atom, nitro, cyano, a C₁₋₁₀ alkyl optionally substituted with one or more substituents selected from a halogen atom and an alkoxy, a C₂₋₁₀ alkenyl optionally substituted with one or more substituents selected from a halogen atom and an alkoxy, a C₂₋₁₀ alkynyl optionally substituted with one or more substituents selected from a halogen atom and an alkoxy, a C₁₋₁₀ alkoxy optionally substituted with one or more substituents selected from a halogen atom and an alkoxy, a C₃₋₁₀ cycloalkyl optionally substituted with one or more substituents selected from a halogen atom and a C₁₋₆ alkoxy, a C₃₋₁₀ cycloalkenyl optionally substituted with one or more substituents selected from a halogen atom and a C₁₋₆ alkoxy, a 3- to 14-membered non-aromatic heterocyclyl optionally substituted with one or more substituents selected from a halogen atom and a C₁₋₆ alkoxy, a C₆₋₁₄ aryl optionally substituted with one or more substituents selected from a halogen atom and a C₁₋₆ alkoxy, a 5- to 14-membered heteroaryl optionally substituted with one or more substituents selected from a halogen atom and a C₁₋₆ alkoxy, a (C₁₋₁₀ alkoxy)carbonyl optionally substituted with one or more substituents selected from a halogen atom and a C₁₋₆ alkoxy, a di(C₁₋₆ alkyl)aminocarbonyl optionally substituted with one or more substituents selected from a halogen atom and a C₁₋₆ alkoxy, a C₁₋₆ alkoxy optionally substituted with one or more substituents selected from a halogen atom and a C₁₋₆ alkoxy, a C₁₋₆ alkylthio optionally substituted with one or more substituents selected from a halogen atom and a C₁₋₆ alkoxy, a C₁₋₆ alkylsulfanyl optionally substituted with one or more substituents selected from a halogen atom and a C₁₋₆ alkoxy, a C₁₋₆ alkylsulfinyl optionally substituted with one or more substituents selected from a halogen atom and a C₁₋₆ alkoxy, a C₁₋₆ alkylsulfonyl optionally substituted with one or more substituents selected from a halogen atom and a C₁₋₆ alkoxy, and oxo;
   each Y¹² is independently selected from a halogen atom, nitro, cyano, a C₁₋₁₀ alkyl optionally substituted with one or more substituents selected from a halogen atom and an alkoxy, a C₂₋₁₀ alkenyl optionally substituted with one or more substituents selected from a halogen atom and an alkoxy, a C₂₋₁₀ alkynyl optionally substituted with one or more substituents selected from a halogen atom and an alkoxy, a C₁₋₁₀ alkoxy optionally substituted with one or more substituents selected from a halogen atom and an alkoxy, a C₃₋₁₀ cycloalkyl optionally substituted with one or more substituents selected from a halogen atom and a C₁₋₆ alkoxy, a C₃₋₁₀ cycloalkenyl optionally substituted with one or more substituents selected from a halogen atom and a C₁₋₆ alkoxy, a 3- to 14-membered non-aromatic heterocyclyl optionally substituted with one or more substituents selected from a halogen atom and a C₁₋₆ alkoxy, a C₆₋₁₄ aryl optionally substituted with one or more substituents selected from a halogen atom and a C₁₋₆ alkoxy, a 5- to 14-membered heteroaryl optionally substituted with one or more substituents selected from a halogen atom and a C₁₋₆ alkoxy, a (C₁₋₁₀ alkoxy)carbonyl optionally substituted with one or more substituents selected from a halogen atom and a C₁₋₆ alkoxy, a di(C₁₋₆ alkyl)aminocarbonyl optionally substituted with one or more substituents selected from a halogen atom and a C₁₋₆ alkoxy, a C₁₋₆ alkoxy optionally substituted with one or more substituents selected from a halogen atom and a C₁₋₆ alkoxy, a C₁₋₆ alkylthio optionally substituted with one or more substituents selected from a halogen atom and a C₁₋₆ alkoxy, a C₁₋₆ alkylsulfanyl optionally substituted with one or more substituents selected from a halogen atom and a C₁₋₆ alkoxy, a C₁₋₆ alkylsulfinyl optionally substituted with one or more substituents selected from a halogen atom and a C₁₋₆ alkoxy, and a C₁₋₆ alkylsulfonyl optionally substituted with one or more substituents selected from a halogen atom and a C₁₋₆ alkoxy;
   each p is independently an integer from 0 to 2;
   R⁵ is selected from a hydrogen atom, benzyl optionally substituted on the benzene ring with one or more substituents selected from Y¹³, benzyloxycarbonyl optionally substituted on the benzene ring with one or more substituents selected from Y¹³, and phenylcarbonyl, (C₁₋₁₀ alkoxy)carbonyl and (C₁₋₁₀ alkyl)carbonyl optionally substituted with one or more substituents selected from Y¹³, a C₁₋₁₀ alkyl optionally substituted with one or more substituents selected from Y¹, a C₂₋₁₀ alkenyl optionally substituted with one or more substituents selected from Y¹, a C₂₋₁₀ alkynyl optionally substituted with one or more substituents selected from Y¹, a C₃₋₁₀ cycloalkyl optionally substituted with one or more substituents selected from Y², a C₃₋₁₀ cycloalkenyl optionally substituted with one or more substituents selected from Y², a 3- to 14-membered non-aromatic heterocyclyl optionally substituted with one or more substituents selected from Y², a C₆₋₁₄ aryl optionally substituted with one or more substituents selected from Y³, and a 5- to 14-membered heteroaryl optionally substituted with one or more substituents selected from Y³;
   R⁶ is selected from a hydrogen atom, benzyl optionally substituted on the benzene ring with one or more substituents selected from Y¹³, benzyloxycarbonyl optionally substituted on the benzene ring with one or more substituents selected from Y¹³, and phenylcarbonyl, (C₁₋₁₀ alkoxy)carbonyl and (C₁₋₁₀ alkyl)carbonyl optionally substituted with one or more substituents selected from Y¹³; or
   R⁵ and R⁶, together with the nitrogen atom to which they are attached, form a 5- to 14-membered nitrogen-containing heterocyclic ring having an imide structure; and
   each Y¹³ is independently selected from a halogen atom, C₁₋₁₀ alkoxy, and C₁₋₁₀ alkyl;
   or a salt thereof.
[1-2] The compound or a salt thereof according to [1-1], wherein n is 0 or 1.
[1-3] The compound or a salt thereof according to [1-1] or [1-2], wherein R¹ is hydrogen atom.
[1-4] The compound or a salt thereof according to any one of [1-1] to [1-3], wherein R⁷ and R⁸ are each hydrogen atom.
[1-5] The compound or a salt thereof according to any of [1-1] to [1-4], wherein X is 2,6-dihalo-4-pyridyl.
[1-6] The compound or a salt thereof according to any of [1-1] to [1-4], wherein X is 2,6-dichloro-4-pyridyl.
[1-7] The compound or a salt thereof according to any of [1-1] to [1-4], wherein X is a perfluoro-C₁₋₃ alkyl.
[1-8] The compound or a salt thereof according to any of [1-1] to [1-4], wherein X is trifluoromethyl.
[1-9] A composition for use in the acylation of a tRNA, containing the compound or a salt thereof of any of [1-1] to [1-8].
[1-10] The composition according to [1-9] for use in the acylation of a tRNA in the presence of a flexizyme.
[1-11] A method for producing a compound represented by formula (I): 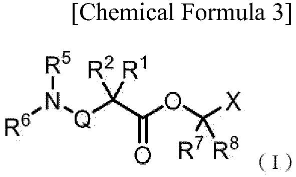
   wherein R¹, R², R⁵, R⁶, R⁷, R⁸, Q and X are as defined in any of [1-1] to [1-8];
   the method including reacting a compound represented by formula (II): 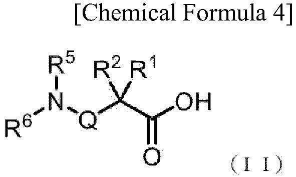
   with a compound represented by formula (III): 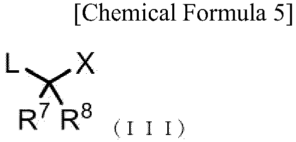
   wherein L is a leaving group.
[1-12] The method according to [1-11], wherein the reaction is carried out in the presence of a base.
[1-13] The method according to [1-12], wherein the base is diisopropylethylamine.
[1-14] A method for producing a tRNA acylated at the 3'-terminus, wherein:
   the acyl group at the 3'-terminus is a group represented by: 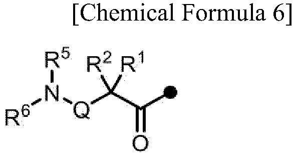
   wherein R¹, R², R⁵, R⁶ and Q are as defined in any of [1-1] to [1-8];
   the method including reacting a compound represented by formula (I): 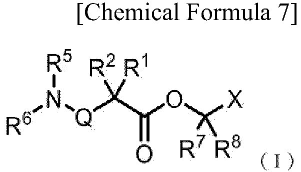
   wherein R¹, R², R⁵, R⁶, R⁷, R⁸, Q and X are as defined in any one of [1-1] to [1-8]; with the tRNA in the presence of a flexizyme.
[1-15] A method for preparing a peptide library, including:
   producing a tRNA acylated at the 3'-terminus by the method according to [1-14];
   preparing an mRNA library; and
   synthesizing a peptide corresponding to each mRNA from the mRNA library with a cell-free translation system to prepare a peptide library.
[1-16] A method for acylating a tRNA at the 3'-terminus, including reacting the tRNA with a compound represented by formula (I): 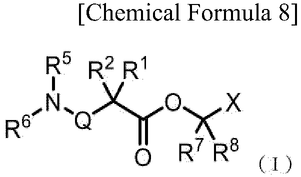
   wherein R¹, R², R⁵, R⁶, R⁷, R⁸, Q and X are as defined in any one of [1-1] to [1-8]; in the presence of a flexizyme, thereby adding an acyl group represented by: 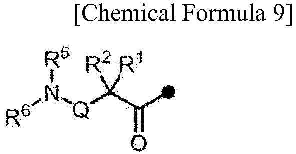
   wherein R¹, R², R⁵, R⁶ and Q are as defined in any of [1-1] to [1-8]; to the tRNA at the 3'-terminus.
[2-1] A compound represented by formula (Ia): 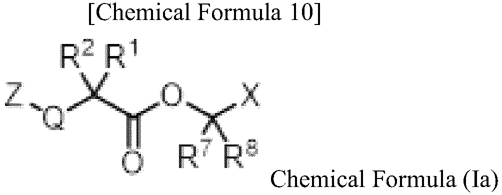 wherein:
   X is pyridyl substituted with two or more halogen atoms, or a C₁₋₃ alkyl substituted with three or more fluorine atoms;
   Z is -NR⁵R⁶ or -OR^{d};
   Q is a group represented by 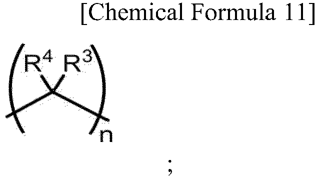
   n is an integer from 0 to 3, and preferably an integer from 0 to 2;
   R⁷ and R⁸ are each independently selected from a hydrogen atom or a C₁₋₃ alkyl;
   R¹ and R² are each independently selected from a hydrogen atom, a halogen atom, cyano, a C₁₋₁₀ alkyl optionally substituted with one or more substituents selected from Y¹, a C₂₋₁₀ alkenyl optionally substituted with one or more substituents selected from Y¹, a C₂₋₁₀ alkynyl optionally substituted with one or more substituents selected from Y¹, a C₃₋₁₀ cycloalkyl optionally substituted with one or more substituents selected from Y², a C₃₋₁₀ cycloalkenyl optionally substituted with one or more substituents selected from Y², a 3- to 14-membered non-aromatic heterocyclyl optionally substituted with one or more substituents selected from Y², a C₆₋₁₄ aryl optionally substituted with one or more substituents selected from Y³, a 5- to 14-membered heteroaryl optionally substituted with one or more substituents selected from Y³, -COOR¹¹, and -CONR¹²R¹³; or
   R¹ and R⁵, together with the carbon atom and the nitrogen atom, respectively, to which they are attached, form a 3- to 10-membered non-aromatic heterocyclic ring optionally substituted with one or more substituents selected from Y²;
   when n is an integer from 1 to 3, R¹ and any one of R³, together with the carbon atoms to which they are attached, form a C₃₋₁₀ carbocyclic ring optionally substituted with one or more substituents selected from Y², or a 3- to 10-membered non-aromatic heterocyclic ring optionally substituted with one or more substituents selected from Y²;
   R¹ and R², together with the carbon atom to which they are attached, form a C₃₋₁₀ carbocyclic ring optionally substituted with one or more substituents selected from Y², or a 3- to 10-membered non-aromatic heterocyclic ring optionally substituted with one or more substituents selected from Y²;
   R³ and R⁴ are each independently selected from a hydrogen atom, a halogen atom, cyano, a C₁₋₁₀ alkyl optionally substituted with one or more substituents selected from Y¹, a C₂₋₁₀ alkenyl optionally substituted with one or more substituents selected from Y¹, a C₂₋₁₀ alkynyl optionally substituted with one or more substituents selected from Y¹, a C₃₋₁₀ cycloalkyl optionally substituted with one or more substituents selected from Y², a C₃₋₁₀ cycloalkenyl optionally substituted with one or more substituents selected from Y², a 3- to 14-membered non-aromatic heterocyclyl optionally substituted with one or more substituents selected from Y², a C₆₋₁₄ aryl optionally substituted with one or more substituents selected from Y³, and a 5- to 14-membered heteroaryl optionally substituted with one or more substituents selected from Y³, -COOR¹¹, and -CONR¹²R¹³; or
   R³ and R⁴, together with the carbon atom to which they are attached, form a C₃₋₁₀ carbocyclic ring optionally substituted with one or more substituents selected from Y², or a 3- to 10-membered non-aromatic heterocyclic ring optionally substituted with one or more substituents selected from Y²;
   when n is an integer from 1 to 3, R⁵ and any one of R³, together with the carbon atom to which they are attached, form a 3- to 10-membered nitrogen-containing non-aromatic heterocyclic ring optionally substituted with one or more substituents selected from Y²;
   R¹¹, R¹² and R¹³ are each independently selected from a C₁₋₁₀ alkyl optionally substituted with one or more substituents selected from Y¹, a C₂₋₁₀ alkenyl optionally substituted with one or more substituents selected from Y¹, a C₂₋₁₀ alkynyl optionally substituted with one or more substituents selected from Y¹, a C₃₋₁₀ cycloalkyl optionally substituted with one or more substituents selected from Y², a C₃₋₁₀ cycloalkenyl optionally substituted with one or more substituents selected from Y², a 3- to 14-membered non-aromatic heterocyclyl optionally substituted with one or more substituents selected from Y², a C₆₋₁₄ aryl optionally substituted with one or more substituents selected from Y³, and a 5- to 14-membered heteroaryl optionally substituted with one or more substituents selected from Y³;
   each Y¹ is independently selected from a halogen atom, nitro, cyano, a C₃₋₁₀ cycloalkyl optionally substituted with one or more substituents selected from Y⁵, a C₃₋₁₀ cycloalkenyl optionally substituted with one or more substituents selected from Y⁵, a 3- to 14-membered non-aromatic heterocyclyl optionally substituted with one or more substituents selected from Y⁵, a C₆₋₁₄ aryl optionally substituted with one or more substituents selected from Y⁶, a 5- to 14-membered heteroaryl optionally substituted with one or more substituents selected from Y⁶, -SH, -COOR²¹, -NR²²R²³, -N₃, -S(O)ₚR²⁴, -OR²⁵, -SO₂NR²⁶R²⁷ and - CONR²⁸R²⁹, and oxo;
   each Y² is independently selected from a halogen atom, nitro, cyano, a C₁₋₁₀ alkyl optionally substituted with one or more substituents selected from Y⁴, a C₂₋₁₀ alkenyl optionally substituted with one or more substituents selected from Y⁴, a C₂₋₁₀ alkynyl optionally substituted with one or more substituents selected from Y⁴, a C₃₋₁₀ cycloalkyl optionally substituted with one or more substituents selected from Y⁵, a C₃₋₁₀ cycloalkenyl optionally substituted with one or more substituents selected from Y⁵, a 3- to 14-membered non-aromatic heterocyclyl optionally substituted with one or more substituents selected from Y⁵, a C₆₋₁₄ aryl optionally substituted with one or more substituents selected from Y⁶, a 5- to 14-membered heteroaryl optionally substituted with one or more substituents selected from Y⁶, -SH, -COOR²¹, -NR²²R²³, -S(O)ₚR²⁴, -OR²⁵, -SO₂NR²⁶R²⁷ and -CONR²⁸R²⁹, and oxo;
   each Y³ is independently selected from a halogen atom, nitro, cyano, a C₁₋₁₀ alkyl optionally substituted with one or more substituents selected from Y⁴, a C₂₋₁₀ alkenyl optionally substituted with one or more substituents selected from Y⁴, a C₂₋₁₀ alkynyl optionally substituted with one or more substituents selected from Y⁴, a C₃₋₁₀ cycloalkyl optionally substituted with one or more substituents selected from Y⁵, a C₃₋₁₀ cycloalkenyl optionally substituted with one or more substituents selected from Y⁵, a 3- to 14-membered non-aromatic heterocyclyl optionally substituted with one or more substituents selected from Y⁵, a C₆₋₁₄ aryl optionally substituted with one or more substituents selected from Y⁶, a 5- to 14-membered heteroaryl optionally substituted with one or more substituents selected from Y⁶, -SH, -COOR²¹, -NR²²R²³, -S(O)ₚR²⁴, -OR²⁵, -SO₂NR²⁶R²⁷ and -CONR²⁸R²⁹;
   R²¹, R²⁶, R²⁷, R²⁸ and R²⁹ are each independently selected from a hydrogen atom, a C₁₋₁₀ alkyl optionally substituted with one or more substituents selected from Y⁴, a C₂₋₁₀ alkenyl optionally substituted with one or more substituents selected from Y⁴, a C₂₋₁₀ alkynyl optionally substituted with one or more substituents selected from Y⁴, a C₃₋₁₀ cycloalkyl optionally substituted with one or more substituents selected from Y⁵, a C₃₋₁₀ cycloalkenyl optionally substituted with one or more substituents selected from Y⁵, a 3- to 14-membered non-aromatic heterocyclyl optionally substituted with one or more substituents selected from Y⁵, a C₆₋₁₄ aryl optionally substituted with one or more substituents selected from Y⁶, and a 5- to 14-membered heteroaryl optionally substituted with one or more substituents selected from Y⁶;
   R²² and R²⁵ are each independently selected from a hydrogen atom, a C₁₋₁₀ alkyl optionally substituted with one or more substituents selected from Y⁴, a C₂₋₁₀ alkenyl optionally substituted with one or more substituents selected from Y⁴, a C₂₋₁₀ alkynyl optionally substituted with one or more substituents selected from Y⁴, a C₃₋₁₀ cycloalkyl optionally substituted with one or more substituents selected from Y⁵, a C₃₋₁₀ cycloalkenyl optionally substituted with one or more substituents selected from Y⁵, a 3- to 14-membered non-aromatic heterocyclyl optionally substituted with one or more substituents selected from Y⁵, a C₆₋₁₄ aryl optionally substituted with one or more substituents selected from Y⁶, a 5- to 14-membered heteroaryl optionally substituted with one or more substituents selected from Y⁶, -COR^{a1}, -CONR^{a2}R^{a3}, -COOR^{a4}, -C(=NR^{a5})NR^{a6}R^{a7}, -S(O)ₚR²⁴ and -SO₂NR^{a8}R^{a9};
   R²³ is selected from a hydrogen atom, a C₁₋₁₀ alkyl optionally substituted with one or more substituents selected from Y⁴, a C₂₋₁₀ alkenyl optionally substituted with one or more substituents selected from Y⁴, a C₂₋₁₀ alkynyl optionally substituted with one or more substituents selected from Y⁴, a C₃₋₁₀ cycloalkyl optionally substituted with one or more substituents selected from Y⁵, a C₃₋₁₀ cycloalkenyl optionally substituted with one or more substituents selected from Y⁵, a 3- to 14-membered non-aromatic heterocyclyl optionally substituted with one or more substituents selected from Y⁵, a C₆₋₁₄ aryl optionally substituted with one or more substituents selected from Y⁶, and a 5- to 14-membered heteroaryl optionally substituted with one or more substituents selected from Y⁶;
   R²⁴ is selected from a C₁₋₁₀ alkyl optionally substituted with one or more substituents selected from Y⁴, a C₂₋₁₀ alkenyl optionally substituted with one or more substituents selected from Y⁴, a C₂₋₁₀ alkynyl optionally substituted with one or more substituents selected from Y⁴, a C₃₋₁₀ cycloalkyl optionally substituted with one or more substituents selected from Y⁵, a C₃₋₁₀ cycloalkenyl optionally substituted with one or more substituents selected from Y⁵, a 3-to 14-membered non-aromatic heterocyclyl optionally substituted with one or more substituents selected from Y⁵, a C₆₋₁₄ aryl optionally substituted with one or more substituents selected from Y⁶, and a 5- to 14-membered heteroaryl optionally substituted with one or more substituents selected from Y⁶;
   R^{a1}, R^{a2}, R^{a3}, R^{a4}, R^{a5} , R^{a6}, R^{a7}, R^{a8} and R^{a9} are each independently selected from a hydrogen atom, a C₁₋₁₀ alkyl optionally substituted with one or more substituents selected from Y⁴, a C₂₋₁₀ alkenyl optionally substituted with one or more substituents selected from Y⁴, a C₂₋₁₀ alkynyl optionally substituted with one or more substituents selected from Y⁴, a C₃₋₁₀ cycloalkyl optionally substituted with one or more substituents selected from Y⁵, a C₃₋₁₀ cycloalkenyl optionally substituted with one or more substituents selected from Y⁵, a 3- to 14-membered non-aromatic heterocyclyl optionally substituted with one or more substituents selected from Y⁵, a C₆₋₁₄ aryl optionally substituted with one or more substituents selected from Y⁶, and a 5- to 14-membered heteroaryl optionally substituted with one or more substituents selected from Y⁶;
   each Y⁴ is independently selected from a halogen atom, nitro, cyano, a C₃₋₁₀ cycloalkyl optionally substituted with one or more substituents selected from Y⁵, a C₃₋₁₀ cycloalkenyl optionally substituted with one or more substituents selected from Y⁵, a 3- to 14-membered non-aromatic heterocyclyl optionally substituted with one or more substituents selected from Y⁵, a C₆₋₁₄ aryl optionally substituted with one or more substituents selected from Y⁶, a 5- to 14-membered heteroaryl optionally substituted with one or more substituents selected from Y⁶, -SH, -COOR³¹, -NR³²R³³, -S(O)ₚR³⁴, -OR³⁵, -SO₂NR³⁶R³⁷ and - CONR³⁸R³⁹, and oxo;
   each Y⁵ is independently selected from a halogen atom, nitro, cyano, a C₁₋₁₀ alkyl optionally substituted with one or more substituents selected from Y⁷, a C₂₋₁₀ alkenyl optionally substituted with one or more substituents selected from Y⁷, a C₂₋₁₀ alkynyl optionally substituted with one or more substituents selected from Y⁷, a C₃₋₁₀ cycloalkyl optionally substituted with one or more substituents selected from Y⁸, a C₃₋₁₀ cycloalkenyl optionally substituted with one or more substituents selected from Y⁸, a 3- to 14-membered non-aromatic heterocyclyl optionally substituted with one or more substituents selected from Y⁸, a C₆₋₁₄ aryl optionally substituted with one or more substituents selected from Y⁹, a 5- to 14-membered heteroaryl optionally substituted with one or more substituents selected from Y⁹, -SH, -COOR³¹, -NR³²R³³, -S(O)ₚR³⁴, -OR³⁵, -SO₂NR³⁶R³⁷, -CONR³⁸R³⁹, and oxo;
   each Y⁶ is independently selected from a halogen atom, nitro, cyano, a C₁₋₁₀ alkyl optionally substituted with one or more substituents selected from Y⁷, a C₂₋₁₀ alkenyl optionally substituted with one or more substituents selected from Y⁷, a C₂₋₁₀ alkynyl optionally substituted with one or more substituents selected from Y⁷, a C₃₋₁₀ cycloalkyl optionally substituted with one or more substituents selected from Y⁸, a C₃₋₁₀ cycloalkenyl optionally substituted with one or more substituents selected from Y⁸, a 3- to 14-membered non-aromatic heterocyclyl optionally substituted with one or more substituents selected from Y⁸, a C₆₋₁₄ aryl optionally substituted with one or more substituents selected from Y⁹, a 5- to 14-membered heteroaryl optionally substituted with one or more substituents selected from Y⁹, -SH, -COOR³¹, -NR³²R³³, -S(O)ₚR³⁴, -OR³⁵, -SO₂NR³⁶R³⁷, and -CONR³⁸R³⁹;
   R³¹, R³⁶, R³⁷, R³⁸ and R³⁹ are each independently selected from a hydrogen atom, a C₁₋₁₀ alkyl optionally substituted with one or more substituents selected from Y⁷, a C₂₋₁₀ alkenyl optionally substituted with one or more substituents selected from Y⁷, a C₂₋₁₀ alkynyl optionally substituted with one or more substituents selected from Y⁷, a C₃₋₁₀ cycloalkyl optionally substituted with one or more substituents selected from Y⁸, a C₃₋₁₀ cycloalkenyl optionally substituted with one or more substituents selected from Y⁸, a 3- to 14-membered non-aromatic heterocyclyl optionally substituted with one or more substituents selected from Y⁸, a C₆₋₁₄ aryl optionally substituted with one or more substituents selected from Y⁹, and a 5- to 14-membered heteroaryl optionally substituted with one or more substituents selected from Y⁹;
   R³² and R³⁵ are each independently selected from a hydrogen atom, a C₁₋₁₀ alkyl optionally substituted with one or more substituents selected from Y⁷, a C₂₋₁₀ alkenyl optionally substituted with one or more substituents selected from Y⁷, a C₂₋₁₀ alkynyl optionally substituted with one or more substituents selected from Y⁷, a C₃₋₁₀ cycloalkyl optionally substituted with one or more substituents selected from Y⁸, a C₃₋₁₀ cycloalkenyl optionally substituted with one or more substituents selected from Y⁸, a 3- to 14-membered non-aromatic heterocyclyl optionally substituted with one or more substituents selected from Y⁸, a C₆₋₁₄ aryl optionally substituted with one or more substituents selected from Y⁹, a 5- to 14-membered heteroaryl optionally substituted with one or more substituents selected from Y⁹, -COR^{b1}, -CONR^{b2}R^{b3}, -CO₂R^{b4}, -C(=NR^{b5})NR^{b6}R^{b7} and -SO₂NR^{b8}R^{b9};
   R³³ is selected from a hydrogen atom, a C₁₋₁₀ alkyl optionally substituted with one or more substituents selected from Y⁷, a C₂₋₁₀ alkenyl optionally substituted with one or more substituents selected from Y⁷, a C₂₋₁₀ alkynyl optionally substituted with one or more substituents selected from Y⁷, a C₃₋₁₀ cycloalkyl optionally substituted with one or more substituents selected from Y⁸, a C₃₋₁₀ cycloalkenyl optionally substituted with one or more substituents selected from Y⁸, a 3- to 14-membered non-aromatic heterocyclyl optionally substituted with one or more substituents selected from Y⁸, a C₆₋₁₄ aryl optionally substituted with one or more substituents selected from Y⁹, and a 5- to 14-membered heteroaryl optionally substituted with one or more substituents selected from Y⁹;
   R³⁴ is selected from a C₁₋₁₀ alkyl optionally substituted with one or more substituents selected from Y⁷, a C₂₋₁₀ alkenyl optionally substituted with one or more substituents selected from Y⁷, a C₂₋₁₀ alkynyl optionally substituted with one or more substituents selected from Y⁷, a C₃₋₁₀ cycloalkyl optionally substituted with one or more substituents selected from Y⁸, a C₃₋₁₀ cycloalkenyl optionally substituted with one or more substituents selected from Y⁸, a 3-to 14-membered non-aromatic heterocyclyl optionally substituted with one or more substituents selected from Y⁸, a C₆₋₁₄ aryl optionally substituted with one or more substituents selected from Y⁹, and a 5- to 14-membered heteroaryl optionally substituted with one or more substituents selected from Y⁹;
   R^{b1}, R^{b2}, R^{b3}, R^{b4}, R^{b5}, R^{b6}, R^{b7}, R^{b8} and R^{b9} are each independently selected from a hydrogen atom, a C₁₋₁₀ alkyl optionally substituted with one or more substituents selected from Y⁷, a C₂₋₁₀ alkenyl optionally substituted with one or more substituents selected from Y⁷, a C₂₋₁₀ alkynyl optionally substituted with one or more substituents selected from Y⁷, a C₃₋₁₀ cycloalkyl optionally substituted with one or more substituents selected from Y⁸, a C₃₋₁₀ cycloalkenyl optionally substituted with one or more substituents selected from Y⁸, a 3- to 14-membered non-aromatic heterocyclyl optionally substituted with one or more substituents selected from Y⁸, a C₆₋₁₄ aryl optionally substituted with one or more substituents selected from Y⁹, and a 5- to 14-membered heteroaryl optionally substituted with one or more substituents selected from Y⁹;
   each Y⁷ is independently selected from a halogen atom, nitro, cyano, a C₁₋₁₀ alkyl optionally substituted with one or more substituents selected from Y¹⁰, a C₂₋₁₀ alkenyl optionally substituted with one or more substituents selected from Y¹⁰, a C₂₋₁₀ alkynyl optionally substituted with one or more substituents selected from Y¹⁰, a C₃₋₁₀ cycloalkyl optionally substituted with one or more substituents selected from Y¹¹, a C₃₋₁₀ cycloalkenyl optionally substituted with one or more substituents selected from Y¹¹, a 3- to 14-membered non-aromatic heterocyclyl optionally substituted with one or more substituents selected from Y¹¹, a C₆₋₁₄ aryl optionally substituted with one or more substituents selected from Y¹², a 5- to 14-membered heteroaryl optionally substituted with one or more substituents selected from Y¹², -SH, -COOR⁴⁰, -NR⁴¹R⁴², -S(O)ₚR⁴³, -OR⁴⁴, -SO₂NR⁴⁵R⁴⁶ and -CONR⁴⁷R⁴⁸, and oxo;
   each Y⁸ is independently selected from a halogen atom, nitro, cyano, a C₁₋₁₀ alkyl optionally substituted with one or more substituents selected from Y¹⁰, a C₂₋₁₀ alkenyl optionally substituted with one or more substituents selected from Y¹⁰, a C₂₋₁₀ alkynyl optionally substituted with one or more substituents selected from Y¹⁰, a C₃₋₁₀ cycloalkyl optionally substituted with one or more substituents selected from Y¹¹, a C₃₋₁₀ cycloalkenyl optionally substituted with one or more substituents selected from Y¹¹, a 3- to 14-membered non-aromatic heterocyclyl optionally substituted with one or more substituents selected from Y¹¹, a C₆₋₁₄ aryl optionally substituted with one or more substituents selected from Y¹², a 5- to 14-membered heteroaryl optionally substituted with one or more substituents selected from Y¹², -SH, -COOR⁴⁰, -NR⁴¹R⁴², -S(O)ₚR⁴³, -OR⁴⁴, -SO₂NR⁴⁵R⁴⁶, -CONR⁴⁷R⁴⁸, and oxo;
   each Y⁹ is independently selected from a halogen atom, nitro, cyano, a C₁₋₁₀ alkyl optionally substituted with one or more substituents selected from Y¹⁰, a C₂₋₁₀ alkenyl optionally substituted with one or more substituents selected from Y¹⁰, a C₂₋₁₀ alkynyl optionally substituted with one or more substituents selected from Y¹⁰, a C₃₋₁₀ cycloalkyl optionally substituted with one or more substituents selected from Y¹¹, a C₃₋₁₀ cycloalkenyl optionally substituted with one or more substituents selected from Y¹¹, a 3- to 14-membered non-aromatic heterocyclyl optionally substituted with one or more substituents selected from Y¹¹, a C₆₋₁₄ aryl optionally substituted with one or more substituents selected from Y¹², a 5- to 14-membered heteroaryl optionally substituted with one or more substituents selected from Y¹², -SH, -COOR⁴⁰, -NR⁴¹R⁴², -S(O)ₚR⁴³, -OR⁴⁴, -SO₂NR⁴⁵R⁴⁶ and -CONR⁴⁷R⁴⁸;
   R⁴⁰, R⁴⁵, R⁴⁶, R⁴⁷ and R⁴⁸ are each independently selected from a hydrogen atom, a C₁₋₁₀ alkyl optionally substituted with one or more substituents selected from Y¹⁰, a C₂₋₁₀ alkenyl optionally substituted with one or more substituents selected from Y¹⁰, a C₂₋₁₀ alkynyl optionally substituted with one or more substituents selected from Y¹⁰, a C₃₋₁₀ cycloalkyl optionally substituted with one or more substituents selected from Y¹¹, a C₃₋₁₀ cycloalkenyl optionally substituted with one or more substituents selected from Y¹¹, a 3- to 14-membered non-aromatic heterocyclyl optionally substituted with one or more substituents selected from Y¹¹, a C₆₋₁₄ aryl optionally substituted with one or more substituents selected from Y¹², and a 5- to 14-membered heteroaryl optionally substituted with one or more substituents selected from Y¹²;
   R⁴¹ and R⁴⁴ are each independently selected from a hydrogen atom, a C₁₋₁₀ alkyl optionally substituted with one or more substituents selected from Y¹⁰, a C₂₋₁₀ alkenyl optionally substituted with one or more substituents selected from Y¹⁰, a C₂₋₁₀ alkynyl optionally substituted with one or more substituents selected from Y¹⁰, a C₃₋₁₀ cycloalkyl optionally substituted with one or more substituents selected from Y¹¹, a C₃₋₁₀ cycloalkenyl optionally substituted with one or more substituents selected from Y¹¹, a 3- to 14-membered non-aromatic heterocyclyl optionally substituted with one or more substituents selected from Y¹¹, a C₆₋₁₄ aryl optionally substituted with one or more substituents selected from Y¹², a 5- to 14-membered heteroaryl optionally substituted with one or more substituents selected from Y¹², -COR^{c1}, -CONR^{c2}R^{c3}, -COOR^{c4}, -C(=NR^{c5})NR^{c6}R^{c7}, and -SO₂NR^{c8}R^{c9};
   R⁴² is selected from a hydrogen atom, a C₁₋₁₀ alkyl optionally substituted with one or more substituents selected from Y¹⁰, a C₂₋₁₀ alkenyl optionally substituted with one or more substituents selected from Y¹⁰, a C₂₋₁₀ alkynyl optionally substituted with one or more substituents selected from Y¹⁰, a C₃₋₁₀ cycloalkyl optionally substituted with one or more substituents selected from Y¹¹, a C₃₋₁₀ cycloalkenyl optionally substituted with one or more substituents selected from Y¹¹, a 3- to 14-membered non-aromatic heterocyclyl optionally substituted with one or more substituents selected from Y¹¹, a C₆₋₁₄ aryl optionally substituted with one or more substituents selected from Y¹², and a 5- to 14-membered heteroaryl optionally substituted with one or more substituents selected from Y¹²;
   R⁴³ is selected from a C₁₋₁₀ alkyl optionally substituted with one or more substituents selected from Y¹⁰, a C₂₋₁₀ alkenyl optionally substituted with one or more substituents selected from Y¹⁰, a C₂₋₁₀ alkynyl optionally substituted with one or more substituents selected from Y¹⁰, a C₃₋₁₀ cycloalkyl optionally substituted with one or more substituents selected from Y¹¹, a C₃₋₁₀ cycloalkenyl optionally substituted with one or more substituents selected from Y¹¹, a 3- to 14-membered non-aromatic heterocyclyl optionally substituted with one or more substituents selected from Y¹¹, a C₆₋₁₄ aryl optionally substituted with one or more substituents selected from Y¹², and a 5- to 14-membered heteroaryl optionally substituted with one or more substituents selected from Y¹²;
   R^{c1}, R^{c2}, R^{c3}, R^{c4}, R^{c5}, R^{c6}, R^{c7}, R^{c8} and R^{c9} are each independently selected from a hydrogen atom, a C₁₋₁₀ alkyl optionally substituted with one or more substituents selected from Y¹⁰, a C₂₋₁₀ alkenyl optionally substituted with one or more substituents selected from Y¹⁰, a C₂₋₁₀ alkynyl optionally substituted with one or more substituents selected from Y¹⁰, a C₃₋₁₀ cycloalkyl optionally substituted with one or more substituents selected from Y¹¹, a C₃₋₁₀ cycloalkenyl optionally substituted with one or more substituents selected from Y¹¹, a 3- to 14-membered non-aromatic heterocyclyl optionally substituted with one or more substituents selected from Y¹¹, a C₆₋₁₄ aryl optionally substituted with one or more substituents selected from Y¹², and a 5- to 14-membered heteroaryl optionally substituted with one or more substituents selected from Y¹²;
   each Y¹⁰ is independently selected from a halogen atom, nitro, cyano, a C₁₋₁₀ alkoxy selected from a halogen atom and an alkoxy, a C₃₋₁₀ cycloalkyl optionally substituted with one or more substituents selected from a halogen atom and a C₁₋₆ alkoxy, a C₃₋₁₀ cycloalkenyl optionally substituted with one or more substituents selected from a halogen atom and a C₁₋₆ alkoxy, a 3- to 14-membered non-aromatic heterocyclyl optionally substituted with one or more substituents selected from a halogen atom and a C₁₋₆ alkoxy, a C₆₋₁₄ aryl optionally substituted with one or more substituents selected from a halogen atom and a C₁₋₆ alkoxy, a 5-to 14-membered heteroaryl optionally substituted with one or more substituents selected from a halogen atom and a C₁₋₆ alkoxy, a (C₁₋₁₀ alkoxy)carbonyl optionally substituted with one or more substituents selected from a halogen atom and a C₁₋₆ alkoxy, a di(C₁₋₆ alkyl)aminocarbonyl optionally substituted with one or more substituents selected from a halogen atom and a C₁₋₆ alkoxy, a C₁₋₆ alkoxy optionally substituted with one or more substituents selected from a halogen atom and a C₁₋₆ alkoxy, a C₁₋₆ alkylthio optionally substituted with one or more substituents selected from a halogen atom and a C₁₋₆ alkoxy, a C₁₋₆ alkylsulfanyl optionally substituted with one or more substituents selected from a halogen atom and a C₁₋₆ alkoxy, a C₁₋₆ alkylsulfinyl optionally substituted with one or more substituents selected from a halogen atom and a C₁₋₆ alkoxy, a C₁₋₆ alkylsulfonyl optionally substituted with one or more substituents selected from a halogen atom and a C₁₋₆ alkoxy, and oxo;
   each Y¹¹ is independently selected from a halogen atom, nitro, cyano, a C₁₋₁₀ alkyl optionally substituted with one or more substituents selected from a halogen atom and an alkoxy, a C₂₋₁₀ alkenyl optionally substituted with one or more substituents selected from a halogen atom and an alkoxy, a C₂₋₁₀ alkynyl optionally substituted with one or more substituents selected from a halogen atom and an alkoxy, a C₁₋₁₀ alkoxy optionally substituted with one or more substituents selected from a halogen atom and an alkoxy, a C₃₋₁₀ cycloalkyl optionally substituted with one or more substituents selected from a halogen atom and a C₁₋₆ alkoxy, a C₃₋₁₀ cycloalkenyl optionally substituted with one or more substituents selected from a halogen atom and a C₁₋₆ alkoxy, a 3- to 14-membered non-aromatic heterocyclyl optionally substituted with one or more substituents selected from a halogen atom and a C₁₋₆ alkoxy, a C₆₋₁₄ aryl optionally substituted with one or more substituents selected from a halogen atom and a C₁₋₆ alkoxy, a 5- to 14-membered heteroaryl optionally substituted with one or more substituents selected from a halogen atom and a C₁₋₆ alkoxy, a (C₁₋₁₀ alkoxy)carbonyl optionally substituted with one or more substituents selected from a halogen atom and a C₁₋₆ alkoxy, a di(C₁₋₆ alkyl)aminocarbonyl optionally substituted with one or more substituents selected from a halogen atom and a C₁₋₆ alkoxy, a C₁₋₆ alkoxy optionally substituted with one or more substituents selected from a halogen atom and a C₁₋₆ alkoxy, a C₁₋₆ alkylthio optionally substituted with one or more substituents selected from a halogen atom and a C₁₋₆ alkoxy, a C₁₋₆ alkylsulfanyl optionally substituted with one or more substituents selected from a halogen atom and a C₁₋₆ alkoxy, a C₁₋₆ alkylsulfinyl optionally substituted with one or more substituents selected from a halogen atom and a C₁₋₆ alkoxy, a C₁₋₆ alkylsulfonyl optionally substituted with one or more substituents selected from a halogen atom and a C₁₋₆ alkoxy, and oxo;
   each Y¹² is independently selected from a halogen atom, nitro, cyano, a C₁₋₁₀ alkyl optionally substituted with one or more substituents selected from a halogen atom and an alkoxy, a C₂₋₁₀ alkenyl optionally substituted with one or more substituents selected from a halogen atom and an alkoxy, a C₂₋₁₀ alkynyl optionally substituted with one or more substituents selected from a halogen atom and an alkoxy, a C₁₋₁₀ alkoxy optionally substituted with one or more substituents selected from a halogen atom and an alkoxy, a C₃₋₁₀ cycloalkyl optionally substituted with one or more substituents selected from a halogen atom and a C₁₋₆ alkoxy, a C₃₋₁₀ cycloalkenyl optionally substituted with one or more substituents selected from a halogen atom and a C₁₋₆ alkoxy, a 3- to 14-membered non-aromatic heterocyclyl optionally substituted with one or more substituents selected from a halogen atom and a C₁₋₆ alkoxy, a C₆₋₁₄ aryl optionally substituted with one or more substituents selected from a halogen atom and a C₁₋₆ alkoxy, a 5- to 14-membered heteroaryl optionally substituted with one or more substituents selected from a halogen atom and a C₁₋₆ alkoxy, a (C₁₋₁₀ alkoxy)carbonyl optionally substituted with one or more substituents selected from a halogen atom and a C₁₋₆ alkoxy, a di(C₁₋₆ alkyl)aminocarbonyl optionally substituted with one or more substituents selected from a halogen atom and a C₁₋₆ alkoxy, a C₁₋₆ alkoxy optionally substituted with one or more substituents selected from a halogen atom and a C₁₋₆ alkoxy, a C₁₋₆ alkylthio optionally substituted with one or more substituents selected from a halogen atom and a C₁₋₆ alkoxy, a C₁₋₆ alkylsulfanyl optionally substituted with one or more substituents selected from a halogen atom and a C₁₋₆ alkoxy, a C₁₋₆ alkylsulfinyl optionally substituted with one or more substituents selected from a halogen atom and a C₁₋₆ alkoxy, and a C₁₋₆ alkylsulfonyl optionally substituted with one or more substituents selected from a halogen atom and a C₁₋₆ alkoxy;
   each p is independently an integer from 0 to 2;
   R⁵ is selected from a hydrogen atom, benzyl optionally substituted on the benzene ring with one or more substituents selected from Y¹³, benzyloxycarbonyl optionally substituted on the benzene ring with one or more substituents selected from Y¹³, phenylcarbonyl and (C₁₋₁₀ alkoxy)carbonyl optionally substituted with one or more substituents selected from Y¹³, (C₁₋₁₀ alkyl)carbonyl optionally substituted with a halogen atom, a C₁₋₁₀ alkyl optionally substituted with one or more substituents selected from Y¹, a C₂₋₁₀ alkenyl optionally substituted with one or more substituents selected from Y¹, a C₂₋₁₀ alkynyl optionally substituted with one or more substituents selected from Y¹, a C₃₋₁₀ cycloalkyl optionally substituted with one or more substituents selected from Y², a C₃₋₁₀ cycloalkenyl optionally substituted with one or more substituents selected from Y², a 3- to 14-membered non-aromatic heterocyclyl optionally substituted with one or more substituents selected from Y², a C₆₋₁₄ aryl optionally substituted with one or more substituents selected from Y³, and a 5- to 14-membered heteroaryl optionally substituted with one or more substituents selected from Y³;
   R⁶ is selected from a hydrogen atom, benzyl optionally substituted on the benzene ring with one or more substituents selected from Y¹³, benzyloxycarbonyl optionally substituted on the benzene ring with one or more substituents selected from Y¹³, and phenylcarbonyl, (C₁₋₁₀ alkoxy)carbonyl and (C₁₋₁₀ alkyl)carbonyl optionally substituted with one or more substituents selected from Y¹³; or
   R⁵ and R⁶, together with the nitrogen atom to which they are attached, form a 5- to 14-membered nitrogen-containing heterocyclyl having an imide structure, or a 3- to 14-membered non-aromatic heterocyclyl containing, as ring atoms, two or more nitrogen atoms optionally substituted with one or more substituents selected from Y²;
   R^{d} is selected from a hydrogen atom, benzyl optionally substituted on the benzene ring with one or more substituents selected from Y¹³, benzyloxycarbonyl optionally substituted on the benzene ring with one or more substituents selected from Y¹³, and phenylcarbonyl, (C₁₋₁₀ alkoxy)carbonyl and (C₁₋₁₀ alkyl)carbonyl optionally substituted with one or more substituents selected from Y¹³;
   each Y¹³ is independently selected from a halogen atom, C₁₋₁₀ alkoxy, and C₁₋₁₀ alkyl;
   when the 3- to 14-membered non-aromatic heterocyclyl is a monocyclic heterocyclyl, the heterocyclyl is optionally fused with a benzene ring; and
   when the C₆₋₁₄ aryl is phenyl, the phenyl is optionally fused with a 5- to 7-membered non-aromatic heterocyclic ring;
      or a salt thereof, except for the compound of formula (Ia) wherein Z is hydroxy, n is 0, R⁷ and
      R⁸ are each hydrogen atom, X is 2,6-dichloropyridin-4-yl, and;
   R¹ and R² are each a hydrogen atom; or
   R¹ is a hydrogen atom and R² is methyl, ethyl, isopropyl, n-butyl, tert-butyl or isobutyl; or
   R¹ is ethyl and R² is methyl or ethyl.
[2-2] The compound or a salt thereof according to [2-1], wherein n is 0 or 1.
[2-3] The compound or a salt thereof according to [2-1] or [2-2], wherein R¹ is hydrogen atom.
[2-4] The compound or a salt thereof according to any one of [2-1] to [2-3], wherein R⁷ and R⁸ are each a hydrogen atom.
[2-5] The compound or a salt thereof according to any of [2-1] to [2-4], wherein X is 2,6-dihalo-4-pyridyl.
[2-6] The compound or a salt thereof according to any of [2-1] to [2-4], wherein X is 2,6-dichloro-4-pyridyl.
[2-7] The compound or a salt thereof according to any of [2-1] to [2-4], wherein X is a perfluoro-C₁₋₃ alkyl.
[2-8] The compound or a salt thereof according to any of [2-1] to [2-4], wherein X is trifluoromethyl.
[2-9] A composition for use in the acylation of a tRNA, including the compound or a salt thereof according to any of [2-1] to [2-8].
[2-10] The composition according to [2-9] for use in the acylation of a tRNA in the presence of a flexizyme.
[2-11] A method for producing a compound represented by formula (Ia): 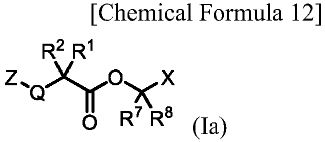
   wherein R¹, R², R⁷, R⁸, Q, X and Z are as defined in any of [2-1] to [2-8];
   the method including reacting a compound represented by formula (IIa): 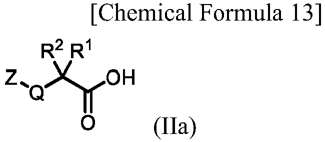
   with a compound represented by formula (III): 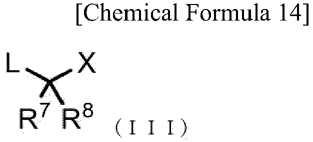
   wherein L is a leaving group.
[2-12] The method according to [2-11], wherein the reaction is carried out in the presence of a base.
[2-13] The method according to [2-12], wherein the base is diisopropylethylamine.
[2-14] A method for producing a tRNA acylated at the 3'-terminus, wherein:
   the acyl group at the 3'-terminus is a group represented by: 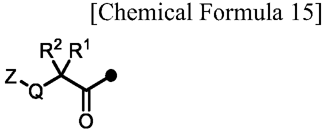
   wherein R¹, R², Q and Z are as defined in any of [2-1] to [2-8];
   the method including reacting a compound represented by formula (Ia): 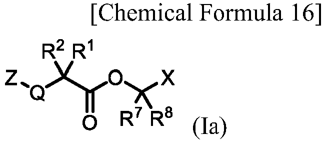
   wherein R¹, R², R⁷, R⁸, Q, X and Z are as defined in any of [2-1] to [2-8]
      with the tRNA in the presence of a flexizyme.
[2-15] A method for preparing a peptide library, including:
   producing a tRNA acylated at the 3'-terminus by the method according to [2-14];
   preparing an mRNA library; and
   synthesizing a peptide corresponding to each mRNA from the mRNA library with a cell-free translation system to prepare a peptide library.
[2-16] A method for acylating a tRNA at the 3'-terminus, including reacting the tRNA with a compound represented by formula (I): 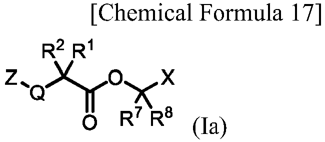
   wherein R¹, R², R⁷, R⁸, Q, X and Z are as defined in any of [2-1] to [2-8]; in the presence of a flexizyme, thereby adding an acyl group represented by: 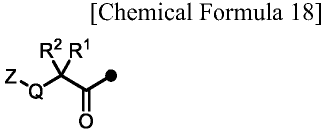
   wherein R¹, R², R⁵, R⁶ and Q are as defined in any of [2-1] to [2-8]; to the tRNA at the 3'-terminus.
[2-17] The compound or a salt thereof according to [2-1], wherein Z is -NR⁵R⁶.

### ADVANTAGEOUS EFFECTS OF INVENTION

The amino acid active ester according to the present invention is useful as a synthetic intermediate, such as a reagent for an acylation reaction. In one aspect of the present invention, the active ester is used for the aminoacylation of a tRNA with a flexizyme and useful for synthesizing special peptides composed of various amino acids or amino acid derivatives including unnatural amino acids. In addition, the novel amino acid active ester according to the present invention is easy to handle during use, storage and disposal, and can also reduce handling costs.

### BRIEF DESCRIPTION OF DRAWINGS

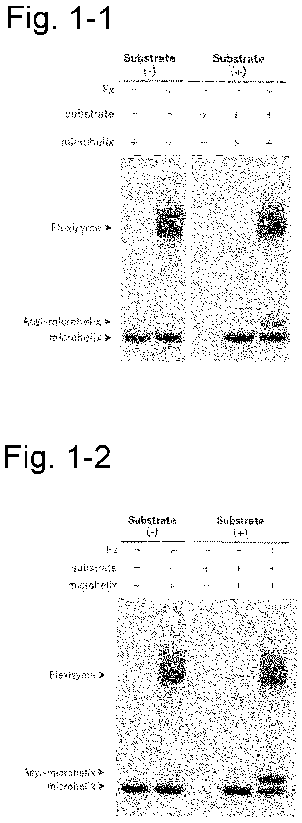

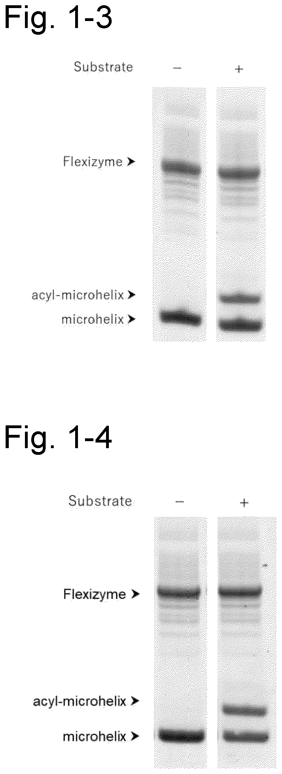

[Fig. 1-1] Fig. 1-1 is a photograph, in lieu of a drawing, showing the results of confirming the efficiency of acylation of (2,6-dichloropyridin-4-yl)methyl L-isoleucinate (Compound No. 21).
[Fig. 1-2] Fig. 1-2 is a photograph, in lieu of a drawing, showing the results of confirming the efficiency of acylation of (2,6-dichloropyridin-4-yl)methyl glycinate (Compound No. 99).
[Fig. 1-3] Fig. 1-3 is a photograph, in lieu of a drawing, showing the results of confirming the efficiency of acylation of 2,2,2-trifluoroethyl (S)-2-amino-4-phenylbutanoate (Compound No. 47).
[Fig. 1-4] Fig. 1-4 is a photograph, in lieu of a drawing, showing the results of confirming the efficiency of acylation of 2,2,2-trifluoroethyl (S)-2-amino-3-(1H-pyrrolo[2,3-b]pyridin-3-yl)propanoate (Compound No. 81).

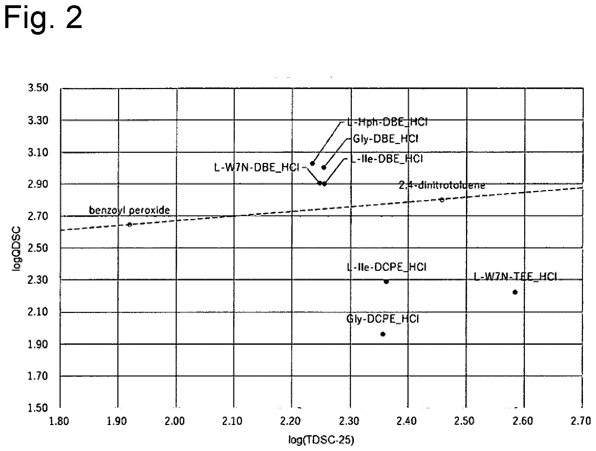

[Fig. 2] Fig. 2 is a graph showing the results of confirming the presence or absence of hazards posed by each amino acid active ester, by using 2,4-dinitrotoluene (Compound No. 115) and benzoyl peroxide (Compound No. 116) as reference compounds, plotting the reference points of Q_{DSC} and T_{DSC} for each of the compounds and drawing a straight line connecting the two points as the hazard determination line.

### DESCRIPTION OF EMBODIMENTS

As used herein, the term "halogen atom" refers to a fluorine atom, a chlorine atom, a bromine atom, an iodine atom, or the like. When a halogen atom is used as a substituent for an aryl, a heteroaryl, or the like, preferred examples thereof include a fluorine atom, a chlorine atom and a bromine atom. When a halogen atom is used herein as a substituent for an alkyl or a group containing an alkyl as a part thereof (alkoxy, alkenyl, alkylthio, or the like), preferred examples thereof include a fluorine atom. Specific examples of the group having a halogen atom as a substituent include trifluoromethyl, pentafluoroethyl, trifluoromethoxy, pentafluoroethoxy, trifluoromethylthio, and pentafluoroethylthio.

As used herein, the term "C₁₋₃ alkyl" refers to a monovalent group derived by removing any one hydrogen atom from a linear or branched saturated aliphatic hydrocarbon having 1 to 3 carbon atoms. Specific examples thereof include methyl, ethyl, n-propyl and isopropyl.

As used herein, the term "C₁₋₁₀ alkyl" refers to a monovalent group derived by removing any one hydrogen atom from a linear or branched saturated aliphatic hydrocarbon having 1 to 10 carbon atoms. Specific examples thereof include methyl, ethyl, n-propyl, isopropyl, n-butyl, isobutyl, sec-butyl, t-butyl, 1-methylpropyl, n-pentyl, isopentyl, 2-methylbutyl, 1,1-dimethylpropyl, 1-ethylpropyl, hexyl, 4-methylpentyl, n-heptyl, 5-methylhexyl, 1-propylbutyl, 2-ethyl-2-methylbutyl, n-octyl, 5-methylheptyl, 2,3-dimethylhexyl, 1-methyl-1-propylbutyl, and 2,2-diethylbutyl, 7-methyloctyl, 5-ethylheptyl, n-decyl, 8-methylnonyl, 5,5-dimethyloctyl, and 4-ethyl-6-methylheptyl.

As used herein, the term "C₂₋₁₀ alkenyl" refers to a monovalent group derived by removing any one hydrogen atom from a linear or branched aliphatic hydrocarbon having 2 to 10 carbon atoms and at least one double bond (two adjacent SP2 carbon atoms). Specific examples thereof include vinyl, 1-propenyl, 2-propenyl, 1-butenyl, 2-butenyl, 3-butenyl, 1-methyl-2-propenyl, 1-pentenyl, 2-pentenyl, 3-pentenyl, 4-pentenyl, 1-methyl-2-butenyl, 1-methyl-3-butenyl, 2-methyl-3-butenyl, 3-methyl-1-butenyl, 1,1-dimethyl-2-propenyl, 1-hexenyl, heptenyl and octenyl.

As used herein, the phrase "C₂₋₁₀ alkenyl optionally substituted with" refers to a C₂₋₁₀ alkenyl which is unsubstituted or in which one or more hydrogen atoms on the alkenyl are substituted with a given substituent. When two or more substituents are present, the substituents may be the same or different. One carbon atom may be substituted with more than one substituent.

As used herein, the term "C₂₋₁₀ alkynyl" refers to a monovalent group derived by removing any one hydrogen atom from a linear or branched aliphatic hydrocarbon having 2 to 10 carbon atoms and at least one triple bond (two adjacent SP carbon atoms). Specific examples thereof include ethynyl, 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-hexynyl, heptynyl, heptadiynyl, octynyl and octadiynyl.

As used herein, the phrase "C₂₋₁₀ alkynyl optionally substituted with" refers to a C₂₋₁₀ alkynyl which is unsubstituted or in which one or more hydrogen atoms on the alkynyl are substituted with a given substituent. When two or more substituents are present, the substituents may be the same or different. One carbon atom may be substituted with more than one substituent.

As used herein, the term "C₁₋₆ alkoxy" refers to a C₁₋₆ alkyl-O- group wherein the C₁₋₆ alkyl is as defined above. Specific examples thereof include methoxy, ethoxy, 1-propoxy, 2-propoxy, n-butoxy, i-butoxy, sec-butoxy, t-butoxy, 1-pentyloxy and 1-hexyloxy.

As used herein, the term "C₁₋₁₀ alkoxy" refers to a C₁₋₁₀ alkyl-O- group wherein the C₁₋₁₀ alkyl is as defined above. Specific examples thereof include methoxy, ethoxy, 1-propoxy, 2-propoxy, n-butoxy, i-butoxy, sec-butoxy, t-butoxy, 1-pentyloxy, 1-hexyloxy, n-heptyloxy, 5-methylhexyloxy, 1-propylbutyloxy, 2-ethyl-2-methylbutyloxy, n-octyloxy, 5-methylheptyloxy, 2,3-dimethylhexyloxy, 1-methyl-1-propylbutyloxy, and 2,2-diethylbutyloxy, 7-methyloctyloxy, 5-ethylheptyloxy, n-decyloxy, 8-methylnonyloxy, 5,5-dimethyloctyloxy, and 4-ethyl-6-methylheptyloxy.

As used herein, the term "C₁₋₆ alkylthio" refers to a C₁₋₆ alkyl-S- group wherein the C₁₋₆ alkyl is as defined above. Specific examples thereof include methylthio, ethylthio, n-propylthio, i-propylthio, n-butylthio, i-butylthio, t-butylthio, sec-butylthio, 1-methylpropylthio, n-pentylthio, isopentylthio, 2-methylbutylthio, 1,1-dimethylpropylthio, 1-ethylpropylthio, hexylthio, 4-methylpentylthio and 2-ethylbutylthio.

As used herein, the term "(C₁₋₁₀ alkyl)carbonyl" refers to a C₁₋₁₀ alkyl-C(O)- group wherein the C₁₋₁₀ alkyl is as defined above. Specific examples thereof include methylcarbonyl, ethylcarbonyl, n-propylcarbonyl, i-propylcarbonyl, n-butylcarbonyl, i-butylcarbonyl, sec-butylcarbonyl, t-butylcarbonyl, 1-methylpropylcarbonyl, n-pentylcarbonyl, isopentylcarbonyl, 2-methylbutylcarbonyl, 1,1-dimethylpropylcarbonyl, 1-ethylpropylcarbonyl, hexylcarbonyl, 4-methylpentylcarbonyl and 2-ethylbutylcarbonyl.

As used herein, the term "di(C₁₋₆ alkyl)aminocarbonyl" refers to carbonyl substituted with a di(C₁₋₆ alkyl)amino. The term "di(C₁₋₆ alkyl)amino" refers to amino substituted with two C₁₋₆ alkyls. Specific examples thereof include dimethylaminocarbonyl and diethylaminocarbonyl.

As used herein, the term "C₁₋₆ alkylsulfanyl" refers to a C₁₋₆ alkyl-S- group wherein the C₁₋₆ alkyl is as defined above. Specific examples thereof include methylsulfanyl, ethylsulfanyl, n-propylsulfanyl, and preferably methylsulfanyl.

As used herein, the term "C₁₋₆ alkylsulfonyl" refers to a C₁₋₆ alkyl-SO₂- group wherein the C₁₋₆ alkyl is as defined above. Specific examples thereof include methylsulfonyl, ethylsulfonyl, n-propylsulfonyl, and preferably methylsulfonyl.

As used herein, the term "C₁₋₆ alkylsulfinyl" refers to a C₁₋₆ alkyl-S(=O)- group wherein the C₁₋₆ alkyl is as defined above. Specific examples thereof include methylsulfinyl, ethylsulfinyl, n-propylsulfinyl, and preferably methylsulfinyl.

As used herein, the term "(C₁₋₁₀ alkoxy)carbonyl" refers to a C₁₋₁₀ alkyl-O-C(O)-group wherein the C₁₋₁₀ alkyl is as defined above. Specific examples thereof include methoxycarbonyl, ethoxycarbonyl, n-propoxycarbonyl, i-propoxycarbonyl, n-butoxycarbonyl, i-butoxycarbonyl, sec-butoxycarbonyl, t-butoxycarbonyl, and 1-methylpropoxycarbonyl, n-pentyloxycarbonyl, isopentyloxycarbonyl, 2-methylbutoxycarbonyl, 1,1-dimethylpropoxycarbonyl, 1-ethylpropoxycarbonyl, hexyloxycarbonyl, 4-methylpentyloxycarbonyl, and 2-ethylbutoxycarbonyl.

As used herein, the term "C₆₋₁₄ aryl" refers to a monovalent aromatic hydrocarbon ring group. Examples of the C₆₋₁₄ aryl include phenyl, 1-naphthyl and 2-naphthyl.

When the C₆₋₁₄ aryl is phenyl, the phenyl is optionally fused with a 5- to 7-membered non-aromatic heterocyclic ring. One example of the C₆₋₁₄ aryl fused with a 5- to 7-membered non-aromatic heterocyclic ring includes 2,3-dihydrobenzo-1,4-dioxinyl.

As used herein, the term "5- to 14-membered heteroaryl" refers to an aromatic ring group containing one or more (for example, 1 to 5, preferably 1 to 3) heteroatoms in 5 to 14 ring member atoms. The ring may be a monocyclic or bicyclic ring. Specific examples of the "5- to 14-membered heteroaryl" include thienyl, pyridazinyl, pyrazinyl, thiazolyl, oxazolyl, isothiazolyl, thiadiazolyl, oxadiazolyl, isoxazolyl, pyrazolyl, quinolinyl, isoquinolyl, benzothiazolyl, benzoxazolyl, benzimidazolyl, pyridinyl, pyrimidinyl, indolyl, imidazolyl, furyl, thioxazolyl, pyrrolyl, tetrazolyl, oxopyrimidinyl, naphthyl, benzodioxinyl, benzoisoxazolyl, benzoisothiazolyl, indazolyl, benzothienyl, benzofuranyl, benzopyranyl and triazolyl.

As used herein, the term "3- to 14-membered non-aromatic heterocyclyl" refers to a non-aromatic cyclic ring or ring system containing at least one heteroatom in 3 to 14 ring member atoms. The heterocyclyl may have any degree of saturation as long as at least one ring in the ring system is not aromatic. The heteroatom may be present in a non-aromatic or aromatic ring in the ring system. In a preferred 6-membered monocyclic heterocyclyl, the heteroatoms are three or less selected from O, N and S, and in a preferred 5-membered monocyclic heterocyclyl, the heteroatoms are two or less selected from O, N and S. Examples of the heterocyclyl include azepinyl, acridinyl, carbazolyl, cinnolinyl, dioxolanyl, imidazolinyl, imidazolidinyl, morpholinyl, oxiranyl, oxepanyl, thiepanyl, pyridyl, piperidinyl, piperazinyl, dioxopiperazinyl, pyrrolidinyl, pyrrolidonyl, pyrrolidinoyl, 4-piperidonyl, pyrazolinyl, pyrazolidinyl, oxazolinyl, oxazolidinyl, oxazolidinonyl, thienyl, thiazolinyl and thiazolidinyl.

When the 3- to 14-membered non-aromatic heterocyclyl is a monocyclic heterocyclyl, the heterocyclyl is optionally fused with a benzene ring. One example of the 3- to 14-membered non-aromatic heterocyclyl fused with a benzene ring includes at least 2,3-dihydrobenzo-1,4-dioxinyl.

As used herein, the term "C₃₋₁₀ carbocyclic ring" is a cycloalkane ring, a cycloalkene or a cycloalkyne ring having 3 to 10 ring member carbon atoms. Examples thereof include cyclopropane, cyclobutane, cyclopentane, cyclohexane, cycloheptane, cyclooctane, cyclononane, cyclodecane, cyclopropene, cyclobutene, cyclopentene, cyclohexene, cycloheptene, cyclooctene, cyclononene, cyclodecene, cyclohexadiene, cyclooctadiene and cyclooctyne.

As used herein, the term "3- to 10-membered heterocyclic ring" refers to a heterocyclic group containing one N as a heteroatom and having 3 to 10 ring member atoms. Specific examples thereof include pyrrolidine, piperidine, azepane and azocane, and particularly pyrrolidine and piperidine.

As used herein, the term "C₃₋₁₀ cycloalkyl" refers to a cyclic saturated aliphatic hydrocarbon group having 3 to 10 carbon atoms. Specific examples thereof include cyclopropyl, cyclobutyl, cyclopentyl, cyclohexyl, cycloheptyl, cyclooctyl, cyclononyl and cyclodecyl.

As used herein, the term "C₃₋₆ cycloalkenyl" refers to a cyclic aliphatic hydrocarbon group having 3 to 6 carbon atoms and at least one double bond (two adjacent SP2 carbon atoms). Specific examples thereof include cyclopropenyl, cyclobutenyl, cyclopentenyl and cyclohexenyl.

As used herein, the term "C₃₋₁₀ cycloalkenyl" refers to a cyclic aliphatic hydrocarbon group having 3 to 10 carbon atoms and at least one double bond (two adjacent SP2 carbon atoms). Specific examples thereof include cyclopropenyl, cyclobutenyl, cyclopentenyl, cyclohexenyl, cycloheptenyl, cyclooctenyl, cyclononenyl and cyclodekenyl.

As used herein, the phrase "5- to 14-membered nitrogen-containing heterocyclic ring having an imide structure" refers to a heterocyclic group, having 5 to 14 ring member atoms, containing N as a ring member atom and having an imide structure in which two carbonyl groups are attached to the N. Specific examples thereof include succinimide, glutarimide and phthalimide.

As used herein, the phrases "optionally substituted with" and "substituted with" refer to "optionally substituted with one substituent" and "substituted with one substituent" respectively, when the number of substituents (such as "one or more", "1 to 3", "1 or 2", "2", or "1") is not specified. For example, "B optionally substituted with A" and "B substituted with A" mean "B optionally substituted with one A" and "B substituted with one A", respectively.

In one aspect of the present invention, in the compound represented by formula (I) or a salt thereof, it is preferred that R⁷ and R⁸ are each a hydrogen atom and X is 2,6-dichloro-4-pyridyl. The dichloropyridinyl methyl ester represented by the following formula is hereinafter sometimes referred to as "DCPE". In one aspect of the present invention, in the compound represented by formula (I) or a salt thereof, R⁷ and R⁸ are each a hydrogen atom and X is trifluoromethyl. The 2,2,2-trifluoroethyl ester represented by the following formula is hereinafter sometimes referred to as "TEE".

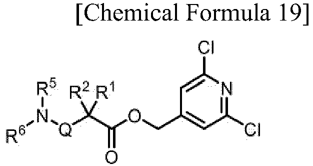

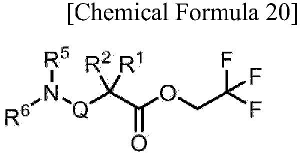

In one aspect of the present invention, a compound represented by formula (V): 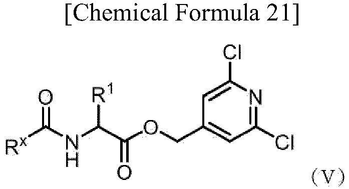 wherein R¹ is a hydrogen atom and R^{x} is 2-methylpropyl or octyl, or R¹ is 2-methylpropyl and R^{x} is methyl;
is excluded from a compound represented by formula (I).

In addition, in the present specification, the main chain amino group of the compound represented by formula (I) may be protected with a general protecting group such as an Fmoc group or a Boc group.

In one aspect of the present invention, provided is a compound represented by formula (IV): 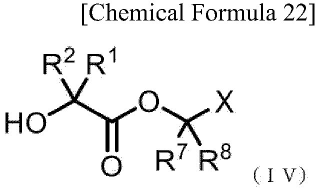
wherein R¹, R², R⁷, R⁸ and X are as defined herein;
   except for the compound of the formula (IV) wherein:
R⁷ and R⁸ are each a hydrogen atom, X is 2,6-dichloropyridin-4-yl;
R¹ and R² are each a hydrogen atom;
R¹ is a hydrogen atom and R² is methyl, ethyl, isopropyl, n-butyl, tert-butyl or isobutyl; or
R¹ is ethyl and R² is methyl or ethyl.

Examples of the salt of the compound represented by formula (I) in the present specification include an acid addition salt and a base addition salt. Examples of the acid addition salt include hydrochloride, hydrobromide, hydroiodide, phosphate, phosphonate, sulfate, and the like; a sulfonate such as methanesulfonate, ethanesulfonate, benzenesulfonate or p-toluenesulfonate; and a carboxylate such as acetate, citrate, malate, tartrate, succinate, salicylate, maleate, fumarate, benzoate, malonate, glycolate, oxalate, glucuronate, adipate, glutarate, ketoglutarate or hippurate. Examples of the base addition salt include an alkali metal salt such as a sodium salt or a potassium salt; an alkaline earth metal salt such as a magnesium salt or a calcium salt; and an ammonium salt such as an ammonium salt, an alkylammonium salt, a dialkylammonium salt, a trialkylammonium salt or a tetraalkylammonium salt; and an amino acid salt such as a lysine salt, an arginine salt, a glycine salt, a valine salt, a threonine salt, a serine salt, a proline salt or an alanine salt. These salts are produced by contacting the compound with an acid or base that can be used in the manufacture of pharmaceuticals.

In the present specification, the compound represented by formula (I) or salt thereof may be an anhydride or may form a solvate such as a hydrate. The term "solvate" as used herein refers to a solid in which a compound molecule and a solvent molecule form a complex. For example, when the solvent is water, it is referred to as a hydrate. Solvates other than a hydrate include a solid containing an alcohol (such as methanol, ethanol or n-propanol), dimethylformamide, or the like.

The compound represented by formula (I) and a salt thereof can also be present in the form of several tautomers such as a keto isomer and an enol isomer, an imine isomer and an enamine isomer, and a mixture thereof. The tautomer is present as a mixture of tautomers in a solution. When the mixture of tautomers is in the form of solid, one tautomer is usually predominant. Although one tautomer may be described, the present invention includes all tautomers of the compound of the present invention.

The present invention includes all stereoisomers of the compound represented by formula (I) (such as enantiomers and diastereomers (including cis and trans geometric isomers)), racemates of such isomers, and other mixtures thereof. For example, the compound of the present invention may have one or more asymmetric centers, and the compound of the present invention includes a racemic mixture, a diastereomeric mixture and enantiomers of such compounds.

When the compound represented by formula (I) is obtained as a free form, it can be converted into a salt that may be formed by the compound, or into a hydrate or solvate thereof, according to any conventional method.

When the compound represented by formula (I) is obtained as a salt, hydrate or solvate thereof, it can be converted into a free form thereof according to any conventional method.

The elements constituting the compound represented by formula (I) may be any isotopes, and the present invention includes the compound of formula (I) containing an isotope. An isotope of the compound is one in which at least one atom has been replaced with an atom having the same atomic number (number of protons) but a different mass number (the sum of the number of protons and the number of neutrons). Examples of the isotope included in the compound of the present invention include isotopes of a hydrogen atom, a carbon atom a nitrogen atom, an oxygen atom, a phosphorus atom, a sulfur atom, a fluorine atom and a chlorine atom, which include ²H, ³H, ¹³C, ¹⁴C, ¹⁵N, ¹⁷O, ¹⁸O, ³¹P, ³²P, ³⁵S, ¹⁸F and ³⁶Cl, respectively. In particular, radioisotopes that decay while emitting radioactivity, such as ³H and ¹⁴C, are useful in testing in vivo tissue distribution of pharmaceuticals or compounds, for example. Since stable isotopes do not decay, hardly change in abundance, and have no radioactivity, they can be used safely. The isotope in the compound of the present invention can be converted according to any conventional method by replacing the reagent used in the synthesis with the reagent containing the corresponding isotope.

In one aspect of the present invention, the compound represented by formula (I) can be used as a reagent for an acylation reaction. Examples of the acylation reaction include an acylation reaction of hydroxy or an amino group optionally having a substituent. In one aspect of the present invention, the compound represented by formula (I) can be used as an activated ester for use in a peptide synthesis reaction.
The peptide synthesis method can be carried out by any technique well known in the art.

In another aspect of the present invention, the compound represented by formula (I) can be used as an activated ester for modifying a group contained in a peptide or protein, such as a hydroxy or an amino group optionally having a substituent, by acylation.

In one aspect of the present invention, the compound represented by formula (I) can be used as a reagent for an aminoacylation reaction of a tRNA. The compound represented by formula (I) is an ester obtained by reacting an amino acid represented by formula (II) with a compound represented by formula (III), and the amino acid represented by formula (II) includes not only a natural amino acid but also an unnatural amino acid. The present inventors have found that the above-described ester can be reacted with a tRNA to attach the amino acid represented by formula (II) to the tRNA at the 3' terminus (aminoacylation reaction). The reaction can be carried out by any known method. In one embodiment, the aminoacylation reaction of a tRNA can be carried out in the presence of a catalyst or enzyme, and preferably in the presence of a flexizyme. The flexizyme is an artificial aminoacylation RNA catalyst, which recognizes and activates only the A (adenosine residue) of CCA at the 3' terminus for all tRNAs. That is, since the flexizyme does not have any strict substrate specificity, the flexizyme can be used to attach various amino acids, including unnatural amino acids, and amino acid derivatives to any tRNA (aminoacylation of a tRNA), thereby synthesizing special peptides having unnatural amino acids or the like incorporated therein. As such a flexizyme, for example, the following:
a prototypic flexizyme Fx
   [5'-GGAUCGAAAGAUUUCCGCAGGCCCGAAAGGGUAUUGGCGUUAGGU-3', 45nt] (SEQ ID NO: 1),
an enhanced flexizyme eFx
   [5'-GGAUCGAAAGAUUUCCGCGGCCCCGAAAGGGGAUUAGCGUUAGGU-3', 45nt]) (SEQ ID NO: 2),
a dinitrobenzyl flexizyme dFx 
[5'-GGAUCGAAAGAUUUCCGCAUCCCCGAAAGGGUACAUGGCGUUAGGU-3',
46nt] (SEQ ID NO: 3),
an amino flexizyme aFx 
[5'-GGAUCGAAAGAUUUCCGCACCCCCGAAAGGGGUAAGUGGCGUUAGGU-3',
47nt]) (SEQ ID NO: 4)
and the like are known (International Publication No. WO 2011/049157), but the flexizyme to be used is not limited thereto, and any substance having a flexizyme activity can be suitably used. Although not limited thereto, dFx can be suitably used for DCPE, and eFx can be suitably used for TEE, respectively.

In one aspect of the present invention, provided is a peptide synthesis method including synthesizing, from an mRNA, a peptide corresponding thereto with a cell-free translation system using an aminoacylated tRNA synthesized by the aminoacylation reaction described above. In addition, in one aspect of the present invention, provided is a method for preparing a peptide library including: preparing an mRNA library, and synthesizing a peptide corresponding to each mRNA from the mRNA library with a cell-free translation system using an aminoacylated tRNA synthesized by the aminoacylation reaction described above, to prepare a peptide library. The mRNA library may be provided by obtaining it from a commercial product or the like, or by preparing it. For example, when preparing an mRNA library, a DNA library may be obtained in accordance with the method described in Chemistry & Biology 18, 1562-1570 (2011) and/or Chemistry & Biology 21, 766-774 (2014), and transcribed in vitro to prepare an mRNA library.

In the present specification, the cell-free translation system is not particularly limited as long as it is a translation system that is free of cells. In one aspect of the present invention, a system in which a peptide or protein of interest is synthesized in vitro by utilizing a protein synthesis function extracted from a cell can be used as a cell-free translation system. Examples of the cell-free translation system that can be used include an Escherichia coli extract, a wheat germ extract, a rabbit red blood cell extract and an insect cell extract. In one aspect of the present invention, a reconstituted cell-free translation system constructed by reconstituting a ribosomal protein, an aminoacyl-tRNA synthetase (aaRS), a ribosomal RNA, an amino acid, an rRNA, a GTP, an ATP, a translation initiation factor (IF), an elongation factor (EF), a release factor (RF) and a ribosome recycling factor (RRF) that have been purified, respectively, as well as other factors necessary for translation can be used as a cell-free translation system.

In the present specification, the cell-free translation system may be a system that includes an RNA polymerase to carry out transcription from DNA in combination. In one aspect of the present invention, the cell-free translation system to be used may be any commercially available system. Examples thereof include a system derived from Escherichia coli such as RTS-100 (R) from Roche Diagnostics; a reconstituted translation system such as PURESYSTEM (R) from PGI or PURExpress (R) In Vitro Protein Synthesis Kit from New England Bio Labs; and a system using wheat germ extract such as a system from ZOEGENE Corporation or a system from CellFree Sciences Co., Ltd.

In addition, as a system using a ribosome of Escherichia coli, for example, the techniques described in the following literature are known: H. F. Kung et al., 1977. The Journal of Biological Chemistry Vol. 252, No. 19, 6889-6894; M. C. Gonza et al., 1985, Proceeding of National Academy of Sciences of the United States of America Vol. 82, 1648-1652; M. Y. Pavlov and M. Ehrenberg, 1996, Archives of Biochemistry and Biophysics Vol. 328, No. 1, 9-16; Y. Shimizu et al., 2001, Nature Biotechnology Vol. 19, No. 8, 751-755; H. Ohashi et al., 2007, Biochemical and Biophysical Research Communications Vol. 352, No. 1, 270-276. The cell-free translation system allows an expression product to be obtained in a highly pure form without purification. Expression of peptides using the cell-free translation system can be carried out, for example, with a flexible in vitro translation system (FIT system) in accordance with the method described in Goto, Y., Katoh, T. & Suga, H. Flexizymes for genetic code reprogramming. Nat Protoc 6, 779-790, (2011).

In one aspect of the present invention, provided is the above-described production method including reacting a compound represented by formula (II): 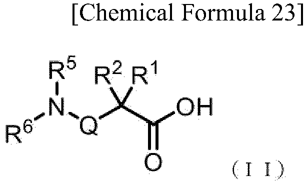
with a compound represented by formula (III): 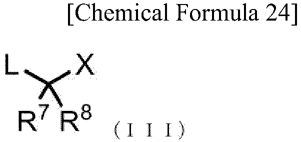
wherein L is a leaving group. In one embodiment of the present invention, the leaving group is selected from a halogen atom and a group represented by R^{D}-SO₂O-, such as a chlorine atom, a bromine atom, an iodine atom, methanesulfonyloxy, benzenesulfonyloxy, toluenesulfonyloxy, trifluoromethanesulfonyloxy or pentafluoroethanesulfonyloxy. The method can be carried out by any method known to those skilled in the art.

In one embodiment of the present invention, the above-described method is carried out in a solvent. Examples of the solvent include DMF.

In one embodiment of the present invention, the above-described method is carried out in the presence of a base. Examples of the base include diisopropylethylamine.

In one embodiment of the present invention, the compound represented by formula (III) can be used in an amount of 0.45 to 1.45 equivalents or 0.65 to 1.25 equivalents, and preferably 0.85 to 1.05 equivalents, relative to the amount of the compound represented by formula (II).

In one embodiment of the present invention, the reaction temperature is set in the range of 0 to 40°C or 0 to 30°C, and preferably 0 to 25°C. In one embodiment of the present invention, the reaction time is set in the range of 30 minutes to 24 hours, 30 minutes to 12 hours, or 30 minutes to 6 hours, and preferably 30 minutes to 2 hours.

In one aspect of the present invention, provided is an amino acid active ester excellent in safety in terms of hazards, such as pyrophoric and explosive properties, in handling as a reagent. The active ester of the present invention is not a nitro compound or a cyanide compound. It is therefore considered to be safer than DBE or CME, which has heretofore been used as an amino acid active ester for the aminoacylation of a tRNA. Assessment tests were carried out to support this. Differential scanning calorimetry (DSC) can be used as a method for determining the hazard of an amino acid active ester (Akiyoshi et al., Netsu Sokutei 2018, 45(4), 161-167.; Sakira Kaneko, Netsu Sokutei 1995, 22(1), 36-43.). DSC is an effective means for assessing the hazard of a self-reactive substance, because it can measure the reaction starting temperature (T_{DSC}) and the reaction heat (Q_{DSC}) with a trace amount of sample. A correlation has been found between the exothermic heat (thermal decomposition energy) obtained by DSC and the fire and explosive properties of chemical substances, and DSC has been conventionally used as a hazard assessment test. In Japan, DSC is used as a test to determine whether a chemical substance falls under the Category V Hazardous materials (self-reactive substances) under the Japanese Fire Service Act. Internationally, in accordance with the UN Recommendation on the Transport of Dangerous Goods (TDG), the need for an explosion assessment test to determine whether a chemical substance is an explosive or not is first determined using the thermal decomposition energy obtained by DSC. If, as a result of such a test, it is determined, for example, that the chemical substance in question falls under the Category V Hazardous materials, careful and complicated measures would be required for storage and handling of the substance, such as: avoiding heat, impact and friction; keeping the substance away from combustibles; keeping the substance away from other chemicals; storing the substance in a cool, dark place; and taking care not to damage the container, and the costs of storage and transportation would also increase.

Examples of the test for determining whether a chemical substance falls under the Category V Hazardous materials (self-reactive substances) in the Japanese Fire Service Act include a method involving using 2,4-dinitrotoluene and benzoyl peroxide as reference compounds, plotting the reference points of Q_{DSC} and T_{DSC} for each of the compounds and drawing a straight line connecting the two points as the hazard determination line. If the chemical substance is on or above the determination line, it is considered "hazardous" (it falls under the Category V Hazardous materials), and if it is below the determination line, it is considered "not hazardous" (it does not fall under the Category V Hazardous materials). The inventors carried out the above method for DBE, which has heretofore been used as an amino acid active ester for the aminoacylation of a tRNA, as well as DCPE and TEE of the present invention, and as a result, DBE was determined to fall under the Category V Hazardous materials, whereas DCPE and TEE were determined not to fall under the Category V Hazardous materials (Test Example). This indicated that the active esters of the present invention are reduced in safety and waste risks, are easy to handle and can also be reduced in disposal costs, compared to the previous active ester.

### EXAMPLES

### Example 1: Synthesis of amino acid active ester

The present invention will be described in more detail below with reference to synthesis examples as Examples, but the present invention is not limited to these Examples.

In present specification, when amino acids and the like are represented by abbreviations, each representation is based on the abbreviation according to the IUPAC-IUB Commission on Biochemical Nomenclature or on the abbreviations commonly used in the art.

The abbreviations in the synthesis examples are as follows:
Fmoc for 9-fluorenylmethyloxycarbonyl or 9-fluorenylmethoxycarbonyl;
Boc for tertiary butoxycarbonyl;
tBu for tertiary butyl;
DMF for N,N-dimethylformamide;
DIPEA for diisopropylethylamine;
EtOAc for ethyl acetate;
Na₂SO₄ for sodium sulfate;
DCM for dichloromethane;
MTHP for 4-methyltetrahydropyran;
MTBE for methyl tertiary butyl ether;
CPME for cyclopentyl methyl ether;
Fx for a flexizyme;
THF for tetrahydrofuran;
HCl for hydrochloric acid;
mL for milliliter (unit);
M for molar (unit: mol/L);
mM for millimolar (unit);
mm for millimeter (unit);
nm for nanometer (unit);
µm for micrometer (unit);
Å for angstrom (unit);
min for minute (unit);
MS for mass spectrometry;
mmol for millimole (unit);
mg for milligram (unit);
LC-MS or LC/MS for liquid chromatography-mass spectrometer; and
tR for retention time.

Unless otherwise stated, proton nuclear magnetic resonance (1H NMR) in the following synthesis examples was measured in deuterated chloroform or deuterated dimethyl sulfoxide solvent using JNM-ECP300, manufactured by JEOL Ltd., or JNM-ECX300, manufactured by JEOL Ltd., or AscendTM500, manufactured by Bruker, and each chemical shift was expressed as a δ value (ppm) relative to that of tetramethylsilane as an internal standard (0.0 ppm).

In the description of each NMR spectrum, "s" refers to singlet, "d" refers to doublet, "t" refers to triplet, "q" refers to quartet, "dd" refers to doublet of doublet, "dt" refers to doublet of triplet, "sept" refers to septet, "m" refers to multiplet, "br" refers to broad, "J" refers to coupling constant, "Hz" refers to hertz, "CDCl3" refers to deuterated chloroform, and "DMSO-d6" refers to deuterated dimethyl sulfoxide.

Unless otherwise stated, high performance liquid chromatography/mass spectrometry was measured using any of ACQUITY UPLC H-Class/QDa, manufactured by Waters Corporation, or ACQUITY UPLC H-Class/SQD2, manufactured by Waters Corporation, or LC-20AD/Triple Tof5600, manufactured by SHIMADZU CORPORATION.

In the descriptions of high-performance liquid chromatography/mass spectrometry, ESI+ is a positive mode of electrospray ionization, M+H refers to a proton adduct, and M + Na refers to a sodium adduct.

In the descriptions of high-performance liquid chromatography/mass spectrometry, ESI- is a negative mode of electrospray ionization, and M-H refers to a proton-deficient form.

The purity of the amino acid active ester synthesized according to each of the following synthesis examples was calculated from the area ratio in the LC/MS chromatogram under the following analytical conditions A to D, and mass spectrometry was carried out with a single quadrupole mass spectrometer and an ESI-MS (+) ion source.

### Analysis condition A

Column: Kinetex (R) EVO C18, 2.6 µm, 2.1 × 150 mm, 100 Å
Mobile phase A: 0.025% TFA in H₂O
Mobile phase B: 0.025% TFA in CH₃CN
Column temperature: 60°C
Gradient (% B): 0-40% over 7.15 minutes, then 40% from 7.16 minutes to 9.00 minutes, then 95-95% over 1.55 minutes; flow rate: 0.5 mL/min
Detection: UV 220 nm.

### Analysis condition B

Column: Kinetex (R) EVO C18, 2.6 µm, 2.1 × 150 mm, 100 Å
Mobile phase A: 0.025% TFA in H₂O
Mobile phase B: 0.025% TFA in CH₃CN
Column temperature: 60°C
Gradient (% B): 5-95% over 7.15 minutes, then 95% from 7.16 minutes to 10.55 minutes;
flow rate: 0.5 mL/min
Detection: UV 220 nm.

### Analysis condition C

Column: Waters XBridge BEH C18 2.5 µm 2.1 × 150 mm 130 Å
Mobile phase A: 0.025% TFA in H₂O
Mobile phase B: 0.025% TFA in CH₃CN
Column temperature: 60°C
Gradient (% B): 0-40% over 7.15 minutes, then 40% from 7.16 minutes to 9.00 minutes, then 95-95% over 1.55 minutes; flow rate: 0.5 mL/min
Detection: UV 220 nm.

### Analysis condition D

Column: Waters ACQUITY UPLC BEH C18 1.7 µm 2.1 × 50 mm, 130 Å
Mobile phase A: 0.025% TFA in H₂O
Mobile phase B: 0.025% TFA in CH₃CN
Column temperature: 60°C
Gradient (% B): 5-95% over 2.78 minutes, then 95% from 2.78 minutes to 3.61 minutes; flow rate: 0.4 mL/min
Detection: UV 220 nm.

The structure of the amino acid active ester synthesized according to each of the following synthesis examples and the results of LC/MS analysis therefor are shown in Table 1.

### Example 1-1: 2,2,2-Trifluoroethyl L-phenylalaninate hydrochloride (Compound No. 39)

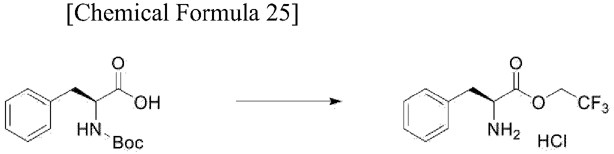

To a flask equipped with a nitrogen balloon were added in sequence (tertbutoxycarbonyl)-L-phenylalanine (7.96 g, 30.0 mmol), DMF (60.0 mL), 2,2,2-trifluoroethyl trifluoromethanesulfonate (6.61 g, 28.5 mmol) and DIPEA (6.29 mL, 36.0 mmol). After stirring at room temperature, a 1 M aqueous HCl solution was added thereto to quench the reaction. The mixture was extracted with EtOAc, and the organic layer was washed with saturated brine, dried over anhydrous Na₂SO₄, filtered through a glass filter and concentrated under reduced pressure. The residue was purified by flash column chromatography (silica gel; EtOAc/hexane = 0/100 → 100/0). DCM (30.0 mL) and 4 N-HCl/MTHP (30.0 mL) were added in sequence to the resulting compound. After stirring at room temperature, the mixture was concentrated under reduced pressure. MTBE was added to the residue, the mixture was filtered, washed with MTBE and dried in vacuum to obtain 2,2,2-trifluoroethyl L-phenylalaninate hydrochloride (7.28 g, 25.7 mmol; yield: 86%) as a colorless solid.

### Example 1-2: 2,2,2-trifluoroethyl (2-chloroacetyl)-L-phenylalaninate (Compound No. 90)

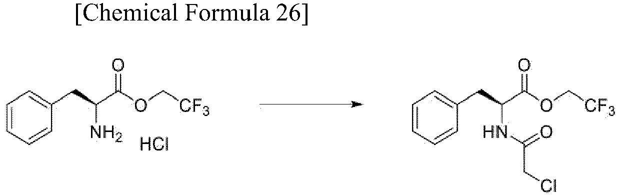

To a flask equipped with a nitrogen balloon were added in sequence 2,2,2-trifluoroethyl L-phenylalaninate hydrochloride (2.84 g, 10.0 mmol), THF (25.0 mL), N-methylmorpholine (2.42 mL, 22.0 mmol) and chloroacetyl chloride (0.881 mL, 11.0 mmol) while maintaining the temperature at -15°C or less. After stirring at -15°C, 1 M HCl was added thereto to quench the reaction. The mixture was extracted with EtOAc, and the organic layer was washed with saturated brine, dried over anhydrous Na₂SO₄, filtered through a glass filter and concentrated under reduced pressure. The residue was purified by flash column chromatography (silica gel; EtOAc/hexane = 0/100 → 100/0) to obtain 2,2,2-trifluoroethyl (2-chloroacetyl)-L-phenylalaninate (3.00 g, 9.27 mmol; yield: 93%) as a colorless solid.

### Example 1-3: (2,6-Dichloropyridin-4-yl)methyl acetyl-L-alaninate (Compound No. 87)

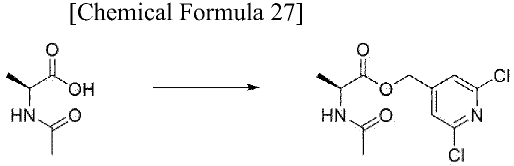

To a flask equipped with a nitrogen balloon were added in sequence acetyl-L-alanine (2.62 g, 20.0 mmol), DMF (40.0 mL), 4-(bromomethyl)-2,6-dichloropyridine (4.58 g, 19.0 mmol) and DIPEA (4.19 ml, 24.0 mmol). After stirring at room temperature, 2 M aqueous HCl solution was added thereto to quench the reaction. The mixture was extracted with EtOAc, and the organic layer was washed with saturated brine, dried over anhydrous Na₂SO₄, filtered through a glass filter and concentrated under reduced pressure. The residue was purified by flash column chromatography (silica gel; EtOAc/hexane = 0/100 → 100/0) to obtain (2,6-dichloropyridin-4-yl)methyl acetyl-L-alaninate (2.93 g, 10.1 mmol; yield: 50%) as a colorless solid.

### Example 1-4: (2,6-Dichloropyridin-4-yl)methyl (2-([1,1'-biphenyl]-4-yl)ethyl)glycinate hydrochloride (Compound No. 35)

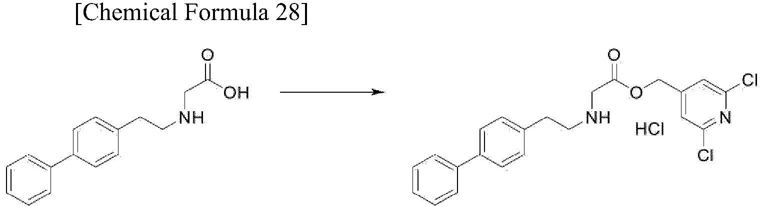

To a flask equipped with a nitrogen balloon were added in sequence (2-([1,1'-biphenyl]-4-yl)ethyl)glycine (1.55 g, 6.07 mmol, CAS Registry Number: 1906593-76-7), 1,4-dioxane (14.0 mL), H₂O (7.00 mL), sodium hydrogen carbonate (1.53 g, 18.2 mmol) and di-tert-butyl dicarbonate (1.32 g, 6.07 mmol, CAS Registry Number: 24424-99-5) under ice cooling. After stirring at room temperature, an aqueous citric acid solution was added thereto to quench the reaction. The mixture was extracted with EtOAc, and the organic layer was washed with saturated brine, dried over anhydrous Na₂SO₄, filtered through a glass filter and concentrated under reduced pressure. The residue was purified by flash column chromatography (silica gel; 0.1% aqueous formic acid solution/acetonitrile = 50/50 → 20/80). DMF (5.6 mL), 4-(bromomethyl)-2,6-dichloropyridine (0.644 g, 2.67 mmol) and DIPEA (0.590 mL, 3.38 mmol) were added in sequence to the resulting compound (1.00 g). After stirring at room temperature, a 1 M aqueous HCl solution was added thereto to quench the reaction. The mixture was extracted with EtOAc, and the organic layer was washed in sequence with an aqueous sodium hydrogen carbonate solution and saturated brine, dried over anhydrous Na₂SO₄, filtered through a glass filter and concentrated under reduced pressure. The residue was purified by flash column chromatography (silica gel; EtOAc/hexane = 20/80 → 40/60). DCM (8.0 mL) and 4 N-HCl/MTHP (8.0 mL) were added in sequence to the resulting compound. After stirring at room temperature, the mixture was concentrated under reduced pressure. Diisopropyl ether was added to the residue, and the mixture was filtered, washed with diisopropyl ether and dried in vacuum to obtain (2,6-dichloropyridin-4-yl)methyl (2-([1,1'-biphenyl]-4-yl)ethyl)glycinate hydrochloride (941 mg, 2.08 mmol) as a colorless solid.

### Example 1-5: (2,6-Dichloropyridin-4-yl)methyl (S)-2-amino-3-(3-guanidinophenyl)propanoate dihydrochloride (Compound No. 74)

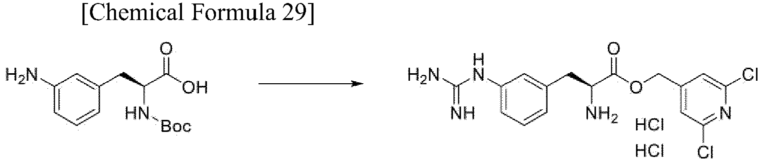

To a flask equipped with a nitrogen balloon were added in sequence (S)-3-(3-aminophenyl)-2-((tert-butoxycarbonyl)amino)propanoic acid (1.03 g, 3.67 mmol), absolute ethanol (15 mL) and N,N'-bis(tert-butoxycarbonyl)-1H-pyrazole-1-carboxamidine (1.14 g, 3.67 mmol, CAS Registry Number: 152120-54-2). After stirring at room temperature, a 1 M aqueous HCl solution was added thereto to quench the reaction. The mixture was extracted with EtOAc, and the organic layer was washed with saturated brine, dried over anhydrous Na₂SO₄, filtered through a glass filter and concentrated under reduced pressure. DMF (60.0 mL), 4-(bromomethyl)-2,6-dichloropyridine (0.841 g, 3.49 mmol) and DIPEA (0.770 mL, 4.41 mmol) were added in sequence to the resulting residue. After stirring at room temperature, a 1 M aqueous HCl solution was added thereto to quench the reaction. The mixture was extracted with EtOAc, and the organic layer was washed in sequence with an aqueous sodium hydrogen carbonate solution and saturated brine, dried over anhydrous Na₂SO₄, filtered through a glass filter and concentrated under reduced pressure. The residue was purified by flash column chromatography (silica gel; EtOAc/hexane = 10/90 → 30/70). DCM (26.0 mL) and 4 N-HCl/MTHP (26.0 mL) were added in sequence to the resulting compound. After stirring at room temperature, the mixture was concentrated under reduced pressure. Diethyl ether was added to the residue, and the mixture was filtered, washed with diethyl ether, and dried in vacuum to obtain (2,6-dichloropyridin-4-yl)methyl (S)-2-amino-3-(3-guanidinophenyl)propanoate dihydrochloride (962 mg, 2.11 mmol) as a colorless solid.

### Example 1-6: (2,6-Dichloropyridin-4-yl)methyl (S)-3-cyclopentyl-2-(methylamino)propanoate hydrochloride (Compound No. 75)

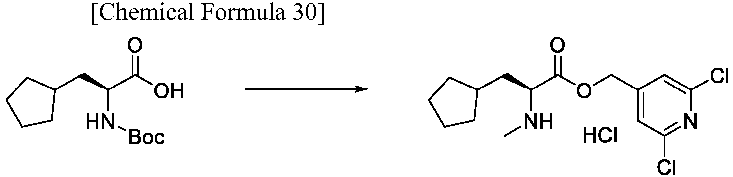

To a flask equipped with a nitrogen balloon were added in sequence (S)-2-((tertbutoxycarbonyl)amino)-3-cyclopentylpropanoic acid (10.0 g, 38.9 mmol, CAS Registry Number: 143415-31-0), THF (300 mL) and 60% sodium hydride (2.80 g, 117 mmol, CAS Registry Number: 7646-69-7) under ice cooling. After stirring the mixture at room temperature for 30 minutes, iodomethane (11.0 g, 77.7 mmol, CAS Registry Number: 74-88-4) was added to this mixture. After stirring the mixture at room temperature overnight, an aqueous citric acid solution was added thereto to quench the reaction. The mixture was extracted with ethyl acetate, and the organic layer was washed with saturated brine, dried over anhydrous sodium sulfate, filtered and concentrated under reduced pressure. The residue was purified by flash column chromatography (silica gel; acetonitrile/0.1% aqueous formic acid solution = 10/90 → 80/20) to obtain (S)-2-((tert-butoxycarbonyl)(methyl)amino)-3-cyclopentylpropanoic acid (5.00 g). To the obtained compound were added in sequence DMF (25 mL), 4-(bromomethyl)-2,6-dichloropyridine (4.30 g, 17.9 mmol, CAS Registry Number: 175204-45-2) and DIPEA (3.92 mL, 22.6 mmol). After stirring the mixture at room temperature for 3 hours, ethyl acetate was added to this mixture. The organic layer was washed with saturated brine, dried over anhydrous sodium sulfate, filtered and concentrated under reduced pressure. The residue was purified by flash column chromatography (silica gel; acetonitrile/0.1% aqueous formic acid solution = 10/90 → 100/0) to obtain (2,6-dichloropyridin-4-yl)methyl (S)-2-((tert-butoxycarbonyl)(methyl)amino)-3-cyclopentylpropanoate (6.67 g). Dichloromethane (26 mL) and 4 M-hydrochloric acid/MTHP (26 mL) were added in sequence to the obtained compound (3.00 g) under ice cooling. The mixture was stirred at room temperature for 2 hours and then concentrated under reduced pressure. The residue was suspended in and washed with diethyl ether and dried in vacuum to obtain (2,6-dichloropyridin-4-yl)methyl (S)-3-cyclopentyl-2-(methylamino)propanoate hydrochloride (2.62 g, 7.13 mmol) as a colorless solid.

### Example 1-7: 2,2,2-trifluoroethyl (S)-2-amino-3-(6-phenylpyridin-3-yl)propanoate dihydrochloride (Compound No. 76)

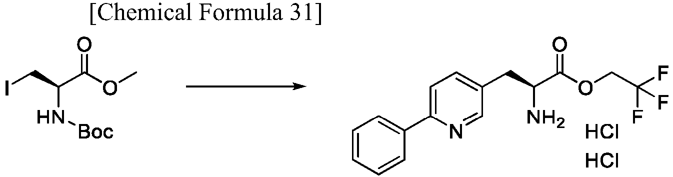

To a flask equipped with a nitrogen balloon were added in sequence zinc (9.22 g, 141 mmol, CAS Registry Number: 7440-66-6), DMF (150 mL) and iodine (1.79 g, 7.05 mmol, CAS Registry Number: 7553-56-2). After stirring the mixture at room temperature for 10 minutes, (S)-methyl 2-(tert-butoxycarbonylamino)-3-iodopropanoate (15.5 g, 47.0 mmol, CAS Registry Number: 93267-04-0) and iodine (1.79 g, 7.05 mmol, CAS Registry Number: 7553-56-2) were added in sequence to this mixture. After stirring the mixture at room temperature for 30 minutes, 5-bromo-2-phenylpyridine (13.2 g, 56.4 mmol, CAS Registry Number: 27012-25-5), 2-dicyclohexylphosphino-2',6'-dimethoxybiphenyl (0.96 g, 2.3 mmol, CAS Registry Number: 657408-07-6) and tris(dibenzylideneacetone)dipalladium-chloroform (1.46 g, 1.41 mmol, CAS Registry Number: 52522-40-4) were added in sequence to this mixture. After stirring at 50°C for 3 hours, this mixture was filtered, extracted with ethyl acetate, and the organic layer was washed with saturated brine, dried over anhydrous sodium sulfate, filtered and concentrated under reduced pressure. The residue was purified by flash column chromatography (silica gel; ethyl acetate/petroleum ether = 1/10 → 1/3) to obtain methyl (S)-2-((tertbutoxycarbonyl)amino)-3-(6-phenylpyridin-3-yl)propanoate (16.0 g). To the obtained compound were added in sequence isopropanol (180 mL), water (60 mL), calcium chloride (79.7 g, 718 mmol, CAS Registry Number: 10043-52-4) and lithium hydroxide monohydrate (7.55 g, 180 mmol, CAS Registry Number: 1310-66-3) under ice cooling. After stirring the mixture at room temperature for 24 hours, sodium dihydrogen phosphate was added thereto to quench the reaction. The mixture was filtered and extracted with ethyl acetate, and the organic layer was washed with saturated brine, dried over anhydrous sodium sulfate, filtered and concentrated under reduced pressure. The residue was purified by flash column chromatography (silica gel; methanol/dichloromethane = 1/50 → 1/10) to obtain (S)-2-((tertbutoxycarbonyl)amino)-3-(6-phenylpyridin-3-yl)propanoic acid (14.1 g). DMF (140 mL), 2,2,2-trifluoroethyl trifluoromethanesulfonate (9.08 g, 39.1 mmol, CAS Registry Number: 6226-25-1) and DIPEA (6.39 g, 49.4 mmol) were added in sequence to the obtained compound. After stirring the mixture at room temperature for 3 hours, water and sodium dihydrogen phosphate were added thereto in sequence to quench the reaction. The mixture was extracted with ethyl acetate, and the organic layer was washed with saturated brine, dried over anhydrous sodium sulfate, filtered and concentrated under reduced pressure. The residue was purified by flash column chromatography (silica gel; ethyl acetate/petroleum ether = 1/10 → 1/1) to obtain 2,2,2-trifluoroethyl (S)-2-((tert-butoxycarbonyl)amino)-3-(6-phenylpyridin-3-yl)propanoate (15.1 g). Dichloromethane (30 mL) and 4 M-hydrochloric acid/MTHP (30 mL) were added in sequence to the obtained compound (1.87 g). The mixture was stirred at room temperature for 3 hours and then concentrated under reduced pressure. The residue was suspended in and washed with diethyl ether and dried in vacuum to obtain 2,2,2-trifluoroethyl (S)-2-amino-3-(6-phenylpyridin-3-yl)propanoate dihydrochloride (1.56 g, 4.32 mmol) as a colorless solid.

### Example 1-8: (2,6-Dichloropyridin-4-yl)methyl (S)-2-amino-4-(quinolin-5-yl)butanoate dihydrochloride (Compound No. 77)

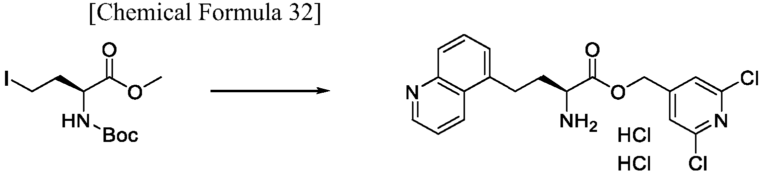

To a flask equipped with a nitrogen balloon were added in sequence zinc (47.2 g, 721 mmol, CAS Registry Number: 7440-66-6), DMF (500 mL) and iodine (36.6 g, 144 mmol, CAS Registry Number: 7553-56-2). After stirring the mixture at room temperature for 5 minutes, methyl (S)-2-((tert-butoxycarbonyl)amino)-4-iodobutanoate (99.0 g, 288 mmol, CAS Registry Number: 101650-14-0) was added to this mixture. After stirring the mixture at room temperature for 1 hour, 5-bromoquinoline (50.0 g, 240 mmol, CAS Registry Number: 4964-7-10), 2-dicyclohexylphosphino-2',6'-dimethoxybiphenyl (9.85 g, 24.0 mmol, CAS Registry Number: 657408-07-6) and tris(dibenzylideneacetone)dipalladium-chloroform (11.0 g, 12.0 mmol, CAS Registry Number: 52522-40-4) were added in sequence to this mixture. After stirring at 50°C for 3 hours, this mixture was filtered and extracted with ethyl acetate, and the organic layer was washed with saturated brine, dried over anhydrous sodium sulfate, filtered and concentrated under reduced pressure. The residue was purified by flash column chromatography (silica gel; ethyl acetate/petroleum ether = 1/1) to obtain methyl (S)-2-((tertbutoxycarbonyl)amino)-4-(quinolin-5-yl)butanoate (40.0 g). THF (200 mL) and lithium hydroxide monohydrate (5.85 g, 139 mmol, CAS Registry Number: 1310-66-3) dissolved in water (200 mL) were added in sequence to the obtained compound under ice cooling. After stirring the mixture at room temperature for 2 hours, an aqueous sodium dihydrogen phosphate solution was added thereto to quench the reaction. The mixture was extracted with ethyl acetate, and the organic layer was washed with saturated brine, dried over anhydrous sodium sulfate, filtered and concentrated under reduced pressure to obtain (S)-2-((tert-butoxycarbonyl)amino)-4-(quinolin-5-yl)butanoic acid (20.0 g). DMF (50 mL), 4-(bromomethyl)-2,6-dichloropyridine (3.46 g, 14.4 mmol, CAS Registry Number: 175204-45-2) and DIPEA (3.17 mL, 18.2 mmol) were added in sequence to the obtained compound (5.00 g). After stirring the mixture at room temperature for 30 minutes, an aqueous sodium dihydrogen phosphate solution was added thereto to quench the reaction. The mixture was extracted with ethyl acetate, and the organic layer was washed with saturated brine, dried over anhydrous sodium sulfate, filtered and concentrated under reduced pressure. The residue was purified by flash column chromatography (silica gel; ethyl acetate/petroleum ether = 1/5) to obtain (2,6-dichloropyridin 4-yl)methyl (S)-2-((tert-butoxycarbonyl)amino)-4-(quinolin-5-yl)butanoate (6.31 g). Dichloromethane (25 mL) and 4 M-hydrochloric acid/MTHP (25 mL) were added in sequence to the obtained compound (3.30 g). The mixture was stirred at room temperature for 2 hours and then concentrated under reduced pressure. The residue was suspended in and washed with diethyl ether and dried in vacuum to obtain (2,6-dichloropyridin-4-yl)methyl (S)-2-amino-4-(quinolin-5-yl)butanoate dihydrochloride (2.92 g, 6.84 mmol) as a colorless solid.

### Example 1-9: (2,6-Dichloropyridin-4-yl)methyl (S)-2-amino-3-(6-aminopyridin-3-yl)propanoate dihydrochloride (Compound No. 78)

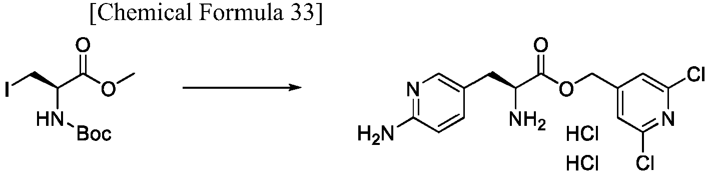

To a flask equipped with a nitrogen balloon were added in sequence zinc (22.7 g, 347 mmol, CAS Registry Number: 7440-66-6), DMF (400 mL) and iodine (8.80 g, 34.7 mmol, CAS Registry Number: 7553-56-2). After stirring the mixture at room temperature, (S)-methyl 2-(tert-butoxycarbonylamino)-3-iodopropanoate (41.9 g, 127 mmol, CAS Registry Number: 93267-04-0) and iodine (8.80 g, 34.7 mmol, CAS Registry Number: 7553-56-2) were added in sequence to this mixture. After stirring the mixture at room temperature for 30 minutes, 2-amino-5-bromopyridine (20.0 g, 116 mmol, CAS Registry Number: 1072-97-5), 2-dicyclohexylphosphino-2',6'-dimethoxybiphenyl (9.49 g, 23.1 mmol, CAS Registry Number: 657408-07-6) and tris(dibenzylideneacetone)dipalladium-chloroform (5.29 g, 5.78 mmol, CAS Registry Number: 52522-40-4) were added in sequence to this mixture. After stirring at 50°C for 3 hours, ethyl acetate was added to this mixture. The mixture was filtered and extracted with ethyl acetate, and the organic layer was washed with saturated brine, dried over anhydrous sodium sulfate, filtered and concentrated under reduced pressure. The residue was purified by flash column chromatography (silica gel; ethyl acetate/petroleum ether = 1/1) to obtain methyl (S)-3-(6-aminopyridin-3-yl)-2-((tertbutoxycarbonyl)amino)propanoate (30.0 g). Dichloromethane (200 mL), tert-butanol (800 mL), di-tert-butyl dicarbonate (35.5 g, 163 mmol, CAS Registry Number: 24424-99-5) and sodium iodide (24.4 g, 163 mmol, CAS Registry Number: 7681-82-5) were added in sequence to the obtained compound (40.0 g). After stirring the mixture at room temperature overnight, this mixture was concentrated under reduced pressure and ethyl acetate was added thereto. The organic layer was washed with saturated brine, dried over anhydrous sodium sulfate, filtered and concentrated under reduced pressure. The residue was purified by flash column chromatography (silica gel; ethyl acetate/petroleum ether = 1/1) to obtain methyl (S)-2-((tert-butoxycarbonyl)amino)-3-(6-((tert-butoxycarbonyl)amino)pyridin-3-yl)propanoate (50.0 g). THF (100 mL), isopropanol (600 mL), water (200 mL), calcium chloride (135 g, 1210 mmol, CAS Registry Number: 10043-52-4) and lithium hydroxide monohydrate (12.8 g, 304 mmol, CAS Registry Number: 1310-66-3) were added in sequence to the obtained compound (30.0 g) under ice cooling. After stirring the mixture at room temperature, sodium dihydrogen phosphate was added thereto to quench the reaction. This mixture was filtered and extracted with ethyl acetate, and the organic layer was washed with saturated brine, dried over anhydrous sodium sulfate, filtered and concentrated under reduced pressure. The residue was purified by flash column chromatography (silica gel; ethyl acetate/dichloromethane = 1/1) to obtain (S)-2-((tert-butoxycarbonyl)amino)-3-(6-phenylpyridin-3-yl)propanoic acid (15.0 g). DMF (50 mL), 4-(bromomethyl)-2,6-dichloropyridine (5.40 g, 22.4 mmol, CAS Registry Number: 175204-45-2) and DIPEA (4.93 mL, 28.3 mmol) were added in sequence to the obtained compound (9.00 g). After stirring the mixture at room temperature for 2 hours, ethyl acetate was added to this mixture. The organic layer was washed in sequence with an aqueous sodium hydrogen carbonate solution and saturated brine, dried over anhydrous sodium sulfate, filtered and concentrated under reduced pressure. The residue was purified by flash column chromatography (silica gel; ethyl acetate/petroleum ether = 1/5) to obtain (2,6-dichloropyridin-4-yl)methyl (S)-2-((tert-butoxycarbonyl)amino)-3-(6-((tert-butoxycarbonyl)amino)pyridin-3-yl)propanoate (11.2 g). Dichloromethane (25 mL) and 4 M-hydrochloric acid/MTHP (25 mL) were added in sequence to the obtained compound (3.00 g). The mixture was stirred at room temperature and then concentrated under reduced pressure. The residue was suspended in and washed with methyl tert-butyl ether and dried in vacuum to obtain (2,6-dichloropyridin-4-yl)methyl (S)-2-amino-3-(6-aminopyridin-3-yl)propanoate dihydrochloride (2.32 g, 5.60 mmol) as a colorless solid.

### Example 1-10: (2,6-Dichloropyridin-4-yl)methyl (S)-2-amino-4-(2-aminopyridin-4-yl)butanoate dihydrochloride (Compound No. 79)

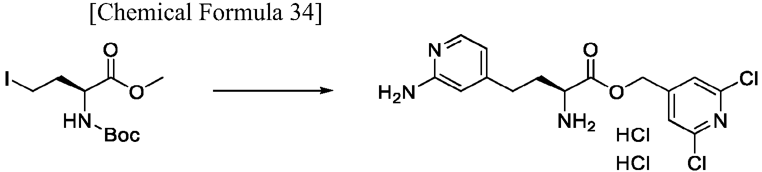

To a flask equipped with a nitrogen balloon were added in sequence zinc (8.57 g, 131 mmol, CAS Registry Number: 7440-66-6), DMF (200 mL) and iodine (6.66 g, 26.2 mmol, CAS Registry Number: 7553-56-2). After stirring the mixture at room temperature for 5 minutes, methyl (S)-2-((tert-butoxycarbonyl)amino)-4-iodobutanoate (15.0 g, 43.7 mmol, CAS Registry Number: 101650-14-0) was added to this mixture. After stirring the mixture at room temperature for 1 hour, 2-amino-4-bromopyridine (8.32 g, 48.1 mmol, CAS Registry Number: 84249-14-9), 2-dicyclohexylphosphino-2',6'-dimethoxybiphenyl (3.59 g, 8.75 mmol, CAS Registry Number: 657408-07-6) and tris(dibenzylideneacetone)dipalladium-chloroform (2.00 g, 2.18 mmol, CAS Registry Number: 52522-40-4) were added in sequence to this mixture. After stirring at 60°C for 3 hours, this mixture was filtered and extracted with ethyl acetate. The organic layer was washed with saturated brine, dried over anhydrous sodium sulfate, filtered and concentrated under reduced pressure. The residue was purified by flash column chromatography (silica gel; ethyl acetate/petroleum ether = 1/2) to obtain methyl (S)-4-(2-aminopyridin-4-yl)-2-((tert-butoxycarbonyl)amino)butanoate (9.00 g). tert-Butanol (90 mL), sodium iodide (5.23 g, 34.9 mmol, CAS Registry Number: 7681-82-5) and di-tert-butyl dicarbonate (7.62 g, 34.9 mmol, CAS Registry Number: 24424-99-5) were added in sequence to the obtained compound. After stirring the mixture at room temperature for 16 hours, ethyl acetate was added to this mixture. The organic layer was washed with saturated brine, dried over anhydrous sodium sulfate, filtered and concentrated under reduced pressure. The residue was purified by flash column chromatography (silica gel; ethyl acetate/petroleum ether = 1/2) to obtain methyl (S)-2-((tert-butoxycarbonyl)amino)-4-(2-((tertbutoxycarbonyl)amino)pyridin-4-yl)butanoate (8.80 g). Isopropanol (120 mL), calcium chloride (38.2 g, 344 mmol, CAS Registry Number: 10043-52-4) and lithium hydroxide monohydrate (3.61 g, 86.0 mmol, CAS Registry Number: 1310-66-3) dissolved in water (40 mL) were added in sequence to the obtained compound under ice cooling. The mixture was stirred at room temperature for 16 hours and then filtered. An aqueous sodium dihydrogen phosphate solution was added thereto to quench the reaction. The mixture was extracted with ethyl acetate, and the organic layer was washed with saturated brine, dried over anhydrous sodium sulfate, filtered and concentrated under reduced pressure. The residue was purified by flash column chromatography (silica gel; ethyl acetate/petroleum ether = 1/1) to obtain (S)-2-((tert-butoxycarbonyl)amino)-4-(2-((tertbutoxycarbonyl)amino)pyridin-4-yl)butanoic acid (8.00 g). DMF (80 mL), 4-(bromomethyl)-2,6-dichloropyridine (4.63 g, 19.2 mmol, CAS Registry Number: 175204-45-2) and DIPEA (4.23 mL, 24.3 mmol) were added in sequence to the obtained compound. After stirring the mixture at room temperature for 30 minutes, an aqueous sodium dihydrogen phosphate solution was added thereto to quench the reaction. The mixture was extracted with ethyl acetate, and the organic layer was washed with saturated brine, dried over anhydrous sodium sulfate, filtered and concentrated under reduced pressure. The residue was purified by flash column chromatography (silica gel; acetonitrile/10 mM aqueous ammonium hydrogen carbonate solution = 10/90 → 100/0) to obtain (2,6-dichloropyridin 4-yl)methyl (S)-2-((tert-butoxycarbonyl)amino)-4-(2-((tert-butoxycarbonyl)amino)pyridin-4-yl)butanoate (5.62 g). Dichloromethane (18 mL), triisopropylsilane (2.14 g, 13.5 mmol, CAS Registry Number: 6485-79-6) and 2,2,2-trifluoroacetic acid (12.5 mL) were added in sequence to the obtained compound (3.00 g). The mixture was stirred at room temperature for 18 hours and then concentrated under reduced pressure, and 4 M-hydrochloric acid/CPME (25 mL) was added thereto. The mixture was stirred at room temperature for 1 hour, and then filtered, washed with diethyl ether and dried in vacuum to obtain (2,6-dichloropyridin-4-yl)methyl (S)-2-amino-4-(2-aminopyridin-4-yl)butanoate dihydrochloride (1.52 g, 3.55 mmol) as a colorless solid.

### Example 1-11: (S)-2-(4-(4-Amino-5-((2,6-dichloropyridin-4-yl)methoxy)-5-oxopentanoyl)piperazin-1-yl)acetic acid dihydrochloride (Compound No. 60)

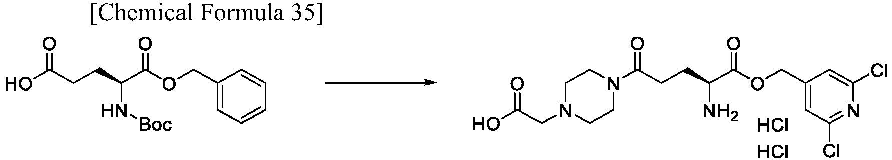

To a flask equipped with a nitrogen balloon were added in sequence (S)-5-(benzyloxy)-4-((tert-butoxycarbonyl)amino)-5-oxopentanoic acid (15.0 g, 44.5 mmol, CAS Registry Number: 30924-93-7), tert-butyl 2-(piperazin-1-yl)acetate (8.90 g, 44.5 mmol, CAS Registry Number: 112257-22-4) and THF (300 mL) under ice cooling. After stirring the mixture at room temperature, ethyl 2-cyano-2-(hydroxyimino)acetate (6.94 g, 48.9 mmol, CAS Registry Number: 3849-21-6) and 1-ethyl-3-(3-dimethylaminopropyl)carbodiimide hydrochloride (9.38 g, 48.9 mmol, CAS Registry Number: 25952-53-8) were added in sequence to this mixture under ice cooling. After stirring the mixture at room temperature overnight, ethyl acetate was added to this mixture. The organic layer was washed with saturated brine, dried over anhydrous sodium sulfate, filtered and concentrated under reduced pressure. The residue was purified by flash column chromatography (silica gel; ethyl acetate/petroleum ether = 1/1) to obtain benzyl (S)-5-(4-(2-(tert-butoxy)-2-oxoethyl)piperazin-1-yl)-2-((tert-butoxycarbonyl)amino)-5-oxopentanoate (13.0 g). Methanol (260 mL) and palladium/carbon (2.60 g, CAS Registry Number: 7440-05-3) were added in sequence to the obtained compound. After stirring at room temperature under a hydrogen atmosphere for 2 hours, this mixture was filtered, extracted with ethyl acetate and concentrated under reduced pressure to obtain (S)-5-(4-(2-(tert-butoxy)-2-oxoethyl)piperazin-1-yl)-2-((tert-butoxycarbonyl)amino)-5-oxopentanoic acid (12.0 g). DMF (100 mL), 4-(bromomethyl)-2,6-dichloropyridine (6.39 g, 26.5 mmol, CAS Registry Number: 175204-45-2) and DIPEA (5.60 mL, 32.1 mmol) were added in sequence to the obtained compound. After stirring the mixture at room temperature for 2 hours, ethyl acetate was added to this mixture. The organic layer was washed in sequence with saturated brine, dried over anhydrous sodium sulfate, filtered and concentrated under reduced pressure. The residue was purified by flash column chromatography (silica gel; ethyl acetate/petroleum ether = 1/1) to obtain (2,6-dichloropyridin-4-yl)methyl (S)-5-(4-(2-(tert-butoxy)-2-oxoethyl)piperazin-1-yl)-2-((tert-butoxycarbonyl)amino)-5-oxopentanoate (9.07 g). Dichloromethane (25 mL) and 4 M-hydrochloric acid/CPME (25 mL) were added in sequence to the obtained compound (3.00 g). The mixture was stirred at room temperature for 3 hours and then concentrated under reduced pressure. The residue was suspended in and washed with methyl tert-butyl ether and dried in vacuum to obtain (S)-2-(4-(4-amino-5-((2,6-dichloropyridin-4-yl)methoxy)-5-oxopentanoyl)piperazin-1-yl)acetic acid dihydrochloride (2.39 g, 4.72 mmol) as a colorless solid.

### Example 1-12: (2,6-Dichloropyridin-4-yl)methyl (3-methoxypropyl)glycinate hydrochloride (Compound No. 61)

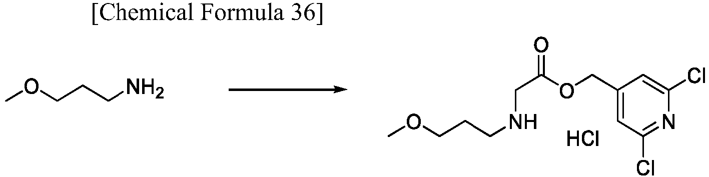

To a flask equipped with a nitrogen balloon were added in sequence 3-methoxypropylamine (10.0 g, 112 mmol, CAS Registry Number: 5332-73-0), ethyl glyoxylate (23.0 mL, 225 mmol, CAS Registry Number.: 924-44-7) and dichloromethane (200 mL). After stirring the mixture at room temperature for 3 minutes, sodium cyanoborohydride (14.0 g, 223 mmol, CAS Registry Number: 25895-60-7) and acetic acid (9.5 mL) were added in sequence to this mixture. After stirring the mixture at room temperature for 30 minutes, triethylamine (31.3 mL, 224 mmol) and di-tert-butyl dicarbonate (37.0 g, 170 mmol, CAS Registry Number: 24424-99-5) were added thereto in sequence. The mixture was stirred at room temperature and then concentrated under reduced pressure. The residue was purified by flash column chromatography (silica gel; ethyl acetate/petroleum ether = 1/1) to obtain ethyl N-(tert-butoxycarbonyl)-N-(3-methoxypropyl)glycinate (11.0 g). 1,4-Dioxane (10 mL), water (10 mL) and lithium hydroxide monohydrate (2.30 g, 54.8 mmol) were added in sequence to the obtained compound (9.50 g) at room temperature. After stirring the mixture at room temperature, an aqueous sodium dihydrogen phosphate solution was added thereto to quench the reaction. The mixture was extracted with ethyl acetate, and the organic layer was washed with saturated brine, dried over anhydrous sodium sulfate, filtered and concentrated under reduced pressure to obtain N-(tert-butoxycarbonyl)-N-(3-methoxypropyl)glycine (8.30 g). DMF (50 mL), 4-(bromomethyl)-2,6-dichloropyridine (5.70 g, 1.91 mmol, CAS Registry Number: 175204-45-2) and DIPEA (6.0 mL, 2.30 mmol) were added in sequence to the obtained compound (7.30 g). The mixture was stirred at room temperature and then purified by flash column chromatography (silica gel; water/methanol = 95/5 → 0/100) to obtain (2,6-dichloropyridin-4-yl)methyl N-(tert-butoxycarbonyl)-N-(3-methoxypropyl)glycinate (6.18 g). Dichloromethane (18 mL) and 4 M-hydrochloric acid/CPME (18 mL) were added in sequence to the obtained compound (1.90 g). The mixture was stirred at room temperature for 2 hours and then concentrated under reduced pressure. The residue was suspended in and washed with diethyl ether and dried in vacuum to obtain (2,6-dichloropyridin-4-yl)methyl (3-methoxypropyl)glycinate (1.31 g, 3.81 mmol) as a colorless solid.

### Example 1-13: 2,2,2-Trifluoroethyl (S)-2-amino-3-(4-(5-((3aS,4S,6aR)-2-oxohexahydro-1H-thieno[3,4-d]imidazol-4-yl)pentanamido)phenyl)propanoate hydrochloride (Compound No. 62)

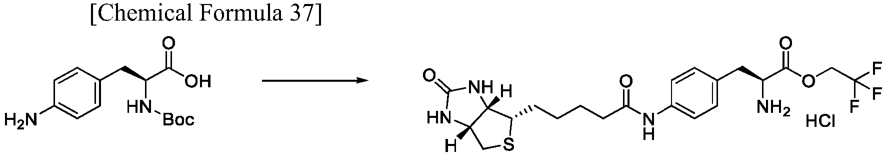

To a flask equipped with a nitrogen balloon were added in sequence (S)-3-(4-aminophenyl)-2-((tert-butoxycarbonyl)amino)propanoic acid (7.01 g, 25 mmol, CAS Registry Number: 55533-24-9), DMF (50 mL), allyl(2,5-dioxopyrrolidin-1-yl)carbonate (4.88 g, 24.5 mmol, CAS Registry Number: 135544-68-2) and DIPEA (4.80 mL, 27.5 mmol) under ice cooling. After stirring the mixture at room temperature, hydrochloric acid was added thereto to quench the reaction. The mixture was extracted with ethyl acetate, and the organic layer was washed in sequence with water and saturated brine, dried over anhydrous sodium sulfate, filtered and concentrated under reduced pressure to obtain (S)-3-(4-((allyloxy)carbonyl)amino)phenyl)-2-((tert-butoxycarbonyl)amino)propanoic acid. DMF (50 mL), 2,2,2-trifluoroethyl trifluoromethanesulfonate (5.80 g, 25.0 mmol, CAS Registry Number: 6226-25-1) and DIPEA (5.24 mL, 30 mmol) were added in sequence to the obtained compound. After stirring the mixture at room temperature, hydrochloric acid was added thereto to quench the reaction. The mixture was extracted with ethyl acetate, and the organic layer was washed in sequence with water and an aqueous sodium hydrogen carbonate solution, dried over anhydrous sodium sulfate, filtered and concentrated under reduced pressure. The residue was purified by flash column chromatography (silica gel; ethyl acetate/heptane = 0/100 → 100/0) to obtain 2,2,2-trifluoroethyl (S)-3-(4-(((allyloxy)carbonyl)amino)phenyl)-2-((tert-butoxycarbonyl)amino)propanoate (3.70 g). Dichloromethane (20 mL), tetrakis(triphenylphosphine)palladium (0.185 g, 0.160 mmol, CAS Registry Number: 14221-01-3) and phenylsilane (2.95 mL, 24.0 mmol, CAS Registry Number: 694-53-1) were added in sequence to the obtained compound (3.57 g) under a nitrogen atmosphere under ice cooling. This mixture was stirred under a nitrogen atmosphere under ice cooling and then purified by flash column chromatography (silica gel; ethyl acetate/heptane = 0/100 → 100/0) to obtain 2,2,2-trifluoroethyl (S)-3-(4-aminophenyl)-2-((tert-butoxycarbonyl)amino)propanoate (2.26 g). DMF (8.0 mL), biotin (0.537 g, 2.20 mmol, CAS Registry Number: 58-85-5), ethyl 2-cyano-2-(hydroxyimino)acetate (0.313 g, 2.20 mmol, CAS Registry Number: 3849-21-6) and 1-ethyl-3-(3-dimethylaminopropyl)carbodiimide hydrochloride (0.422 g, 2.20 mmol, CAS Registry Number: 25952-53-8) were added in sequence to the obtained compound (0.725 g). After stirring the mixture at room temperature, water was added thereto to quench the reaction. The resulting residue was filtered, washed with water and purified by flash column chromatography (silica gel; methanol/dichloromethane = 3/97 → 20/80) to obtain 2,2,2-trifluoroethyl (S)-2-((tert-butoxycarbonyl)amino)-3-(4-(5-((3aS,4S,6aR)-2-oxohexahydro-1H-thieno[3,4-d]imidazol-4-yl)pentanamido)phenyl)propanoate (0.965 g). Dichloromethane (8.2 mL) and 4 M-hydrochloric acid/MTHP (8.2 mL) were added in sequence to the obtained compound. The mixture was stirred at room temperature and then concentrated under reduced pressure. The residue was suspended in and washed with methyl tert-butyl ether and dried in vacuum to obtain 2,2,2-trifluoroethyl (S)-2-amino-3-(4-(5-((3aS,4S,6aR)-2-oxohexahydro-1H-thieno[3,4-d]imidazol-4-yl)pentanamido)phenyl)propanoate (0.501 g, 0.954 mmol) as a colorless solid.

### Example 1-14: (2,6-Dichloropyridin-4-yl)methyl (S)-2-(methylamino)-5-ureidopentanoate hydrochloride (Compound No. 63)

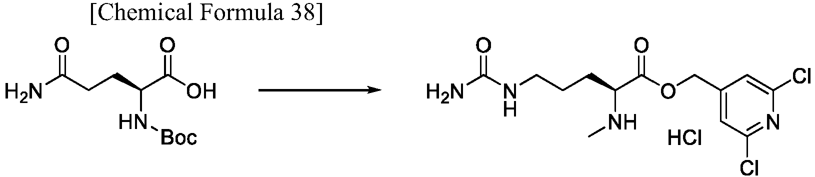

To a flask equipped with a nitrogen balloon were added in sequence N-(tertbutoxycarbonyl)-L-glutamine (30.0 g, 122 mmol, CAS Registry Number: 13726-85-7), pyridine (210 mL) and dicyclohexylcarbodiimide (22.6 g, 110 mmol, CAS Registry Number: 538-75-0) under ice cooling. The mixture was stirred at room temperature for 3 hours, and then filtered and extracted with dichloromethane. The organic layer was washed in sequence with hydrochloric acid and saturated brine, dried over anhydrous sodium sulfate, filtered and concentrated under reduced pressure. The residue was purified by flash column chromatography (silica gel; ethyl acetate/petroleum ether = 1/1) to obtain (S)-2-((tertbutoxycarbonyl)amino)-4-cyanobutanoic acid (23.0 g). THF (150 mL) and 60% sodium hydride (8.00 g, 200 mmol, CAS Registry Number: 7646-69-7) were added to the obtained compound at 10°C. After stirring the mixture for 30 minutes, iodomethane (110 g, 775 mmol, CAS Registry Number: 74-88-4) was added to this mixture. After stirring the mixture at room temperature overnight, water was added thereto to quench the reaction. The mixture was extracted with ethyl acetate, and the organic layer was washed in sequence with citric acid and saturated brine, dried over anhydrous sodium sulfate, filtered and concentrated under reduced pressure to obtain (S)-2-((tert-butoxycarbonyl)(methyl)amino)-4-cyanobutanoic acid (13.0 g). Isopropanol (100 mL), concentrated hydrochloric acid (3 mL) and platinum dioxide (1.00 g, 4.48 mmol, CAS Registry Number: 1314-15-4) were added in sequence to the obtained compound. The mixture was stirred at room temperature under a hydrogen atmosphere for 6 hours, and then filtered and concentrated under reduced pressure to obtain (S)-5-amino-2-((tert-butoxycarbonyl)(methyl)amino)pentanoic acid hydrochloride (13.0 g). THF (200 mL), water (130 mL) and potassium cyanate (12.9 g, 159 mmol, CAS Registry Number: 590-28-3) were added in sequence to the obtained compound. After stirring the mixture at room temperature overnight, hydrochloric acid was added thereto to quench the reaction. The mixture was extracted with ethyl acetate, and the organic layer was washed with saturated brine, dried over anhydrous sodium sulfate, filtered and concentrated under reduced pressure. The residue was purified by flash column chromatography (silica gel; acetonitrile/0.1% aqueous formic acid solution = 10/90 → 50/50) to obtain (S)-2-((tert-butoxycarbonyl)(methyl)amino)-5-ureidopentanoic acid (5.03 g). DMF (50 mL), 4-(bromomethyl)-2,6-dichloropyridine (2.77 g, 11.5 mmol, CAS Registry Number: 175204-45-2) and DIPEA (2.54 mL, 14.5 mmol) were added in sequence to the obtained compound (3.50 g). The mixture was stirred at room temperature for 16 hours, and then purified by flash column chromatography (silica gel; methanol/dichloromethane = 1/99 → 20/80) to obtain (2,6-dichloropyridin-4-yl)methyl (S)-2-((tertbutoxycarbonyl)(methyl)amino)-5-ureidopentanoate (4.00 g). Dichloromethane (25 mL) and 4 M-hydrochloric acid/CPME (25 mL) were added in sequence to the obtained compound (3.00 g). The mixture was stirred at room temperature for 2 hours and then concentrated under reduced pressure. The residue was suspended in and washed with diethyl ether to obtain (2,6-dichloropyridin-4-yl)methyl (S)-2-(methylamino)-5-ureidopentanoate (2.17 g, 5.63 mmol) as a colorless solid.

### Example 1-15: (S)-2-(4-(2-Amino-3-((2,6-dichloropyridin-4-yl)methoxy)-3-oxopropyl)piperidin-1-yl)acetic acid dihydrochloride (Compound No. 64)

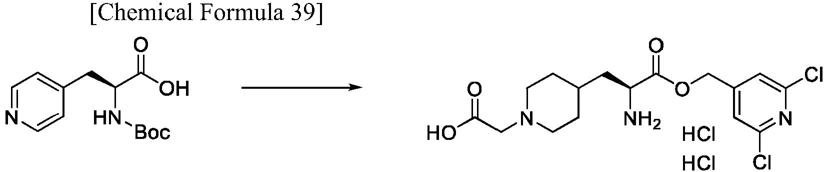

To a flask were added in sequence (S)-2-((tert-butoxycarbonyl)amino)-3-(pyridin 4-yl)propanoic acid (45.0 g, 169 mmol, CAS Registry Number: 37535-57-2), isopropanol (675 mL), a 1 M aqueous hydrochloric acid solution (169 mL) and platinum dioxide (6.52 g, 28.7 mmol, CAS Registry Number: 1314-15-4). After stirring at room temperature under a hydrogen atmosphere for 16 hours, this mixture was filtered and concentrated under reduced pressure to obtain (S)-2-((tert-butoxycarbonyl)amino)-3-(piperidin-4-yl)propanoic acid hydrochloride (50.0 g). THF (1000 mL) and bis(trimethylsilyl)acetamide (115 g, 567 mmol, CAS Registry Number: 10416-59-8) were added in sequence to the obtained compound under ice cooling. After stirring under ice cooling for 1 hour, DIPEA (62.8 mL, 243 mmol) and tert-butyl bromoacetate (47.4 g, 243 mmol, CAS Registry Number: 5292-43-3) were added in sequence to this mixture under ice cooling. After stirring under ice cooling for 1 hour, this mixture was stirred at room temperature for another 1 hour and concentrated under reduced pressure. The residue was purified by flash column chromatography (silica gel; methanol/dichloromethane = 1/100 → 1/1) to obtain (S)-3-(1-(2-(tert-butoxy)-2-oxoethyl)piperidin-4-yl)-2-((tert-butoxycarbonyl)amino)propanoic acid (55.0 g). DMF (80 mL), 4-(bromomethyl)-2,6-dichloropyridine (4.86 g, 20.2 mmol, CAS Registry Number: 175204-45-2) and DIPEA (4.43 mL, 25.5 mmol) were added in sequence to the obtained compound (8.20 g). After stirring the mixture at room temperature for 2 hours, an aqueous sodium dihydrogen phosphate solution was added thereto to quench the reaction. The mixture was extracted with isopropyl acetate, and the organic layer was washed in sequence with an aqueous sodium hydrogen carbonate solution and saturated brine, dried over anhydrous sodium sulfate, filtered and concentrated under reduced pressure. The residue was purified by flash column chromatography (silica gel; ethyl acetate/petroleum ether = 1/1) to obtain (2,6-dichloropyridin-4-yl)methyl (S)-3-(1-(2-(tert-butoxy)-2-oxoethyl)piperidin-4-yl)-2-((tert-butoxycarbonyl)amino)propanoate (7.06 g). Dichloromethane (15 mL) and 4 M-hydrochloric acid/MTHP (15 mL) were added in sequence to the obtained compound (1.60 g). The mixture was stirred at room temperature for 2 hours and then concentrated under reduced pressure. The residue was suspended in and washed with diethyl ether and dried in vacuum to obtain (S)-2-(4-(2-amino-3-((2,6-dichloropyridin-4-yl)methoxy)-3-oxopropyl)piperidin-1-yl)acetic acid dihydrochloride (1.49 g, 3.22 mmol) as a colorless solid.

### Example 1-16: (2,6-Dichloropyridin-4-yl)methyl (S)-2-amino-3-(1-carbamoylpiperidin-4-yl)propanoate hydrochloride (Compound No. 65)

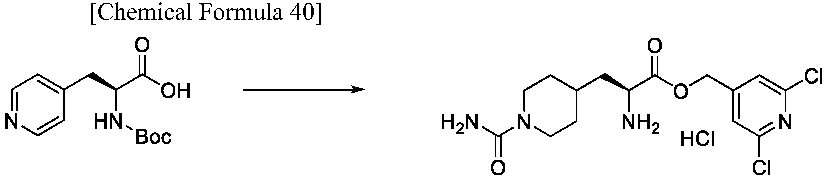

THF (600 mL), water (400 mL) and potassium cyanate (36.0 g, 444 mmol, CAS Registry Number: 590-28-3) were added in sequence to (S)-2-((tert-butoxycarbonyl)amino)-3-(piperidin-4-yl)propanoic acid hydrochloride (80.0 g, 259 mmol) obtained from Example 1-15. After stirring the mixture at room temperature for 16 hours, an aqueous sodium dihydrogen phosphate solution was added thereto to quench the reaction. The mixture was extracted with ethyl acetate, and the organic layer was washed with saturated brine, dried over anhydrous sodium sulfate, filtered and concentrated under reduced pressure to obtain (S)-2-((tert-butoxycarbonyl)amino)-3-(1-carbamoylpiperidin-4-yl)propanoic acid (80.0 g). DMF (150 mL), 4-(bromomethyl)-2,6-dichloropyridine (10.9 g, 45.2 mmol, CAS Registry Number: 175204-45-2) and DIPEA (9.94 mL, 57.1 mmol) were added in sequence to the obtained compound (15.0 g). After stirring the mixture at room temperature for 1 hour, water was added thereto to quench the reaction. The mixture was extracted with ethyl acetate, washed with saturated brine, dried over anhydrous sodium sulfate, filtered and concentrated under reduced pressure. The residue was purified by flash column chromatography (silica gel; methanol/dichloromethane = 1/10) to obtain (2,6-dichloropyridin-4-yl)methyl (S)-2-((tert-butoxycarbonyl)amino)-3-(1-carbamoylpiperidin-4-yl)propanoate (10.7 g). Dichloromethane (25 mL) and 4 M-hydrochloric acid/MTHP (25 mL) were added in sequence to the obtained compound (3.00 g). The mixture was stirred at room temperature for 30 minutes and then concentrated under reduced pressure. The residue was suspended in and washed with diethyl ether and dried in vacuum to obtain (2,6-dichloropyridin-4-yl)methyl (S)-2-amino-3-(1-carbamoylpiperidin-4-yl)propanoate hydrochloride (2.90 g, 6.48 mmol) as a colorless solid.

### Example 1-17: (S)-2-(4-((5-Amino-6-((2,6-dichloropyridin-4-yl)methoxy)-6-oxohexyl)carbamoyl)piperazin-1-yl)acetic acid dihydrochloride (Compound No. 66)

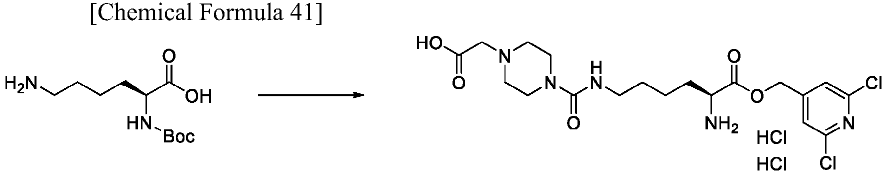

To a flask were added in sequence (tert-butoxycarbonyl)-L-lysine (10.0 g, 40.6 mmol, CAS Registry Number: 13734-28-6), THF (66 mL) and bis(trimethylsilyl)acetamide (18.2 g, 89.3 mmol, CAS Registry Number: 10416-59-8) under ice cooling. After stirring the mixture at room temperature for 30 minutes, DIPEA (8.49 mL, 48.7 mmol) and 4-nitrophenyl chloroformate (7.77 g, 38.6 mmol, CAS Registry Number: 7693-46-1) dissolved in THF (17 mL) were added in sequence to this mixture under ice cooling. After stirring the mixture at 10°C or less for 1.5 hours, hydrochloric acid was added thereto to quench the reaction. The mixture was extracted with ethyl acetate, and the organic layer was washed with water and saturated brine, dried over anhydrous sodium sulfate, filtered and concentrated under reduced pressure. The residue was purified by flash column chromatography (silica gel; ethyl acetate/petroleum ether = 1/1) to obtain N²-(tert-butoxycarbonyl)-N⁶-((4-nitrophenoxy)carbonyl)-L-lysine (10.6 g). THF (90 mL), 1,1-dimethylethyl 1-piperazineacetate (5.32 g, 26.5 mmol) dissolved in DMF (10 mL), and DIPEA (4.84 mL, 27.8 mmol) were added in sequence to the obtained compound (10.4 g) under ice cooling. After stirring the mixture at room temperature for 4 hours, an aqueous sodium dihydrogen phosphate solution was added thereto to quench the reaction. The mixture was extracted with ethyl acetate, and the organic layer was washed with saturated brine, dried over anhydrous sodium sulfate, filtered and concentrated under reduced pressure. The residue was purified by flash column chromatography (silica gel; methanol/dichloromethane = 8/92) to obtain N⁶-(4-(2-(tert-butoxy)-2-oxoethyl)piperazine-1-carbonyl)-N²-(tert-butoxycarbonyl)-L-lysine (8.00 g). DMF (80 mL), 4-(bromomethyl)-2,6-dichloropyridine (3.87 g, 16.1 mmol, CAS Registry Number: 175204-45-2) and DIPEA (2.63 g, 20.3 mmol) were added in sequence to the obtained compound. After stirring the mixture at room temperature for 1 hour, water was added thereto to quench the reaction. The mixture was extracted with ethyl acetate, and the organic layer was washed with saturated brine, dried over anhydrous sodium sulfate, filtered and concentrated under reduced pressure. The residue was purified by flash column chromatography (silica gel; methanol/dichloromethane = 4/96) to obtain (2,6-dichloropyridin-4-yl)methyl N⁶-(4-(2-(tert-butoxy)-2-oxoethyl)piperazine-1-carbonyl)-N²-(tert-butoxycarbonyl)-L-lysinate (8.24 g). Dichloromethane (10 mL) and 2,2,2-trifluoroacetic acid (12.5 mL) were added in sequence to the obtained compound (5.07 g). The mixture was stirred at room temperature for 20 hours and then concentrated under reduced pressure, and acetonitrile and 4 M-hydrochloric acid/MTHP (25 mL) were added thereto in sequence. The mixture was filtered, washed with THF and dried in vacuum to obtain (S)-2-(4-((5-amino-6-((2,6-dichloropyridin-4-yl)methoxy)-6-oxohexyl)carbamoyl)piperazin-1-yl)acetic acid dihydrochloride (4.10 g, 8.01 mmol) as a colorless solid.

### Example 1-18:

### (2,6-Dichloropyridin-4-yl)methyl (S)-2-amino-4-(benzylsulfonyl)butanoate hydrochloride (Compound No. 67)

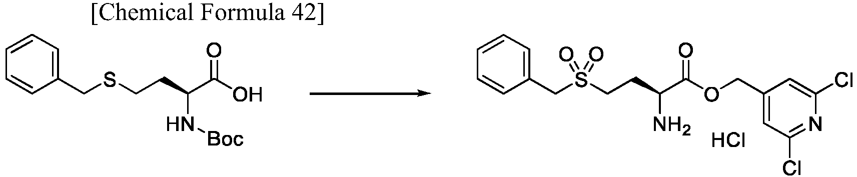

To a flask were added in sequence S-benzyl-N-(tert-butoxycarbonyl)-L-homocysteine (0.325 g, 1.00 mmol, CAS Registry Number: 16947-99-2), methanol (2.0 mL) and magnesium bis(monoperoxyphthalate) hexahydrate (0.594 g, 1.20 mmol, CAS Registry Number: 84665-66-7) under ice cooling. After stirring the mixture at room temperature, hydrochloric acid was added thereto to quench the reaction. The mixture was extracted with ethyl acetate, and the organic layer was washed with saturated brine, dried over anhydrous sodium sulfate, filtered and concentrated under reduced pressure to obtain (S)-4-(benzylsulfonyl)-2-((tert-butoxycarbonyl)amino)butanoic acid (0.357 g). DMF (2.0 mL), 4-(bromomethyl)-2,6-dichloropyridine (0.229 g, 0.95 mmol, CAS Registry Number: 175204-45-2) and DIPEA (0.210 mL, 1.20 mmol) were added in sequence to the obtained compound. After stirring the mixture at room temperature, hydrochloric acid was added thereto to quench the reaction. The mixture was extracted with ethyl acetate, and the organic layer was washed with saturated brine, dried over anhydrous sodium sulfate, filtered and concentrated under reduced pressure. The residue was purified by flash column chromatography (silica gel; ethyl acetate/hexane = 0/100 → 100/0) to obtain (2,6-dichloropyridin-4-yl)methyl (S)-4-(benzylsulfonyl)-2-((tert-butoxycarbonyl)amino)butanoate (0.474 g). Dichloromethane (4.6 mL) and 4 M-hydrochloric acid/MTHP (4.6 mL) were added in sequence to the obtained compound. The mixture was stirred at room temperature and then concentrated under reduced pressure. The residue was suspended in and washed with methyl tert-butyl ether and dried in vacuum to obtain (2,6-dichloropyridin-4-yl)methyl (S)-2-amino-4-(benzylsulfonyl)butanoate hydrochloride (0.384 g, 0.845 mmol) as a colorless solid.

### Example 1-19: (2,6-Dichloropyridin-4-yl)methyl (S)-2-amino-3-(2-carbamoyl-1H-imidazol-4-yl)propanoate dihydrochloride (Compound No. 68)

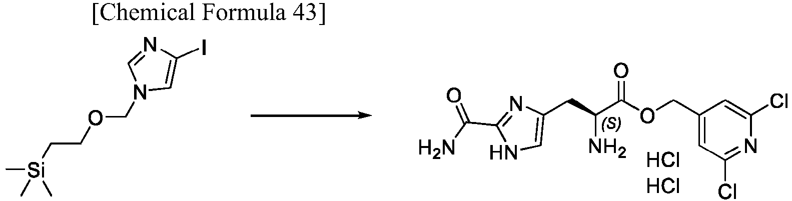

To a flask equipped with a nitrogen balloon were added in sequence 4-iodo-1-((2-(trimethylsilyl)ethoxy)methyl)-1H-imidazole (80.0 g, 247 mmol, CAS Registry Number: 518329-44-7) and THF (1400 mL). After stirring the mixture at room temperature for 30 minutes, 2 M-LDA/THF (148 mL, 296 mmol, CAS Registry Number: 4111-54-0) was added to this mixture at -78°C. This mixture was stirred at -78°C for 30 minutes, and then added to a mixture of ethyl chloroformate (93.7 g, 864 mmol, CAS Registry Number: 541-41-3) and THF (500 mL) at -78°C. After stirring at -78°C for 30 minutes, water was added thereto to quench the reaction. The mixture was extracted with ethyl acetate, and the organic layer was washed with saturated brine, dried over anhydrous sodium sulfate, filtered and concentrated under reduced pressure. The residue was purified by flash column chromatography (silica gel; ethyl acetate/petroleum ether = 20/80 → 50/50) to obtain ethyl 4-iodo-1-((2-(trimethylsilyl)ethoxy)methyl)-1H-imidazole-2-carboxylate (80.0 g). 4 M-NH₃/MeOH (1600 mL, CAS Registry Number: 7664-41-7) was added to the obtained compound (80.0 g). This mixture was stirred at 60°C for 2.5 hours and then concentrated under reduced pressure. The residue was purified by flash column chromatography (silica gel; ethyl acetate/petroleum ether = 20/80 → 50/50) to obtain 4-iodo-1-((2-(trimethylsilyl)ethoxy)methyl)-1H-imidazole-2-carboxamide (52.0 g). To a flask equipped with a nitrogen balloon were added in sequence zinc (23.5 g, 359 mmol, CAS Registry Number: 7440-66-6), DMF (880 mL) and iodine (9.12 g, 35.9 mmol, CAS Registry Number: 7553-56-2). After stirring at 60°C for 30 minutes, (S)-methyl 2-(tert-butoxycarbonylamino)-3-iodopropanoate (78.9 g, 240 mmol, CAS Registry Number: 93267-04-0) was added to this mixture and stirred at room temperature for 1.5 hours to prepare an organozinc reagent. 2-Dicyclohexylphosphino-2',6'-dimethoxybiphenyl (4.92 g, 12.0 mmol, CAS Registry Number: 657408-07-6), tris(dibenzylideneacetone)dipalladium-chloroform (2.74 g, 3.00 mmol, CAS Registry Number: 52522-40-4) and DMF (40 mL) were added in sequence to a flask equipped with a nitrogen balloon, and stirred at 60°C for 30 minutes to prepare a palladium complex solution.

The prepared palladium complex solution and 4-iodo-1-((2-(trimethylsilyl)ethoxy)methyl)-1H-imidazole-2-carboxamide (44.0 g) dissolved in DMF (160 mL) were added in sequence to the prepared organozinc reagent at room temperature. After stirring at 60°C for 1 hour, this mixture was filtered and extracted with ethyl acetate. The organic layer was washed with saturated brine, dried over anhydrous sodium sulfate, filtered and concentrated under reduced pressure. The residue was purified by flash column chromatography (silica gel; ethyl acetate/petroleum ether = 20/80 → 40/60) to obtain methyl (S)-2-((tert-butoxycarbonyl)amino)-3-(2-carbamoyl-1-((2-(trimethylsilyl)ethoxy)methyl)-1H-imidazol-4-yl)propanoate (30.0 g). Isopropanol (900 mL), water (300 mL), calcium chloride (120 g, 1080 mmol, CAS Registry Number: 10043-52-4) and lithium hydroxide monohydrate (11.4 g, 271 mmol, CAS Registry Number: 1310-66-3) were added in sequence to the obtained compound under ice cooling. After stirring the mixture at room temperature for 2 hours, sodium dihydrogen phosphate was added thereto to quench the reaction. This mixture was filtered and extracted with ethyl acetate, and the organic layer was washed with saturated brine, dried over anhydrous sodium sulfate, filtered and concentrated under reduced pressure. The residue was purified by flash column chromatography (silica gel; ethyl acetate/petroleum ether = 40/60 → 60/40) to obtain (S)-2-((tert-butoxycarbonyl)amino)-3-(2-carbamoyl-1-((2-(trimethylsilyl)ethoxy)methyl)-1H-imidazol-4-yl)propanoic acid (20.0 g). DMF (400 mL), 4-(bromomethyl)-2,6-dichloropyridine (10.7 g, 44.3 mmol, CAS Registry Number: 175204-45-2) and DIPEA (7.24 g, 56.0 mmol) were added in sequence to the obtained compound (20.0 g). After stirring the mixture at room temperature for 2 hours, water was added thereto to quench the reaction. The mixture was extracted with ethyl acetate, and the organic layer was washed with saturated brine, dried over anhydrous sodium sulfate, filtered and concentrated under reduced pressure. The residue was purified by flash column chromatography (silica gel; acetonitrile/0.1% aqueous formic acid solution = 40/60 → 60/40) to obtain (2,6-dichloropyridin-4-yl)methyl (S)-2-((tert-butoxycarbonyl)amino)-3-(2-carbamoyl-1-((2-(trimethylsilyl)methoxy))-1H-imidazol-4-yl)propanoate (10.7 g). Dichloromethane (32 mL) and 4 M-hydrochloric acid/MTHP (32 mL) were added in sequence to the obtained compound (5.01 g). The mixture was stirred at room temperature for 3 days and then concentrated under reduced pressure. The residue was suspended in and washed with diethyl ether and dried in vacuum. The residue was purified by flash column chromatography (silica gel; acetonitrile/water = 2/98 → 15/85), water and acetonitrile were added thereto, and the mixture was freeze-dried to obtain (2,6-dichloropyridin-4-yl)methyl (S)-2-amino-3-(6-aminopyridin-3-yl)propanoate dihydrochloride (0.368 g, 0.854 mmol) as a colorless solid.

### Example 1-20: (2,6-Dichloropyridin-4-yl)methyl N⁵-(4-aminobutyl)-L-glutamate dihydrochloride (Compound No. 69)

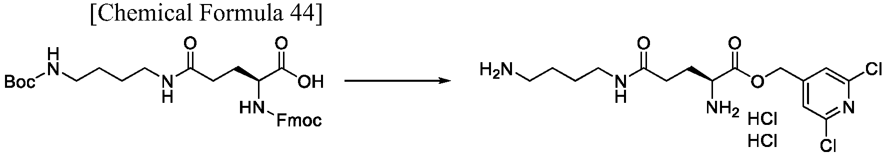

To a flask were added in sequence N²-(((9H-fluoren-9-yl)methoxy)carbonyl)-N⁵-(4-((tert-butoxycarbonyl)amino)butyl)-L-glutamine (2.70 g, 5.00 mmol, purchased from Amatek Chemical Co., Ltd. (AS02649)), acetonitrile (10 mL) and triethylamine (2.53 g, 25.0 mmol) at room temperature. This mixture was stirred at 60°C for 1 hour and then concentrated under reduced pressure. Diethyl ether was added to the residue, and the mixture was filtered, washed with diethyl ether and dried in vacuum to obtain N⁵-(4-((tert-butoxycarbonyl)amino)butyl)-L-glutamine. 1,4-Dioxane (15 mL), water (10 mL), sodium carbonate (1.59 g, 15.0 mmol) and di-tert-butyl dicarbonate (1.31 g, 6.00 mmol, CAS Registry Number: 24424-99-5) were added in sequence to the obtained compound at room temperature. The mixture was stirred at room temperature and then concentrated under reduced pressure to obtain N²-(tert-butoxycarbonyl)-N⁵-(4-((tert-butoxycarbonyl)amino)butyl)-L-glutamine. DMF (10 mL), DIPEA (0.775 g, 6.00 mmol) and 4-(bromomethyl)-2,6-dichloropyridine (1.14 g, 4.75 mmol, CAS Registry Number: 175204-45-2) were added in sequence to the obtained compound. After stirring the mixture at room temperature, water was added to this mixture to quench the reaction. The mixture was extracted with isopropyl acetate, and the organic layer was washed in sequence with water and saturated brine, dried over anhydrous sodium sulfate, filtered and concentrated under reduced pressure. The residue was purified by flash column chromatography (silica gel; ethyl acetate/heptane = 0/100 → 75/25) to obtain (2,6-dichloropyridin-4-yl)methyl N²-(tert-butoxycarbonyl)-N⁵-(4-((tert-butoxycarbonyl)amino)butyl)-L-glutamate. Dichloromethane (25 mL) and 4 M-hydrochloric acid/MTHP (25 mL) were added in sequence to the obtained compound. The mixture was stirred at room temperature and then concentrated under reduced pressure. The residue was suspended in and washed with diethyl ether to obtain (2,6-dichloropyridin-4-yl)methyl N⁵-(4-aminobutyl)-L-glutamate dihydrochloride (1.12 g, 2.49 mmol) as a colorless solid.

### Example 1-21: (2,6-Dichloropyridin-4-yl)methyl O-isobutyl-L-homoserinate hydrochloride (Compound No. 70)

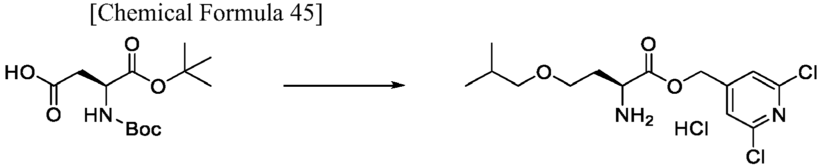

To a flask were added in sequence (S)-4-(tert-butoxy)-3-((tert-butoxycarbonyl)amino)-4-oxobutanoic acid (50.0 g, 173 mmol, CAS Registry Number: 34582-32-6), THF (500 mL), 4-methylmorpholine (22.7 g, 224 mmol, CAS Registry Number; 109-02-4) and isopropyl chloroformate (23.1 g, 188 mmol, CAS Registry Number: 108-23-6) at -10°C. After stirring for 30 minutes at -10°C, sodium borohydride (13.1 g, 346 mmol, CAS Registry Number: 16940-66-2) was added to this mixture at -10°C. After stirring at -10°C for 30 minutes, sodium borohydride (13.1 g, 346 mmol) dissolved in water (150 mL) was added thereto at -10°C. After stirring at -10°C for 2 hours, an aqueous ammonium chloride solution was added to this mixture to quench the reaction. The mixture was extracted with ethyl acetate, washed in sequence with an aqueous ammonium chloride solution and saturated brine, dried over anhydrous sodium sulfate, filtered and concentrated under reduced pressure to obtain tert-butyl (tert-butoxycarbonyl)-L-homoserinate (45.0 g). tert-Butyl (tert-butoxycarbonyl)-L-homoserinate (50.0 g) dissolved in acetonitrile (150 mL) was added to thionyl chloride (56.2 g, 472 mmol, CAS Registry Number: 7719-09-7) dissolved in acetonitrile (850 mL) at -40°C. After stirring at -40°C for 15 minutes, pyridine (86.2 g, 1090 mmol) was added to this mixture at -40°C. After stirring at 0°C for 20 minutes, water was added to this mixture to quench the reaction. The mixture was extracted with dichloromethane, washed with a 1 M aqueous hydrochloric acid solution and saturated brine, dried over anhydrous sodium sulfate, filtered and concentrated under reduced pressure. The residue was purified by flash column chromatography (silica gel; ethyl acetate/petroleum ether = 1/10) to obtain di-tert-butyl (4S)-1,2,3-oxathiazinane-3,4-dicarboxylate 2-oxide (30.0 g). Dimethyl carbonate (300 mL) and ruthenium trichloride hydrate (0.21 g, 0.933 mmol, CAS Number: 14898-67-0) dissolved in water, and sodium periodate (59.9 g, 280 mmol, CAS Number: 14898-67-0) were added in sequence to the obtained compound under ice cooling. The mixture was stirred at room temperature for 3 hours, and then filtered, extracted with ethyl acetate, washed with saturated brine, dried over anhydrous sodium sulfate, filtered and concentrated under reduced pressure. The residue was purified by flash column chromatography (silica gel; ethyl acetate/petroleum ether = 1/10) to obtain di-tert-butyl (S)-1,2,3-oxathiazinane-3,4-dicarboxylate 2,2-dioxide (25.0 g). Sodium dihydrogen phosphate (20.6 g, 172 mmol, CAS Registration Number; 7558-79-4) and 2-methylpropan-1-ol (79.6 g, 1070 mmol, CAS Registration Number; 78-83-1) were added to the obtained compound (14.5 g) at room temperature. This mixture was stirred at 55°C for 3 hours and then concentrated under reduced pressure. The mixture was dissolved in ethyl acetate, washed with water and saturated brine, dried over anhydrous sodium sulfate, filtered and concentrated under reduced pressure to obtain tert-butyl N-(tert-butoxycarbonyl)-O-isobutyl-L-homoserinate (13.6 g). 1,4-Dioxane (35 mL) and a 4 M aqueous hydrochloric acid solution (105 mL) were added in sequence to the obtained compound at room temperature. This mixture was stirred at 85°C for 3 hours and then concentrated under reduced pressure to obtain O-isobutyl-L-homoserine hydrochloride (8.60 g). 1,4-Dioxane (90 mL), water (60 mL), sodium hydrogen carbonate (13.6 g, 162 mmol, CAS Registry Number: 497-19-8) and di-tert-butyl dicarbonate (10.6 g, 48.6 mmol, CAS Registry Number: 24424-99-5) were added in sequence to the obtained compound under ice cooling. After stirring the mixture at room temperature for 5 hours, a 1 M aqueous hydrochloric acid solution was added thereto to quench the reaction. The mixture was extracted with ethyl acetate, and the organic layer was washed in sequence with saturated brine, dried over anhydrous sodium sulfate, filtered and concentrated under reduced pressure. The residue was purified by flash column chromatography (silica gel; methanol/dichloromethane = 1/10) to obtain N-(tert-butoxycarbonyl)-O-isobutyl-L-homoserine (10.4 g). DMF (100 mL), DIPEA (5.86 g, 45.3 mmol) and 4-(bromomethyl)-2,6-dichloropyridine (8.64 g, 35.9 mmol, CAS Registry Number: 175204-45-2) were added in sequence to the obtained compound. After stirring the mixture at room temperature for 1 hour, an aqueous sodium dihydrogen phosphate solution was added to this mixture to quench the reaction. The mixture was extracted with ethyl acetate, and the organic layer was washed in sequence with an aqueous sodium hydrogen carbonate solution and saturated brine, dried over anhydrous sodium sulfate, filtered and concentrated under reduced pressure. The residue was purified by flash column chromatography (silica gel; ethyl acetate/petroleum ether = 1/5) to obtain (2,6-dichloropyridin-4-yl)methyl N-(tert-butoxycarbonyl)-O-isobutyl-L-homoserinate (9.95 g). Dichloromethane (42 mL) and 4 M-hydrochloric acid/MTHP (43 mL) were added in sequence to the obtained compound (4.98 g). The mixture was stirred at room temperature for 17 hours and then concentrated under reduced pressure. The residue was suspended in and washed with diethyl ether to obtain (2,6-dichloropyridin-4-yl)methyl O-isobutyl-L-homoserinate hydrochloride (3.11 g, 8.37 mmol) as a colorless solid.

### Example 1-22: (2,6-Dichloropyridin-4-yl)methyl N²,N⁵ ,N⁵-trimethyl-L-glutamate hydrochloride (Compound No. 71)

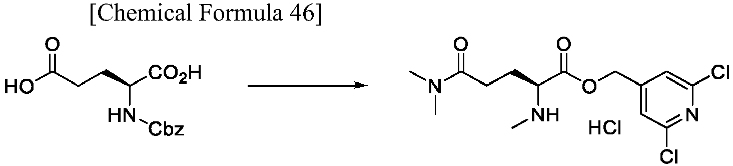

To a flask were added in sequence ((benzyloxy)carbonyl)-L-glutamic acid (50.0 g, 178 mmol, CAS Registry Number: 1155-62-0), paraformaldehyde (16.0 g, 533 mmol, CAS Registry Number: 30525-89-4), 10-camphorsulfonic acid (8.25 g, 35.6 mmol, CAS Registry Number: 3144-16-9) and toluene (800 mL). After stirring at 80°C overnight, ethyl acetate was added to this mixture. The organic layer was washed with saturated brine, dried over anhydrous sodium sulfate, filtered and concentrated under reduced pressure. The residue was purified by flash column chromatography (silica gel; methanol/dichloromethane = 1/10) to obtain (S)-3-(3-((benzyloxy)carbonyl)-5-oxooxazolidin-4-yl)propanoic acid (50.0 g). DMF (300 mL), dimethylamine hydrochloride (8.34 g, 102 mmol, CAS Registry Number: 506-59-2), 1-[bis(dimethylamino)methylene]-1H-1,2,3-triazolo[4,5-b]pyridinium 3-oxide hexafluorophosphate (51.9 g, 136 mmol, CAS Registry Number: 148893-10-1) and DIPEA (35.3 g, 273 mmol) were added in sequence to the obtained compound (20.0 g) at room temperature. After stirring the mixture at room temperature overnight, ethyl acetate was added to this mixture. The organic layer was washed with saturated brine, dried over anhydrous sodium sulfate, filtered and concentrated under reduced pressure. The residue was purified by flash column chromatography (silica gel; ethyl acetate/petroleum ether = 1/1) to obtain benzyl (S)-4-(3-(dimethylamino)-3-oxopropyl)-5-oxooxazolidine-3-carboxylate (20.0 g). Dichloromethane (30 mL), triisopropylsilane (10.5 g, 66.5 mmol, CAS Registry Number: 6485-79-6) and 2,2,2-trifluoroacetic acid (30.0 mL) were added in sequence to the obtained compound (6.50 g) at room temperature. The mixture was stirred at room temperature overnight and then concentrated under reduced pressure to obtain N²-((benzyloxy)carbonyl)-N²,N⁵ ,N⁵-trimethyl-L-glutamine. Methanol (170 mL) and palladium/carbon (1.77 g, CAS Registry Number: 7440-05-3) were added in sequence to the obtained compound. After stirring at room temperature under a hydrogen atmosphere overnight, this mixture was filtered, extracted with methanol and concentrated under reduced pressure to obtain N²,N⁵,N⁵-trimethyl-L-glutamine. 1,4-Dioxane (100 mL) and water (100 mL) were added to the obtained compound. After stirring the mixture at room temperature, sodium carbonate (21.4 g, 202 mmol, CAS Registry Number: 497-19-8) and di-tert-butyl dicarbonate (33.1 g, 151 mmol, CAS Registry Number: 24424-99-5) were added thereto in sequence under ice cooling. After stirring the mixture at room temperature overnight, hydrochloric acid was added thereto to quench the reaction. The mixture was extracted with ethyl acetate, and the organic layer was washed with saturated brine, dried over anhydrous sodium sulfate, filtered and concentrated under reduced pressure to obtain N²-(tert-butoxycarbonyl)-N²,N⁵,N⁵-trimethyl-L-glutamine. DMF (100 mL), 4-(bromomethyl)-2,6-dichloropyridine (7.62 g, 31.6 mmol, CAS Registry Number: 175204-45-2) and DIPEA (5.16 g, 40.0 mmol) were added in sequence to the obtained compound. After stirring the mixture at room temperature for 1 hour, water was added thereto to quench the reaction. The mixture was extracted with ethyl acetate, and the organic layer was washed with saturated brine, dried over anhydrous sodium sulfate, filtered and concentrated under reduced pressure. The residue was purified by flash column chromatography (silica gel; ethyl acetate/petroleum ether = 1/1) to obtain (2,6-dichloropyridin-4-yl)methyl N²-(tert-butoxycarbonyl)-N²,N⁵,N⁵-trimethyl-L-glutamate (7.01 g). Dichloromethane (25 mL) and 4 M-hydrochloric acid/MTHP (25 mL) were added in sequence to the obtained compound (4.48 g). The mixture was stirred at room temperature for 17 hours and then concentrated under reduced pressure. The residue was suspended in and washed with methyl tert-butyl ether and dried in vacuum to obtain (2,6-dichloropyridin-4-yl)methyl N²,N⁵,N⁵-trimethyl-L-glutamate hydrochloride (3.54 g, 9.21 mmol) as a colorless solid.

### Example 1-23: (2,6-Dichloropyridin-4-yl)methyl (S)-3-(5-hydroxypyridin-3-yl)-2-(methylamino)propanoate dihydrochloride (Compound No. 72)

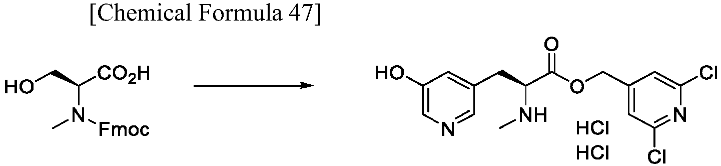

To a flask equipped with a nitrogen balloon were added in sequence N-(((9H-fluoren-9-yl)methoxy)carbonyl)-N-methyl-L-serine (150 g, 439 mmol, CAS Registry Number: 291311-48-3), methanol (1500 mL), and concentrated sulfuric acid (50 mL). The mixture was stirred at 60°C and then concentrated under reduced pressure. Ethyl acetate was added to this mixture, and the organic layer was washed with saturated brine, dried over anhydrous sodium sulfate, filtered and concentrated under reduced pressure to obtain methyl N-(((9H-fluoren-9-yl)methoxy)carbonyl)-N-methyl-L-serinate (120 g). Dichloromethane (500 mL), triphenylphosphine (78.6 g, 300 mmol, CAS Registry Number: 603-35-0), imidazole (20.4 g, 300 mmol, CAS Registry Number: 288-32-4) and iodine (76.1 g, 300 mmol, CAS Registry Number: 7553-56-2) were added in sequence to the obtained compound (71.0 g) under ice cooling. After stirring the mixture at room temperature for 30 minutes, dichloromethane was added to this mixture. The organic layer was washed with saturated brine, dried over anhydrous sodium sulfate, filtered and concentrated under reduced pressure. The residue was purified by flash column chromatography (silica gel; ethyl acetate/petroleum ether = 1/1) to obtain methyl (R)-2-((((9H-fluoren-9-yl)methoxy)carbonyl)(methyl)amino)-3-iodopropanoate (70.0 g). To a flask equipped with a nitrogen balloon were added in sequence zinc (19.0 g, 290 mmol, CAS Registry Number: 7440-66-6), DMF (400 mL) and iodine (14.7 g, 58.0 mmol, CAS Registry Number: 7553-56-2). After stirring the mixture at room temperature for 2 minutes, methyl (R)-2-((((9H-fluoren-9-yl)methoxy)carbonyl)(methyl)amino)-3-iodopropanoate (45.0 g) was added to this mixture. After stirring the mixture at room temperature for 30 minutes, 5-bromopyridin-3-ol (20.2 g, 116 mmol, CAS Registry Number: 74115-13-2), 2-dicyclohexylphosphino-2',6'-dimethoxybiphenyl (1.98 g, 4.83 mmol, CAS Registry Number: 657408-07-6) and tris(dibenzylideneacetone)dipalladium-chloroform (2.21 g, 2.42 mmol, CAS Registry Number: 52522-40-4) were added in sequence to this mixture at room temperature. After stirring at 50°C for 3 hours, water was added to this mixture, and the mixture was filtered and extracted with ethyl acetate. The organic layer was washed with saturated brine, dried over anhydrous sodium sulfate, filtered and concentrated under reduced pressure. The residue was purified by flash column chromatography (silica gel; ethyl acetate/petroleum ether = 1/3) to obtain methyl (S)-2-((((9H-fluoren-9-yl)methoxy)carbonyl)(methyl)amino)-3-(5-hydroxypyridin-3-yl)propanoate (18.0 g).

Isopropanol (120 mL), water (40 mL), calcium chloride (41.1 g, 370 mmol, CAS Registry Number: 10043-52-4) and lithium hydroxide monohydrate (3.88 g, 92.5 mmol, CAS Registry Number: 1310-66-3) were added in sequence to the obtained compound (10.0 g) under ice cooling. After stirring the mixture at room temperature overnight, sodium dihydrogen phosphate was added thereto to quench the reaction. This mixture was filtered and extracted with ethyl acetate, and the organic layer was washed with saturated brine, dried over anhydrous sodium sulfate, filtered and concentrated under reduced pressure. The residue was purified by flash column chromatography (silica gel; ethyl acetate/petroleum ether = 1/5) to obtain (S)-2-(((9H-fluoren-9-yl)methoxy)carbonyl)(methyl)amino)-3-(5-hydroxypyridin-3-yl)propanoic acid (8.00 g). THF (20 mL) and diethylamine (3.51 g, 48.0 mmol, CAS Registry Number: 109-89-7) were added in sequence to the obtained compound (6.7 g). The mixture was stirred at room temperature for 1 hour and then concentrated under reduced pressure to obtain (S)-3-(5-hydroxypyridin-3-yl)-2-(methylamino)propanoic acid (3.14 g). THF (30 mL), di-tert-butyl dicarbonate (7.68 g, 35.2 mmol, CAS Registry Number: 24424-99-5) and DIPEA (4.13 g, 31.2 mmol) were added in sequence to the obtained compound under ice cooling. The mixture was stirred at room temperature overnight and then concentrated under reduced pressure to obtain (S)-2-((tert-butoxycarbonyl)(methyl)amino)-3-(5-((tert-butoxycarbonyl)oxy)pyridin-3-yl)propanoic acid (6.00 g). DMF (30 mL), 4-(bromomethyl)-2,6-dichloropyridine (3.45 g, 14.3 mmol, CAS Registry Number: 175204-45-2) and DIPEA (2.35 g, 18.2 mmol) were added in sequence to the obtained compound. After stirring the mixture at room temperature for 1 hour, an aqueous sodium dihydrogen phosphate solution was added thereto to quench the reaction. The mixture was extracted with ethyl acetate, and the organic layer was washed in sequence with an aqueous sodium hydrogen carbonate solution and saturated brine, dried over anhydrous sodium sulfate, filtered and concentrated under reduced pressure. The residue was purified by flash column chromatography (silica gel; ethyl acetate/petroleum ether = 1/4) to obtain (2,6-dichloropyridin-4-yl)methyl (S)-2-((tert-butoxycarbonyl)(methyl)amino)-3-(5-((tert-butoxycarbonyl)oxy)pyridin-3-yl)propanoic acid (4.95 g). Dichloromethane (21.3 mL) and 4 M-hydrochloric acid/MTHP (21.3 mL) were added in sequence to the obtained compound (2.62 g). The mixture was stirred at room temperature for 4 hours and then concentrated under reduced pressure. The residue was suspended in and washed with diethyl ether and dried in vacuum to obtain (2,6-dichloropyridin-4-yl)methyl (S)-3-(5-hydroxypyridin-3-yl)-2-(methylamino)propanoate dihydrochloride (1.99 g, 4.64 mmol) as a light brown solid.

### Example 1-24: (2,6-Dichloropyridin-4-yl)methyl (S)-3-(6-aminopyridin-3-yl)-2-(methylamino)propanoate dihydrochloride (Compound No. 73)

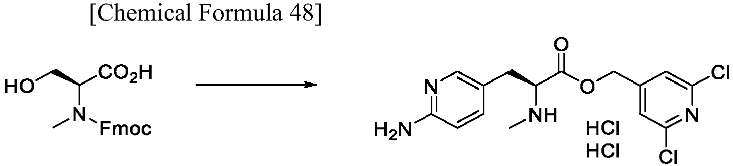

To a flask equipped with a nitrogen balloon were added in sequence zinc (22.4 g, 343 mmol, CAS Registry Number: 7440-66-6), DMF (450 mL) and iodine (17.4 g, 68.6 mmol, CAS Registry Number: 7553-56-2). After stirring the mixture at room temperature for 10 minutes, methyl (R)-2-((((9H-fluoren-9-yl)methoxy)carbonyl)(methyl)amino)-3-iodopropanoate (53.2 g) obtained from Example 1-23 was added to this mixture. After stirring the mixture at room temperature for 1 hour, 5-bromopyridin-2-amine (20.0 g, 116 mmol, CAS Registry Number: 1072-97-5), 2-dicyclohexylphosphino-2',6'-dimethoxybiphenyl (9.40 g, 22.9 mmol, CAS Registry Number: 657408-07-6) and tris(dibenzylideneacetone)dipalladium-chloroform (5.80 g, 5.72 mmol, CAS Registry Number: 52522-40-4) were added in sequence to this mixture at room temperature. After stirring at 50°C for 16 hours, water was added to this mixture, and the mixture was filtered and extracted with ethyl acetate. The organic layer was washed in sequence with water and saturated brine, dried over anhydrous sodium sulfate, filtered and concentrated under reduced pressure. The residue was purified by flash column chromatography (silica gel; ethyl acetate/petroleum ether = 2/1) to obtain methyl ((S)-2-((((9H-fluoren-9-yl)methoxy)carbonyl)(methyl)amino)-3-(6-aminopyridin-3-yl)propanoate (30.0 g). tert-Butanol (600 mL), di-tert-butyl dicarbonate (18.2 g, 83.4 mmol, CAS Registry Number: 24424-99-5) and sodium iodide (12.5 g, 83.4 mmol, CAS Registry Number: 7681-82-5) were added in sequence to the obtained compound. The mixture was stirred at room temperature for 16 hours and then concentrated under reduced pressure. The residue was purified by flash column chromatography (silica gel; ethyl acetate/petroleum ether = 1/4) to obtain methyl (S)-2-(((9H-fluoren-9-yl)methoxy)carbonyl)(methyl)amino)-3-(6-((tert-butoxycarbonyl)amino)pyridin-3-yl)propanoate (20.0 g). Isopropanol (360 mL), calcium chloride (66.8 g, 602 mmol, CAS Registry Number: 10043-52-4), water (120 mL) and lithium hydroxide monohydrate (3.61 g, 151 mmol, CAS Registry Number: 1310-66-3) were added in sequence to the obtained compound under ice cooling. After stirring the mixture at room temperature for 16 hours, hydrochloric acid was added thereto to quench the reaction. This mixture was filtered and extracted with ethyl acetate, and the organic layer was washed with saturated brine, dried over anhydrous sodium sulfate, filtered and concentrated under reduced pressure to obtain (S)-2-(((9H-fluoren-9-yl)methoxy)carbonyl)(methyl)amino)-3-(6-((tert-butoxycarbonyl)amino)pyridin-3-yl)propanoic acid. THF (50 mL) and diethylamine (5.50 g, 75.2 mmol, CAS Registry Number: 109-89-7) were added in sequence to the obtained compound. The mixture was stirred at room temperature for 5 hours and then concentrated under reduced pressure to obtain (S)-3-(6-((tert-butoxycarbonyl)amino)pyridin-3-yl)-2-(methylamino)propanoic acid.

THF, TEA (11.4 g, 75.2 mmol) and di-tert-butyl dicarbonate (9.86 g, 45.2 mmol, CAS Registry Number: 24424-99-5) were added in sequence to the obtained compound. The mixture was stirred at room temperature for 16 hours and then concentrated under reduced pressure to obtain (S)-2-((tert-butoxycarbonyl)(methyl)amino)-3-(6-((tert-butoxycarbonyl)amino)pyridin-3-yl)propanoic acid. DMF (200 mL), 4-(bromomethyl)-2,6-dichloropyridine (10.8 g, 44.9 mmol, CAS Registry Number: 175204-45-2) and DIPEA (7.33 g, 56.7 mmol) were added in sequence to the obtained compound. After stirring the mixture at room temperature for 1 hour, an aqueous sodium dihydrogen phosphate solution was added thereto to quench the reaction. The mixture was extracted with ethyl acetate, and the organic layer was washed in sequence with an aqueous sodium hydrogen carbonate solution and saturated brine, dried over anhydrous sodium sulfate, filtered and concentrated under reduced pressure. The residue was purified by flash column chromatography (silica gel; ethyl acetate/petroleum ether = 1/5) to obtain (2,6-dichloropyridin-4-yl)methyl (S)-2-((tert-butoxycarbonyl)(methyl)amino)-3-(6-((tert-butoxycarbonyl)amino)pyridin-3-yl)propanoate (11.3 g). Dichloromethane (20 mL) and 4 M-hydrochloric acid/MTHP (20 mL) were added in sequence to the obtained compound (4.44 g). The mixture was stirred at room temperature and then concentrated under reduced pressure. The residue was suspended in and washed with heptane, dried in a vacuum, dissolved by adding water and acetonitrile, and then freeze-dried to obtain (2,6-dichloropyridin-4-yl)methyl (S)-3-(6-aminopyridin-3-yl)-2-(methylamino)propanoate dihydrochloride (3.33 g, 7.77 mmol) as a colorless solid.

### Example 1-25: (S)-2-(4-((6-((2,6-Dichloropyridin-4-yl)methyl)-5-(methylamino)-6-oxohexyl)carbamoyl)piperazin-1-yl)acetic acid dihydrochloride (Compound No. 74)

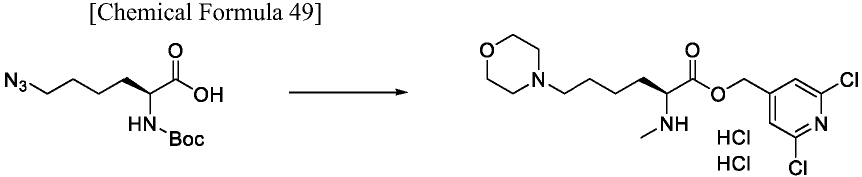

To a flask equipped with a nitrogen balloon were added in sequence N²-(tert-butoxycarbonyl)-N⁶-diazo-L-lysine (50.0 g, 183 mmol, CAS Registry Number: 846549-33-5), THF (500 mL) and 60% sodium hydride (18.4 g, 459 mmol, CAS Registry Number: 7646-69-7) under ice cooling. After stirring under ice cooling for 1 hour, iodomethane (78.2 g, 551 mmol, CAS Registry Number: 74-88-4) was added to this mixture under ice cooling. After stirring the mixture at room temperature overnight, water was added thereto to quench the reaction. The mixture was extracted with ethyl acetate, and the organic layer was washed in sequence with an aqueous sodium dihydrogen phosphate solution and saturated brine, dried over anhydrous sodium sulfate, filtered and concentrated under reduced pressure to obtain N²-(tert-butoxycarbonyl)-N⁶-diazo-N²-methyl-L-lysine (38.6 g). Methanol (400 mL) and palladium/carbon (4.00 g, CAS Registry Number: 7440-05-3) were added in sequence to the obtained compound. After stirring at room temperature under a hydrogen atmosphere overnight, this mixture was filtered, extracted with methanol and concentrated under reduced pressure to obtain N²-(tert-butoxycarbonyl)-N²-methyl-L-lysine (35.0 g). Methanol (300 mL), bis(trimethylsilyl)acetamide (46.9 g, 230 mmol, CAS Registry Number: 10416-59-8), 1-bromo-2-(2-bromoethoxy)ethane (28.1 g, 121 mmol, CAS Registry Number: 5414-19-7) and DIPEA (44.7 g, 346 mmol) were added in sequence to the obtained compound (30.0 g) at room temperature. The mixture was stirred at 60°C for 24 hours and then concentrated under reduced pressure to obtain (S)-2-((tert-butoxycarbonyl)(methyl)amino)-6-morpholinohexanoic acid (30.0 g). DMF (300 mL), 4-(bromomethyl)-2,6-dichloropyridine (26.3 g, 109 mmol, CAS Registry Number: 175204-45-2) and DIPEA (17.6 g, 136 mmol) were added in sequence to the obtained compound. After stirring the mixture at room temperature for 2 hours, an aqueous sodium dihydrogen phosphate solution was added thereto to quench the reaction. The mixture was extracted with ethyl acetate, and the organic layer was washed in sequence with water and saturated brine, dried over anhydrous sodium sulfate, filtered and concentrated under reduced pressure. The residue was purified by flash column chromatography (silica gel; acetonitrile/10 mM aqueous ammonium hydrogen carbonate solution = 20/80 → 60/40) to obtain (2,6-dichloropyridin-4-yl)methyl (S)-2-((tert-butoxycarbonyl)(methyl)amino)-6-morpholinohexanoate (9.80 g). Dichloromethane (25 mL) and 4 M-hydrochloric acid/MTHP (25 mL) were added in sequence to the obtained compound (4.90 g). This mixture was stirred at room temperature and then concentrated under reduced pressure. Methyl tert-butyl ether was added to the residue, and the mixture was filtered, washed with methyl tert-butyl ether and dried in vacuum to obtain (2,6-dichloropyridin-4-yl)methyl (S)-2-(methylamino)-6-morpholinohexanoate dihydrochloride (39.1 g, 8.45 mmol) as a colorless solid.

### Example 1-26: (2,6-Dichloropyridin-4-yl)methyl (S)-2-(methylamino)-6-morpholinohexanoate dihydrochloride (Compound No. 75)

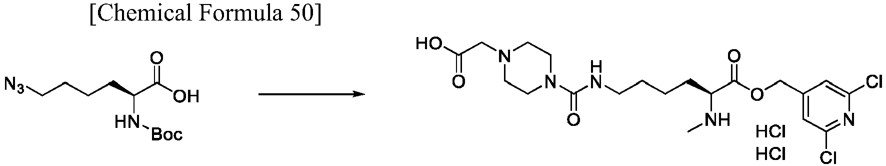

To a flask were added in sequence N²-(tert-butoxycarbonyl)-N²-methyl-L-lysine (35.0 g) obtained from Example 1-23, THF (500 mL) and bis(trimethylsilyl)acetamide (60.2 g, 296 mmol, CAS Registry Number: 10416-59-8), DIPEA (20.9 g, 161 mmol) and 4-nitrophenyl chloroformate (25.2 g, 128 mmol, CAS Registry Number: 7693-46-1) under ice cooling. After stirring the mixture at room temperature for 2 hours, hydrochloric acid was added thereto under ice cooling to quench the reaction. The mixture was extracted with ethyl acetate, and the organic layer was washed with water and saturated brine, dried over anhydrous sodium sulfate, filtered and concentrated under reduced pressure. The residue was purified by flash column chromatography (silica gel; ethyl acetate/petroleum ether = 1/1) to obtain N²-(tert-butoxycarbonyl)-N²-methyl-N⁶-((4-nitrophenoxy)carbonyl)-L-lysine (12.0 g). THF (108 mL), DMF (12 mL) and 1,1-dimethylethyl 1-piperazineacetate (5.99 g, 29.6 mmol) were added in sequence to the obtained compound under ice cooling. After stirring the mixture at 30°C overnight, an aqueous sodium hydrogen carbonate solution was added thereto to quench the reaction. The mixture was extracted with ethyl acetate, and the organic layer was washed with saturated brine, dried over anhydrous sodium sulfate, filtered and concentrated under reduced pressure to obtain N⁶-(4-(2-(tert-butoxy)-2-oxoethyl)piperazine-1-carbonyl)-N²-(tert-butoxycarbonyl)-N²-methyl-L-lysine (13.0 g). DMF (60 mL), 4-(bromomethyl)-2,6-dichloropyridine (6.11 g, 25.4 mmol, CAS Registry Number: 175204-45-2) and DIPEA (4.14 g, 32.1 mmol) were added in sequence to the obtained compound. After stirring the mixture at room temperature for 1 hour, an aqueous sodium dihydrogen phosphate solution was added thereto to quench the reaction. The mixture was extracted with ethyl acetate, and the organic layer was washed in sequence with an aqueous sodium hydrogen carbonate solution and saturated brine, dried over anhydrous sodium sulfate, filtered and concentrated under reduced pressure. The residue was purified by flash column chromatography (silica gel; acetonitrile/0.1% aqueous ammonium hydrogen carbonate solution = 30/70 → 50/50) to obtain (2,6-dichloropyridin-4-yl)methyl N⁶-(4-(2-(tert-butoxy)-2-oxoethyl)piperazine-1-carbonyl)-N²-(tert-butoxycarbonyl)-N²-methyl-L-lysinate (3.51 g). Dichloromethane (14 mL) and 2,2,2-trifluoroacetic acid (14 mL) were added in sequence to the obtained compound (3.02 g). The mixture was stirred at room temperature for 23 hours and then concentrated under reduced pressure, and acetonitrile and 4 M-hydrochloric acid/MTHP were added thereto in sequence. The mixture was filtered, washed with diethyl ether and dried in vacuum to obtain (S)-2-(4-((6-((2,6-dichloropyridin-4-yl)methoxy)-5-(methylamino)-6-oxohexyl)carbamoyl)piperazin-1-yl)acetic acid dihydrochloride (2.18 g, 4.67 mmol) as a pale yellow solid.

### Example 1-27: (2,6-Dichloropyridin-4-yl)methyl (S)-2-amino-3-(2-oxo-1,2-dihydropyridin-4-yl)propanoate hydrochloride (Compound No. 76)

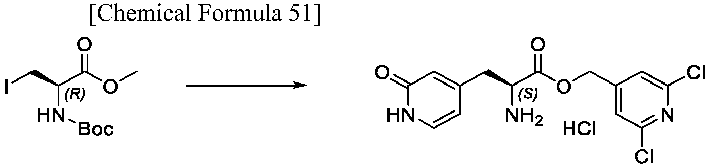

To a flask equipped with a nitrogen balloon were added in sequence zinc (20.9 g, 319 mmol, CAS Registry Number: 7440-66-6), DMF (600 mL) and iodine (16.2 g, 63.8 mmol, CAS Registry Number: 7553-56-2). After stirring the mixture at room temperature, (S)-methyl 2-(tert-butoxycarbonylamino)-3-iodopropanoate (42.0 g, 128 mmol, CAS Registry Number: 93267-04-0) was added to this mixture. After stirring the mixture at room temperature for 60 minutes, 4-bromo-2-methoxypyridine (20.0 g, 106 mmol, CAS Registry Number: 100367-39-3), 2-dicyclohexylphosphino-2',6'-dimethoxybiphenyl (8.73 g, 21.3 mmol, CAS Registry Number: 657408-07-6) and tris(dibenzylideneacetone)dipalladium-chloroform (5.44 g, 5.32 mmol, CAS Registry Number: 52522-40-4) were added in sequence to this mixture. After stirring at 50°C for 16 hours, water was added to this mixture, and the mixture was filtered and extracted with ethyl acetate. The organic layer was washed with saturated brine, dried over anhydrous sodium sulfate, filtered and concentrated under reduced pressure. The residue was purified by flash column chromatography (silica gel; ethyl acetate/petroleum ether = 1/5) to obtain methyl (S)-2-(((tert-butoxycarbonyl)amino)-3-(2-methoxypyridin-4-yl)propanoate (25.0 g). 1,4-Dioxane (250 mL) and a 48% aqueous hydrogen bromide solution (250 mL) were added in sequence to the obtained compound. The mixture was stirred at 100°C overnight and concentrated under reduced pressure to obtain (S)-2-amino-3-(2-oxo-1,2-dihydropyridin-4-yl)propanoic acid hydrobromide. 1,4-Dioxane (300 mL), water (300 mL), DIPEA (49.4 g, 380 mmol) and di-tert-butyl dicarbonate (24.9 g, 114 mmol, CAS Registry Number: 24424-99-5) were added in sequence to the obtained compound under ice cooling. After stirring the mixture at room temperature for 4 hours, an aqueous hydrochloric acid solution was added thereto to quench the reaction, and this mixture was concentrated under reduced pressure. The residue was purified by flash column chromatography (silica gel; acetonitrile/10 mM aqueous ammonium hydrogen carbonate solution= 10/90 → 50/50) to obtain (S)-2-((tert-butoxycarbonyl)amino)-3-(2-oxo-1,2-dihydropyridin-4-yl)propanoic acid (20.0 g). DMF (150 mL), DIPEA (11.1 g, 63.8 mmol) and 4-(bromomethyl)-2,6-dichloropyridine (12.2 g, 50.5 mmol, CAS Registry Number: 175204-45-2) were added in sequence to the obtained compound. After stirring the mixture at room temperature for 2 hours, a 1 M aqueous hydrochloric acid solution was added thereto to quench the reaction. The mixture was extracted with ethyl acetate, and the organic layer was washed in sequence with an aqueous sodium hydrogen carbonate solution and saturated brine, dried over anhydrous sodium sulfate, filtered and concentrated under reduced pressure. The residue was purified by flash column chromatography (silica gel; methanol/dichloromethane = 1/10) to obtain (2,6-dichloropyridin-4-yl)methyl (S)-2-((tert-butoxycarbonyl)amino)-3-(2-oxo-1,2-dihydropyridin-4-yl)propanoate (10.8 g). Dichloromethane (42 mL) and 4 M-hydrochloric acid/MTHP (42 mL) were added in sequence to the obtained compound (5.00 g). The mixture was stirred at room temperature for 5 hours and then concentrated under reduced pressure. The residue was suspended in and washed with diethyl ether and dried in vacuum to obtain (2,6-dichloropyridin-4-yl)methyl (S)-2-amino-3-(2-oxo-1,2-dihydropyridin-4-yl)propanoate hydrochloride (4.85 g, 12.81 mmol) as a colorless solid.

### Example 1-28: (2,6-Dichloropyridin-4-yl)methyl (S)-2-amino-4-(2-oxo-1,2-dihydroquinolin-6-yl)butanoate hydrochloride (Compound No. 77)

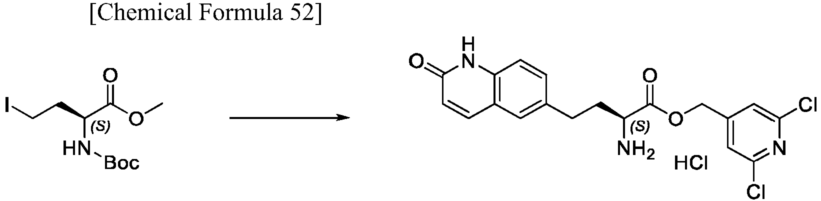

To a flask equipped with a nitrogen balloon were added in sequence zinc (16.5 g, 252 mmol, CAS Registry Number: 7440-66-6), DMF (500 mL) and iodine (6.40 g, 25.2 mmol, CAS Registry Number: 7553-56-2). After stirring the mixture at room temperature, (S)-2-((tert-butoxycarbonyl)amino)-4-iodobutanoate (34.6 g, 101 mmol, CAS Registry Number: 101650-14-0) was added to this mixture. After stirring the mixture at room temperature for 60 minutes, 6-bromo-2-methoxyquinoline (20.0 g, 84.0 mmol, CAS Registry Number: 99455-05-7), 2-dicyclohexylphosphino-2',6'-dimethoxybiphenyl (6.90 g, 16.8 mmol, CAS Registry Number: 657408-07-6) and tris(dibenzylideneacetone)dipalladium-chloroform (4.35 g, 4.20 mmol, CAS Registry Number: 52522-40-4) were added in sequence to this mixture. After stirring at 50°C for 3 hours, water was added to this mixture, and the mixture was filtered and extracted with ethyl acetate. The organic layer was washed with saturated brine, dried over anhydrous sodium sulfate, filtered and concentrated under reduced pressure. The residue was purified by flash column chromatography (silica gel; ethyl acetate/petroleum ether = 1/5) to obtain methyl (S)-2-(((tert-butoxycarbonyl)amino)-4-(2-methoxyquinolin-6-yl)butanoate (25.0 g). 1,4-Dioxane (300 mL) and a 48% aqueous hydrogen bromide solution (300 mL) were added in sequence to the obtained compound. This mixture was stirred at 100°C overnight and then concentrated under reduced pressure to obtain (S)-2-amino-4-(2-oxo-1,2-dihydroquinolin-6-yl)butanoic acid hydrobromide. 1,4-Dioxane (200 mL), water (200 mL), DIPEA (34.8 g, 269 mmol) and di-tert-butyl dicarbonate (17.6 g, 80.7 mmol, CAS Registry Number: 24424-99-5) were added to the obtained compound under ice cooling. After stirring the mixture at room temperature overnight, an aqueous hydrochloric acid solution was added thereto to quench the reaction, and this mixture was concentrated under reduced pressure. The residue was purified by flash column chromatography (silica gel; methanol/dichloromethane = 1/5) to obtain (S)-2-((tert-butoxycarbonyl)amino)-4-(2-oxo-1,2-dihydroquinolin-6-yl)butanoic acid (20.0 g). DMF (120 mL), DIPEA (5.37 g, 41.6 mmol) and 4-(bromomethyl)-2,6-dichloropyridine (7.93 g, 32.9 mmol, CAS Registry Number: 175204-45-2) were added in sequence to the obtained compound (12.0 g). After stirring the mixture at room temperature for 2 hours, a 1 M aqueous sodium hydrogen carbonate solution was added thereto to quench the reaction. The mixture was extracted with ethyl acetate, and the organic layer was washed in sequence with an aqueous sodium hydrogen carbonate solution and saturated brine, dried over anhydrous sodium sulfate, filtered and concentrated under reduced pressure. The residue was purified by flash column chromatography (silica gel; ethyl acetate/dichloromethane = 1/1) to obtain (2,6-dichloropyridin-4-yl)methyl (S)-2-((tert-butoxycarbonyl)amino)-4-(2-oxo-1,2-dihydroquinolin-6-yl)butanoate (10.1 g). Dichloromethane (27 mL) and 4 M-hydrochloric acid/MTHP (27 mL) were added in sequence to the obtained compound (3.66 g). The mixture was stirred at room temperature for 16 hours and then concentrated under reduced pressure. Diethyl ether was added to the residue, and the mixture was filtered, washed with diethyl ether and dried in vacuum to obtain (2,6-dichloropyridin-4-yl)methyl (S)-2-amino-4-(2-oxo-1,2-dihydroquinolin-6-yl)butanoate hydrochloride (3.14 g, 7.09 mmol) as a colorless solid.

### Example 1-29: 2,2,2-Trifluoroethyl (S)-2-amino-3-(4-(2-aminopyridin-3-yl)phenyl)propanoate dihydrochloride (Compound No. 78)

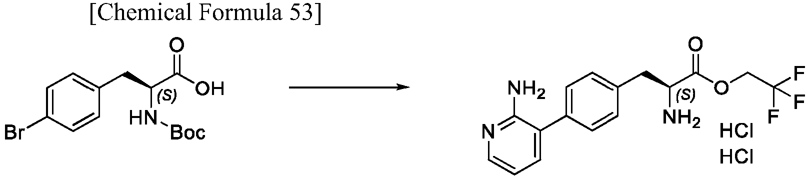

To a flask were added in sequence (S)-3-(4-bromophenyl)-2-(((tert-butoxycarbonyl)amino)propanoic acid (20.0 g, 58.1 mmol, CAS Registry Number: 62129-39-9), 1,4-dioxane (160 mL), water (40 mL), (2-((tert-butoxycarbonyl)amino)pyridin-3-yl)boronic acid (16.6 g, 69.7 mmol, CAS Registry Number: 863753-35-9), potassium carbonate (24.1 g, 174 mmol) and [1,1'-bis(di-tert-butylphosphino)ferrocene]dichloropalladium (3.03 g, 4.65 mmol, CAS Registry Number: 95408-45-0) at room temperature. After stirring at 50°C under a nitrogen atmosphere for 6 hours, a 1 M aqueous hydrochloric acid solution was added thereto to quench the reaction. The mixture was extracted with ethyl acetate, washed with saturated brine, dried over anhydrous sodium sulfate, filtered and concentrated under reduced pressure. The residue was purified by flash column chromatography (silica gel; methanol/dichloroethane = 1/5) to obtain (S)-2-((tert-butoxycarbonyl)amino)-3-(4-(2-((tert-butoxycarbonyl)amino)pyridin-3-yl)phenyl)propanoic acid (14.5 g). DMF (150 mL), 2,2,2-trifluoroethyl trifluoromethanesulfonate (6.99 g, 30.1 mmol, CAS Registry Number: 6226-25-1) and DIPEA (4.92 g, 38.0 mmol) were added in sequence to the obtained compound. After stirring the mixture at room temperature for 3 hours, an aqueous sodium dihydrogen phosphate solution was added thereto to quench the reaction. The mixture was extracted with ethyl acetate, and the organic layer was washed with an aqueous sodium hydrogen carbonate solution and saturated brine, dried over anhydrous sodium sulfate, filtered and concentrated under reduced pressure. The residue was purified by flash column chromatography (silica gel; ethyl acetate/petroleum ether = 1/1) to obtain 2,2,2-trifluoroethyl (S)-2-((tert-butoxycarbonyl)amino)-3-(4-(2-((tert-butoxycarbonyl)amino)pyridin-3-yl)phenyl)propanoate (10.3 g). Dichloromethane (20 mL) and 4 M-hydrochloric acid/MTHP (20 mL) were added in sequence to the obtained compound (4.32 g). The mixture was stirred at room temperature and then concentrated under reduced pressure. The residue was triturated and washed with methyl tert-butyl ether and then dried in vacuum to obtain 2,2,2-trifluoroethyl (S)-2-amino-3-(4-(2-aminopyridin-3-yl)phenyl)propanoate dihydrochloride (3.33 g, 8.10 mmol) as a light beige solid.

### Example 1-30: (2,6-Dichloropyridin-4-yl)methyl (S)-2-amino-3-(2-carbamoyl-1H-imidazol-4-yl)propanoate dihydrochloride (Compound No. 79)

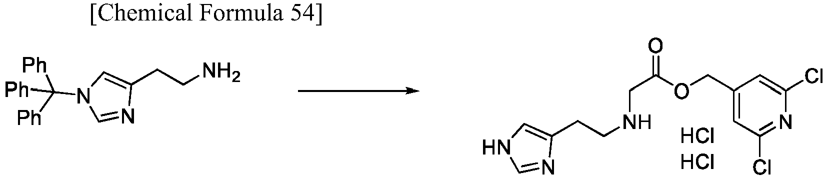

To a flask were added in sequence 2-(1-trityl-1H-imidazol-4-yl)ethan-1-amine (11.7 g, 33.1 mmol, CAS Registry Number: 195053-92-0), acetonitrile (300 mL), DIPEA (8.56 g, 66.2 mmol) and benzyl 2-bromoacetate (8.34 g, 36.4 mmol, CAS Registry Number: 5437-45-6) under ice cooling. After stirring the mixture at room temperature for 5 hours, water was added thereto to quench the reaction. The mixture was extracted with ethyl acetate, washed with saturated brine, dried over anhydrous sodium sulfate, filtered and concentrated under reduced pressure. The residue was purified by flash column chromatography (silica gel; methanol/dichloroethane = 0/100 → 7/93) to obtain benzyl (2-(1-trityl-1H-imidazol-4-yl)ethyl)glycinate (13.6 g). Methanol (120 mL) and palladium/carbon (4.08 g, CAS Registry Number: 7440-05-3) were added in sequence to the obtained compound. After stirring at room temperature under a hydrogen atmosphere for 4 hours, this mixture was filtered, extracted with methanol and concentrated under reduced pressure to obtain (2-(1-trityl-1H-imidazol-4-yl)ethyl)glycine (11.5 g). 1,4-Dioxane (30 mL), water (10 mL), sodium hydrogen carbonate (2.33 g, 27.7 mmol) and di-tert-butyl dicarbonate (3.63 g, 16.6 mmol, CAS Registry Number: 24424-99-5) were added in sequence to the obtained compound (5.70 g) under ice cooling. After stirring the mixture at room temperature for 2 hours, an aqueous citric acid solution was added thereto to quench the reaction. The mixture was extracted with ethyl acetate, washed with saturated brine, dried over anhydrous sodium sulfate, filtered and concentrated under reduced pressure. The residue was purified by flash column chromatography (silica gel; methanol/dichloroethane = 1/100 → 1/10) to obtain N-(tert-butoxycarbonyl)-N-(2-(1-trityl-1H-imidazol-4-yl)ethyl)glycine (4.50 g). DMF (20 mL), 4-(bromomethyl)-2,6-dichloropyridine (2.01 g, 8.36 mmol, CAS Registry Number: 175204-45-2) and DIPEA (1.36 g, 4.69 mmol) were added in sequence to the obtained compound. After stirring the mixture at room temperature for 2 hours, water was added thereto to quench the reaction. The mixture was extracted with ethyl acetate, and the organic layer was washed in sequence with water and saturated brine, dried over anhydrous sodium sulfate, filtered and concentrated under reduced pressure. The residue was purified by flash column chromatography (silica gel; ethyl acetate/petroleum ether = 1/100 → 1/1) to obtain (2,6-dichloropyridin-4-yl)methyl N-(tert-butoxycarbonyl)-N-(2-(1-trityl-1H-imidazol-4-yl)ethyl)glycinate (2.69 g). Dichloromethane (3.5 mL), triisopropylsilane (1.0 mL) and 2,2,2-trifluoroacetic acid (3.4 mL) were added in sequence to the obtained compound (1.50 g). The mixture was stirred at room temperature for 2 hours and then concentrated under reduced pressure, and acetonitrile and 4 M-hydrochloric acid/MTHP were added thereto in sequence. The mixture was stirred at room temperature for 30 minutes, and then filtered, washed with diethyl ether and dried in vacuum to obtain (2,6-dichloropyridin-4-yl)methyl (S)-2-amino-3-(2-carbamoyl-1H-imidazol-4-yl)propanoate dihydrochloride (0.487 g, 1.21 mmol) as a gray solid.

### Example 1-31: (2,6-Dichloropyridin-4-yl)methyl (S)-2-amino-3-(3-((3-aminopropyl)carbamoyl)phenyl)propanoate dihydrochloride (Compound No. 80)

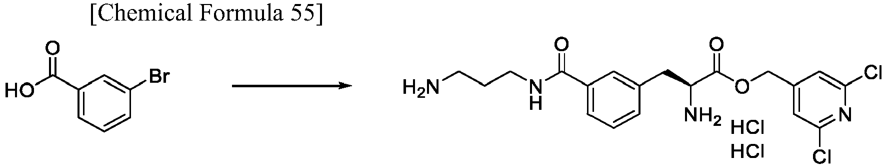

To a flask were added in sequence 3-bromobenzoic acid (10.0 g, 49.7 mmol, CAS Registry Number: 585-76-2), DMF (200 mL), tert-butyl (3-aminopropyl)carbamate (10.4 g, 59.7 mmol, CAS Registry Number: 75178-96-0), 1-ethyl-3-(3'-dimethylaminopropyl)carbodiimide hydrochloride (10.5 g, 54.7 mmol, CAS Registry Number: 25952-53-8) and ethyl 2-cyano-2-(hydroxyimino)acetate (7.78 g, 54.7 mmol, CAS Registry Number: 3849-21-6) at room temperature. After stirring the mixture at room temperature for 2 hours, water was added thereto to quench the reaction. The mixture was extracted with ethyl acetate, washed with saturated brine, dried over anhydrous sodium sulfate, filtered and concentrated under reduced pressure. The residue was purified by flash column chromatography (silica gel; ethyl acetate/petroleum ether = 1/1) to obtain tert-butyl (3-(3-bromobenzamido)propyl)carbamate (15.0 g). To a flask equipped with a nitrogen balloon were added in sequence zinc (8.24 g, 126 mmol, CAS Registry Number: 7440-66-6), DMF (300 mL) and iodine (3.20 g, 12.6 mmol, CAS Registry Number: 7553-56-2). After stirring the mixture at room temperature, methyl (R)-2-((((9H-fluoren-9-yl)methoxy)carbonyl)amino)-3-iodopropanoate (20.8 g, 46.2 mmol, CAS Registry Number: 156017-42-4) and iodine (3.20 g, 12.6 mmol, CAS Registry Number: 7553-56-2) were added in sequence to this mixture. After stirring the mixture at room temperature for 30 minutes, the obtained tert-butyl (3-(3-bromobenzamido)propyl)carbamate (15.0 g), 2-dicyclohexylphosphino-2',6'-dimethoxybiphenyl (3.45 g, 8.40 mmol, CAS Registry Number: 657408-07-6) and tris(dibenzylideneacetone)dipalladium-chloroform (1.92 g, 2.10 mmol, CAS Registry Number: 52522-40-4) were added in sequence to this mixture at room temperature. After stirring at 50°C for 3 hours, this mixture was filtered, ethyl acetate was added thereto. The organic layer was washed with saturated brine, dried over anhydrous sodium sulfate, filtered and concentrated under reduced pressure. The residue was suspended in and washed with diethyl ether and dried in vacuum to obtain methyl (S)-2-(((9H-fluoren-9-yl)methoxy)carbonyl)amino)-3-(3-(((tert-butoxycarbonyl)amino)propyl)carbamoyl)phenyl)propanoate (20.0 g).

Isopropyl alcohol (300 mL), water (100 mL), THF (50 mL), calcium chloride (67.9 g, 612 mmol, CAS Registry Number: 10043-52-4) and lithium hydroxide monohydrate (6.42 g, 153 mmol, CAS Registry Number: 1310-66-3) were added in sequence to the obtained compound (23.0 g) under ice cooling. After stirring the mixture at room temperature, an aqueous sodium dihydrogen phosphate solution was added thereto to quench the reaction. This mixture was filtered and extracted with ethyl acetate, and the organic layer was washed with saturated brine, dried over anhydrous sodium sulfate, filtered and concentrated under reduced pressure. The residue was purified by flash column chromatography (silica gel; ethyl acetate/petroleum ether = 1/1) to obtain (S)-2-((((9H-fluoren-9-yl)methoxy)carbonyl)amino)-3-(3-(((tert-butoxycarbonyl)amino)propyl)carbamoyl)phenyl)propanoic acid (19.5 g). Acetonitrile (10 mL) and triethylamine (1.01 g, 10.0 mmol) were added in sequence to the obtained compound (1.20 g) at room temperature. The mixture was stirred at 60°C for 1 hour and then concentrated under reduced pressure. The residue was suspended in and washed with diethyl ether and dried in vacuum to obtain (S)-2-amino-3-(3-(((tert-butoxycarbonyl)amino)propyl)carbamoyl)phenyl)propanoic acid. 1,4-Dioxane (6.0 mL), water (4.0 mL), sodium carbonate (0.636 g, 6.00 mmol) and di-tert-butyl dicarbonate (0.524 g, 2.4 mmol, CAS Registry Number: 24424-99-5) were added in sequence to the obtained compound at room temperature.

The mixture was stirred at room temperature and then concentrated under reduced pressure to obtain (S)-2-((tert-butoxycarbonyl)amino)-3-(3-((tert-butoxycarbonyl)amino)propyl)phenyl)propanoic acid. DMF (4.0 mL) and 4-(bromomethyl)-2,6-dichloropyridine (0.458 g, 1.90 mmol, CAS Registry Number: 175204-45-2) were added in sequence to the obtained compound. After stirring the mixture at room temperature, water was added thereto to quench the reaction. The mixture was extracted with isopropyl acetate, and the organic layer was washed in sequence with water and saturated brine, dried over anhydrous sodium sulfate, filtered and concentrated under reduced pressure. The residue was purified by flash column chromatography (silica gel; ethyl acetate/heptane = 0/100 → 80/20) to obtain (2,6-dichloropyridin-4-yl)methyl (S)-2-((tert-butoxycarbonyl)amino)-3-(3-((tert-butoxycarbonyl)amino)carbamoyl)phenyl)propanoate. Dichloromethane (25 mL) and 4 M-hydrochloric acid/MTHP (25 mL) were added in sequence to the obtained compound. The mixture was stirred at room temperature and then concentrated under reduced pressure. The residue was suspended in and washed with diethyl ether and dried in vacuum to obtain (2,6-dichloropyridin-4-yl)methyl (S)-2-amino-3-(3-((3-aminopropyl)carbamoyl)phenyl)propanoate dihydrochloride (0.262 g, 0.525 mmol) as a colorless solid.

### Example 1-32: (2,6-Dichloropyridin-4-yl)methyl (S)-2-amino-4-(3,3-difluoropiperidin-1-yl)butanoate dihydrochloride (Compound No. 81)

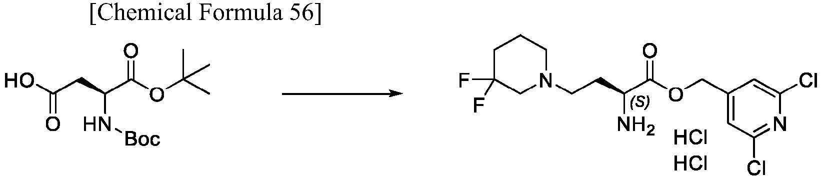

To a flask were added in sequence (S)-4-(tert-butoxy)-3-((tert-butoxycarbonyl)amino)-4-oxobutanoic acid (40.0 g, 138 mmol, CAS Registry Number: 34582-32-6), DMF (800 mL), 3,3-difluoropiperidine hydrochloride (24.0 g, 152 mmol, CAS Registry Number: 496807-97-7), 1-ethyl-3-(3'-dimethylaminopropyl)carbodiimide hydrochloride (29.2 g, 152 mmol, CAS Registry Number: 25952-53-8), ethyl 2-cyano-2-(hydroxyimino)acetate (21.6 g, 152 mmol, CAS Registry Number: 3849-21-6) and DIPEA (26.8 g, 207 mmol) under ice cooling. After stirring the mixture at room temperature overnight, an aqueous sodium hydrogen carbonate solution was added thereto to quench the reaction. The mixture was extracted with ethyl acetate, washed with saturated brine, dried over anhydrous sodium sulfate, filtered and concentrated under reduced pressure. The residue was purified by flash column chromatography (silica gel; methanol/dichloromethane = 1/20) to obtain tert-butyl (S)-2-((tert-butoxycarbonyl)amino)-4-(3,3-difluoropiperidin-1-yl)-4-oxobutanoate (44.0 g). tert-Butyl (S)-2-((tert-butoxycarbonyl)amino)-4-(3,3-difluoropiperidin-1-yl)-4-oxobutanoate (44.0 g) dissolved in THF (800 mL) was added to 1,1,3,3-tetramethyldisiloxane (120 g, 897 mmol, CAS Registry Number: 3277-26-7) and triruthenium dodecacarbonyl (3.58 g, 5.61 mmol, CAS Registry Number: 15243-33-1) dissolved in THF. This mixture was stirred at 40°C under an air atmosphere overnight and then concentrated under reduced pressure to obtain tert-butyl (S)-2-(((tert-butoxycarbonyl)amino)-4-(3,3-difluoropiperidin-1-yl)butanoate. 1,4-Dioxane (300 mL) and a 4 M aqueous hydrochloric acid solution (300 mL) were added in sequence to the obtained compound at room temperature. The mixture was stirred at 100°C overnight and then concentrated under reduced pressure to obtain (S)-2-amino-4-(3,3-difluoropiperidin-1-yl)butanoic acid dihydrochloride. 1,4-Dioxane (300 mL), water (300 mL), sodium carbonate (57.5 g, 542 mmol, CAS Registry Number: 497-19-8) and di-tert-butyl dicarbonate (35.5 g, 163 mmol, CAS Registry Number: 24424-99-5) were added in sequence to the obtained compound under ice cooling. After stirring the mixture at room temperature overnight, an aqueous sodium dihydrogen phosphate solution was added thereto to quench the reaction.

The mixture was extracted with ethyl acetate, and the organic layer was washed in sequence with saturated brine, dried over anhydrous sodium sulfate, filtered and concentrated under reduced pressure to obtain (S)-2-((tert-butoxycarbonyl)amino)-4-(3,3-difluoropiperidin-1-yl)butanoic acid (14.0 g). DMF (150 mL), DIPEA (6.74 g, 52.1 mmol) and 4-(bromomethyl)-2,6-dichloropyridine (9.94 g, 41.3 mmol, CAS Registry Number: 175204-45-2) were added in sequence to the obtained compound. After stirring the mixture at room temperature for 1 hour, a 1 M aqueous hydrochloric acid solution was added thereto to quench the reaction. The mixture was extracted with ethyl acetate, and the organic layer was washed in sequence with saturated brine, dried over anhydrous sodium sulfate, filtered and concentrated under reduced pressure. The residue was purified by flash column chromatography (silica gel; acetonitrile/0.1% aqueous formic acid solution = 5/95 → 100/0) to obtain (2,6-dichloropyridin-4-yl)methyl (S)-2-((tert-butoxycarbonyl)amino)-4-(3,3-difluoropiperidin-1-yl)butanoate (7.30 g). Dichloromethane (20 mL) and 4 M-hydrochloric acid/MTHP (20 mL) were added in sequence to the obtained compound (3.22 g). The mixture was stirred at room temperature for 4 hours and then concentrated under reduced pressure. The residue was suspended in and washed with diethyl ether and dried in vacuum to obtain (2,6-dichloropyridin-4-yl)methyl (S)-2-amino-4-(3,3-difluoropiperidin-1-yl)butanoate dihydrochloride (2.68 g, 5.89 mmol) as a colorless solid.

Each of the following compounds was synthesized using the corresponding starting amino acid, esterification reagent and deprotection condition in each of the following Tables according to the procedure of Example 1-1.

**[Table 1-1]**

| | |
|---|---|
| ExampleNo. | Structure |
| | Compound name |
| | ¹HNMR spectrum analysis results |
| | LC/MS analysis results |
| | tR (LC/MS analysis condition) |
| | Starting amino acid (CAS Registry Number) |
| | Esterification reagent (CAS Registry Number) |
| | Deprotection condition (CAS Registry Number) |
| 1-1-1 | 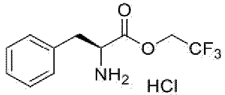 |
| | 2,2,2-Trifluoroethyl L-phenylalaninate hydrochloride |
| | ¹H NMR (500 MHz, DMSO-d*₆*) δ 3.10 (dd, 1H, *J* = 7.5, 14.0 Hz), 3.22 (dd, 1H, *J* = 5.5, 14.0 Hz), 4.46 (dd, 1H, *J* = 5.5, 7.5 Hz), 4.82-4.85 (m, 2H), 7.25-7.35 (m, 5H), 8.72 (brs, 3H) |
| | LC-MS (ESI) *m*/*z* [M+H]⁻: 248.05 |
| | tR 4.564 min (A) |
| | (tert-Butoxycarbonyl)-L-phenylalanine (13734-34-4) |
| | 2,2,2-Trifluoroethyl trifluoromethanesulfonate (6226-25-1) |
| | 4N-HCl/MTHP |

**[Table 1-2]**

| | |
|---|---|
| 1-1-2 | 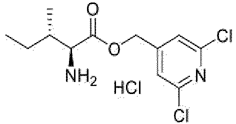 |
| | (2,6-Dichloropyridin-4-yl)methyl L-isoleucinate hydrochloride |
| | ¹H NMR (500 MHz, DMSO-d*₆*) δ 0.88-0.95 (m, 6H), 1.26-1.32 (m, 1H), 1.44-1.52 (1H), 1.95-2.04 (m, 1H), 4.12-4.13 (m, 1H), 5.28-5.29 (m, 2H), 7,67 (s, 2H), 8.61 (brs, 3H) |
| | LC-MS (ESI) *m*/*z* [M+H]⁺; 291.00 |
| | tR 3.248 min (B) |
| | (tert-Butoxycarbonyl)-L-isoleucine (13139-16-7) |
| | 4-(Bromomethyl)-2,6-dichloropyridine (175204-45-2) |
| | 4N-HCl/CPME |
| 1-1-3 | 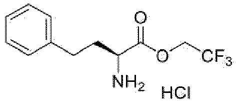 |
| | 2,2,2-Trifluoroethyl (S)-2-amino-4-phenylbutanoate hydrochloride |
| | 1H NMR (500 MHz, DMSO-d6) δ ppm 2.11 (q, 2 H *J* = 7.5 Hz) 2.57 - 2.65 (m, 1 H) 2.74 - 2.82 (m, 1 H) 4.22 (br t, 1 H, *J* = 6.0 Hz) 4.88 - 5.02 (m, 2 H) 7.19 - 7.24 (m, 3 H) 7.32 (t, 2 H, *J* = 7.0 Hz) 8.69 (br s, 3 H) |
| | LC-MS (ESI) *m*/*z* [M+H]⁺: 262.06 |
| | tR 3.132 min (B) |
| | (tert-Butoxycarbonyl)-(S)-2-amino-4-phenylbutanoic acid (100564-78-1) |
| | 2,2,2-Trifluoroethyl trifluoromethanesulfonate (6226-25-1) |
| | 4N-HCl/MTHP |

**[Table 1-3]**

| | |
|---|---|
| 1-1-4 | 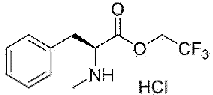 |
| | 2,2,2-Trifluoroethyl methyl-L-phenylalaninate hydrochloride |
| | ¹H NMR (500 MHz, DMSO-*d₆*) δ 2.62 (s, 3H), 3.13 (dd, 1H, *J* = 8.0, 14.0 Hz), 3.37-3.39 (m, 1H), 4.51-4.52 (m, 1H), 4.79-4.83 (m, 2H), 7.25-7.35 (m, 5H), 9.60-9.88 (m, 2H) |
| | LC-MS (ESI) *m*/*z* [M+H]⁺: 262.05 |
| | tR 2.904 min (B) |
| | (tert-Butoxycarbonyl)-methyl-L-phenylalanine (37553-65-4) |
| | 2,2,2-Trifluoroethyl trifluoromethanesulfonate (6226-25-1) |
| | 4N-HCl/MTHP |
| 1-1-5 | 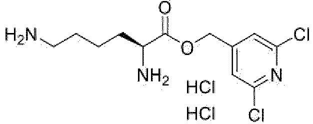 |
| | (2,6-Dichloropyridin-4-yl)methyl-L-lysinate dihydrochloride |
| | 1H NMR (500 MHz, DMSO-d6) δ ppm 1.34 - 1.63 (m, 4 H) 1.72 - 1.89 (m, 2 H) 2.76 (br t, 2 H, *J* = 7.5 Hz) 4.13 - 4.18 (m, 1 H) 5.22 - 5.42 (m, 2 H) 7.68 (s, 2 H) 7.73 - 8.81 (m, δ H) |
| | LC-MS (ESI) *m*/*z* [M+H]⁺: 305.98 |
| | tR 4.444 min (A) |
| | Di-(tert-Butoxycarbonyl)-L-lysine (2483-46-7) |
| | 4-(Bromomethyl)-2,6-dichloropyridine (175204-45-2) |
| | 4N-HCl/CPME |

**[Table 1-4]**

| | |
|---|---|
| 1-1-6 | 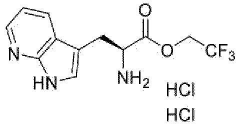 |
| | 2,2,2-Trifluoroethyl (S)-2-amino-3-(1H-pyrrolo[2,3-b]pyridin-3-yl) propanoate dihydrochloride |
| | ¹H NMR (500 MHz, DMSO-d*₆*) *δ* 3.33-3.42 (m, 2H), 4.51-4.52 (m, 1H), 4.84-4.91 (m, 2H), 7.30 (dd, 1H, *J* = 5.0, 7.5 Hz), 7.52 (d, 1H, J = 2.0 Hz), 8.34-8.37 (m, 2H), 8.76 (brs, 3H), 12.2 (brs, 1H) |
| | LC-MS (ESI) *m*/*z* [M+H]⁻: 288.07 |
| | tR 5.548 min (A) |
| | (S)-2-((tert-Butoxycarbonyl)amino)-3-(1H-pyrrolo[2,3-b]pyridin-3-yl) propionic acid (146610-21-1) |
| | 2,2,2-Trifluoroethyl trifluoromethanesulfonate (6226-25-1) |
| | 4N-HCl/MTHP |
| 1-1-7 | 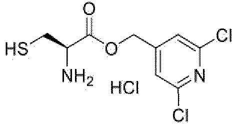 |
| | (2,6-Dichloropyridin-4-yl)methyl-L-cysteinate hydrochloride |
| | 1H NMR (500 MHz, DMSO-d6) δ ppm 3.05-3.19 (m, 2 H) 3.36 - 3.41 (m, 1 H) 4.54 - 4.60 (m, 1 H) 5.30 - 5.40 (m, 2 H) 7.70 (s, 2 H) 8.81 (br s, 3 H) |
| | LC-MS (ESI) *m*/*z* [M+H]⁺: 280.90 |
| | tR 4.428 min (A) |
| | N-(tert-Butoxycarbonyl)-S-trityl-L-cysteine (21947-98-8) |
| | 4-(Bromomethyl)-2,6-dichloropyridine (175204-45-2) |
| | 2,2,2-Trifluoroacetic acid (76-05-1) |

**[Table 1-5]**

| | |
|---|---|
| 1-1-8 | 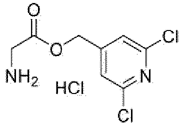 |
| | (2,6-Dichloropyridin-4-yl)methyl glycinate hydrochloride |
| | ¹H NMR (500 MHz, DMSO-*d₆*) δ 3.99 (s, 2H), 5.33 (s, 2H), 7.66 (s, 2H), 8.42 (brs, 3H) |
| | LC-MS (ESI) *m*/*z* [M+H]⁺: 235.05 |
| | tR 2.012 min (B) |
| | (tert-Butoxycarbonyl)-glycine (4530-20-5) |
| | 4-(Bromomethyl)-2,6-dichloropyridine (175204-45-2) |
| | 4N-HCl/CPME |
| 1-1-9 | 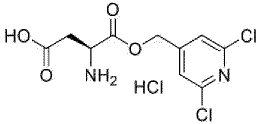 |
| | (S)-3-Amino-4-((2,6-dichloropyridin-4-yl)methoxy)-4-oxobutanoic acid hydrochloride |
| | LC-MS (ESI) *m*/*z* [M+H]⁺: 292.92 |
| | tR 3.552 min (A) |
| | (tert-Butoxycarbonyl)-L-aspartic acid 4-tert-butyl ester (1676-90-0) |
| | 4-(Bromomethyl)-2,6-dichloropyridine (175204-45-2) |
| | 2,2,2-Trifluoroacetic acid (76-05-1) |

**[Table 1-6]**

| | |
|---|---|
| 1-1-10 | 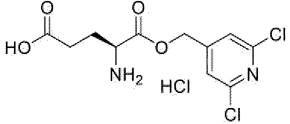 |
| | (S)-3-Amino-5-((2,6-dichloropyridin-4-yl)methoxy)-5-oxopentanoic acid hydrochloride |
| | LC-MS (ESI) *m*/*z* [M+H]⁺: 306.98 |
| | tR 3.588 min (A) |
| | (tert-Butoxycarbonyl)-L-glutamic acid 5-tert-butyl ester (13726-84-6) |
| | 4-(Bromomethyl)-2,6-dichloropyridine (175204-45-2) |
| | 2,2,2-Trifluoroacetic acid (76-05-1) |
| 1-1-11 | 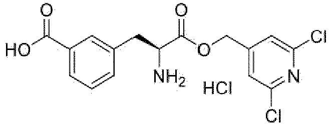 |
| | (S)-3-(2-Amino-3-((2,6-dichloropyridin-4-yl)methoxy)-3-oxopropyl) benzoic acid hydrochloride |
| | LC-MS (ESI) *m*/*z* [M+H]⁺: 368.97 |
| | tR 3.104 min (B) |
| | (S)-2-((tert-Butoxycarbonyl)amino)-3-(3-(tert-butoxycarbonyl)phenyl) propionic acid (2245801-13-0) |
| | 4-(Bromomethyl)-2,6-dichloropyridine (175204-45-2) |
| | 4N-HCl/CPME |

**[Table 1-7]**

| | |
|---|---|
| 1-1-12 | 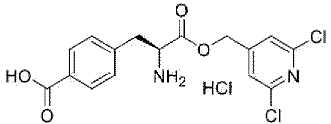 |
| | (S)-4-(2-Amino-3-((2,6-dichloropyridin-4-yl)methoxy)-3-oxopropyl) benzoic acid hydrochloride |
| | LC-MS (ESI) *m*/*z* [M+H]⁺: 368.98 |
| | tR 2.876 min (B) |
| | (S)-2-((tert-Butoxycarbonyl)amino)-3-(4-(tert-butoxycarbonyl)phenyl) propionic acid (214750-69-3) |
| | 4-(Bromomethyl)-2,6-dichloropyridine (175204-45-2) |
| | 4N-HCl/MTHP |
| 1-1-13 | 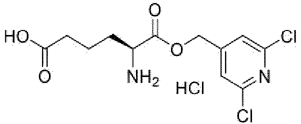 |
| | (S)-5-Amino-6-((2,6-dichloropyridin-4-yl)methoxy)-6-oxohexanoic acid hydrochloride |
| | LC-MS (ESI) *m*/*z* [M+H]⁺: 320.96 |
| | tR 4.072 min (A) |
| | (S)-6-(tert-Butoxy)-2-((tert-butoxycarbonyl)amino)-6-oxohexanoic acid (1242267-49-7) |
| | 4-(Bromomethyl)-2,6-dichloropyridine (175204-45-2) |
| | 4N-HCl/MTHP |

**[Table 1-8]**

| | |
|---|---|
| 1-1-14 | 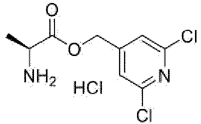 |
| | (2,6-Dichloropyridin-4-yl)methyl-L-alanate hydrochloride |
| | LC-MS (ESI) *m*/*z* [M+H]⁺: 248.90 |
| | tR 2.393 min (B) |
| | (tert-Butoxycarbonyl)-L-alanine (15761-38-3) |
| | 4-(Bromomethyl)-2,6-dichloropyridine (175204-45-2) |
| | 4N-HCl/CPME |
| 1-1-15 | 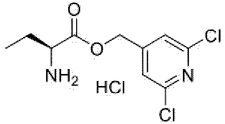 |
| | (2,6-Dichloropyridin-4-yl)methyl (S)-2-aminobutanoate hydrochloride |
| | LC-MS (ESI) *m*/*z* [M+H]⁺: 263.00 |
| | tR 2.720 min (B) |
| | (S)-2-((tert-Butoxycarbonyl)amino)butanoic acid (34306-42-8) |
| | 4-(Bromomethyl)-2,6-dichloropyridine (175204-45-2) |
| | 4N-HCl/CPME |

**[Table 1-9]**

| | |
|---|---|
| 1-1-16 | 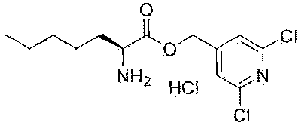 |
| | (2,6-Dichloropyridin-4-yl)methyl (S)-2-aminoheptanoate hydrochloride |
| | LC-MS (ESI) *m*/*z* [M+H]⁺: 304.98 |
| | tR 3.612 min (B) |
| | (S)-2-((tert-Butoxycarbonyl)amino)heptanoic acid (71066-01-8) |
| | 4-(Bromomethyl)-2,6-dichloropyridine (175204-45-2) |
| | 4N-HCl/CPME |
| 1-1-17 | 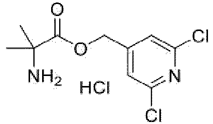 |
| | (2,6-Dichloropyridin-4-yl)methyl 2-amino-2-methylpropanoate hydrochloride |
| | LC-MS (ESI) *m*/*z* [M+H]⁺: 262.99 |
| | tR 4.232 min (A) |
| | 2-((tert-Butoxycarbonyl)amino)-2-methylpropionic acid (30992-29-1) |
| | 4-(Bromomethyl)-2,6-dichloropyridine (175204-45-2) |
| | 4N-HCl/MTHP |

**[Table 1-10]**

| | |
|---|---|
| 1-1-18 | 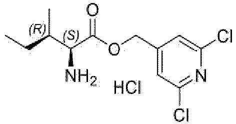 |
| | (2,6-Dichloropyridin-4-yl)methyl-L-alloisoleucinate hydrochloride |
| | LC-MS (ESI) *m*/*z* [M+H]⁺: 291.03 |
| | tR 3.216 min (B) |
| | (tert-Butoxycarbonyl)-L-alloisoleucine (35264-07-4) |
| | 4-(Bromomethyl)-2,6-dichloropyridine (175204-45-2) |
| | 4N-HCl/MTHP |
| 1-1-19 | 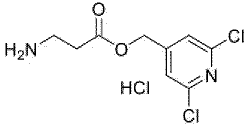 |
| | (2,6-Dichloropyridin-4-yl)methyl 3-aminopropanoate hydrochloride |
| | LC-MS (ESI) *m*/*z* [M+H]⁺: 248.98 |
| | tR 3.548 min (A) |
| | 3-((tert-Butoxycarbonyl)amino)propionic acid (3303-84-2) |
| | 4-(Bromomethyl)-2,6-dichloropyridine (175204-45-2) |
| | 4N-HCl/MTHP |

**[Table 1-11]**

| | |
|---|---|
| 1-1-20 | 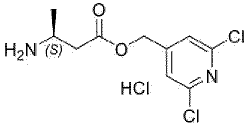 |
| | (2,6-Dichloropyridin-4-yl)methyl (S)-3-aminobutanoate hydrochloride |
| | LC-MS (ESI) *m*/*z* [M+H]⁺: 262.99 |
| | tR 2.628 min (B) |
| | (S)-3-((tert-Butoxycarbonyl)amino)butanoic acid (158851-30-0) |
| | 4-(Bromomethyl)-2,6-dichloropyridine (175204-45-2) |
| | 4N-HCl/MTHP |
| 1-1-21 | 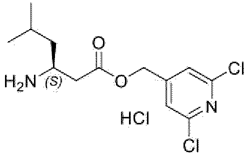 |
| | (2,6-Dichloropyridin-4-yl)methyl (S)-3-amino-5-methylhexanoate hydrochloride |
| | LC-MS (ESI) *m*/*z* [M+H]⁺: 305.04 |
| | tR 3.416 min (B) |
| | (S)-3-((tert-Butoxycarbonyl)amino)-5-methylhexanoic acid (132549-43-0) |
| | 4-(Bromomethyl)-2,6-dichloropyridine (175204-45-2) |
| | 4N-HCl/MTHP |

**[Table 1-12]**

| | |
|---|---|
| 1-1-22 | 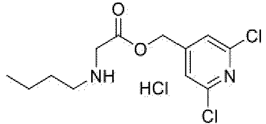 |
| | (2,6-Dichloropyridin-4-yl)methyl butylglycinate hydrochloride |
| | LC-MS (ESI) *m*/*z* [M+H]⁻: 290.99 |
| | tR 3.044 min (B) |
| | N-(tert-Butoxycarbonyl)-N-butylglycine (439287-56-6) |
| | 4-(Bromomethyl)-2,6-dichloropyridine (175204-45-2) |
| | 4N-HCl/MTHP |
| 1-1-23 | 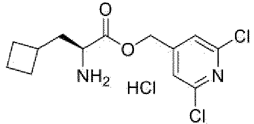 |
| | (2,6-Dichloropyridin-4-yl)methyl (S)-2-amino-3-cyclobutylpropanoate hydrochloride |
| | LC-MS (ESI) *m*/*z* [M+H]⁺: 302.99 |
| | tR 3.452 min (B) |
| | (S)-2-((tert-Butoxycarbonyl)amino)-3-cyclobutylpropionic acid (478183-60-7) |
| | 4-(Bromomethyl)-2,6-dichloropyridine (175204-45-2) |
| | 4N-HCl/MTHP |

**[Table 1-13]**

| | |
|---|---|
| 1-1-24 | 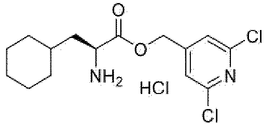 |
| | (2,6-Dichloropyridin-4-yl)methyl (S)-2-amino-3-cyclohexylpropionate hydrochloride |
| | LC-MS (ESI) *m*/*z* [M+H]⁺: 331.03 |
| | tR 3.876 min (B) |
| | (S)-2-((tert-Butoxycarbonyl)amino)-3-cyclohexylpropionic acid (37736-82-6) |
| | 4-(Bromomethyl)-2,6-dichloropyridine (175204-45-2) |
| | 4N-HCl/MTHP |
| 1-1-25 | 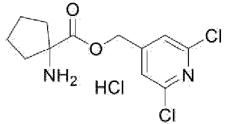 |
| | (2,6-Dichloropyridin-4-yl)methyl 1-aminocyclopentane-1-carboxylate hydrochloride |
| | LC-MS (ESI) *m*/*z* [M+H]⁺: 288.99 |
| | tR 2.992 min (B) |
| | 1-((tert-Butoxycarbonyl)amino)cyclopentane-1-carboxylic acid (35264-09-6) |
| | 4-(Bromomethyl)-2,6-dichloropyridine (175204-45-2) |
| | 4N-HCl/CPME |

**[Table 1-14]**

| | |
|---|---|
| 1-1-26 | 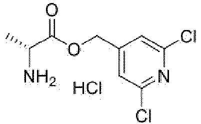 |
| | (2,6-Dichloropyridin-4-yl)methyl D-alaninate hydrochloride |
| | LC-MS (ESI) *m*/*z* [M+H]⁺: 249.00 |
| | tR 2.464 min (B) |
| | (tert-Butoxycarbonyl)-D-alanine (7764-95-6) |
| | 4-(Bromomethyl)-2,6-dichloropyridine (175204-45-2) |
| | 4N-HCl/CPME |
| 1-1-27 | 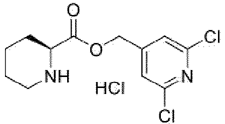 |
| | (2,6-Dichloropyridin-4-yl)methyl (S)-piperidine-2-carboxylate hydrochloride |
| | LC-MS (ESI) *m*/*z* [M+H]⁺: 288.99 |
| | tR 2.800 min (B) |
| | (S)-1-(tert-Butoxycarbonyl)piperidine-2-carboxylic acid (26250-84-0) |
| | 4-(Bromomethyl)-2,6-dichloropyridine (175204-45-2) |
| | 4N-HCl/MTHP |

**[Table 1-15]**

| | |
|---|---|
| 1-1-28 | 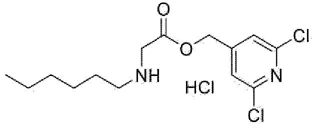 |
| | (2,6-Dichloropyridin-4-yl)methyl hexylglycinate hydrochloride |
| | LC-MS (ESI) *m*/*z* [M+H]⁺: 319.06 |
| | tR 3.660 min (B) |
| | N-(tert-Butoxycarbonyl)-N-hexylglycine (110106-56-4) |
| | 4-(Bromomethyl)-2,6-dichloropyridine (175204-45-2) |
| | 4N-HCl/CPME |

**[Table 1-16]**

| | |
|---|---|
| 1-1-29 | 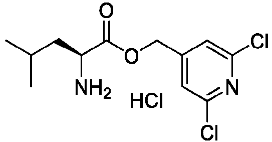 |
| | (2,6-Dichloropyridin-4-yl)methyl L-leucinate hydrochloride |
| | LC-MS (ESI) *m*/*z* [M+H]⁺: 291.02 |
| | tR 3.304 min (B) |
| | (tert-Butoxycarbonyl)-L-leucine (13139-15-6) |
| | 4-(Bromomethyl)-2,6-dichloropyridine (175204-45-2) |
| | 4N-HCl/CPME |
| 1-1-30 | 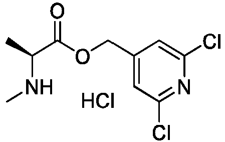 |
| | (2,6-Dichloropyridin-4-yl)methyl methyl-L-alaninate hydrochloride |
| | LC-MS (ESI) *m*/*z* [M+H]⁺: 262.99 |
| | tR 2.572 min (B) |
| | N-(tert-Butoxycarbonyl)-N-methyl-L-alanine (16948-16-6) |
| | 4-(Bromomethyl)-2,6-dichloropyridine (175204-45-2) |
| | 4N-HCl/CPME |

**[Table 1-17]**

| | |
|---|---|
| 1-1-31 | 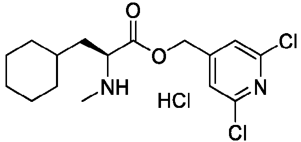 |
| | (2,6-Dichloropyridin-4-yl)methyl (S)-3-cyclohexyl-2-(methylamino)propanoate hydrochloride |
| | LC-MS (ESI) *m*/*z* [M+H]⁺: 344.97 |
| | tR 4.052 min (B) |
| | (S)-2-((tert-Butoxycarbonyl)(methyl)amino)-3-cyclohexylpropionic acid (97269-22-2) |
| | 4-(Bromomethyl)-2,6-dichloropyridine (175204-45-2) |
| | 4N-HCl/MTHP |
| 1-1-32 | 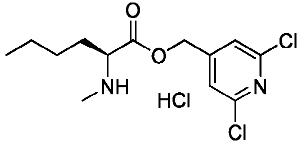 |
| | (2,6-Dichloropyridin-4-yl)methyl (S)-2-(methylamino)hexanoate hydrochloride |
| | LC-MS (ESI) *m*/*z* [M+H]⁺: 305.02 |
| | tR 3.432 min (B) |
| | (S)-2-((tert-Butoxycarbonyl)(methyl)amino)hexanoic acid (117903-25-0) |
| | 4-(Bromomethyl)-2,6-dichloropyridine (175204-45-2) |
| | 4N-HCl/MTHP |

**[Table 1-18]**

| | |
|---|---|
| 1-1-33 | 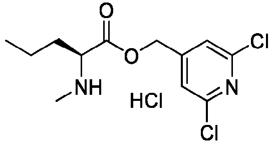 |
| | (2,6-Dichloropyridin-4-yl)methyl (S)-2-(methylamino)pentanoate hydrochloride |
| | LC-MS (ESI) *m*/*z* [M+H]⁺: 290.97 |
| | tR 3.272 min (B) |
| | (S)-2-((tert-Butoxycarbonyl)(methyl)amino)pentanoic acid (136092-78-9) |
| | 4-(Bromomethyl)-2,6-dichloropyridine (175204-45-2) |
| | 4N-HCl/MTHP |
| 1-1-34 | 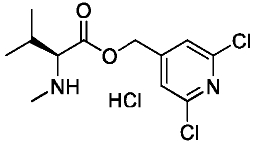 |
| | (2,6-Dichloropyridin-4-yl)methyl methyl-L-valinate hydrochloride |
| | LC-MS (ESI) *m*/*z* [M+H]⁺: 291.04 |
| | tR 3.028 min (B) |
| | N-(tert-Butoxycarbonyl)-N-methyl-L-valine (45170-31-8) |
| | 4-(Bromomethyl)-2,6-dichloropyridine (175204-45-2) |
| | 4N-HCl/CPME |
| 1-1-35 | 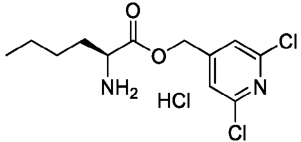 |
| | (2,6-Dichloropyridin-4-yl)methyl (S)-2-aminohexanoate hydrochloride |
| | LC-MS (ESI) *m*/*z* [M+H]⁺: 291.01 |
| | tR 3.336 min (B) |
| | (S)-2-((tert-Butoxycarbonyl)amino)hexanoic acid (6404-28-0) |
| | 4-(Bromomethyl)-2,6-dichloropyridine (175204-45-2) |
| | 4N-HCl/CPME |

**[Table 1-19]**

| | |
|---|---|
| 1-1-36 | 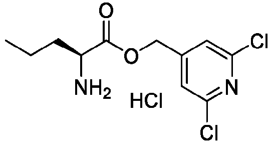 |
| | (2,6-Dichloropyridin-4-yl)methyl (S)-2-aminopentanoate hydrochloride |
| | LC-MS (ESI) *m*/*z* [M+H]⁺: 276.98 |
| | tR 3.184 min (B) |
| | (S)-2-((tert-Butoxycarbonyl)amino)pentanoic acid (53308-95-5) |
| | 4-(Bromomethyl)-2,6-dichloropyridine (175204-45-2) |
| | 4N-HCl/MTHP |
| 1-1-37 | 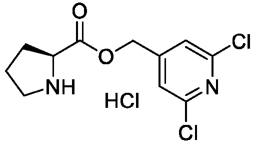 |
| | (2,6-Dichloropyridin-4-yl)methyl L-prolinate hydrochloride |
| | LC-MS (ESI) *m*/*z* [M+H]⁺: 274.97 |
| | tR 2.604 min (B) |
| | (tert-Butoxycarbonyl)-L-proline (15761-39-4) |
| | 4-(Bromomethyl)-2,6-dichloropyridine (175204-45-2) |
| | 4N-HCl/CPME |

**[Table 1-20]**

| | |
|---|---|
| 1-1-38 | 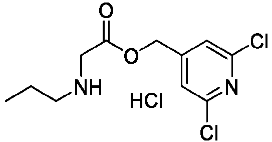 |
| | (2,6-Dichloropyridin-4-yl)methyl propylglycinate hydrochloride |
| | LC-MS (ESI) *m*/*z* [M+H]⁺: 277.01 |
| | tR 2.716 min (B) |
| | N-(tert-Butoxycarbonyl)-N-propylglycine (165607-76-1) |
| | 4-(Bromomethyl)-2,6-dichloropyridine (175204-45-2) |
| | 4N-HCl/MTHP |
| 1-1-39 | 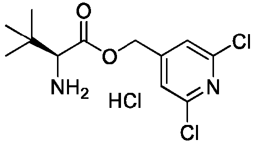 |
| | (2,6-Dichloropyridin-4-yl)methyl (S)-2-amino-3,3-dimethylbutanoate hydrochloride |
| | LC-MS (ESI) *m*/*z* [M+H]⁺: 291.03 |
| | tR 3.180 min (B) |
| | (S)-2-((tert-Butoxycarbonyl)amino)-3,3-dimethylbutanoic acid (62965-35-9) |
| | 4-(Bromomethyl)-2,6-dichloropyridine (175204-45-2) |
| | 4N-HCl/MTHP |
| 1-1-40 | 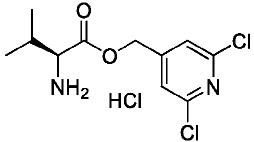 |
| | (2,6-Dichloropyridin-4-yl)methyl L-valinate hydrochloride |
| | LC-MS (ESI) *m*/*z* [M+H]⁺: 276.99 |
| | tR 2.964 min (B) |
| | (tert-Butoxycarbonyl)-L-valine (13734-41-3) |
| | 4-(Bromomethyl)-2,6-dichloropyridine (175204-45-2) |
| | 4N-HCl/MTHP |

**[Table 1-21]**

| | |
|---|---|
| 1-1-41 | 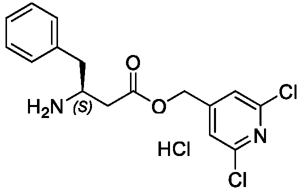 |
| | (2,6-Dichloropyridin-4-yl)methyl (S)-3-amino-4-phenylbutanoate hydrochloride |
| | LC-MS (ESI) *m*/*z* [M+H]⁺: 338.92 |
| | tR 3.576 min (B) |
| | (S)-3-((tert-Butoxycarbonyl)amino)-4-phenylbutanoic acid (51871-62-6) |
| | 4-(Bromomethyl)-2,6-dichloropyridine (175204-45-2) |
| | 4N-HCl/MTHP |
| 1-1-42 | 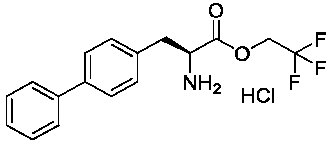 |
| | 2,2,2-Trifluoroethyl (S)-3-((1,1'-biphenyl)-4-yl)-2-aminopropanoate hydrochloride |
| | LC-MS (ESI) *m*/*z* [M+H]⁺: 324.02 |
| | tR 3.936 min (B) |
| | (S)-3-((1,1'-Biphenyl)-4-yl)-2-((tert-butoxycarbonyl)amino)propanoic acid (147923-08-8) |
| | 2,2,2-Trifluoroethyl trifluoromethanesulfonate (6226-25-1) |
| | 4N-HCl/MTHP |

**[Table 1-22]**

| | |
|---|---|
| 1-1-43 | 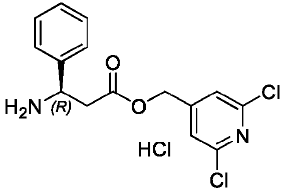 |
| | (2,6-Dichloropyridin-4-yl)methyl (R)-3-amino-3-phenylpropanoate hydrochloride |
| | LC-MS (ESI) *m*/*z* [M+H]⁺: 326.92 |
| | tR 3.412 min (B) |
| | (R)-3-((tert-Butoxycarbonyl)amino)-3-phenylpropanoic acid (161024-80-2) |
| | 4-(Bromomethyl)-2,6-dichloropyridine (175204-45-2) |
| | 4N-HCl/MTHP |
| 1-1-44 | 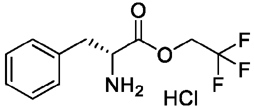 |
| | 2,2,2-Trifluoroethyl D-phenylalaninate hydrochloride |
| | LC-MS (ESI) *m*/*z* [M+H]⁺: 248.07 |
| | tR 2.820 min (B) |
| | (tert-Butoxycarbonyl)-D-phenylalanine (18942-49-9) |
| | 2,2,2- Trifluoroethyl trifluoromethanesulfonate (6226-25-1) |
| | 4N-HCl/MTHP |

**[Table 1-23]**

| | |
|---|---|
| 1-1-45 | 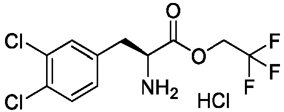 |
| | 2,2,2-Trifluoroethyl (S)-2-amino-3-(3,4-dichlorophenyl)propanoate hydrochloride |
| | LC-MS (ESI) *m*/*z* [M+H]⁺: 315.89 |
| | tR 3.660 min (B) |
| | (S)-2-((tert-Butoxycarbonyl)amino)-3-(3,4-dichlorophenyl)propanoic acid (80741-39-5) |
| | 2,2,2-Trifluoroethyl trifluoromethanesulfonate (6226-25-1) |
| | 4N-HCl/MTHP |
| 1-1-46 | 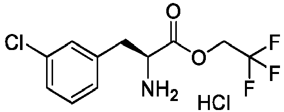 |
| | 2,2,2-Trifluoroethyl (S)-2-amino-3-(3-chlorophenyl)propanoate hydrochloride |
| | LC-MS (ESI) *m*/*z* [M+H]⁺: 281.94 |
| | tR 3.344 min (B) |
| | (S)-2-((tert-Butoxycarbonyl)amino)-3-(3-chlorophenyl)propanoic acid (114873-03-9) |
| | 2,2,2-Trifluoroethyl trifluoromethanesulfonate (6226-25-1) |
| | 4N-HCl/MTHP |

**[Table 1-24]**

| | |
|---|---|
| 1-1-47 | 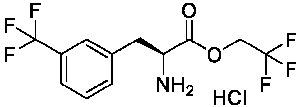 |
| | 2,2,2-Trifluoroethyl (S)-2-amino-3-(3-(trifluoromethyl)phenyl)propanoate hydrochloride |
| | LC-MS (ESI) *m*/*z* [M+H]⁺: 315.97 |
| | tR 3.596 min (B) |
| | (S)-2-((tert-Butoxycarbonyl)amino)-3-(3-(trifluoromethyl)phenyl)propanoic acid (142995-31-1) |
| | 2,2,2-Trifluoroethyl trifluoromethanesulfonate (6226-25-1) |
| | 4N-HCl/MTHP |
| 1-1-48 | 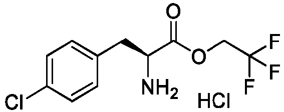 |
| | 2,2,2-Trifluoroethyl (S)-2-amino-3-(4-chlorophenyl)propanoate hydrochloride |
| | LC-MS (ESI) *m*/*z* [M+H]⁺: 282.00 |
| | tR 5.428 min (A) |
| | (S)-2-((tert-Butoxycarbonyl)amino)-3-(4-chlorophenyl)propanoic acid (68090-88-0) |
| | 2,2,2-Trifluoroethyl trifluoromethanesulfonate (6226-25-1) |
| | 4N-HCl/MTHP |

**[Table 1-25]**

| | |
|---|---|
| 1-1-49 | 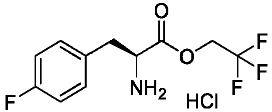 |
| | 2,2,2-Trifluoroethyl (S)-2-amino-3-(4-fluorophenyl)propanoate hydrochloride |
| | LC-MS (ESI) *m*/*z* [M+H]⁺: 266.05 |
| | tR 2.876 min (B) |
| | (S)-2-((tert-Butoxycarbonyl)amino)-3-(4-fluorophenyl)propanoic acid (41153-30-4) |
| | 2,2,2-Trifluoroethyl trifluoromethanesulfonate (6226-25-1) |
| | 4N-HCl/MTHP |
| 1-1-50 | 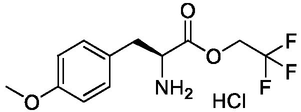 |
| | 2,2,2-Trifluoroethyl (S)-2-amino-3-(4-methoxyphenyl)propanoate hydrochloride |
| | LC-MS (ESI) *m*/*z* [M+H]⁺: 278.09 |
| | tR 4.744 min (A) |
| | (S)-2-((tert-Butoxycarbonyl)amino)-3-(4-methoxyphenyl)propanoic acid (53267-93-9) |
| | 2,2,2-Trifluoroethyl trifluoromethanesulfonate (6226-25-1) |
| | 4N-HCl/MTHP |

**[Table 1-26]**

| | |
|---|---|
| 1-1-51 | 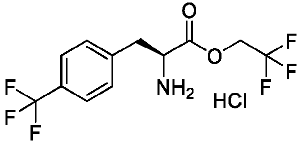 |
| | 2,2,2-Trifluoroethyl (S)-2-amino-3-(4-(trifluoromethyl)phenyl)propanoate hydrochloride |
| | LC-MS (ESI) *m*/*z* [M+H]⁺: 315.94 |
| | tR 3.616 min (B) |
| | (S)-2-((tert-Butoxycarbonyl)amino)-3-(4-(trifluoromethyl)phenyl)propanoic acid (114873-07-3) |
| | 2,2,2-Trifluoroethyl trifluoromethanesulfonate (6226-25-1) |
| | 4N-HCl/MTHP |
| 1-1-52 | 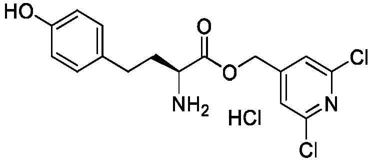 |
| | (2,6-Dichloropyridin-4-yl)methyl (S)-2-amino-4-(4-hydroxyphenyl)butanoate hydrochloride |
| | LC-MS (ESI) *m*/*z* [M+H]⁺: 356.92 |
| | tR 3.108 min (B) |
| | (R)-2-((tert-Butoxycarbonyl)amino)-4-(4-hydroxyphenyl)butanoic acid (198473-94-8) |
| | 4-(Bromomethyl)-2,6-dichloropyridine (175204-45-2) |
| | 4N-HCl/CPME |

**[Table 1-27]**

| | |
|---|---|
| 1-1-53 | 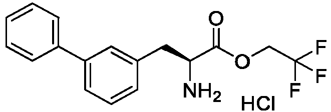 |
| | 2,2,2-Trifluoroethyl (S)-3-((1,1'-biphenyl)-3-yl)-2-aminopropanoate hydrochloride |
| | LC-MS (ESI) *m*/*z* [M+H]⁺: 324.00 |
| | tR 3.920 min (B) |
| | (S)-3-((1,1'-Biphenyl)-3-yl)-2-((tert-butoxycarbonyl)amino)propanoic acid (608528-91-2) |
| | 2,2,2-Trifluoroethyl trifluoromethanesulfonate (6226-25-1) |
| | 4N-HCl/MTHP |
| 1-1-54 | 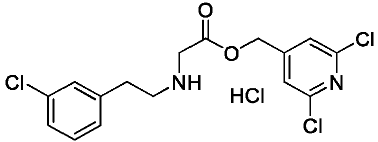 |
| | (2,6-Dichloropyridin-4-yl)methyl (3-chlorophenethyl)glycinate hydrochloride |
| | LC-MS (ESI) *m*/*z* [M+H]⁺: 372.89 |
| | tR 3.876 min (B) |
| | N-(tert-Butoxycarbonyl)-N-(3-chlorophenethyl)glycine (M03771, WATANABE CHEMICAL INDUSTRIES, LTD.) |
| | 4-(Bromomethyl)-2,6-dichloropyridine (175204-45-2) |
| | 4N-HCl/CPME |

**[Table 1-28]**

| | |
|---|---|
| 1-1-55 | 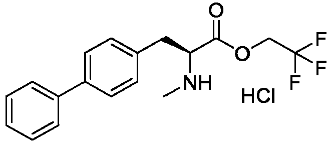 |
| | 2,2,2-Trifluoroethyl (S)-3-((1,1'-biphenyl)-4-yl)-2-(methylamino)propanoate hydrochloride |
| | LC-MS (ESI) *m*/*z* [M+H]⁺: 337.99 |
| | tR 4.012 min (B) |
| | (S)-3-([1,1'-Biphenyl]-4-yl)-2-((tert-butoxycarbonyl)(methyl)amino)propanoic acid (2050910-29-5) |
| | 2,2,2-Trifluoroethyl trifluoromethanesulfonate (6226-25-1) |
| | 4N-HCl/MTHP |
| 1-1-56 | 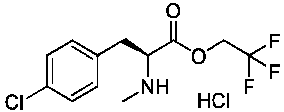 |
| | 2,2,2-Trifluoroethyl (S)-3-(4-chlorophenyl)-2-(methylamino)propanoate hydrochloride |
| | LC-MS (ESI) *m*/*z* [M+H]⁺: 296.06 |
| | tR 3.308 min (B) |
| | (S)-2-((tert-Butoxycarbonyl)(methyl)amino)-3-(4-chlorophenyl)propanoic acid (179033-68-2) |
| | 2,2,2-Trifluoroethyl trifluoromethanesulfonate (6226-25-1) |
| | 4N-HCl/MTHP |

**[Table 1-29]**

| | |
|---|---|
| 1-1-57 | 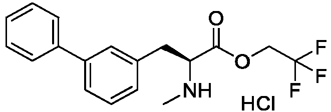 |
| | 2,2,2-Trifluoroethyl (S)-3-((1,1'-biphenyl)-3-yl)-2-(methylamino)propanoate hydrochloride |
| | LC-MS (ESI) *m*/*z* [M+H]⁺: 338.09 |
| | tR 3.872 min (B) |
| | (S)-3-((1,1'-Biphenyl)-3-yl)-2-((tert-butoxycarbonyl)(methyl)amino)propanoic acid (M03659, WATANABE CHEMICAL INDUSTRIES, LTD.) |
| | 2,2,2-Trifluoroethyl trifluoromethanesulfonate (6226-25-1) |
| | 4N-HCl/MTHP |
| 1-1-58 | 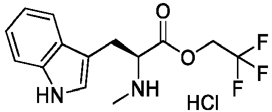 |
| | 2,2,2-Trifluoroethyl methyl-L-tryptophanate hydrochloride |
| | LC-MS (ESI) *m*/*z* [M+H]⁺: 301.03 |
| | tR 3.080 min (B) |
| | Na,1-bis(tert-Butoxycarbonyl)-Na-methyl-L-tryptophan (K22864, Mimotopes) |
| | 2,2,2-Trifluoroethyl trifluoromethanesulfonate (6226-25-1) |
| | 4N-HCl/MTHP |

**[Table 1-30]**

| | |
|---|---|
| 1-1-59 | 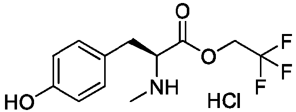 |
| | 2,2,2-Trifluoroethyl methyl-L-tyrosinate hydrochloride |
| | LC-MS (ESI) *m*/*z* [M+H]⁺: 278.07 |
| | tR 5.984 min (A) |
| | Dicyclohexylamine N-(tert-butoxycarbonyl)-N-methyl-L-tyrosinate (95105-25-2) |
| | 2,2,2-Trifluoroethyl trifluoromethanesulfonate (6226-25-1) |
| | 4N-HCl/MTHP |
| 1-1-60 | 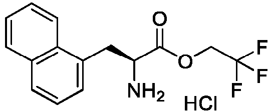 |
| | 2,2,2-Trifluoroethyl (S)-2-amino-3-(naphthalen-1-yl)propanoate hydrochloride |
| | LC-MS (ESI) *m*/*z* [M+H]⁺: 298.03 |
| | tR 3.468 min (B) |
| | (S)-2-((tert-Butoxycarbonyl)amino)-3-(naphthalen-1-yl)propanoic acid (55447-00-2) |
| | 2,2,2-Trifluoroethyl trifluoromethanesulfonate (6226-25-1) |
| | 4N-HCl/MTHP |

**[Table 1-31]**

| | |
|---|---|
| 1-1-61 | 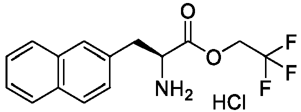 |
| | 2,2,2-Trifluoroethyl (S)-2-amino-3-(naphthalen-2-yl)propanoate hydrochloride |
| | LC-MS (ESI) *m*/*z* [M+H]⁺: 298.03 |
| | tR 3.512 min (B) |
| | (S)-2-((tert-Butoxycarbonyl)amino)-3-(naphthalen-2-yl)propanoic acid (58438-04-3) |
| | 2,2,2-Trifluoroethyl trifluoromethanesulfonate (6226-25-1) |
| | 4N-HCl/MTHP |
| 1-1-62 | 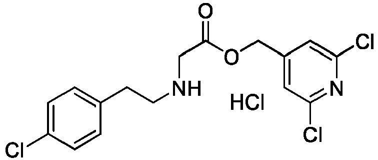 |
| | (2,6-Dichloropyridin-4-yl)methyl (4-chlorophenethyl)glycinate hydrochloride |
| | LC-MS (ESI) *m*/*z* [M+H]⁺: 374.98 |
| | tR 3.776 min (B) |
| | N-(tert-Butoxycarbonyl)-N-(4-chlorophenethyl)glycine (M03784, WATANABE CHEMICAL INDUSTRIES, LTD.) |
| | 4-(Bromomethyl)-2,6-dichloropyridine (175204-45-2) |
| | 4N-HCl/CPME |

**[Table 1-32]**

| | |
|---|---|
| 1-1-63 | 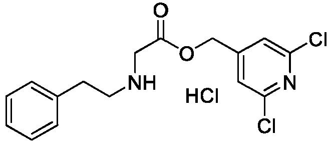 |
| | (2,6-Dichloropyridin-4-yl)methyl phenethylglycinate hydrochloride |
| | LC-MS (ESI) *m*/*z* [M+H]⁺: 338.99 |
| | tR 3.448 min (B) |
| | N-(tert-Butoxycarbonyl)-N-phenethylglycine (172834-25-2) |
| | 4-(Bromomethyl)-2,6-dichloropyridine (175204-45-2) |
| | 4N-HCl/MTHP |
| 1-1-64 | 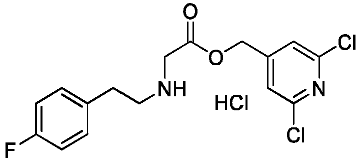 |
| | (2,6-Dichloropyridin-4-yl)methyl (4-fluorophenethyl)glycinate hydrochloride |
| | LC-MS (ESI) *m*/*z* [M+H]⁺: 357.00 |
| | tR 3.524 min (B) |
| | N-(tert-Butoxycarbonyl)-N-(4-fluorophenethyl)glycine (M03782, WATANABE CHEMICAL INDUSTRIES, LTD.) |
| | 4-(Bromomethyl)-2,6-dichloropyridine (175204-45-2) |
| | 4N-HCl/MTHP |
| 1-1-65 | 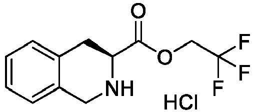 |
| | 2,2,2-Trifluoroethyl (S)-1,2,3,4-tetrahydroisoquinoline-3-carboxylate hydrochloride |
| | LC-MS (ESI) *m*/*z* [M+H]⁺: 260.03 |
| | tR 4.344 min (A) |
| | (S)-2-(tert-Butoxycarbonyl)-1,2,3,4-tetrahydroisoquinoline-3-carboxylic acid (78879-20-6) |
| | 2,2,2-Trifluoroethyl trifluoromethanesulfonate (6226-25-1) |
| | 4N-HCl/MTHP |

**[Table 1-33]**

| | |
|---|---|
| 1-1-66 | 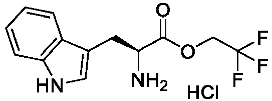 |
| | 2,2,2-Trifluoroethyl L-tryptophanate hydrochloride |
| | LC-MS (ESI) *m*/*z* [M+H]⁺: 287.04 |
| | tR 3.032 min (B) |
| | (tert-Butoxycarbonyl)-L-tryptophan (13139-14-5) |
| | 2,2,2-Trifluoroethyl trifluoromethanesulfonate (6226-25-1) |
| | 4N-HCl/MTHP |
| 1-1-67 | 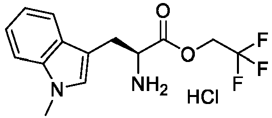 |
| | 2,2,2-Trifluoroethyl 1-methyl-L-tryptophanate hydrochloride |
| | LC-MS (ESI) *m*/*z* [M+H]⁺: 300.99 |
| | tR 3.348 min (B) |
| | Na-(tert-Butoxycarbonyl)-1-methyl-L-tryptophan (109927-44-8) |
| | 2,2,2- Trifluoroethyl trifluoromethanesulfonate (6226-25-1) |
| | 4N-HCl/MTHP |
| 1-1-68 | 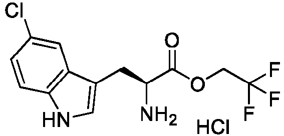 |
| | 2,2,2-Trifluoroethyl (S)-2-amino-3-(5-chloro-1H-indol-3-yl)propanoate hydrochloride |
| | LC-MS (ESI) *m*/*z* [M+H]⁺: 320.94 |
| | tR 3.544 min (B) |
| | (S)-2-((tert-Butoxycarbonyl)amino)-3-(5-chloro-1H-indol-3-yl)propanoic acid (114873-08-4) |
| | 2,2,2-Trifluoroethyl trifluoromethanesulfonate (6226-25-1) |
| | 4N-HCl/MTHP |

**[Table 1-34]**

| | |
|---|---|
| 1-1-69 | 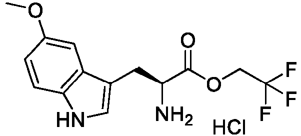 |
| | 2,2,2-Trifluoroethyl (S)-2-amino-3-(5-methoxy-1H-indol-3-yl)propanoate hydrochloride |
| | LC-MS (ESI) *m*/*z* [M+H]⁺: 317.29 |
| | tR 3.136 min (B) |
| | (S)-2-((tert-Butoxycarbonyl)amino)-3-(5-methoxy-1H-indol-3-yl)propanoic acid (114903-30-9) |
| | 2,2,2-Trifluoroethyl trifluoromethanesulfonate (6226-25-1) |
| | 4N-HCl/MTHP |
| 1-1-70 | 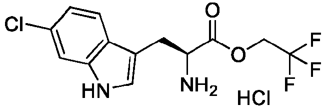 |
| | 2,2,2-Trifluoroethyl (S)-2-amino-3-(6-chloro-1H-indol-3-yl)propanoate hydrochloride |
| | LC-MS (ESI) *m*/*z* [M+H]⁺: 320.94 |
| | tR 3.548 min (B) |
| | (S)-2-((tert-Butoxycarbonyl)amino)-3-(6-chloro-1H-indol-3-yl)propanoic acid (1234875-52-5) |
| | 2,2,2-Trifluoroethyl trifluoromethanesulfonate (6226-25-1) |
| | 4N-HCl/MTHP |

**[Table 1-35]**

| | |
|---|---|
| 1-1-71 | 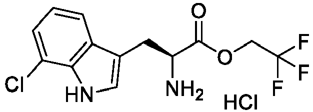 |
| | 2,2,2-Trifluoroethyl (S)-2-amino-3-(7-chloro-1H-indol-3-yl)propanoate hydrochloride |
| | LC-MS (ESI) *m*/*z* [M+H]⁺: 320.97 |
| | tR 3.516 min (B) |
| | (S)-2-((tert-Butoxycarbonyl)amino)-3-(7-chloro-1H-indol-3-yl)propanoic acid (2306826-87-7) |
| | 2,2,2-Trifluoroethyl trifluoromethanesulfonate (6226-25-1) |
| | 4N-HCl/MTHP |
| 1-1-72 | 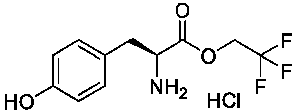 |
| | 2,2,2-Trifluoroethyl L-tyrosinate hydrochloride |
| | LC-MS (ESI) *m*/*z* [M+H]⁺: 264.04 |
| | tR 2.332 min (B) |
| | (S)-3-(4-(tert-Butoxy)phenyl)-2-((tert-butoxycarbonyl)amino)propanoic acid (47375-34-8) |
| | 2,2,2-Trifluoroethyl trifluoromethanesulfonate (6226-25-1) |
| | 4N-HCl/MTHP |

**[Table 1-36]**

| | |
|---|---|
| 1-1-73 | 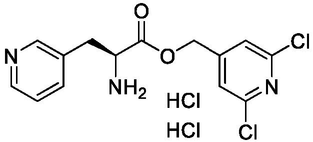 |
| | (2,6-Dichloropyridin-4-yl)methyl (S)-2-amino-3-(pyridin-3-yl)propanoate dihydrochloride |
| | LC-MS (ESI) *m*/*z* [M+H]⁺: 325.97 |
| | tR 3.280 min (A) |
| | (S)-2-((tert-Butoxycarbonyl)amino)-3-(pyridin-3-yl)propanoic acid (117142-26-4) |
| | 4-(Bromomethyl)-2,6-dichloropyridine (175204-45-2) |
| | 4N-HCl/MTHP |
| 1-1-74 | 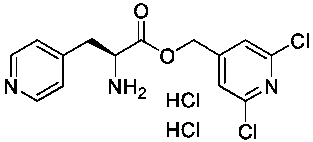 |
| | (2,6-Dichloropyridin-4-yl)methyl (S)-2-amino-3-(pyridin-4-yl)propanoate dihydrochloride |
| | LC-MS (ESI) *m*/*z* [M+H]⁺: 325.96 |
| | tR 2.760 min (A) |
| | (S)-2-((tert-Butoxycarbonyl)amino)-3-(pyridin-4-yl)propanoic acid (37535-57-2) |
| | 4-(Bromomethyl)-2,6-dichloropyridine (175204-45-2) |
| | 4N-HCl/MTHP |

**[Table 1-37]**

| | |
|---|---|
| 1-1-75 | 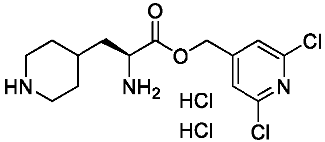 |
| | (2,6-Dichloropyridin-4-yl)methyl (S)-2-amino-3-(piperidin-4-yl)propanoate dihydrochloride |
| | LC-MS (ESI) *m*/*z* [M+H]⁺: 332.01 |
| | tR 1.952 min (A) |
| | (S)-2-((tert-Butoxycarbonyl)amino)-3-(1-(tert-butoxycarbonyl)piperidin-4-yl)propanoic acid (483369-18-2) |
| | 4-(Bromomethyl)-2,6-dichloropyridine (175204-45-2) |
| | 4N-HCl/MTHP |
| 1-1-76 | 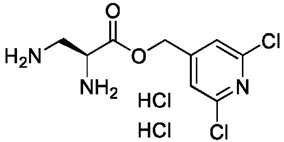 |
| | (2,6-Dichloropyridin-4-yl)methyl (S)-2,3-diaminopropanoate dihydrochloride |
| | LC-MS (ESI) *m*/*z* [M+H]⁺: 263.94 |
| | tR 2.636 min (B) |
| | (S)-2,3-bis((tert-Butoxycarbonyl)amino)propanoic acid (88971-40-8) |
| | 4-(Bromomethyl)-2,6-dichloropyridine (175204-45-2) |
| | 4N-HCl/CPME |

**[Table 1-38]**

| | |
|---|---|
| 1-1-77 | 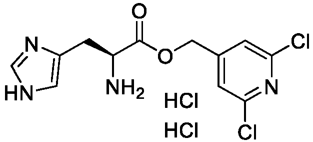 |
| | (2,6-Dichloropyridin-4-yl)methyl L-histidinate dihydrochloride |
| | LC-MS (ESI) *m*/*z* [M+H]⁺: 314.98 |
| | tR 1.812 min (A) |
| | Na,Nt-bis(tert-Butoxycarbonyl)-L-histidine (20866-46-0) |
| | 4-(Bromomethyl)-2,6-dichloropyridine (175204-45-2) |
| | 4N-HCl/CPME |
| 1-1-78 | 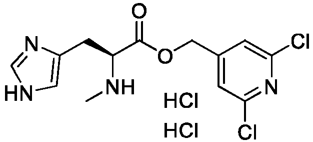 |
| | (2,6-Dichloropyridin-4-yl)methyl L-histidinate dihydrochloride |
| | LC-MS (ESI) *m*/*z* [M+H]⁺: 329.02 |
| | tR 2.136 min (A) |
| | Na-(tert-Butoxycarbonyl)-Na-methyl-Nt-trityl-L-histidine (1217610-35-9) |
| | 4-(Bromomethyl)-2,6-dichloropyridine (175204-45-2) |
| | 2,2,2-Trifluoroacetic acid (76-05-1) and triisopropylsilane (6485-79-6) |
| 1-1-79 | 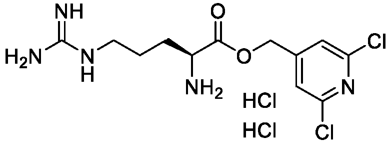 |
| | (2,6-Dichloropyridin-4-yl)methyl L-argininate dihydrochloride |
| | LC-MS (ESI) *m*/*z* [M+H]⁺: 334.02 |
| | tR 2.624 min (A) |
| | N2-(tert-Butoxycarbonyl)-Nw-((2,2,4,6,7-pentamethyl-2,3-dihydrobenzofuran-5-yl)sulfonyl)-L-arginine (200124-22-7) |
| | 4-(Bromomethyl)-2,6-dichloropyridine (175204-45-2) |
| | 2,2,2-Trifluoroacetic acid (76-05-1) |

**[Table 1-39]**

| | |
|---|---|
| 1-1-80 | 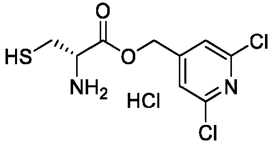 |
| | (2,6-Dichloropyridin-4-yl)methyl D-cysteinate hydrochloride |
| | LC-MS (ESI) *m*/*z* [M+H]⁺: 280.95 |
| | tR 2.812 min (B) |
| | N-(tert-Butoxycarbonyl)-S-trityl-D-cysteine (87494-13-1) |
| | 4-(Bromomethyl)-2,6-dichloropyridine (175204-45-2) |
| | 2,2,2-Trifluoroacetic acid (76-05-1) and triisopropylsilane (6485-79-6) |
| 1-1-81 | 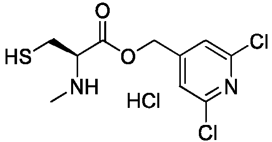 |
| | (2,6-Dichloropyridin-4-yl)methyl methyl-L-cysteinate hydrochloride |
| | LC-MS (ESI) *m*/*z* [M+H]⁺: 294.98 |
| | tR 4.292 min (A) |
| | N-(tert-Butoxycarbonyl)-N-methyl-S-trityl-L-cysteine (91292-54-5) |
| | 4-(Bromomethyl)-2,6-dichloropyridine (175204-45-2) |
| | 2,2,2-Trifluoroacetic acid (76-05-1) and triisopropylsilane (6485-79-6) |

**[Table 1-40]**

| | |
|---|---|
| 1-1-82 | 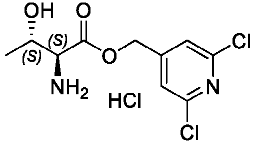 |
| | (2,6-Dichloropyridin-4-yl)methyl L-allothreoninate hydrochloride |
| | LC-MS (ESI) *m*/*z* [M+H]⁺: 278.93 |
| | tR 2.432 min (B) |
| | N-(tert-Butoxycarbonyl)-O-(tert-butyl)-L-allothreoninate dicyclohexylamine salt (M01520, WATANABE CHEMICAL INDUSTRIES, LTD.) |
| | 4-(Bromomethyl)-2,6-dichloropyridine (175204-45-2) |
| | 4N-HCl/MTHP |
| 1-1-83 | 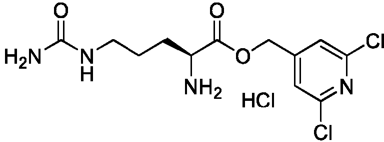 |
| | (2,6-Dichloropyridin-4-yl)methyl (S)-2-amino-5-ureidopentanoate hydrochloride |
| | LC-MS (ESI) *m*/*z* [M+H]⁺: 335.04 |
| | tR 3.576 min (A) |
| | (S)-2-((tert-Butoxycarbonyl)amino)-5-ureidopentanoic acid (45234-13-7) |
| | 4-(Bromomethyl)-2,6-dichloropyridine (175204-45-2) |
| | 4N-HCl/MTHP |

**[Table 1-41]**

| | |
|---|---|
| 1-1-84 | 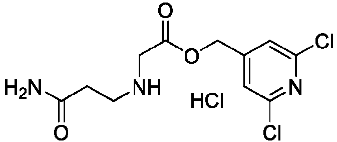 |
| | (2,6-Dichloropyridin-4-yl)methyl (3-amino-3-oxopropyl)glycinate hydrochloride |
| | LC-MS (ESI) *m*/*z* [M+H]⁺: 306.02 |
| | tR 3.184 min (A) |
| | N-(tert-Butoxycarbonyl)-N-(3-oxo-3-(tritylamino)propyl)glycine (M03777, WATANABE CHEMICAL INDUSTRIES, LTD.) |
| | 4-(Bromomethyl)-2,6-dichloropyridine (175204-45-2) |
| | 2,2,2-Trifluoroacetic acid (76-05-1) and triisopropylsilane (6485-79-6) |
| 1-1-85 | 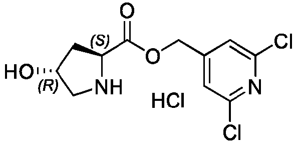 |
| | (2,6-Dichloropyridin-4-yl)methyl (2S,4R)-4-hydroxypyrrolidine-2-carboxylate hydrochloride |
| | LC-MS (ESI) *m*/*z* [M+H]⁺: 290.93 |
| | tR 2.284 min (B) |
| | (2S,4R)-4-(tert-Butoxy)-1-(tert-butoxycarbonyl)pyrrolidine-2-carboxylic acid (148983-07-7) |
| | 4-(Bromomethyl)-2,6-dichloropyridine (175204-45-2) |
| | 4N-HCl/MTHP |

**[Table 1-42]**

| | |
|---|---|
| 1-1-86 | 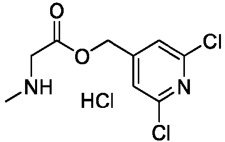 |
| | (2,6-Dichloropyridin-4-yl)methyl N-methylglycinate hydrochloride |
| | LC-MS (ESI) *m*/*z* [M+H]⁺: 249.06 |
| | tR 2.220 min (B) |
| | N-(tert-Butoxycarbonyl)-N-methylglycine (13734-36-6) |
| | 4-(Bromomethyl)-2,6-dichloropyridine (175204-45-2) |
| | 4N-HCl/CPME |
| 1-1-87 | 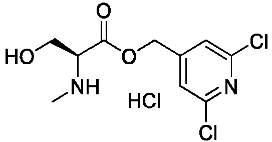 |
| | (2,6-Dichloropyridin-4-yl)methyl N-methyl-L-serinate hydrochloride |
| | LC-MS (ESI) *m*/*z* [M+H]⁺: 278.99 |
| | tR 2.180 min (B) |
| | N-(tert-Butoxycarbonyl)-O-(tert-butyl)-L-serine (13734-38-8) |
| | 4-(Bromomethyl)-2,6-dichloropyridine (175204-45-2) |
| | 4N-HCl/MTHP |
| 1-1-88 | 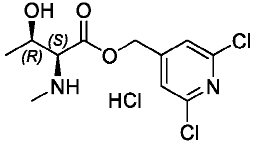 |
| | (2,6-Dichloropyridin-4-yl)methyl methyl-L-threoninate hydrochloride |
| | LC-MS (ESI) *m*/*z* [M+H]⁺: 293.02 |
| | tR 2.552 min (B) |
| | N-(tert-Butoxycarbonyl)-O-(tert-butyl)-N-methyl-L-threonine (2568486-04-2) |
| | 4-(Bromomethyl)-2,6-dichloropyridine (175204-45-2) |
| | 4N-HCl/MTHP |

**[Table 1-43]**

| | |
|---|---|
| 1-1-89 | 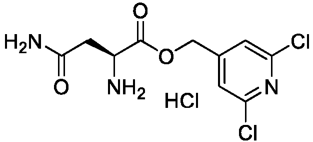 |
| | (2,6-Dichloropyridin-4-yl)methyl L-asparaginate hydrochloride |
| | LC-MS (ESI) *m*/*z* [M+H]⁺: 291.92 |
| | tR 3.244 min (A) |
| | N2-(tert-Butoxycarbonyl)-N4-trityl-L-asparagine (132388-68-2) |
| | 4-(Bromomethyl)-2,6-dichloropyridine (175204-45-2) |
| | 2,2,2-Trifluoroacetic acid (76-05-1) and triisopropylsilane (6485-79-6) |
| 1-1-90 | 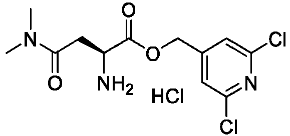 |
| | (2,6-Dichloropyridin-4-yl)methyl N4,N4-dimethyl-L-asparaginate hydrochloride |
| | LC-MS (ESI) *m*/*z* [M+H]⁺: 320.00 |
| | tR 4.232 min (A) |
| | N2-(tert-Butoxycarbonyl)-N4,N4-dimethyl-L-asparagine (70232-19-8) |
| | 4-(Bromomethyl)-2,6-dichloropyridine (175204-45-2) |
| | 4N-HCl/MTHP |
| 1-1-91 | 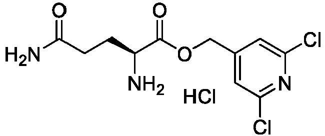 |
| | (2,6-Dichloropyridin-4-yl)methyl L-glutamate hydrochloride |
| | LC-MS (ESI) *m*/*z* [M+H]⁺: 306.02 |
| | tR 3.176 min (B) |
| | N2-(tert-Butoxycarbonyl)-N5-trityl-L-glutamine (132388-69-3) |
| | 4-(Bromomethyl)-2,6-dichloropyridine (175204-45-2) |
| | 2,2,2-Trifluoroacetic acid (76-05-1) |

**[Table 1-44]**

| | |
|---|---|
| 1-1-92 | 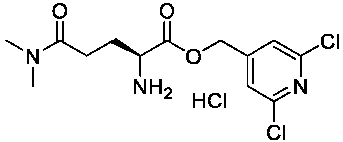 |
| | (2,6-Dichloropyridin-4-yl)methyl N5,N5-dimethyl-L-glutamate hydrochloride |
| | LC-MS (ESI) *m*/*z* [M+H]⁺: 334.03 |
| | tR 4.428 min (A) |
| | N2-(tert-Butoxycarbonyl)-N5,N5-dimethyl-L-glutamine (72449-42-4) |
| | 4-(Bromomethyl)-2,6-dichloropyridine (175204-45-2) |
| | 4N-HCl/MTHP |
| 1-1-93 | 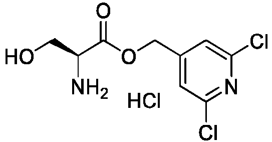 |
| | (2,6-Dichloropyridin-4-yl)methyl-L-serinate hydrochloride |
| | LC-MS (ESI) *m*/*z* [M+H]⁺: 264.95 |
| | tR 2.936 min (B) |
| | N-(tert-Butoxycarbonyl)-O-(tert-butyl)-L-serine (13734-38-8) |
| | 4-(Bromomethyl)-2,6-dichloropyridine (175204-45-2) |
| | 2,2,2-Trifluoroacetic acid (CAS RN: 76-05-1) |

**[Table 1-45]**

| | |
|---|---|
| 1-1-94 | 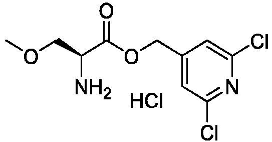 |
| | (2,6-Dichloropyridin-4-yl)methyl O-methyl-L-serinate hydrochloride |
| | LC-MS (ESI) *m*/*z* [M+H]⁺: 278.98 |
| | tR 4.092 min (A) |
| | N-(tert-Butoxycarbonyl)-O-methyl-L-serine (51293-47-1) |
| | 4-(Bromomethyl)-2,6-dichloropyridine (175204-45-2) |
| | 4N-HCl/MTHP |
| 1-1-95 | 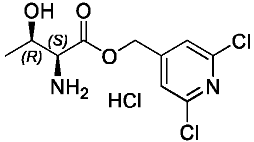 |
| | (2,6-Dichloropyridin-4-yl)methyl-L-threoninate hydrochloride |
| | LC-MS (ESI) *m*/*z* [M+H]⁺: 278.98 |
| | tR 3.096 min (B) |
| | N-(tert-Butoxycarbonyl)-O-(tert-butyl)-L-threonine (13734-40-2) |
| | 4-(Bromomethyl)-2,6-dichloropyridine (175204-45-2) |
| | 2,2,2-Trifluoroacetic acid (76-05-1) |

**[Table 1-46]**

| | |
|---|---|
| Example No. | Structure |
| | Compound name |
| | LC/MS analysis results |
| | tR (LC/MS analysis condition) |
| | Starting amino acid (CAS Registry Number) |
| | Esterification reagent (CAS Registry Number) |
| | Deprotection reagent |
| 1-1-96 | 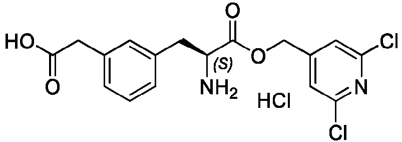 |
| | (S)-2-(3-(2-Amino-3-((2,6-dichloropyridin-4-yl)methoxy)-3-oxopropyl)phenyl)acetic acid hydrochloride |
| | LC-MS (ESI) m/z [M+H]⁺: 382.98 |
| | tR 3.144 min (B) |
| | (S)-3-(3-(2-(tert-Butoxy)-2-oxoethyl)phenyl)-2-((tert-butoxycarbonyl)amino)propanoic acid (K26210, Wuxi Asiapeptide Biotechnology Co. Ltd.) |
| | 4-(Bromomethyl)-2,6-dichloropyridine (175204-45-2) |
| | 4M-HCl/MTHP and 2,2,2-Trifluoroacetic acid |
| 1-1-97 | 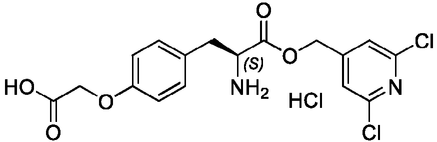 |
| | (S)-2-(4-(2-Amino-3-((2,6-dichloropyridin-4-yl)methoxy)-3-oxopropyl)phenoxy)acetic acid hydrochloride |
| | LC-MS (ESI) m/z [M+H]⁺: 398.97 |
| | tR 3.036 min (B) |
| | (S)-3-(4-(2-(tert-Butoxy)-2-oxoethoxy)phenyl)-2-((tert-butoxycarbonyl)amino)propanoic acid (2131098-10-5) |
| | 4-(Bromomethyl)-2,6-dichloropyridine (175204-45-2) |
| | 4M-HCl/MTHP |

**[Table 1-47]**

| | |
|---|---|
| 1-1-98 | 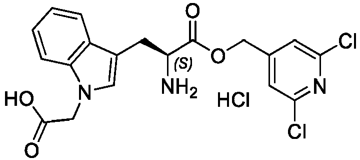 |
| | (S)-2-(3-(2-Amino-3-((2,6-dichloropyridin-4-yl)methoxy)-3-oxopropyl)-1H-indol-1-yl)acetic acid hydrochloride |
| | LC-MS (ESI) m/z [M+H]⁺: 422.02 |
| | tR 3.536 min (B) |
| | 1-(2-(tert-Butoxy)-2-oxoethyl)-N^{a}-(tert-butoxycarbonyl)-L-tryptophan (1629658-35-0) |
| | 4-(Bromomethyl)-2,6-dichloropyridine (175204-45-2) |
| | 4M-HCl/MTHP and 2,2,2-Trifluoroacetic acid |
| 1-1-99 | 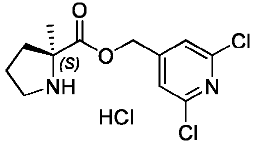 |
| | (2,6-Dichloropyridin-4-yl)methyl (S)-2-methylpyrrolidine-2-carboxylate hydrochloride |
| | LC-MS (ESI) m/z [M+H]⁺: 289.00 |
| | tR 4.448 min (A) |
| | (S)-1-(tert-Butoxycarbonyl)-2-(tert-butoxycarbonyl)-2-methylpyrrolidine-2-carboxylic acid (103336-06-7) |
| | 4-(Bromomethyl)-2,6-dichloropyridine (175204-45-2) |
| | 4M-HCl/MTHP |
| 1-1-100 | 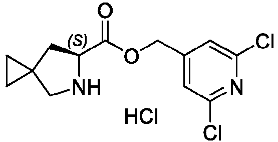 |
| | (2,6-Dichloropyridin-4-yl)methyl (S)-5-azaspiro[2.4]heptane-6-carboxylate hydrochloride |
| | LC-MS (ESI) m/z [M+H]⁺: 301.03 |
| | tR 4.848 min (A) |
| | (S)-5-(tert-Butoxycarbonyl)-5-azaspiro[2.4]heptane-6-carboxylic acid (1129634-44-1) |
| | 4-(Bromomethyl)-2,6-dichloropyridine (175204-45-2) |
| | 4M-HCl/MTHP |

**[Table 1-48]**

| | |
|---|---|
| 1-1-101 | 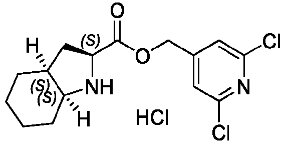 |
| | (2,6-Dichloropyridin-4-yl)methyl (2S,3aS,7aS)-octahydro-1H-indole-2-carboxylate hydrochloride |
| | LC-MS (ESI) m/z [M+H]⁺: 329.03 |
| | tR 3.388 min (B) |
| | (2S,3aS,7aS)-1-(tert-Butoxycarbonyl)octahydro-1H-indole-2-carboxylic acid (109523-13-9) |
| | 4-(Bromomethyl)-2,6-dichloropyridine (175204-45-2) |
| | 4M-HCl/MTHP |
| 1-1-102 | 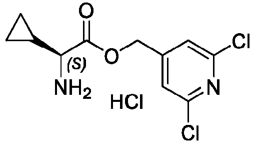 |
| | (2,6-Dichloropyridin-4-yl)methyl (S)-2-amino-2-cyclopropyl acetate hydrochloride |
| | LC-MS (ESI) m/z [M+H]⁺: 274.95 |
| | tR 4.160 min (A) |
| | (S)-2-((tert-Butoxycarbonyl)amino)-2-cyclopropylacetic acid (155976-13-9) |
| | 4-(Bromomethyl)-2,6-dichloropyridine (175204-45-2) |
| | 4M-HCl/MTHP |

**[Table 1-49]**

| | |
|---|---|
| 1-1-103 | 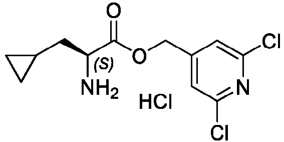 |
| | (2,6-Dichloropyridin-4-yl)methyl (S)-2-amino-3-cyclopropylpropanoate hydrochloride |
| | LC-MS (ESI) m/z [M+H]⁺: 289.02 |
| | tR 3.056 min (B) |
| | (S)-2-((tert-Butoxycarbonyl)amino)-3-cyclopropylpropanoic acid (89483-06-7) |
| | 4-(Bromomethyl)-2,6-dichloropyridine (175204-45-2) |
| | 4M-HCl/MTHP |
| 1-1-104 | 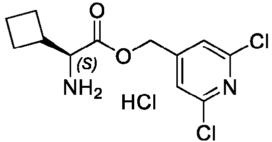 |
| | (2,6-Dichloropyridin-4-yl)methyl (S)-2-amino-2-cyclobutylacetate hydrochloride |
| | LC-MS (ESI) m/z [M+H]⁺: 288.97 |
| | tR 4.784 min (A) |
| | (S)-2-((tert-Butoxycarbonyl)amino)-2-cyclobutylacetic acid (155905-77-4) |
| | 4-(Bromomethyl)-2,6-dichloropyridine (175204-45-2) |
| | 4M-HCl/MTHP |
| 1-1-105 | 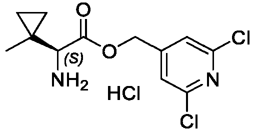 |
| | (2,6-Dichloropyridin-4-yl)methyl (S)-2-amino-2-(1-methylcyclopropyl)acetate hydrochloride |
| | LC-MS (ESI) m/z [M+H]⁺: 288.98 |
| | tR 4.920 min (A) |
| | (S)-2-((tert-Butoxycarbonyl)amino)-2-(1-methylcyclopropyl)acetic acid (928758-14-9) |
| | 4-(Bromomethyl)-2,6-dichloropyridine (175204-45-2) |
| | 4M-HCl/MTHP |

**[Table 1-50]**

| | |
|---|---|
| 1-1-106 | 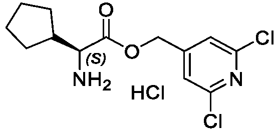 |
| | (2,6-Dichloropyridin-4-yl)methyl (S)-2-amino-2-cyclopentyl acetate hydrochloride |
| | LC-MS (ESI) m/z [M+H]⁺: 303.01 |
| | tR 3.560 min (B) |
| | (S)-2-((tert-Butoxycarbonyl)amino)-2-cyclopentylacetic acid (109183-72-4) |
| | 4-(Bromomethyl)-2,6-dichloropyridine (175204-45-2) |
| | 4M-HCl/MTHP |
| 1-1-107 | 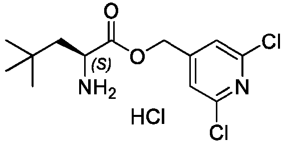 |
| | (2,6-Dichloropyridin-4-yl)methyl (S)-2-amino-4,4-dimethylpentanoate hydrochloride |
| | LC-MS (ESI) m/z [M+H]⁺: 305.02 |
| | tR 3.532 min (B) |
| | (S)-2-((tert-Butoxycarbonyl)amino)-4,4-dimethylpentanoic acid (79777-82-5) |
| | 4-(Bromomethyl)-2,6-dichloropyridine (175204-45-2) |
| | 4M-HCl/MTHP |
| 1-1-108 | 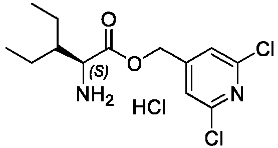 |
| | (2,6-Dichloropyridin-4-yl)methyl (S)-2-amino-3-ethylpentanoate hydrochloride |
| | LC-MS (ESI) m/z [M+H]⁺: 305.02 |
| | tR 3.524 min (B) |
| | (S)-2-((tert-Butoxycarbonyl)amino)-3-ethylpentanoic acid (35264-04-1) |
| | 4-(Bromomethyl)-2,6-dichloropyridine (175204-45-2) |
| | 4M-HCl/MTHP |

**[Table 1-51]**

| | |
|---|---|
| 1-1-109 | 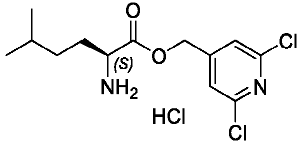 |
| | (2,6-Dichloropyridin-4-yl)methyl (S)-2-amino-5-methylhexanoate hydrochloride |
| | LC-MS (ESI) m/z [M+H]⁺: 305.00 |
| | tR 5.624 min (A) |
| | (S)-2-((tert-Butoxycarbonyl)amino)-5-methylhexanoic acid (208522-10-5) |
| | 4-(Bromomethyl)-2,6-dichloropyridine (175204-45-2) |
| | 4M-HCl/MTHP |
| 1-1-110 | 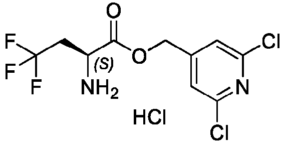 |
| | (2,6-Dichloropyridin-4-yl)methyl (S)-2-amino-4,4,4-trifluorobutanoate hydrochloride |
| | LC-MS (ESI) m/z [M+H]⁺: 316.89 |
| | tR 3.084 min (B) |
| | (S)-2-((tert-Butoxycarbonyl)amino)-4,4,4-trifluorobutanoic acid (181128-25-6) |
| | 4-(Bromomethyl)-2,6-dichloropyridine (175204-45-2) |
| | 4M-HCl/MTHP |

**[Table 1-52]**

| | |
|---|---|
| 1-1-111 | 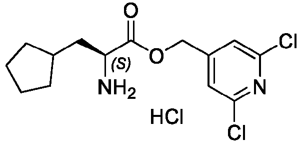 |
| | (2,6-Dichloropyridin-4-yl)methyl (S)-2-amino-3-cyclopentylpropanoate hydrochloride |
| | LC-MS (ESI) m/z [M+H]⁺: 317.06 |
| | tR 3.668 min (B) |
| | (S)-2-((tert-Butoxycarbonyl)amino)-3-cyclopentylpropanoic acid (143415-31-0) |
| | 4-(Bromomethyl)-2,6-dichloropyridine (175204-45-2) |
| | 4M-HCl/MTHP |
| 1-1-112 | 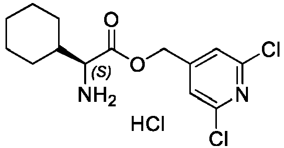 |
| | (2,6-Dichloropyridin-4-yl)methyl (S)-2-amino-2-cyclohexyl acetate hydrochloride |
| | LC-MS (ESI) m/z [M+H]⁺: 317.01 |
| | tR 3.748 min (B) |
| | (S)-2-((tert-Butoxycarbonyl)amino)-2-cyclohexylacetic acid (109183-71-3) |
| | 4-(Bromomethyl)-2,6-dichloropyridine (175204-45-2) |
| | 4M-HCl/MTHP |
| 1-1-113 | 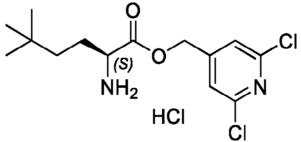 |
| | (2,6-Dichloropyridin-4-yl)methyl (S)-2-amino-5,5-dimethylhexanoate hydrochloride |
| | LC-MS (ESI) m/z [M+H]⁺: 319.07 |
| | tR 3.772 min (B) |
| | (S)-2-((tert-Butoxycarbonyl)amino)-5,5-dimethylhexanoic acid (752237-71-1) |
| | 4-(Bromomethyl)-2,6-dichloropyridine (175204-45-2) |
| | 4M-HCl/MTHP |

**[Table 1-53]**

| | |
|---|---|
| 1-1-114 | 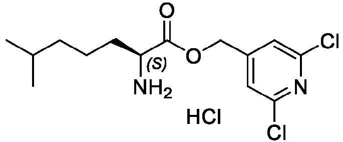 |
| | (2,6-Dichloropyridin-4-yl)methyl (S)-2-amino-6-methylheptanoate hydrochloride |
| | LC-MS (ESI) m/z [M+H]⁺: 318.98 |
| | tR 3.964 min (B) |
| | (S)-2-((tert-Butoxycarbonyl)amino)-6-methylheptanoic acid (329270-49-7) |
| | 4-(Bromomethyl)-2,6-dichloropyridine (175204-45-2) |
| | 4M-HCl/MTHP |
| 1-1-115 | 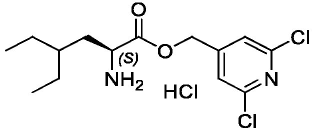 |
| | (2,6-Dichloropyridin-4-yl)methyl (S)-2-amino-4-ethylhexanoate hydrochloride |
| | LC-MS (ESI) m/z [M+H]⁺: 319.03 |
| | tR 3.824 min (B) |
| | (S)-2-((tert-Butoxycarbonyl)amino)-4-ethylhexanoic acid (1372404-73-3) |
| | 4-(Bromomethyl)-2,6-dichloropyridine (175204-45-2) |
| | 4M-HCl/MTHP |

**[Table 1-54]**

| | |
|---|---|
| 1-1-116 | 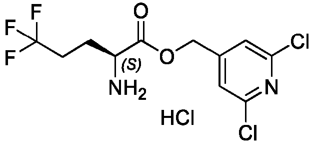 |
| | (2,6-Dichloropyridin-4-yl)methyl (S)-2-amino-5,5,5-trifluoropentanoate hydrochloride |
| | LC-MS (ESI) m/z [M+H]⁺: 330.93 |
| | tR 3.372 min (B) |
| | (S)-2-((tert-Butoxycarbonyl)amino)-5,5,5-trifluoropentanoic acid (453556-65-5) |
| | 4-(Bromomethyl)-2,6-dichloropyridine (175204-45-2) |
| | 4M-HCl/MTHP |
| 1-1-117 | 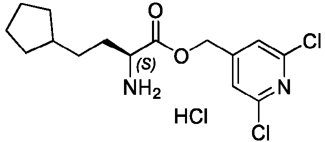 |
| | (2,6-Dichloropyridin-4-yl)methyl (S)-2-amino-4-cyclopentylbutanoate hydrochloride |
| | LC-MS (ESI) m/z [M+H]⁺: 331.03 |
| | tR 4.084 min (B) |
| | (S)-2-((tert-Butoxycarbonyl)amino)-4-cyclopentylbutanoic acid (2349652-68-0) |
| | 4-(Bromomethyl)-2,6-dichloropyridine (175204-45-2) |
| | 4M-HCl/MTHP |

**[Table 1-55]**

| | |
|---|---|
| 1-1-118 | 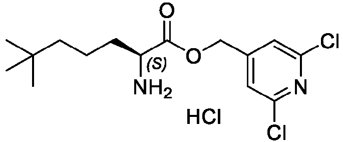 |
| | (2,6-Dichloropyridin-4-yl)methyl (S)-2-amino-6,6-dimethylheptanoate hydrochloride |
| | LC-MS (ESI) m/z [M+H]⁺: 333.02 |
| | tR 4.144 min (B) |
| | (S)-2-((tert-Butoxycarbonyl)amino)-6,6-dimethylheptanoic acid (2349881-94-1) |
| | 4-(Bromomethyl)-2,6-dichloropyridine (175204-45-2) |
| | 4M-HCl/MTHP |
| 1-1-119 | 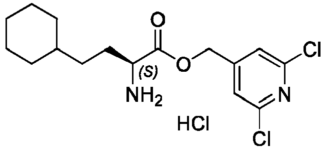 |
| | (2,6-Dichloropyridin-4-yl)methyl (S)-2-amino-4-cyclohexylbutanoate hydrochloride |
| | LC-MS (ESI) m/z [M+H]⁺: 345.04 |
| | tR 4.076 min (B) |
| | (S)-2-((tert-Butoxycarbonyl)amino)-4-cyclohexylbutanoic acid (143415-51-4) |
| | 4-(Bromomethyl)-2,6-dichloropyridine (175204-45-2) |
| | 4M-HCl/MTHP |

**[Table 1-56]**

| | |
|---|---|
| 1-1-120 | 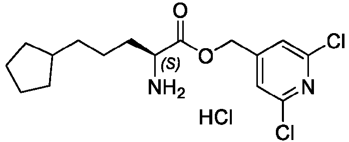 |
| | (2,6-Dichloropyridin-4-yl)methyl (S)-2-amino-5-cyclopentylpentanoate hydrochloride |
| | LC-MS (ESI) m/z [M+H]⁺: 345.04 |
| | tR 4.124 min (B) |
| | (S)-2-((tert-Butoxycarbonyl)amino)-5-cyclopentylpentanoic acid (2350685-02-6) |
| | 4-(Bromomethyl)-2,6-dichloropyridine (175204-45-2) |
| | 4M-HCl/CPME |
| 1-1-121 | 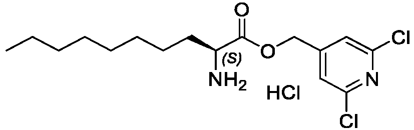 |
| | (2,6-Dichloropyridin-4-yl)methyl (S)-2-aminodecanoate hydrochloride |
| | LC-MS (ESI) m/z [M+H]⁺: 348.30 |
| | tR 4.524 min (B) |
| | (S)-2-((tert-Butoxycarbonyl)amino)decanoic acid (67862-03-7) |
| | 4-(Bromomethyl)-2,6-dichloropyridine (175204-45-2) |
| | 4M-HCl/MTHP |

**[Table 1-57]**

| | |
|---|---|
| 1-1-122 | 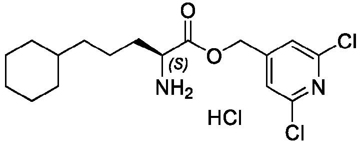 |
| | (2,6-Dichloropyridin-4-yl)methyl (S)-2-amino-5-cyclohexylpentanoate hydrochloride |
| | LC-MS (ESI) m/z [M+H]⁺: 359.02 |
| | tR 4.324 min (B) |
| | (S)-2-((tert-Butoxycarbonyl)amino)-5-cyclohexylpentanoic acid (2349792-96-5) |
| | 4-(Bromomethyl)-2,6-dichloropyridine (175204-45-2) |
| | 4M-HCl/MTHP |
| 1-1-123 | 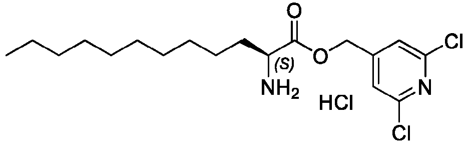 |
| | (2,6-Dichloropyridin-4-yl)methyl (S)-2-aminododecanoate hydrochloride |
| | LC-MS (ESI) m/z [M+H]⁺: 376.40 |
| | tR 4.772 min (B) |
| | (S)-2-((tert-Butoxycarbonyl)amino)dodecanoic acid (146276-04-2) |
| | 4-(Bromomethyl)-2,6-dichloropyridine (175204-45-2) |
| | 4M-HCl/MTHP |
| 1-1-124 | 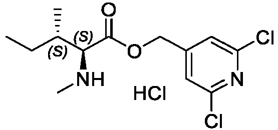 |
| | (2,6-Dichloropyridin-4-yl)methyl L-isoleucinate hydrochloride |
| | LC-MS (ESI) m/z [M+H]⁺: 305.01 |
| | tR 5.444 min (A) |
| | N-(tert-Butoxycarbonyl)-N-methyl-L-isoleucine (52498-32-5) |
| | 4-(Bromomethyl)-2,6-dichloropyridine (175204-45-2) |
| | 4M-HCl/MTHP |

**[Table 1-58]**

| | |
|---|---|
| 1-1-125 | 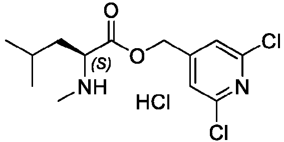 |
| | (2,6-Dichloropyridin-4-yl)methyl L-leucinate hydrochloride |
| | LC-MS (ESI) m/z [M+H]⁺: 305.04 |
| | tR 5.184 min (A) |
| | N-(tert-Butoxycarbonyl)-N-methyl-L-leucine (53363-89-6) |
| | 4-(Bromomethyl)-2,6-dichloropyridine (175204-45-2) |
| | 4M-HCl/MTHP |
| 1-1-126 | 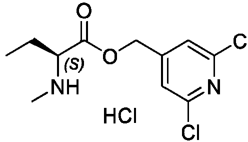 |
| | (2,6-Dichloropyridin-4-yl)methyl (S)-2-(methylamino)butanoate hydrochloride |
| | LC-MS (ESI) m/z [M+H]⁺: 276.98 |
| | tR 4.360 min (A) |
| | (S)-2-((tert-Butoxycarbonyl)(methyl)amino)butanoic acid (101759-74-4) |
| | 4-(Bromomethyl)-2,6-dichloropyridine (175204-45-2) |
| | 4M-HCl/MTHP |

**[Table 1-59]**

| | |
|---|---|
| 1-1-127 | 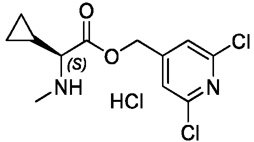 |
| | (2,6-Dichloropyridin-4-yl)methyl (S)-2-cyclopropyl-2-(methylamino)acetate hydrochloride |
| | LC-MS (ESI) m/z [M+H]⁺: 288.97 |
| | tR 4.308 min (A) |
| | (S)-2-((tert-Butoxycarbonyl)(methyl)amino)-2-cyclopropylacetic acid (1202452-81-0) |
| | 4-(Bromomethyl)-2,6-dichloropyridine (175204-45-2) |
| | 4M-HCl/MTHP |
| 1-1-128 | 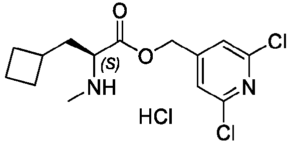 |
| | (2,6-Dichloropyridin-4-yl)methyl (S)-3-cyclobutyl-2-(methylamino)propanoate hydrochloride |
| | LC-MS (ESI) m/z [M+H]⁺: 316.98 |
| | tR 3.468 min (B) |
| | (S)-2-((tert-Butoxycarbonyl)(methyl)amino)-3-cyclobutylpropanoic acid (2102636-45-1) |
| | 4-(Bromomethyl)-2,6-dichloropyridine (175204-45-2) |
| | 4M-HCl/MTHP |

**[Table 1-60]**

| | |
|---|---|
| 1-1-129 | 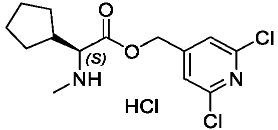 |
| | (2,6-Dichloropyridin-4-yl)methyl (S)-2-cyclopentyl-2-(methylamino)acetate hydrochloride |
| | LC-MS (ESI) m/z [M+H]⁺: 316.98 |
| | tR 5.648 min (A) |
| | (S)-2-((tert-Butoxycarbonyl)(methyl)amino)-2-cyclopentylacetic acid (2704466-35-1) |
| | 4-(Bromomethyl)-2,6-dichloropyridine (175204-45-2) |
| | 4M-HCl/MTHP |
| 1-1-130 | 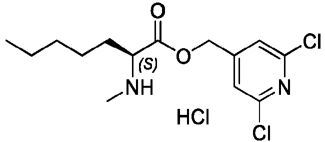 |
| | (2,6-Dichloropyridin-4-yl)methyl (S)-2-(methylamino)heptanoate hydrochloride |
| | LC-MS (ESI) m/z [M+H]⁺: 318.98 |
| | tR 3.792 min (B) |
| | (S)-2-((tert-Butoxycarbonyl)(methyl)amino)heptanoic acid (2389078-55-9) |
| | 4-(Bromomethyl)-2,6-dichloropyridine (175204-45-2) |
| | 4M-HCl/MTHP |

**[Table 1-61]**

| | |
|---|---|
| 1-1-131 | 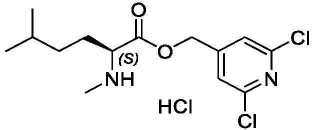 |
| | (2,6-Dichloropyridin-4-yl)methyl (S)-5-methyl-2-(methylamino)hexanoate hydrochloride |
| | LC-MS (ESI) m/z [M+H]⁺: 319.01 |
| | tR 6.188 min (A) |
| | (S)-2-((tert-Butoxycarbonyl)(methyl)amino)-5-methylhexanoic acid A-19110, Amatek Chemical Co. Ltd. |
| | 4-(Bromomethyl)-2,6-dichloropyridine (175204-45-2) |
| | 4M-HCl/MTHP |
| 1-1-132 | 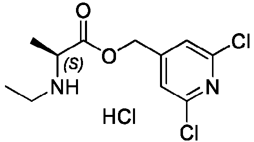 |
| | (2,6-Dichloropyridin-4-yl)methyl ethyl-L-alaninate hydrochloride |
| | LC-MS (ESI) m/z [M+H]⁺: 277.03 |
| | tR 4.080 min (A) |
| | N-(tert-Butoxycarbonyl)-N-ethyl-L-alanine (91292-56-7) |
| | 4-(Bromomethyl)-2,6-dichloropyridine (175204-45-2) |
| | 4M-HCl/MTHP |
| 1-1-133 | 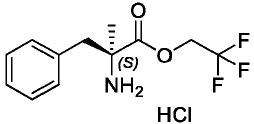 |
| | 2,2,2-Trifluoroethyl (S)-2-amino-2-methyl-3-phenylpropanoate hydrochloride |
| | LC-MS (ESI) m/z [M+H]⁺: 262.05 |
| | tR 4.844 min (A) |
| | (S)-2-((tert-Butoxycarbonyl)amino)-2-methyl-3-phenylpropanoic acid (53940-88-8) |
| | 2,2,2-Trifluoroethyl trifluoromethanesulfonate (6226-25-1) |
| | 4M-HCl/MTHP |

**[Table 1-62]**

| | |
|---|---|
| 1-1-134 | 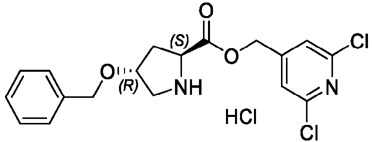 |
| | (2,6-Dichloropyridin-4-yl)methyl (2S,4R)-4-(benzyloxy)pyrrolidine-2-carboxylate hydrochloride |
| | LC-MS (ESI) m/z [M+H]⁺: 381.02 |
| | tR 3.732 min (B) |
| | (2S,4R)-4-(Benzyloxy)-1-(tert-butoxycarbonyl)pyrrolidine-2-carboxylic acid (54631-81-1) |
| | 4-(Bromomethyl)-2,6-dichloropyridine (175204-45-2) |
| | 4M-HCl/CPME |
| 1-1-135 | 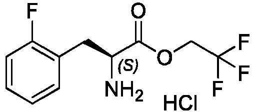 |
| | 2,2,2-Trifluoroethyl (S)-2-amino-3-(2-fluorophenyl)propanoate hydrochloride |
| | LC-MS (ESI) m/z [M+H]⁺: 265.98 |
| | tR 4.424 min (A) |
| | (S)-2-((tert-Butoxycarbonyl)amino)-3-(2-fluorophenyl)propanoic acid (114873-00-6) |
| | 2,2,2-Trifluoroethyl trifluoromethanesulfonate (6226-25-1) |
| | 4M-HCl/MTHP |

**[Table 1-63]**

| | |
|---|---|
| 1-1-136 | 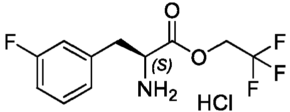 |
| | 2,2,2-Trifluoroethyl (S)-2-amino-3-(3-fluorophenyl)propanoate hydrochloride |
| | LC-MS (ESI) m/z [M+H]⁺: 265.99 |
| | tR 2.796 min (B) |
| | (S)-2-((tert-Butoxycarbonyl)amino)-3-(3-fluorophenyl)propanoic acid (114873-01-7) |
| | 2,2,2-Trifluoroethyl trifluoromethanesulfonate (6226-25-1) |
| | 4M-HCl/MTHP |
| 1-1-137 | 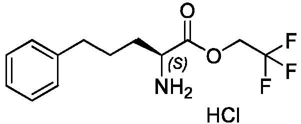 |
| | 2,2,2-Trifluoroethyl (S)-2-amino-5-phenylpentanoate hydrochloride |
| | LC-MS (ESI) m/z [M+H]⁺: 276.04 |
| | tR 3.388 min (B) |
| | (S)-2-((tert-Butoxycarbonyl)amino)-5-phenylpentanoic acid (98628-27-4) |
| | 2,2,2-Trifluoroethyl trifluoromethanesulfonate (6226-25-1) |
| | 4M-HCl/MTHP |
| 1-1-138 | 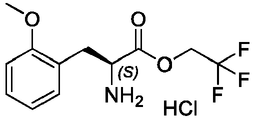 |
| | 2,2,2-Trifluoroethyl (S)-2-amino-3-(2-methoxyphenyl)propanoate hydrochloride |
| | LC-MS (ESI) m/z [M+H]⁺: 278.00 |
| | tR 2.956 min (B) |
| | (S)-2-((tert-Butoxycarbonyl)amino)-3-(2-methoxyphenyl)propanoic acid (143415-63-8) |
| | 2,2,2-Trifluoroethyl trifluoromethanesulfonate (6226-25-1) |
| | 4M-HCl/MTHP |

**[Table 1-64]**

| | |
|---|---|
| 1-1-139 | 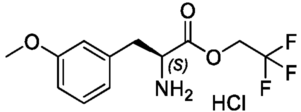 |
| | 2,2,2-Trifluoroethyl (S)-2-amino-3-(3-methoxyphenyl)propanoate hydrochloride |
| | LC-MS (ESI) m/z [M+H]⁺: 278.00 |
| | tR 4.632 min (A) |
| | (S)-2-((tert-Butoxycarbonyl)amino)-3-(3-methoxyphenyl)propane (261360-71-8) |
| | 2,2,2-Trifluoroethyl trifluoromethanesulfonate (6226-25-1) |
| | 4M-HCl/MTHP |
| 1-1-140 | 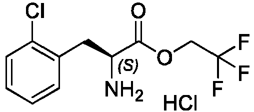 |
| | 2,2,2-Trifluoroethyl (S)-2-amino-3-(2-chlorophenyl)propanoate hydrochloride |
| | LC-MS (ESI) m/z [M+H]⁺: 281.97 |
| | tR 2.988 min (A) |
| | (S)-2-((tert-Butoxycarbonyl)amino)-3-(2-chlorophenyl)propanoic acid (114873-02-8) |
| | 2,2,2-Trifluoroethyl trifluoromethanesulfonate (6226-25-1) |
| | 4M-HCl/MTHP |

**[Table 1-65]**

| | |
|---|---|
| 1-1-141 | 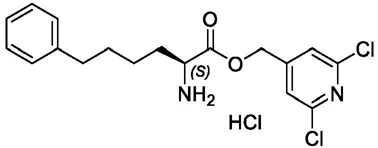 |
| | (2,6-Dichloropyridin-4-yl)methyl (S)-2-amino-6-phenylhexanoate hydrochloride |
| | LC-MS (ESI) m/z [M+H]⁺: 366.95 |
| | tR 4.024 min (B) |
| | (S)-2-((tert-Butoxycarbonyl)amino)-6-phenylhexanoic acid (150722-68-2) |
| | 4-(Bromomethyl)-2,6-dichloropyridine (150722-68-2) |
| | 4M-HCl/MTHP |
| 1-1-142 | 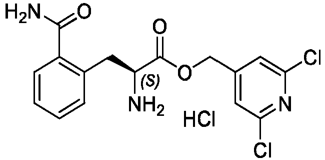 |
| | (2,6-Dichloropyridin-4-yl)methyl (S)-2-amino-3-(2-carbamoylphenyl)propanoate hydrochloride |
| | LC-MS (ESI) m/z [M+H]⁺: 367.94 |
| | tR 4.560 min (A) |
| | (S)-2-((tert-Butoxycarbonyl)amino)-3-(2-carbamoylphenyl)propanoic acid (959573-27-4) |
| | 4-(Bromomethyl)-2,6-dichloropyridine (175204-45-2) |
| | 4M-HCl/MTHP |

**[Table 1-66]**

| | |
|---|---|
| 1-1-143 | 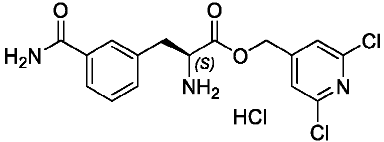 |
| | (2,6-Dichloropyridin-4-yl)methyl (S)-2-amino-3-(3-carbamoylphenyl)propanoate hydrochloride |
| | LC-MS (ESI) m/z [M+H]⁺: 368.00 |
| | tR 4.680 min (A) |
| | (S)-2-((tert-Butoxycarbonyl)amino)-3-(3-carbamoylphenyl)propanoic acid (943449-15-8) |
| | 4-(Bromomethyl)-2,6-dichloropyridine (175204-45-2) |
| | 4M-HCl/MTHP |
| 1-1-144 | 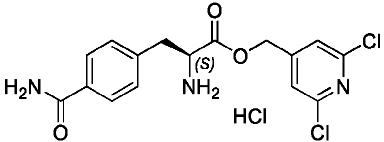 |
| | (2,6-Dichloropyridin-4-yl)methyl (S)-2-amino-3-(4-carbamoylphenyl)propanoate hydrochloride |
| | LC-MS (ESI) m/z [M+H]⁺: 368.02 |
| | tR 2.632 min (B) |
| | (S)-2-((tert-Butoxycarbonyl)amino)-3-(4-carbamoylphenyl)propanoic acid (205126-71-2) |
| | 4-(Bromomethyl)-2,6-dichloropyridine (175204-45-2) |
| | 2,2,2-Trifluoroacetic acid and 4M-HCl/CPME |

**[Table 1-67]**

| | |
|---|---|
| 1-1-145 | 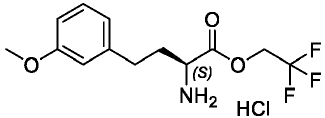 |
| | 2,2,2-Trifluoroethyl (S)-2-amino-4-(3-methoxyphenyl)butanoate hydrochloride |
| | LC-MS (ESI) m/z [M+H]⁺: 292.05 |
| | tR 3.104 min (B) |
| | (S)-2-((tert-Butoxycarbonyl)amino)-4-(3-methoxyphenyl)butanoic acid (942065-44-3) |
| | 2,2,2-Trifluoroethyl trifluoromethanesulfonate (6226-25-1) |
| | 4M-HCl/MTHP |
| 1-1-146 | 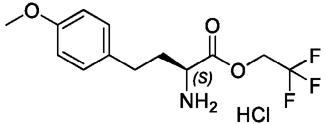 |
| | 2,2,2-Trifluoroethyl (S)-2-amino-4-(4-methoxyphenyl)butanoate hydrochloride |
| | LC-MS (ESI) m/z [M+H]⁺: 292.03 |
| | tR 5.084 min (A) |
| | (S)-2-((tert-Butoxycarbonyl)amino)-4-(4-methoxyphenyl)butanoic acid (162633-83-2) |
| | 2,2,2-Trifluoroethyl trifluoromethanesulfonate (6226-25-1) |
| | 4M-HCl/MTHP |

**[Table 1-68]**

| | |
|---|---|
| 1-1-147 | 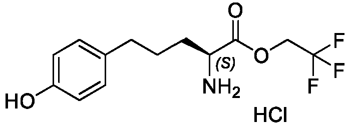 |
| | 2,2,2-Trifluoroethyl (S)-2-amino-5-(4-hydroxyphenyl)pentanoate hydrochloride |
| | LC-MS (ESI) m/z [M+H]⁺: 292.03 |
| | tR 4.476 min (A) |
| | (S)-2-((tert-Butoxycarbonyl)amino)-5-(4-hydroxyphenyl)pentanoic acid (2349678-11-9) |
| | 2,2,2-Trifluoroethyl trifluoromethanesulfonate (6226-25-1) |
| | 4M-HCl/MTHP |
| 1-1-148 | 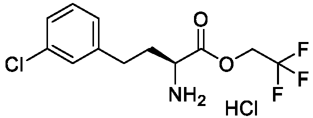 |
| | 2,2,2-Trifluoroethyl (S)-2-amino-4-(3-chlorophenyl)butanoate hydrochloride |
| | LC-MS (ESI) m/z [M+H]⁺: 296.02 |
| | tR 3.432 min (B) |
| | (S)-2-((tert-Butoxycarbonyl)amino)-4-(3-chlorophenyl)butanoic acid (1260589-43-2) |
| | 2,2,2-Trifluoroethyl trifluoromethanesulfonate (6226-25-1) |
| | 4M-HCl/MTHP |
| 1-1-149 | 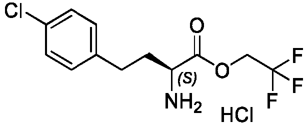 |
| | 2,2,2-Trifluoroethyl (S)-2-amino-4-(4-chlorophenyl)butanoate hydrochloride |
| | LC-MS (ESI) m/z [M+H]⁺: 296.02 |
| | tR 3.460 min (B) |
| | (S)-2-((tert-Butoxycarbonyl)amino)-4-(4-chlorophenyl)butanoic acid (157683-99-3) |
| | 2,2,2-Trifluoroethyl trifluoromethanesulfonate (6226-25-1) |
| | 4M-HCl/MTHP |

**[Table 1-69]**

| | |
|---|---|
| 1-1-150 | 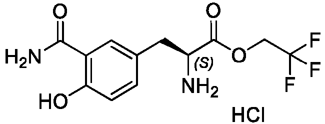 |
| | 2,2,2-Trifluoroethyl (S)-2-amino-3-(3-carbamoyl-4-hydroxyphenyl)propanoate hydrochloride |
| | LC-MS (ESI) m/z [M+H]⁺: 307.08 |
| | tR 3.948 min (A) |
| | (S)-2-((tert-Butoxycarbonyl)amino)-3-(3-carbamoyl-4-hydroxyphenyl)propanoic acid (1073243-35-2) |
| | 2,2,2-Trifluoroethyl trifluoromethanesulfonate (6226-25-1) |
| | 4M-HCl/MTHP |
| 1-1-151 | 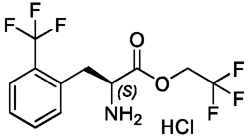 |
| | 2,2,2-Trifluoroethyl (S)-2-amino-3-(2-(trifluoromethyl)phenyl)propanoate hydrochloride |
| | LC-MS (ESI) m/z [M+H]⁺: 315.99 |
| | tR 3.240 min (B) |
| | (S)-2-((tert-Butoxycarbonyl)amino)-3-(2-(trifluoromethyl)phenyl)propanoic acid (167993-21-7) |
| | 2,2,2-Trifluoroethyl trifluoromethanesulfonate (6226-25-1) |
| | 4M-HCl/MTHP |

**[Table 1-70]**

| | |
|---|---|
| 1-1-152 | 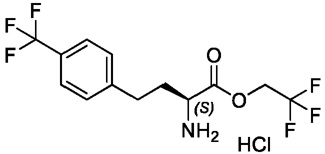 |
| | 2,2,2-Trifluoroethyl (S)-2-amino-4-(4-(trifluoromethyl)phenyl)butanoate hydrochloride |
| | LC-MS (ESI) m/z [M+H]⁺: 329.99 |
| | tR 3.800 min (B) |
| | (S)-2-((tert-Butoxycarbonyl)amino)-4-(4-(trifluoromethyl)phenyl)butanoic acid (1260592-66-2) |
| | 2,2,2-Trifluoroethyl trifluoromethanesulfonate (6226-25-1) |
| | 4M-HCl/MTHP |
| 1-1-153 | 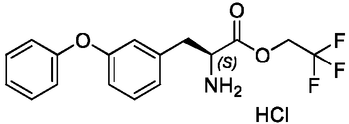 |
| | 2,2,2-Trifluoroethyl (S)-2-amino-3-(3-phenoxyphenyl)propanoate hydrochloride |
| | LC-MS (ESI) m/z [M+H]⁺: 340.04 |
| | tR 3.756 min (B) |
| | (S)-2-((tert-Butoxycarbonyl)amino)-3-(3-phenoxyphenyl)propanoic acid (1213415-33-8) |
| | 2,2,2-Trifluoroethyl trifluoromethanesulfonate (6226-25-1) |
| | 4M-HCl/MTHP |

**[Table 1-71]**

| | |
|---|---|
| 1-1-154 | 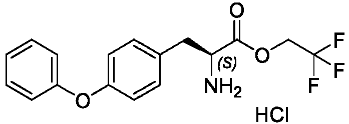 |
| | 2,2,2-Trifluoroethyl (S)-2-amino-3-(4-phenoxyphenyl)propanoate hydrochloride |
| | LC-MS (ESI) m/z [M+H]⁺: 340.02 |
| | tR 3.792 min (B) |
| | (S)-2-((tert-Butoxycarbonyl)amino)-3-(4-phenoxyphenyl)propanoic acid (150351-65-8) |
| | 2,2,2-Trifluoroethyl trifluoromethanesulfonate (6226-25-1) |
| | 4M-HCl/CPME |
| 1-1-155 | 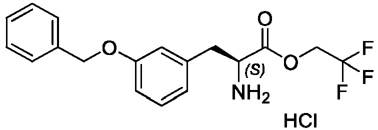 |
| | 2,2,2-Trifluoroethyl (S)-2-amino-3-(3-(benzyloxy)phenyl)propanoate hydrochloride |
| | LC-MS (ESI) m/z [M+H]⁺: 354.07 |
| | tR 3.832 min (B) |
| | (S)-3-(3-(Benzyloxy)phenyl)-2-((tert-butoxycarbonyl)amino)propanoic acid (162536-46-1) |
| | 2,2,2-Trifluoroethyl trifluoromethanesulfonate (6226-25-1) |
| | 4M-HCl/MTHP |

**[Table 1-72]**

| | |
|---|---|
| 1-1-156 | 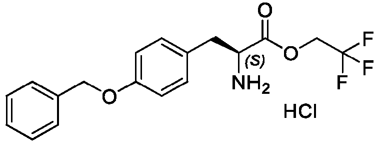 |
| | 2,2,2-Trifluoroethyl (S)-2-amino-3-(4-(benzyloxy)phenyl)propanoate hydrochloride |
| | LC-MS (ESI) m/z [M+H]⁺: 354.09 |
| | tR 3.860 min (B) |
| | (S)-3-(3-(Benzyloxy)phenyl)-2-((tert-butoxycarbonyl)amino)propanoic acid (2130-96-3) |
| | 2,2,2-Trifluoroethyl trifluoromethanesulfonate (6226-25-1) |
| | 4M-HCl/MTHP |
| 1-1-157 | 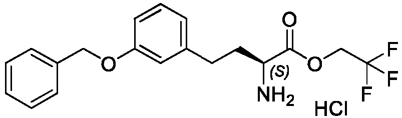 |
| | 2,2,2-Trifluoroethyl (S)-2-amino-4-(3-(benzyloxy)phenyl)butanoate hydrochloride |
| | LC-MS (ESI) m/z [M+H]⁺: 368.00 |
| | tR 4.076 min (B) |
| | (S)-4-(3-(Benzyloxy)phenyl)-2-((tert-butoxycarbonyl)amino)butanoic acid (2349588-16-3) |
| | 2,2,2-Trifluoroethyl trifluoromethanesulfonate (6226-25-1) |
| | 4M-HCl/MTHP |

**[Table 1-73]**

| | |
|---|---|
| 1-1-158 | 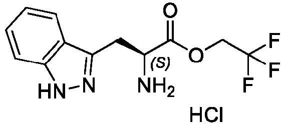 |
| | 2,2,2-Trifluoroethyl (S)-2-amino-3-(1H-indazol-3-yl)propanoate hydrochloride |
| | LC-MS (ESI) m/z [M+H]⁺: 288.02 |
| | tR 2.624 min (B) |
| | (S)-3-(1-(tert-Butoxycarbonyl)-1H-indazol-3-yl)-2-((tertbutoxycarbonyl)amino)propanoic acid (2387561-67-1) |
| | 2,2,2-Trifluoroethyl trifluoromethanesulfonate (6226-25-1) |
| | 4M-HCl/MTHP |
| 1-1-159 | 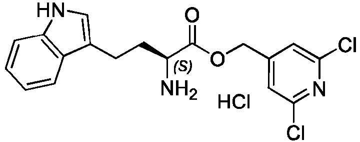 |
| | (2,6-Dichloropyridin-4-yl)methyl (S)-2-amino-4-(1H-indol-3-yl)butanoate hydrochloride |
| | LC-MS (ESI) m/z [M+H]⁺: 378.03 |
| | tR 3.608 min (B) |
| | (S)-2-((tert-Butoxycarbonyl)amino)-4-(1H-indol-3-yl)butanoic acid (2348344-80-7) |
| | 4-(Bromomethyl)-2,6-dichloropyridine (175204-45-2) |
| | 4M-HCl/MTHP |
| 1-1-160 | 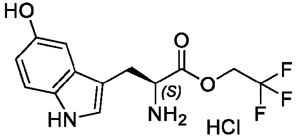 |
| | 2,2,2-Trifluoroethyl (S)-2-amino-3-(5-hydroxy-1H-indol-3-yl)propanoate hydrochloride |
| | LC-MS (ESI) m/z [M+H]⁺: 303.01 |
| | tR 3.656 min (A) |
| | (S)-2-((tert-Butoxycarbonyl)amino)-3-(5-hydroxy-1H-indol-3-yl)propanoic acid (119768-45-5) |
| | 2,2,2-Trifluoroethyl trifluoromethanesulfonate (6226-25-1) |
| | 4M-HCl/MTHP |

**[Table 1-74]**

| | |
|---|---|
| 1-1-161 | 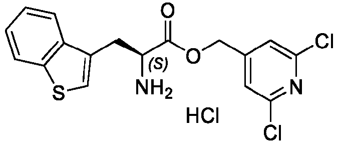 |
| | (2,6-Dichloropyridin-4-yl)methyl (S)-2-amino-3-(benzo[b]thiophen-3-yl)propanoate hydrochloride |
| | LC-MS (ESI) m/z [M+H]⁺: 380.97 |
| | tR 3.804 min (B) |
| | (S)-3-(Benzo[b]thiophen-3-yl)-2-((tert-butoxycarbonyl)amino)propanoic acid (154902-51-9) |
| | 4-(Bromomethyl)-2,6-dichloropyridine (175204-45-2) |
| | 4M-HCl/MTHP |
| 1-1-162 | 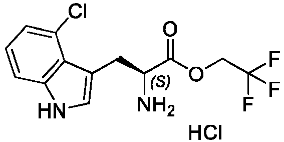 |
| | 2,2,2-Trifluoroethyl (S)-2-amino-3-(4-chloro-1H-indol-3-yl)propanoate hydrochloride |
| | LC-MS (ESI) m/z [M+H]⁺: 320.98 |
| | tR 3.464 min (B) |
| | (S)-2-((tert-Butoxycarbonyl)amino)-3-(4-chloro-1H-indol-3-yl)propanoic acid (2413973-50-7) |
| | 2,2,2-Trifluoroethyl trifluoromethanesulfonate (6226-25-1) |
| | 4M-HCl/MTHP |

**[Table 1-75]**

| | |
|---|---|
| 1-1-163 | 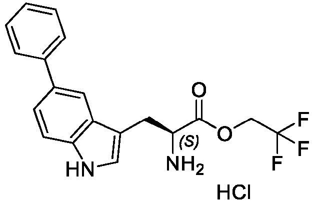 |
| | 2,2,2-Trifluoroethyl (S)-2-amino-3-(5-phenyl-1H-indol-3-yl)propanoate hydrochloride |
| | LC-MS (ESI) m/z [M+H]⁺: 363.08 |
| | tR 3.872 min (B) |
| | (S)-2-((tert-Butoxycarbonyl)amino)-3-(5-phenyl-1H-indol-3-yl)propanoic acid (1910135-36-2) |
| | 2,2,2-Trifluoroethyl trifluoromethanesulfonate (6226-25-1) |
| | 4M-HCl/MTHP |
| 1-1-164 | 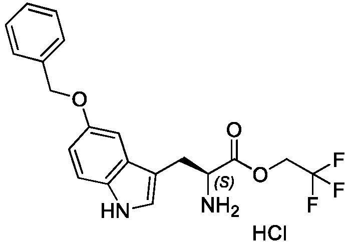 |
| | 2,2,2-Trifluoroethyl (S)-2-amino-3-(5-(benzyloxy)-1H-indol-3-yl)propanoate hydrochloride |
| | LC-MS (ESI) m/z [M+H]⁺: 393.08 |
| | tR 3.924 min (B) |
| | (S)-3-(5-(Benzyloxy)-1H-indol-3-yl)-2-((tert-butoxycarbonyl)amino)propanoic acid (1462877-41-3) |
| | 2,2,2-Trifluoroethyl trifluoromethanesulfonate (6226-25-1) |
| | 4M-HCl/MTHP |

**[Table 1-76]**

| | |
|---|---|
| 1-1-165 | 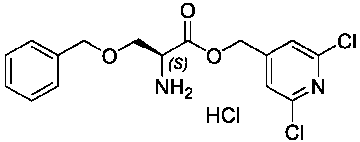 |
| | (2,6-Dichloropyridin-4-yl)methyl O-benzyl-L-serinate hydrochloride |
| | LC-MS (ESI) m/z [M+H]⁺: 355.02 |
| | tR 3.620 min (B) |
| | O-Benzyl-N-(tert-butoxycarbonyl)-L-serine (23680-31-1) |
| | 4-(Bromomethyl)-2,6-dichloropyridine (175204-45-2) |
| | 4M-HCl/MTHP |
| 1-1-166 | 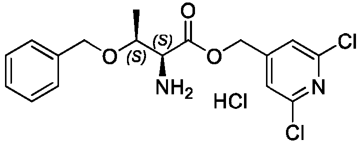 |
| | (2,6-Dichloropyridin-4-yl)methyl O-benzyl-L-allothreoninate hydrochloride |
| | LC-MS (ESI) m/z [M+H]⁺: 368.98 |
| | tR 3.672 min (B) |
| | O-Benzyl-N-(tert-butoxycarbonyl)-L-allothreonine (128609-40-5) |
| | 4-(Bromomethyl)-2,6-dichloropyridine (175204-45-2) |
| | 4M-HCl/MTHP |

**[Table 1-77]**

| | |
|---|---|
| 1-1-167 | 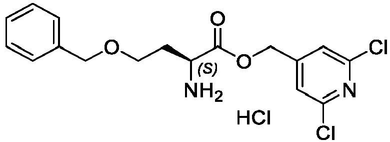 |
| | (2,6-Dichloropyridin-4-yl)methyl O-benzyl-L-homoserinate hydrochloride |
| | LC-MS (ESI) m/z [M+H]⁺: 368.99 |
| | tR 3.732 min (B) |
| | O-Benzyl-N-(tert-butoxycarbonyl)-L-homoserine (59408-74-1) |
| | 4-(Bromomethyl)-2,6-dichloropyridine (175204-45-2) |
| | 4M-HCl/MTHP |
| 1-1-168 | 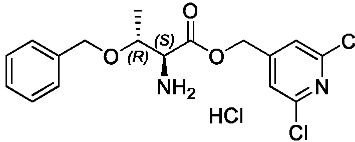 |
| | (2,6-Dichloropyridin-4-yl)methyl O-benzyl-L-threoninate hydrochloride |
| | LC-MS (ESI) m/z [M+H]⁺: 369.00 |
| | tR 3.724 min (B) |
| | O-Benzyl-N-(tert-Butoxycarbonyl)-L-threonine (15260-10-3) |
| | 4-(Bromomethyl)-2,6-dichloropyridine (175204-45-2) |
| | 4M-HCl/MTHP |
| 1-1-169 | 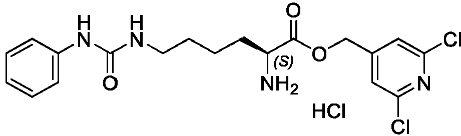 |
| | (2,6-Dichloropyridin-4-yl)methyl N⁶-(phenylcarbamoyl)-L-lysinate hydrochloride |
| | LC-MS (ESI) m/z [M+H]⁺: 425.08 |
| | tR 3.536 min (B) |
| | N²-(tert-Butoxycarbonyl)-N⁶-(phenylcarbamoyl)-L-lysine (K31151,Mimotopes) |
| | 4-(Bromomethyl)-2,6-dichloropyridine (175204-45-2) |
| | 4M-HCl/MTHP |

**[Table 1-78]**

| | |
|---|---|
| 1-1-170 | 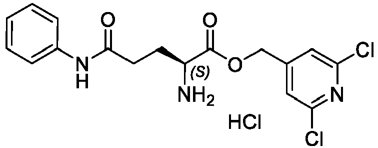 |
| | (2,6-Dichloropyridin-4-yl)methyl N⁵-phenyl-L-glutamate hydrochloride |
| | LC-MS (ESI) m/z [M+H]⁺: 382.03 |
| | tR 3.420 min (B) |
| | N²-(tert-Butoxycarbonyl)-N⁵-phenyl-L-glutamine (198134-13-3) |
| | 4-(Bromomethyl)-2,6-dichloropyridine (175204-45-2) |
| | 4M-HCl/MTHP |
| 1-1-171 | 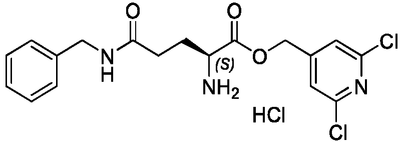 |
| | (2,6-Dichloropyridin-4-yl)methyl N⁵-benzyl-L-glutamate hydrochloride |
| | LC-MS (ESI) m/z [M+H]⁺: 396.03 |
| | tR 3.432 min (B) |
| | N³-Benzyl-N²-(tert-butoxycarbonyl)-L-glutamine (150212-95-6) |
| | 4-(Bromomethyl)-2,6-dichloropyridine (175204-45-2) |
| | 4M-HCl/MTHP |
| 1-1-172 | 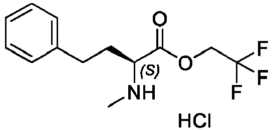 |
| | 2,2,2-Trifluoroethyl (S)-2-(methylamino)-4-phenylbutanoate hydrochloride |
| | LC-MS (ESI) m/z [M+H]⁺: 276.01 |
| | tR 3.232 min (B) |
| | (S)-2-((tert-Butoxycarbonyl)(methyl)amino)-4-phenylbutanoic acid (110755-73-2) |
| | 2,2,2-Trifluoroethyl trifluoromethanesulfonate (6226-25-1) |
| | 4M-HCl/MTHP |

**[Table 1-79]**

| | |
|---|---|
| 1-1-173 | 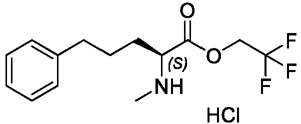 |
| | 2,2,2-Trifluoroethyl (S)-2-(methylamino)-5-phenylpentanoate hydrochloride |
| | LC-MS (ESI) m/z [M+H]⁺: 290.03 |
| | tR 3.488 min (B) |
| | (S)-2-((tert-Butoxycarbonyl)(methyl)amino)-5-phenylpentanoic acid (1274904-40-3) |
| | 2,2,2-Trifluoroethyl trifluoromethanesulfonate (6226-25-1) |
| | 4M-HCl/MTHP |
| 1-1-174 | 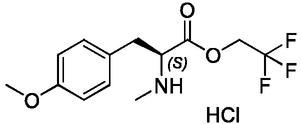 |
| | 2,2,2-Trifluoroethyl (S)-3-(4-methoxyphenyl)-2-(methylamino)propanoate hydrochloride |
| | LC-MS (ESI) m/z [M+H]⁺: 292.02 |
| | tR 3.004 min (B) |
| | (S)-2-((tert-Butoxycarbonyl)(methyl)amino)-3-(4-methoxyphenyl)propanoic acid (73584-84-6) |
| | 2,2,2-Trifluoroethyl trifluoromethanesulfonate (6226-25-1) |
| | 4M-HCl/MTHP |

**[Table 1-80]**

| | |
|---|---|
| 1-1-175 | 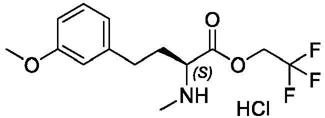 |
| | 2,2,2-Trifluoroethyl (S)-4-(3-methoxyphenyl)-2-(methylamino)butanoate hydrochloride |
| | LC-MS (ESI) m/z [M+H]⁺: 306.05 |
| | tR 5.236 min (A) |
| | (S)-2-((tert-Butoxycarbonyl)(methyl)amino)-4-(3-methoxyphenyl)butanoic acid (A-19094, Amatek Chemical Co., Ltd.) |
| | 2,2,2-Trifluoroethyl trifluoromethanesulfonate (6226-25-1) |
| | 4M-HCl/MTHP |
| 1-1-176 | 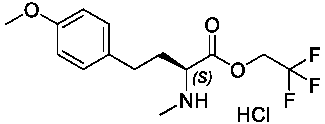 |
| | 2,2,2-Trifluoroethyl (S)-4-(4-methoxyphenyl)-2-(methylamino)butanoate hydrochloride |
| | LC-MS (ESI) m/z [M+H]⁺: 306.12 |
| | tR 3.136 min (B) |
| | (S)-2-((tert-Butoxycarbonyl)(methyl)amino)-4-(4-methoxyphenyl)butanoic acid (A-19093, Amatek Chemical Co., Ltd.) |
| | 2,2,2-Trifluoroethyl trifluoromethanesulfonate (6226-25-1) |
| | 4M-HCl/MTHP |

**[Table 1-81]**

| | |
|---|---|
| 1-1-177 | 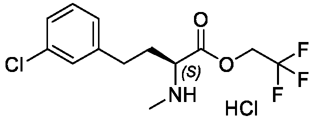 |
| | 2,2,2-Trifluoroethyl (S)-4-(3-chlorophenyl)-2-(methylamino)butanoate hydrochloride |
| | LC-MS (ESI) m/z [M+H]⁺: 309.99 |
| | tR 3.500 min (B) |
| | (S)-2-((tert-Butoxycarbonyl)(methyl)amino)-4-(3-chlorophenyl)butanoic acid (A-19092, Amatek Chemical Co., Ltd.) |
| | 2,2,2-Trifluoroethyl trifluoromethanesulfonate (6226-25-1) |
| | 4M-HCl/MTHP |
| 1-1-178 | 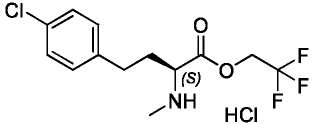 |
| | 2,2,2-Trifluoroethyl (S)-4-(4-chlorophenyl)-2-(methylamino)butanoate hydrochloride |
| | LC-MS (ESI) m/z [M+H]⁺: 309.97 |
| | tR 3.496 min (B) |
| | (S)-2-((tert-Butoxycarbonyl)(methyl)amino)-4-(3-methoxyphenyl)butanoic acid (A-19091, Amatek Chemical Co., Ltd.) |
| | 2,2,2-Trifluoroethyl trifluoromethanesulfonate (6226-25-1) |
| | 4M-HCl/MTHP |

**[Table 1-82]**

| | |
|---|---|
| 1-1-179 | 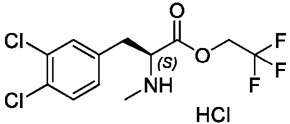 |
| | 2,2,2-Trifluoroethyl (S)-3-(3,4-dichlorophenyl)-2-(methylamino)propanoate hydrochloride |
| | LC-MS (ESI) m/z [M+H]⁺: 329.92 |
| | tR 3.604 min (B) |
| | (S)-2-((tert-Butoxycarbonyl)(methyl)amino)-3-(3,4-dichlorophenyl)propanoic acid (A-19067, Amatek Chemical) |
| | 2,2,2-Trifluoroethyl trifluoromethanesulfonate (6226-25-1) |
| | 4M-HCl/MTHP |
| 1-1-180 | 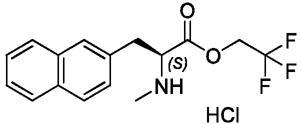 |
| | 2,2,2-Trifluoroethyl (S)-2-(methylamino)-3-(naphthalen-2-yl)propanoate hydrochloride |
| | LC-MS (ESI) m/z [M+H]⁺: 311.98 |
| | tR 3.652 min (B) |
| | (S)-2-((tert-Butoxycarbonyl)(methyl)amino)-3-(naphthalen-2-yl)propanoic acid (145232-51-5) |
| | 2,2,2-Trifluoroethyl trifluoromethanesulfonate (6226-25-1) |
| | 4M-HCl/MTHP |

**[Table 1-83]**

| | |
|---|---|
| 1-1-181 | 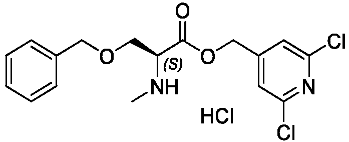 |
| | (2,6-Dichloropyridin-4-yl)methyl O-benzyl-N-methyl-L-serinate hydrochloride |
| | LC-MS (ESI) m/z [M+H]⁺: 369.02 |
| | tR 3.648 min (B) |
| | O-Benzyl-N-(tert-butoxycarbonyl)-N-methyl-L-serine (64263-84-9) |
| | 4-(Bromomethyl)-2,6-dichloropyridine (175204-45-2) |
| | 4M-HCl/CPME |
| 1-1-182 | 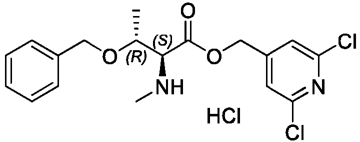 |
| | (2,6-Dichloropyridin-4-yl)methyl O-benzyl-N-methyl-L-threoninate hydrochloride |
| | LC-MS (ESI) m/z [M+H]⁺: 383.04 |
| | tR 3.772 min (B) |
| | O-Benzyl-N-(tert-butoxycarbonyl)-N-methyl-L-threonine (64263-80-5) |
| | 4-(Bromomethyl)-2,6-dichloropyridine (175204-45-2) |
| | 4M-HCl/CPME |

**[Table 1-84]**

| | |
|---|---|
| 1-1-183 | 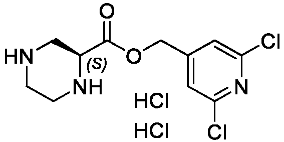 |
| | (2,6-Dichloropyridin-4-yl)methyl (S)-piperazine-2-carboxylate dihydrochloride |
| | LC-MS (ESI) m/z [M+H]⁺: 289.97 |
| | tR 4.788 min (C) |
| | (S)-1,4-Bis(tert-butoxycarbonyl)piperazine-2-carboxylic acid (788799-69-9) |
| | 4-(Bromomethyl)-2,6-dichloropyridine (175204-45-2) |
| | 4M-HCl/MTHP |
| 1-1-184 | 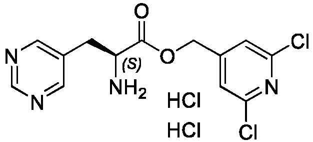 |
| | (2,6-Dichloropyridin-4-yl)methyl (S)-2-amino-3-(pyrimidin-5-yl)propanoate dihydrochloride |
| | LC-MS (ESI) m/z [M+H]⁺: 327.00 |
| | tR 3.912 min (A) |
| | (S)-2-((tert-Butoxycarbonyl)amino)-3-(pyrimidin-5-yl)propanoic acid (1251904-63-8) |
| | 4-(Bromomethyl)-2,6-dichloropyridine (175204-45-2) |
| | 4M-HCl/MTHP |

**[Table 1-85]**

| | |
|---|---|
| 1-1-185 | 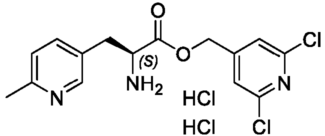 |
| | (2,6-Dichloropyridin-4-yl)methyl (S)-2-amino-3-(6-methylpyridin-3-yl)propanoate dihydrochloride |
| | LC-MS (ESI) m/z [M+H]⁺: 340.0 |
| | tR 3.120 min (A) |
| | (S)-2-((tert-Butoxycarbonyl)amino)-3-(6-methylpyridin-3-yl)propanoic acid (1992820-29-7) |
| | 4-(Bromomethyl)-2,6-dichloropyridine (175204-45-2) |
| | 4M-HCl/MTHP |
| 1-1-186 | 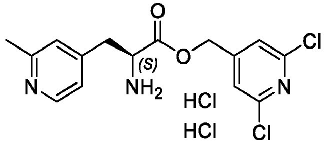 |
| | (2,6-Dichloropyridin-4-yl)methyl (S)-2-amino-3-(2-methylpyridin-4-yl)propanoate dihydrochloride |
| | LC-MS (ESI) m/z [M+H]⁺: 340.02 |
| | tR 2.844 min (A) |
| | (S)-2-((tert-Butoxycarbonyl)amino)-3-(2-methylpyridin-4-yl)propanoic acid (2281916-98-9) |
| | 4-(Bromomethyl)-2,6-dichloropyridine (175204-45-2) |
| | 4M-HCl/MTHP |

**[Table 1-86]**

| | |
|---|---|
| 1-1-187 | 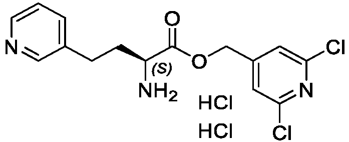 |
| | (2,6-Dichloropyridin-4-yl)methyl (S)-2-amino-4-(pyridin-3-yl)butanoate dihydrochloride |
| | LC-MS (ESI) m/z [M+H]⁺: 340.02 |
| | tR 3.176 min (A) |
| | (S)-2-((tert-Butoxycarbonyl)amino)-4-(pyridin-3-yl)butanoic acid (99461-44-6) |
| | 4-(Bromomethyl)-2,6-dichloropyridine (175204-45-2) |
| | 4M-HCl/MTHP |
| 1-1-188 | 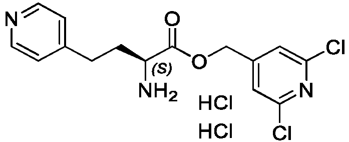 |
| | (2,6-Dichloropyridin-4-yl)methyl (S)-2-amino-4-(pyridin-4-yl)butanoate dihydrochloride |
| | LC-MS (ESI) m/z [M+H]⁺: 339.97 |
| | tR 3.280 min (C) |
| | (S)-2-((tert-Butoxycarbonyl)amino)-4-(pyridin-4-yl)butanoic acid (273222-03-0) |
| | 4-(Bromomethyl)-2,6-dichloropyridine (175204-45-2) |
| | 4M-HCl/MTHP |

**[Table 1-87]**

| | |
|---|---|
| 1-1-189 | 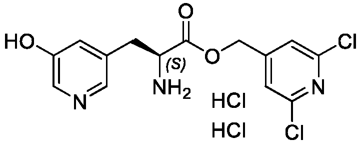 |
| | (2,6-Dichloropyridin-4-yl)methyl (S)-2-amino-3-(5-hydroxypyridin-3-yl)propanoate dihydrochloride |
| | LC-MS (ESI) m/z [M+H]⁺: 341.99 |
| | tR 3.116 min (A) |
| | (S)-2-((tert-Butoxycarbonyl)amino)-3-(5-hydroxypyridin-3-yl)propanoic acid (1241679-66-2) |
| | 4-(Bromomethyl)-2,6-dichloropyridine (175204-45-2) |
| | 4M-HCl/MTHP |
| 1-1-190 | 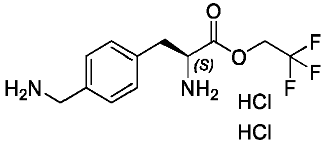 |
| | 2,2,2-Trifluoroethyl (S)-2-amino-3-(4-(aminomethyl)phenyl)propanoate dihydrochloride |
| | LC-MS (ESI) m/z [M+H]⁺: 277.04 |
| | tR 3.980 min (C) |
| | (S)-2-(((tert-Butoxycarbonyl)amino)-3-(4-((tert-butoxycarbonyl)amino)methyl)phenyl)propanoic acid (1212909-48-2) |
| | 2,2,2-Trifluoroethyl trifluoromethanesulfonate (6226-25-1) |
| | 4M-HCl/MTHP |

**[Table 1-88]**

| | |
|---|---|
| 1-1-191 | 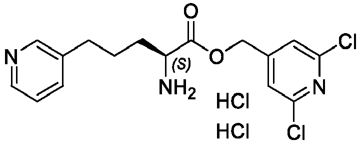 |
| | (2,6-Dichloropyridin-4-yl)methyl (S)-2-amino-5-(pyridin-3-yl)pentanoate dihydrochloride |
| | LC-MS (ESI) m/z [M+H]⁺: 3.5394 |
| | tR 3.224 min (A) |
| | (S)-2-((tert-Butoxycarbonyl)amino)-5-(pyridin-3-yl)pentanoic acid (2349518-36-9) |
| | 4-(Bromomethyl)-2,6-dichloropyridine (175204-45-2) |
| | 4M-HCl/MTHP |
| 1-1-192 | 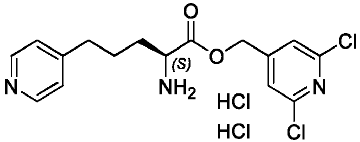 |
| | (2,6-Dichloropyridin-4-yl)methyl (S)-2-amino-5-(pyridin-4-yl)pentanoate dihydrochloride |
| | LC-MS (ESI) m/z [M+H]⁺: 354.01 |
| | tR 2.704 min (A) |
| | (S)-2-((tert-Butoxycarbonyl)amino)-5-(pyridin-4-yl)pentanoic acid (2349528-84-1) |
| | 4-(Bromomethyl)-2,6-dichloropyridine (175204-45-2) |
| | 4M-HCl/MTHP |

**[Table 1-89]**

| | |
|---|---|
| 1-1-193 | 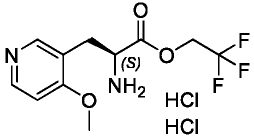 |
| | 2,2,2-Trifluoroethyl (S)-2-amino-3-(4-methoxypyridin-3-yl)propanoate dihydrochloride |
| | LC-MS (ESI) m/z [M+H]⁺: 278.99 |
| | tR 3.012 min (C) |
| | (S)-2-((tert-Butoxycarbonyl)amino)-3-(4-methoxypyridin-3-yl)propanoic acid (2350395-56-9) |
| | 2,2,2-Trifluoroethyl trifluoromethanesulfonate (6226-25-1) |
| | 4M-HCl/MTHP |
| 1-1-194 | 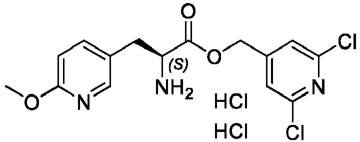 |
| | (2,6-Dichloropyridin-4-yl)methyl (S)-2-amino-3-(6-methoxypyridin-3-yl)propanoate dihydrochloride |
| | LC-MS (ESI) m/z [M+H]⁺: 356.03 |
| | tR 3.100 min (B) |
| | (S)-2-((tert-Butoxycarbonyl)amino)-3-(6-methoxypyridin-3-yl)propanoic acid (879559-97-4) |
| | 4-(Bromomethyl)-2,6-dichloropyridine (175204-45-2) |
| | 4M-HCl/MTHP |

**[Table 1-90]**

| | |
|---|---|
| 1-1-195 | 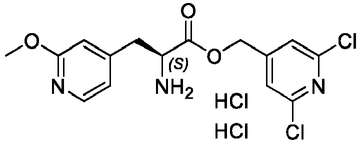 |
| | (2,6-Dichloropyridin-4-yl)methyl (S)-2-amino-3-(2-methoxypyridin-4-yl)propanoate dihydrochloride |
| | LC-MS (ESI) m/z [M+H]⁺: 356.04 |
| | tR 4.424 min (A) |
| | (S)-2-((tert-Butoxycarbonyl)amino)-3-(2-methoxypyridin-4-yl)propanoic acid (1430057-99-0) |
| | 4-(Bromomethyl)-2,6-dichloropyridine (175204-45-2) |
| | 4M-HCl/MTHP |
| 1-1-196 | 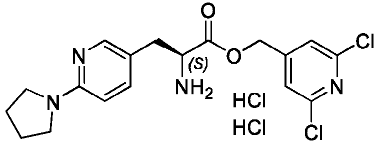 |
| | (2,6-Dichloropyridin-4-yl)methyl (S)-2-amino-3-(6-(pyrrolidin-1-yl)pyridin-3-yl)propanoate dihydrochloride |
| | LC-MS (ESI) m/z [M+H]⁺: 395.05 |
| | tR 5.180 min (C) |
| | (S)-2-((tert-Butoxycarbonyl)amino)-3-(6-(pyrrolidin-1-yl)pyridin-3-yl)propanoic acid (2350426-82-1) |
| | 4-(Bromomethyl)-2,6-dichloropyridine (175204-45-2) |
| | 4M-HCl/MTHP |

**[Table 1-91]**

| | |
|---|---|
| 1-1-197 | 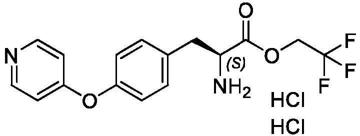 |
| | 2,2,2-Trifluoroethyl (S)-2-amino-3-(4-(pyridin-4-yloxy)phenyl)propanoate dihydrochloride |
| | LC-MS (ESI) m/z [M+H]⁺: 340.99 |
| | tR 4.756 min (C) |
| | (S)-2-((tert-Butoxycarbonyl)amino)-3-(4-(pyridin-4-yloxy)phenyl)propanoic acid (TM00050, Amatek Chemical Co., Ltd.) |
| | 2,2,2-Trifluoroethyl trifluoromethanesulfonate (6226-25-1) |
| | 4M-HCl/MTHP |
| 1-1-198 | 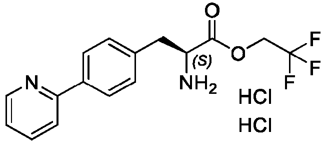 |
| | 2,2,2-Trifluoroethyl (S)-2-amino-3-(4-(pyridin-2-yl)phenyl)propanoate dihydrochloride |
| | LC-MS (ESI) m/z [M+H]⁺: 325.02 |
| | tR 3.984 min (A) |
| | (S)-2-((tert-Butoxycarbonyl)amino)-3-(4-(pyridin-2-yl)phenyl)propanoic acid (1211448-59-7) |
| | 2,2,2-Trifluoroethyl trifluoromethanesulfonate (6226-25-1) |
| | 4M-HCl/CPME |

**[Table 1-92]**

| | |
|---|---|
| 1-1-199 | 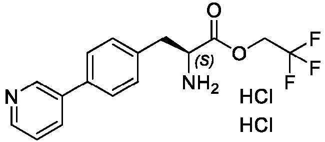 |
| | 2,2,2-Trifluoroethyl (S)-2-amino-3-(4-(pyridin-3-yl)phenyl)propanoate dihydrochloride |
| | LC-MS (ESI) m/z [M+H]⁺: 324.96 |
| | tR 3.436 min (A) |
| | (S)-2-((tert-Butoxycarbonyl)amino)-3-(4-(pyridin-3-yl)phenyl)propanoic acid (1211448-60-0) |
| | 2,2,2-Trifluoroethyl trifluoromethanesulfonate (6226-25-1) |
| | 4M-HCl/CPME |
| 1-1-200 | 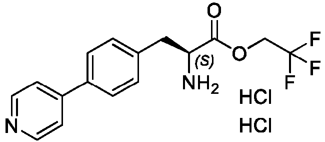 |
| | 2,2,2-Trifluoroethyl (S)-2-amino-3-(4-(pyridin-4-yl)phenyl)propanoate dihydrochloride |
| | LC-MS (ESI) m/z [M+H]⁺: 325.06 |
| | tR 3.200 min (A) |
| | (S)-2-((tert-Butoxycarbonyl)amino)-3-(4-(pyridin-4-yl)phenyl)propanoic acid (1211448-61-1) |
| | 2,2,2-Trifluoroethyl trifluoromethanesulfonate (6226-25-1) |
| | 4M-HCl/CPME |

**[Table 1-93]**

| | |
|---|---|
| 1-1-201 | 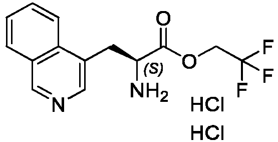 |
| | 2,2,2-Trifluoroethyl (S)-2-amino-3-(isoquinolin-4-yl)propanoate dihydrochloride |
| | LC-MS (ESI) m/z [M+H]⁺: 299.07 |
| | tR 3.224 min (A) |
| | 2-((tert-Butoxycarbonyl)amino)-3-(isoquinolin-4-yl)propanoic acid (2002509-06-8) |
| | 2,2,2-Trifluoroethyl trifluoromethanesulfonate (6226-25-1) |
| | 4M-HCl/MTHP |
| 1-1-202 | 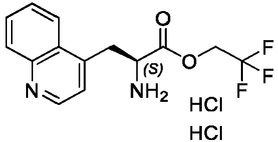 |
| | 2,2,2-Trifluoroethyl (S)-2-amino-3-(quinolin-4-yl)propanoate dihydrochloride |
| | LC-MS (ESI) m/z [M+H]⁺: 298.94 |
| | tR 3.344 min (A) |
| | (S)-2-((tert-butoxycarbonyl)amino)-3-(quinolin-4-yl)propanoic acid (2015385-62-1) |
| | 2,2,2-Trifluoroethyl trifluoromethanesulfonate (6226-25-1) |
| | 4M-HCl/MTHP |

**[Table 1-94]**

| | |
|---|---|
| 1-1-203 | 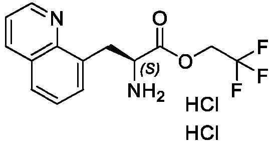 |
| | 2,2,2-Trifluoroethyl (S)-2-amino-3-(quinolin-8-yl)propanoate dihydrochloride |
| | LC-MS (ESI) m/z [M+H]⁺: 299.02 |
| | tR 3.044 min (B) |
| | (S)-2-((tert-Butoxycarbonyl)amino)-3-(quinolin-8-yl)propanoic acid (200864-51-3) |
| | 2,2,2-Trifluoroethyl trifluoromethanesulfonate (6226-25-1) |
| | 4M-HCl/MTHP |
| 1-1-204 | 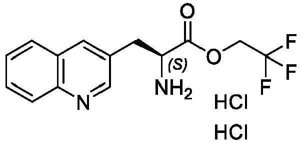 |
| | 2,2,2-Trifluoroethyl (S)-2-amino-3-(quinolin-3-yl)propanoate dihydrochloride |
| | LC-MS (ESI) m/z [M+H]⁺: 299.09 |
| | tR 3.452 min (A) |
| | (S)-2-((tert-Butoxycarbonyl)amino)-3-(quinolin-3-yl)propanoic acid (135101-20-1) |
| | 2,2,2-Trifluoroethyl trifluoromethanesulfonate (6226-25-1) |
| | 4M-HCl/MTHP |

**[Table 1-95]**

| | |
|---|---|
| 1-1-205 | 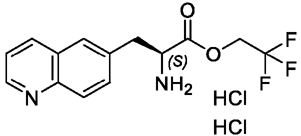 |
| | 2,2,2-Trifluoroethyl (S)-2-amino-3-(quinolin-6-yl)propanoate dihydrochloride |
| | LC-MS (ESI) m/z [M+H]⁺: 299.02 |
| | tR 3.152 min (A) |
| | (S)-2-((tert-Butoxycarbonyl)amino)-3-(quinolin-6-yl)propanoic acid (162677-20-5) |
| | 2,2,2-Trifluoroethyl trifluoromethanesulfonate (6226-25-1) |
| | 4M-HCl/MTHP |
| 1-1-206 | 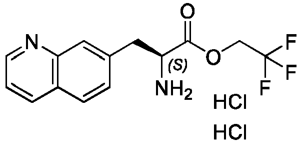 |
| | 2,2,2-Trifluoroethyl (S)-2-amino-3-(quinolin-7-yl)propanoate dihydrochloride |
| | LC-MS (ESI) m/z [M+H]⁺: 299.03 |
| | tR 3.312 min (A) |
| | (S)-2-((tert-Butoxycarbonyl)amino)-3-(quinolin-7-yl)propanoic acid (2349646-35-9) |
| | 2,2,2-Trifluoroethyl trifluoromethanesulfonate (6226-25-1) |
| | 4M-HCl/MTHP |

**[Table 1-96]**

| | |
|---|---|
| 1-1-207 | 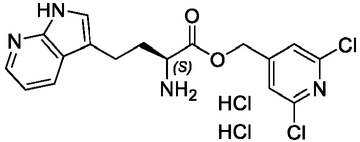 |
| | (2,6-Dichloropyridin-4-yl)methyl (S)-2-amino-4-(1H-pyrrolo[2,3-b]pyridin-3-yl)butanoate dihydrochloride |
| | LC-MS (ESI) m/z [M+H]⁺: 379.04 |
| | tR 2.644 min (B) |
| | (S)-2-((tert-Butoxycarbonyl)amino)-4-(1H-pyrrolo[2,3-b]pyridin-3-yl)butanoic acid (2349312-77-0) |
| | 4-(Bromomethyl)-2,6-dichloropyridine (175204-45-2) |
| | 4M-HCl/CPME |
| 1-1-208 | 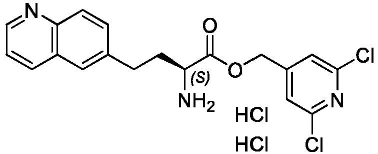 |
| | (2,6-Dichloropyridin-4-yl)methyl (S)-2-amino-4-(quinolin-6-yl)butanoate dihydrochloride |
| | LC-MS (ESI) m/z [M+H]⁺: 390.04 |
| | tR 4.316 min (A) |
| | (S)-2-((tert-Butoxycarbonyl)amino)-4-(quinolin-6-yl)butanoic acid (2350783-85-4) |
| | 4-(Bromomethyl)-2,6-dichloropyridine (175204-45-2) |
| | 4M-HCl/MTHP |

**[Table 1-97]**

| | |
|---|---|
| 1-1-209 | 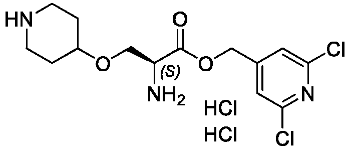 |
| | (2,6-Dichloropyridin-4-yl)methyl O-(piperidin-4-yl)-L-serinate dihydrochloride |
| | LC-MS (ESI) m/z [M+H]⁺: 347.99 |
| | tR 4.136 min (C) |
| | N-(tert-Butoxycarbonyl)-O-(piperidin-4-yl)-L-serine (K26638, Mimotopes) |
| | 4-(Bromomethyl)-2,6-dichloropyridine (175204-45-2) |
| | 4M-HCl/MTHP |
| 1-1-210 | 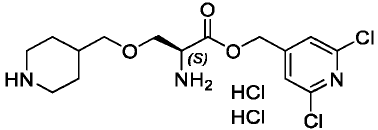 |
| | (2,6-Dichloropyridin-4-yl)methyl O-(piperidin-4-ylmethyl)-L-serinate dihydrochloride |
| | LC-MS (ESI) m/z [M+H]⁺: 362.05 |
| | tR 4.768 min (C) |
| | N-(tert-Butoxycarbonyl)-O-((1-(tert-butoxycarbonyl)piperidin-4-yl)methyl)-L-serine (K26642, Mimotopes) |
| | 4-(Bromomethyl)-2,6-dichloropyridine (175204-45-2) |
| | 4M-HCl/MTHP |

**[Table 1-98]**

| | |
|---|---|
| 1-1-211 | 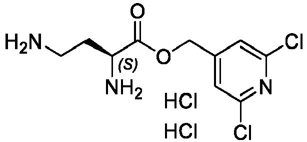 |
| | (2,6-Dichloropyridin-4-yl)methyl (S)-2,4-diaminobutanoate dihydrochloride |
| | LC-MS (ESI) m/z [M+H]⁺: 277.97 |
| | tR 4.656 min (C) |
| | (S)-2,4-bis((tert-Butoxycarbonyl)amino)butanoic acid (34404-27-8) |
| | 4-(Bromomethyl)-2,6-dichloropyridine (175204-45-2) |
| | 4M-HCl/MTHP |
| 1-1-212 | 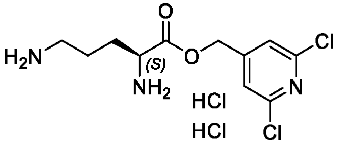 |
| | (2,6-Dichloropyridin-4-yl)methyl (S)-2,5-diaminopentanoate dihydrochloride |
| | LC-MS (ESI) m/z [M+H]⁺: 291.98 |
| | tR 4.916 min (C) |
| | (S)-2,5-bis((tert-Butoxycarbonyl)amino)pentanoic acid (57133-29-6) |
| | 4-(Bromomethyl)-2,6-dichloropyridine (175204-45-2) |
| | 4M-HCl/MTHP |

**[Table 1-99]**

| | |
|---|---|
| 1-1-213 | 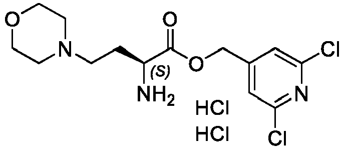 |
| | (2,6-Dichloropyridin-4-yl)methyl (S)-2-amino-4-morpholinobutanoate dihydrochloride |
| | LC-MS (ESI) m/z [M+H]⁺: 347.98 |
| | tR 4.140 min (C) |
| | (S)-2-((tert-Butoxycarbonyl)amino)-4-morpholinobutanoic acid (879219-12-2) |
| | 4-(Bromomethyl)-2,6-dichloropyridine (175204-45-2) |
| | 4M-HCl/MTHP |
| 1-1-214 | 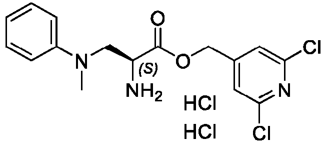 |
| | (2,6-Dichloropyridin-4-yl)methyl (S)-2-amino-3-(methyl(phenyl)amino)propanoate dihydrochloride |
| | LC-MS (ESI) m/z [M+H]⁺: 354.03 |
| | tR 3.632 min (B) |
| | (S)-2-((tert-Butoxycarbonyl)amino)-3-(methyl(phenyl)amino)propanoic acid (M04119, Watanabe Chemical) |
| | 4-(Bromomethyl)-2,6-dichloropyridine (175204-45-2) |
| | 4M-HCl/MTHP |

**[Table 1-100]**

| | |
|---|---|
| 1-1-215 | 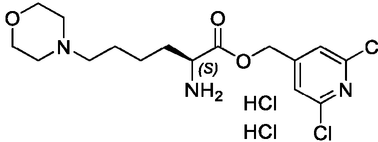 I |
| | (2,6-Dichloropyridin-4-yl)methyl (S)-2-amino-6-morpholinohexanoate dihydrochloride |
| | LC-MS (ESI) m/z [M+H]⁺: 376.05 |
| | tR 4.524 min (C) |
| | (S)-2-((tert-Butoxycarbonyl)amino)-6-morpholinohexanoic acid (2349732-93-8) |
| | 4-(Bromomethyl)-2,6-dichloropyridine (175204-45-2) |
| | 4M-HCl/MTHP |
| 1-1-216 | 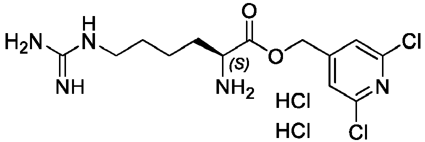 |
| | (2,6-Dichloropyridin-4-yl)methyl N⁶-carbamimidoyl-L-lysinate dihydrochloride |
| | LC-MS (ESI) m/z [M+H]⁺: 348.02 |
| | tR 3.404 min (A) |
| | N²-(tert-Butoxycarbonyl)-N⁶-(2,2,10,10-tetramethyl-4,8-dioxo-3,9-dioxa-5,7-diazaundecan-6-ylidene)-L-lysine (605654-82-8) |
| | 4-(Bromomethyl)-2,6-dichloropyridine (175204-45-2) |
| | 4M-HCl/MTHP |

**[Table 1-101]**

| | |
|---|---|
| 1-1-217 | 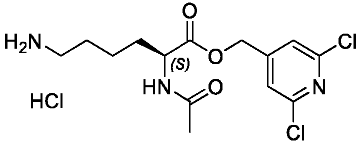 |
| | (2,6-Dichloropyridin-4-yl)methyl acetyl-L-lysinate hydrochloride |
| | LC-MS (ESI) m/z [M+H]⁺: 348.02 |
| | tR 2.856 min (B) |
| | N²-Acetyl-N⁶-(tert-Butoxycarbonyl)-L-lysine (23500-04-1) |
| | 4-(Bromomethyl)-2,6-dichloropyridine (175204-45-2) |
| | 4M-HCl/MTHP |
| 1-1-218 | 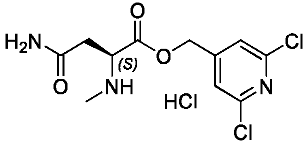 |
| | (2,6-Dichloropyridin-4-yl)methyl methyl-L-asparaginate hydrochloride |
| | LC-MS (ESI) m/z [M+H]⁺: 305.97 |
| | tR 3.220 min (A) |
| | N²-(tert-Butoxycarbonyl)-N²-methyl-N⁴-trityl-L-asparagine (M04100, Watanabe Chemical) |
| | 4-(Bromomethyl)-2,6-dichloropyridine (175204-45-2) |
| | 2,2,2-Trifluoroacetic acid (76-05-1) and triisopropylsilane (6485-79-6) |

**[Table 1-102]**

| | |
|---|---|
| 1-1-219 | 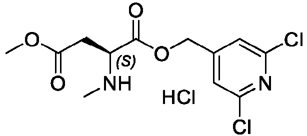 |
| | 1-((2,6-Dichloropyridin-4-yl)methyl)4-methyl methyl-L-aspartate hydrochloride |
| | LC-MS (ESI) m/z [M+H]⁺: 320.96 |
| | tR 4.512 min (A) |
| | (S)-2-((tert-Butoxycarbonyl)(methyl)amino)-4-methoxy-4-oxobutanoic acid (2044709-98-8) |
| | 4-(Bromomethyl)-2,6-dichloropyridine (175204-45-2) |
| | 4M-HCl/MTHP |
| 1-1-220 | 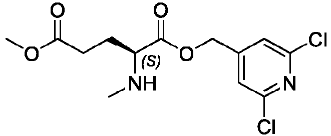 |
| | 1-((2,6-Dichloropyridin-4-yl)methyl)5-methyl methyl-L-glutamate hydrochloride |
| | LC-MS (ESI) m/z [M+H]⁺: 334.99 |
| | tR 2.880 min (B) |
| | (S)-2-((tert-Butoxycarbonyl)(methyl)amino)-5-methoxy-5-oxopentanoic acid (2044710-75-8) |
| | 4-(Bromomethyl)-2,6-dichloropyridine (175204-45-2) |
| | 4M-HCl/MTHP |

**[Table 1-103]**

| | |
|---|---|
| 1-1-221 | 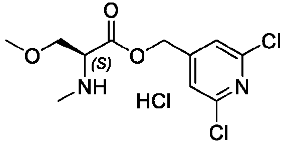 |
| | (2,6-Dichloropyridin-4-yl)methyl N,O-dimethyl-L-serinate hydrochloride |
| | LC-MS (ESI) m/z [M+H]⁺: 292.92 |
| | tR 4.188 min (A) |
| | N-(tert-Butoxycarbonyl)-N,O-dimethyl-L-serine (184104-28-7) |
| | 4-(Bromomethyl)-2,6-dichloropyridine (175204-45-2) |
| | 4M-HCl/MTHP |
| 1-1-222 | 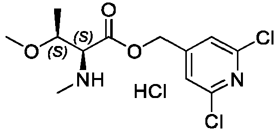 |
| | (2,6-Dichloropyridin-4-yl)methyl N,O-dimethyl-L-allothreoninate hydrochloride |
| | LC-MS (ESI) m/z [M+H]⁺: 306.96 |
| | tR 4.808 min (A) |
| | N-(tert-Butoxycarbonyl)-N,O-dimethyl-L-allothreonine (C132541, CRI Life Sciences Inc.) |
| | 4-(Bromomethyl)-2,6-dichloropyridine (175204-45-2) |
| | 4M-HCl/MTHP |

**[Table 1-104]**

| | |
|---|---|
| 1-1-223 | 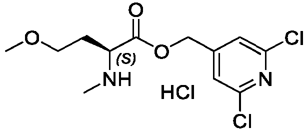 |
| | 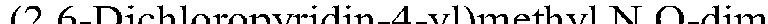 (2,6-dichloropyridin-4yl)metyl N,O-dimethyl-L-homoserinate hydrochloride |
| | LC-MS (ESI) m/z [M+H]⁺: 306.96 |
| | tR 4.512 min (A) |
| | N-(tert-Butoxycarbonyl)-N,O-dimethyl-L-homoserine (862372-17-6) |
| | 4-(Bromomethyl)-2,6-dichloropyridine (175204-45-2) |
| | 4M-HCl/MTHP |
| 1-1-224 | 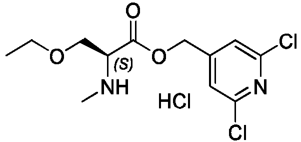 |
| | (2,6-Dichloropyridin-4-yl)methyl O-ethyl-N-methyl-L-serinate hydrochloride |
| | LC-MS (ESI) m/z [M+H]⁺: 307.00 |
| | tR 4.368 min (A) |
| | N-(tert-Butoxycarbonyl)-O-ethyl-N-methyl-L-serine (R00810, Nanjing Peptide Biotechnology Co., Ltd.) |
| | 4-(Bromomethyl)-2,6-dichloropyridine (175204-45-2) |
| | 4M-HCl/MTHP |

**[Table 1-105]**

| | |
|---|---|
| 1-1-225 | 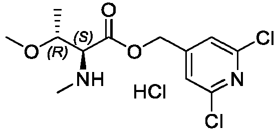 |
| | (2,6-Dichloropyridin-4-yl)methyl N,O-dimethyl-L-threoninate hydrochloride |
| | LC-MS (ESI) m/z [M+H]⁺: 306.98 |
| | tR 2.860 min (B) |
| | N-(tert-Butoxycarbonyl)-N,O-dimethyl-L-threonine (136092-75-6) |
| | 4-(Bromomethyl)-2,6-dichloropyridine (175204-45-2) |
| | 4M-HCl/MTHP |
| 1-1-226 | 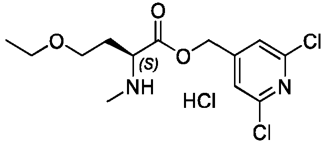 |
| | (2,6-Dichloropyridin-4-yl)methyl O-ethyl-N-methyl-L-homoserinate hydrochloride |
| | LC-MS (ESI) m/z [M+H]⁺: 321.01 |
| | tR 4.848 min (A) |
| | N-(tert-Butoxycarbonyl)-O-ethyl-N-methyl-L-homoserine (K23292, Mimotopes) |
| | 4-(Bromomethyl)-2,6-dichloropyridine (175204-45-2) |
| | 4M-HCl/MTHP |

**[Table 1-106]**

| | |
|---|---|
| 1-1-227 | 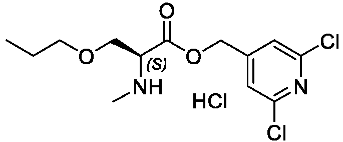 |
| | (2,6-Dichloropyridin-4-yl)methyl N-methyl-O-propyl-L-serinate hydrochloride |
| | LC-MS (ESI) m/z [M+H]⁺: 320.94 |
| | tR 3.276 min (B) |
| | N-(tert-Butoxycarbonyl)-N-methyl-O-propyl-L-serine (R00796, Nanjing Peptide Biotechnology Co., Ltd.) |
| | 4-(Bromomethyl)-2,6-dichloropyridine (175204-45-2) |
| | 4M-HCl/MTHP |
| 1-6-228 | 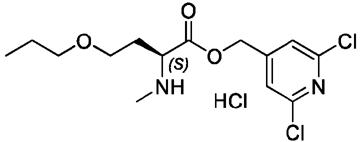 |
| | (2,6-Dichloropyridin-4-yl)methyl N-methyl-O-propyl-L-homoserinate hydrochloride |
| | LC-MS (ESI) m/z [M+H]⁺: 334.97 |
| | tR 3.404 min (B) |
| | N-(tert-Butoxycarbonyl)-N-methyl-O-propyl-L-homoserine (R00798, Nanjing Peptide Biotechnology Co., Ltd.) |
| | 4-(Bromomethyl)-2,6-dichloropyridine (175204-45-2) |
| | 4M-HCl/MTHP |

**[Table 1-107]**

| | |
|---|---|
| 1-1-229 | 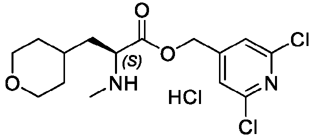 |
| | (2,6-Dichloropyridin-4-yl)methyl (S)-2-(methylamino)-3-(tetrahydro-2H-pyran-4-yl)propanoate hydrochloride |
| | LC-MS (ESI) m/z [M+H]⁺: 347.02 |
| | tR 4.692 min (A) |
| | (S)-2-((tert-Butoxycarbonyl)(methyl)amino)-3-(tetrahydro-2H-pyran-4-yl)propanoic acid (1093865-13-4) |
| | 4-(Bromomethyl)-2,6-dichloropyridine (175204-45-2) |
| | 4M-HCl/MTHP |
| 1-1-230 | 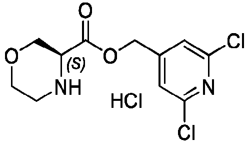 |
| | (2,6-Dichloropyridin-4-yl)methyl (S)-morpholine-3-carboxylate hydrochloride |
| | LC-MS (ESI) m/z [M+H]⁺: 290.93 |
| | tR 2.512 min (B) |
| | (S)-4-(tert-Butoxycarbonyl)morpholine-3-carboxylic acid (783350-37-8) |
| | 4-(Bromomethyl)-2,6-dichloropyridine (175204-45-2) |
| | 4M-HCl/MTHP |

**[Table 1-108]**

| | |
|---|---|
| 1-1-231 | 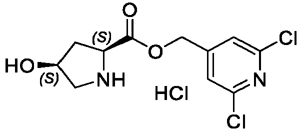 |
| | (2,6-Dichloropyridin-4-yl)methyl (2S,4S)-4-hydroxypyrrolidine-2-carboxylate hydrochloride |
| | LC-MS (ESI) m/z [M+H]⁺: 290.94 |
| | tR 3.516 min (A) |
| | (2S,4S)-1-(tert-Butoxycarbonyl)-4-hydroxypyrrolidine-2-carboxylic acid (87691-27-8) |
| | 4-(Bromomethyl)-2,6-dichloropyridine (175204-45-2) |
| | 4M-HCl/MTHP |
| 1-1-232 | 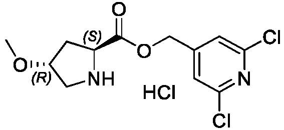 |
| | (2,6-Dichloropyridin-4-yl)methyl (2S,4R)-4-methoxypyrrolidine-2-carboxylate hydrochloride |
| | LC-MS (ESI) m/z [M+H]⁺: 305.01 |
| | tR 4.576 min (A) |
| | (2S,4R)-1-(tert-Butoxycarbonyl)-4-methoxypyrrolidine-2-carboxylic acid (83624-01-5) |
| | 4-(Bromomethyl)-2,6-dichloropyridine (175204-45-2) |
| | 4M-HCl/MTHP |

**[Table 1-109]**

| | |
|---|---|
| 1-1-233 | 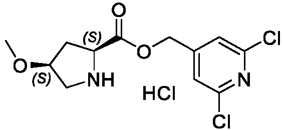 |
| | (2,6-Dichloropyridin-4-yl)methyl (2S,4S)-4-methoxypyrrolidine-2-carboxylate hydrochloride |
| | LC-MS (ESI) m/z [M+H]⁺: 304.97 |
| | tR 4.580 min (A) |
| | (2S,4S)-1-(tert-Butoxycarbonyl)-4-methoxypyrrolidine-2-carboxylic acid (83623-93-2) |
| | 4-(Bromomethyl)-2,6-dichloropyridine (175204-45-2) |
| | 4M-HCl/MTHP |
| 1-1-234 | 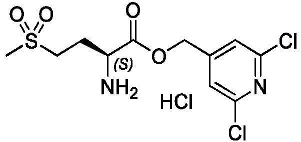 |
| | (2,6-Dichloropyridin-4-yl)methyl (S)-2-amino-4-(methylsulfonyl)butanoate hydrochloride |
| | LC-MS (ESI) m/z [M+H]⁺: 340.92 |
| | tR 3.396 min (A) |
| | (S)-2-((tert-Butoxycarbonyl)amino)-4-(methylsulfonyl)butanoic acid (60280-45-7) |
| | 4-(Bromomethyl)-2,6-dichloropyridine (175204-45-2) |
| | 4M-HCl/CPME |

**[Table 1-110]**

| | |
|---|---|
| 1-1-235 | 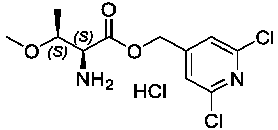 |
| | (2,6-Dichloropyridin-4-yl)methyl O-methyl-L-allothreoninate hydrochloride |
| | LC-MS (ESI) m/z [M+H]⁺: 292.97 |
| | tR 4.728 min (A) |
| | N-(tert-Butoxycarbonyl)-O-methyl-L-allothreonine (630424-73-6) |
| | 4-(Bromomethyl)-2,6-dichloropyridine (175204-45-2) |
| | 4M-HCl/MTHP |
| 1-1-236 | 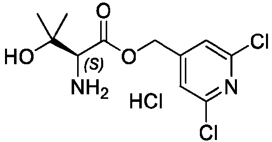 |
| | (2,6-Dichloropyridin-4-yl)methyl (S)-2-amino-3-hydroxy-3-methylbutanoate hydrochloride |
| | LC-MS (ESI) m/z [M+H]⁺: 292.95 |
| | tR 3.652 min (A) |
| | (S)-2-((tert-Butoxycarbonyl)amino)-3-hydroxy-3-methylbutanoic acid (102507-13-1) |
| | 4-(Bromomethyl)-2,6-dichloropyridine (175204-45-2) |
| | 4M-HCl/MTHP |

**[Table 1-111]**

| | |
|---|---|
| 1-1-237 | 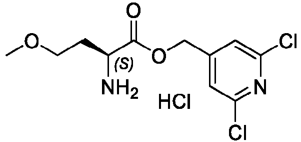 |
| | (2,6-Dichloropyridin-4-yl)methyl O-methyl-L-homoserinate hydrochloride |
| | LC-MS (ESI) m/z [M+H]⁺: 292.96 |
| | tR 4.048 min (A) |
| | N-(tert-Butoxycarbonyl)-O-methyl-L-homoserine (104839-08-9) |
| | 4-(Bromomethyl)-2,6-dichloropyridine (175204-45-2) |
| | 4M-HCl/MTHP |
| 1-1-238 | 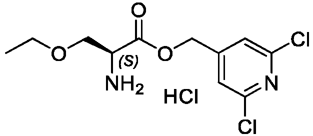 |
| | (2,6-Dichloropyridin-4-yl)methyl O-ethyl-L-serinate hydrochloride |
| | LC-MS (ESI) m/z [M+H]⁺: 292.97 |
| | tR 4.844 min (A) |
| | N-(tert-Butoxycarbonyl)-O-ethyl-L-serine (104839-00-1) |
| | 4-(Bromomethyl)-2,6-dichloropyridine (175204-45-2) |
| | 4M-HCl/MTHP |

**[Table 1-112]**

| | |
|---|---|
| 1-1-239 | 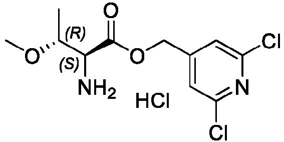 |
| | (2,6-Dichloropyridin-4-yl)methyl O-methyl-L-threoninate hydrochloride |
| | LC-MS (ESI) m/z [M+H]⁺: 292.93 |
| | tR 4.364 min (A) |
| | N-(tert-Butoxycarbonyl)-O-methyl-L-threonine (48068-25-3) |
| | 4-(Bromomethyl)-2,6-dichloropyridine (175204-45-2) |
| | 4M-HCl/MTHP |
| 1-1-240 | 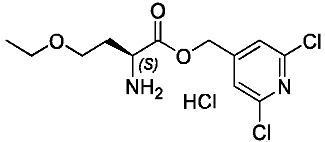 |
| | (2,6-Dichloropyridin-4-yl)methyl O-ethyl-L-homoserinate hydrochloride |
| | LC-MS (ESI) m/z [M+H]⁺: 307.01 |
| | tR 3.004 min (B) |
| | N-(tert-Butoxycarbonyl)-O-ethyl-L-homoserine (1616273-34-7) |
| | 4-(Bromomethyl)-2,6-dichloropyridine (175204-45-2) |
| | 4M-HCl/MTHP |

**[Table 1-113]**

| | |
|---|---|
| 1-1-241 | 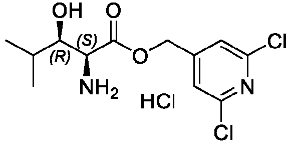 |
| | (2,6-Dichloropyridin-4-yl)methyl (2S,3R)-2-amino-3-hydroxy-4-methylpentanoate hydrochloride |
| | LC-MS (ESI) m/z [M+H]⁺: 307.03 |
| | tR 2.948 min (B) |
| | (S)-2-((tert-Butoxycarbonyl)amino)-3-hydroxy-3-methylbutanoic acid (929198-84-5) |
| | 4-(Bromomethyl)-2,6-dichloropyridine (175204-45-2) |
| | 4M-HCl/MTHP |
| 1-1-242 | 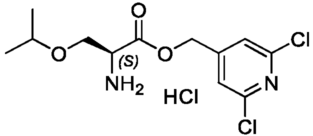 |
| | (2,6-Dichloropyridin-4-yl)methyl O-isopropyl-L-serinate hydrochloride |
| | LC-MS (ESI) m/z [M+H]⁺: 306.97 |
| | tR 5.084 min (A) |
| | N-(tert-Butoxycarbonyl)-O-isopropyl-L-serine (711018-10-9) |
| | 4-(Bromomethyl)-2,6-dichloropyridine (175204-45-2) |
| | 4M-HCl/MTHP |

**[Table 1-114]**

| | |
|---|---|
| 1-1-243 | 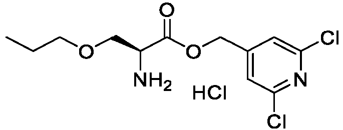 |
| | (2,6-Dichloropyridin-4-yl)methyl O-propyl-L-serinate hydrochloride |
| | LC-MS (ESI) m/z [M+H]⁺: 306.97 |
| | tR 5.524 min (A) |
| | N-(tert-Butoxycarbonyl)-O-propyl-L-serine (398526-37-9) |
| | 4-(Bromomethyl)-2,6-dichloropyridine (175204-45-2) |
| | 4M-HCl/MTHP |
| 1-1-244 | 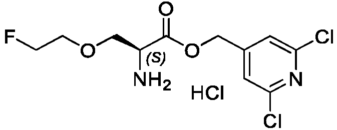 |
| | (2,6-Dichloropyridin-4-yl)methyl O-(2-fluoroethyl)-L-serinate hydrochloride |
| | LC-MS (ESI) m/z [M+H]⁺: 310.93 |
| | tR 4.288 min (A) |
| | N-(tert-Butoxycarbonyl)-O-(2-fluoroethyl)-L-serine (K26618, Mimotopes) |
| | 4-(Bromomethyl)-2,6-dichloropyridine (175204-45-2) |
| | 4M-HCl/MTHP |

**[Table 1-115]**

| | |
|---|---|
| 1-1-245 | 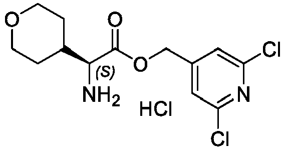 |
| | (2,6-Dichloropyridin-4-yl)methyl (S)-2-amino-2-(tetrahydro-2H-pyran-4-yl)acetate hydrochloride |
| | LC-MS (ESI) m/z [M+H]⁺: 318.98 |
| | tR 4.124 min (A) |
| | (S)-2-((tert-Butoxycarbonyl)amino)-2-(tetrahydro-2H-pyran-4-yl)acetic acid (711017-85-5) |
| | 4-(Bromomethyl)-2,6-dichloropyridine (175204-45-2) |
| | 4M-HCl/MTHP |
| 1-1-246 | 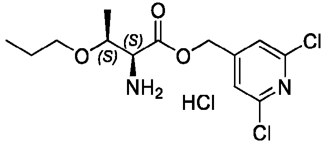 |
| | (2,6-Dichloropyridin-4-yl)methyl O-propyl-L-allothreoninate hydrochloride |
| | LC-MS (ESI) m/z [M+H]⁺: 320.94 |
| | tR 3.440 min (B) |
| | N-(tert-Butoxycarbonyl)-O-propyl-L-allothreonine (R00782, Nanjing Peptide Biotechnology Co. Ltd.) |
| | 4-(Bromomethyl)-2,6-dichloropyridine (175204-45-2) |
| | 4M-HCl/MTHP |

**[Table 1-116]**

| | |
|---|---|
| 1-1-247 | 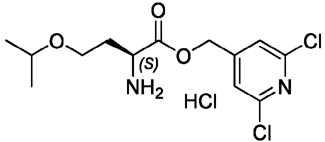 |
| | (2,6-Dichloropyridin-4-yl)methyl O-isopropyl-L-homoserinate hydrochloride |
| | LC-MS (ESI) m/z [M+H]⁺: 320.92 |
| | tR 3.316 min (B) |
| | N-(tert-Butoxycarbonyl)-O-isopropyl-L-homoserine (2349312-43-0) |
| | 4-(Bromomethyl)-2,6-dichloropyridine (175204-45-2) |
| | 4M-HCl/MTHP |
| 1-1-248 | 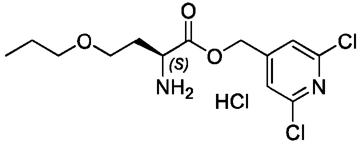 |
| | (2,6-Dichloropyridin-4-yl)methyl O-propyl-L-homoserinate hydrochloride |
| | LC-MS (ESI) m/z [M+H]⁺: 321.00 |
| | tR 3.332 min (B) |
| | N-(tert-Butoxycarbonyl)-O-propyl-L-homoserine (145205-93-2) |
| | 4-(Bromomethyl)-2,6-dichloropyridine (175204-45-2) |
| | 4M-HCl/MTHP |

**[Table 1-117]**

| | |
|---|---|
| 1-1-249 | 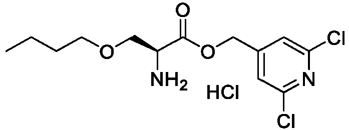 |
| | (2,6-Dichloropyridin-4-yl)methyl O-butyl-L-serinate hydrochloride |
| | LC-MS (ESI) m/z [M+H]⁺: 322.95 |
| | tR 3.596 min (B) |
| | N-(tert-Butoxycarbonyl)-O-butyl-L-serine (23358-68-1) |
| | 4-(Bromomethyl)-2,6-dichloropyridine (175204-45-2) |
| | 4M-HCl/MTHP |
| 1-1-250 | 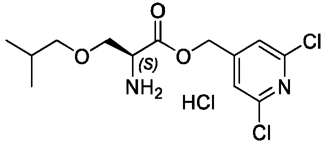 |
| | (2,6-Dichloropyridin-4-yl)methyl O-isobutyl-L-serinate hydrochloride |
| | LC-MS (ESI) m/z [M+H]⁺: 321.00 |
| | tR 5.748 min (A) |
| | N-(tert-Butoxycarbonyl)-O-isobutyl-L-serine (2382157-77-7) |
| | 4-(Bromomethyl)-2,6-dichloropyridine (175204-45-2) |
| | 4M-HCl/MTHP |

**[Table 1-118]**

| | |
|---|---|
| 1-1-251 | 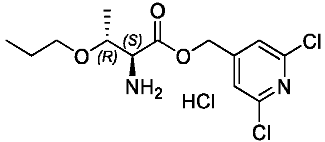 |
| | (2,6-Dichloropyridin-4-yl)methyl O-propyl-L-threoninate hydrochloride |
| | LC-MS (ESI) m/z [M+H]⁺: 321.00 |
| | tR 5.428 min (A) |
| | N-(tert-Butoxycarbonyl)-O-propyl-L-threonine (R00779, Nanjing Peptide Biotechnology Co. Ltd.) |
| | 4-(Bromomethyl)-2,6-dichloropyridine (175204-45-2) |
| | 4M-HCl/MTHP |
| 1-1-252 | 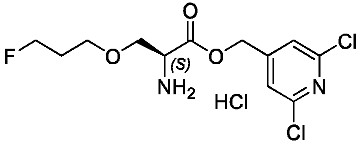 |
| | (2,6-Dichloropyridin-4-yl)methyl O-(3-fluoropropyl)-L-serinate hydrochloride |
| | LC-MS (ESI) m/z [M+H]⁺: 324.97 |
| | tR 4.628 min (A) |
| | N-(tert-Butoxycarbonyl)-O-(3-fluoropropyl)-L-serine (K26622, Mimotopes) |
| | 4-(Bromomethyl)-2,6-dichloropyridine (175204-45-2) |
| | 4M-HCl/MTHP |

**[Table 1-119]**

| | |
|---|---|
| 1-1-253 | 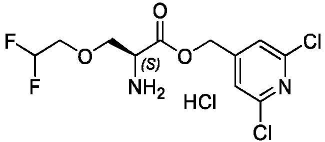 |
| | (2,6-Dichloropyridin-4-yl)methyl O-(2,2-difluoroethyl)-L-serinate hydrochloride |
| | LC-MS (ESI) m/z [M+H]⁺: 328.96 |
| | tR 4.456 min (A) |
| | N-(tert-Butoxycarbonyl)-O-(2,2-difluoroethyl)-L-serine (K26626, Mimotopes) |
| | 4-(Bromomethyl)-2,6-dichloropyridine (175204-45-2) |
| | 4M-HCl/MTHP |
| 1-1-254 | 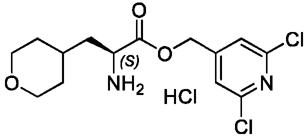 |
| | (2,6-Dichloropyridin-4-yl)methyl (S)-2-amino-3-(tetrahydro-2H-pyran-4-yl)propanoate hydrochloride |
| | LC-MS (ESI) m/z [M+H]⁺: 333.02 |
| | tR 5.024 min (A) |
| | (S)-2-((tert-Butoxycarbonyl)amino)-3-(tetrahydro-2H-pyran-4-yl)propanoic acid (368866-33-5) |
| | 4-(Bromomethyl)-2,6-dichloropyridine (175204-45-2) |
| | 4M-HCl/MTHP |

**[Table 1-120]**

| | |
|---|---|
| 1-1-255 | 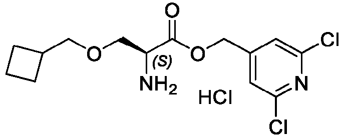 |
| | (2,6-Dichloropyridin-4-yl)methyl O-(cyclobutylmethyl)-L-serinate hydrochloride |
| | LC-MS (ESI) m/z [M+H]⁺: 333.02 |
| | tR 3.532 min (B) |
| | N-(tert-Butoxycarbonyl)-O-(cyclobutylmethyl)-L-serine (A-19108, Amatek Chemical Co. Ltd.) |
| | 4-(Bromomethyl)-2,6-dichloropyridine (175204-45-2) |
| | 4M-HCl/MTHP |
| 1-1-256 | 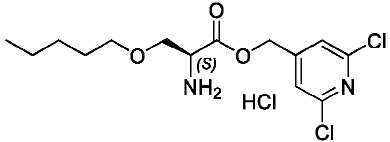 |
| | (2,6-Dichloropyridin-4-yl)methyl O-pentyl-L-serinate hydrochloride |
| | LC-MS (ESI) m/z [M+H]⁺: 335.02 |
| | tR 3.716 min (B) |
| | N-(tert-Butoxycarbonyl)-O-pentyl-L-serine (150715-03-0) |
| | 4-(Bromomethyl)-2,6-dichloropyridine (175204-45-2) |
| | 4M-HCl/MTHP |

**[Table 1-121]**

| | |
|---|---|
| 1-1-257 | 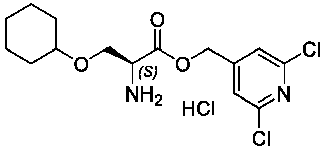 |
| | (2,6-Dichloropyridin-4-yl)methyl O-cyclohexyl-L-serinate hydrochloride |
| | LC-MS (ESI) m/z [M+H]⁺: 347.02 |
| | tR 3.700 min (B) |
| | N-(tert-Butoxycarbonyl)-O-cyclohexyl-L-serine (221057-18-7) |
| | 4-(Bromomethyl)-2,6-dichloropyridine (175204-45-2) |
| | 4M-HCl/MTHP |
| 1-1-258 | 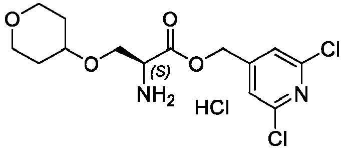 |
| | (2,6-Dichloropyridin-4-yl)methyl O-(tetrahydro-2H-pyran-4-yl)-L-serinate hydrochloride |
| | LC-MS (ESI) m/z [M+H]⁺: 348.98 |
| | tR 4.620 min (A) |
| | N-(tert-Butoxycarbonyl)-O-(tetrahydro-2H-pyran-4-yl)-L-serine (2375248-87-4) |
| | 4-(Bromomethyl)-2,6-dichloropyridine (175204-45-2) |
| | 4M-HCl/MTHP |

**[Table 1-122]**

| | |
|---|---|
| 1-1-259 | 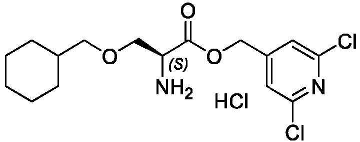 |
| | (2,6-Dichloropyridin-4-yl)methyl O-(cyclohexylmethyl)-L-serinate hydrochloride |
| | LC-MS (ESI) m/z [M+H]⁺: 361.02 |
| | tR 3.968 min (B) |
| | N-(tert-Butoxycarbonyl)-O-(cyclohexylmethyl)-L-serine (219626-93-4) |
| | 4-(Bromomethyl)-2,6-dichloropyridine (175204-45-2) |
| | 4M-HCl/MTHP |
| 1-1-260 | 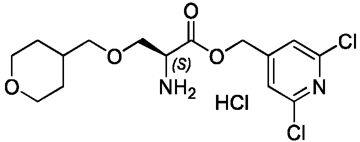 |
| | (2,6-Dichloropyridin-4-yl)methyl O-((tetrahydro-2H-pyran-4-yl)methyl)-L-serinate hydrochloride |
| | LC-MS (ESI) m/z [M+H]⁺: 363.05 |
| | tR 4.760 min (A) |
| | N-(tert-Butoxycarbonyl)-O-(cyclohexylmethyl)-L-serine (K26634, Mimotopes) |
| | 4-(Bromomethyl)-2,6-dichloropyridine (175204-45-2) |
| | 4M-HCl/MTHP |

**[Table 1-123]**

| | |
|---|---|
| 1-1-261 | 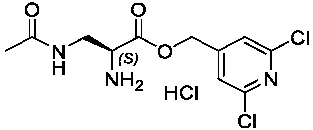 |
| | (2,6-Dichloropyridin-4-yl)methyl (S)-3-acetamido-2-aminopropanoate hydrochloride |
| | LC-MS (ESI) m/z [M+H]⁺: 305.97 |
| | tR 3.800 min (A) |
| | (S)-3-Acetamido-2-((tert-butoxycarbonyl)amino)propanoic acid (158220-97-4) |
| | 4-(Bromomethyl)-2,6-dichloropyridine (175204-45-2) |
| | 4M-HCl/MTHP |
| 1-1-262 | 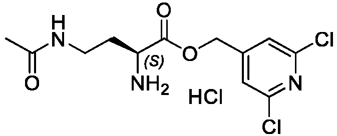 |
| | (2,6-Dichloropyridin-4-yl)methyl (S)-4-acetamido-2-aminobutanoate hydrochloride |
| | LC-MS (ESI) m/z [M+H]⁺: 319.98 |
| | tR 3.936 min (A) |
| | (S)-4-Acetamido-2-((tert-butoxycarbonyl)aminobutanoic acid (1562429-84-8) |
| | 4-(Bromomethyl)-2,6-dichloropyridine (175204-45-2) |
| | 4M-HCl/MTHP |

**[Table 1-124]**

| | |
|---|---|
| 1-1-263 | 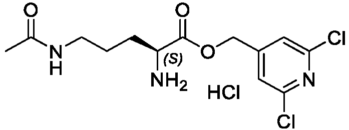 |
| | (2,6-Dichloropyridin-4-yl)methyl (S)-5-acetamido-2-aminopentanoate hydrochloride |
| | LC-MS (ESI) m/z [M+H]⁺: 334.01 |
| | tR 4.140 min (A) |
| | (S)-5-Acetamido-2-((tert-butoxycarbonyl)amino)pentanoic acid (125630-00-4) |
| | 4-(Bromomethyl)-2,6-dichloropyridine (175204-45-2) |
| | 4M-HCl/MTHP |
| 1-1-264 | 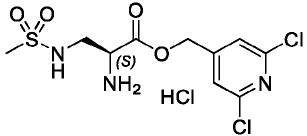 |
| | (2,6-Dichloropyridin-4-yl)methyl (S)-2-amino-3-(methylsulfonamido)propanoate hydrochloride |
| | LC-MS (ESI) m/z [M+H]⁺: 341.92 |
| | tR 5.648 min (C) |
| | (S)-2-((tert-Butoxycarbonyl)amino)-3-(methylsulfonamido)propanoic acid (1056123-61-5) |
| | 4-(Bromomethyl)-2,6-dichloropyridine (175204-45-2) |
| | 4M-HCl/MTHP |

**[Table 1-125]**

| | |
|---|---|
| 1-1-265 | 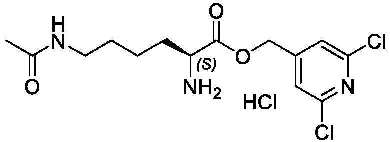 |
| | (2,6-Dichloropyridin-4-yl)methyl N⁶-acetyl-L-lysinate hydrochloride |
| | LC-MS (ESI) m/z [M+H]⁺: 348.00 |
| | tR 4.412 min (A) |
| | N⁶-Acetyl-N²-(tert-butoxycarbonyl)-L-lysine (6404-26-8) |
| | 4-(Bromomethyl)-2,6-dichloropyridine (175204-45-2) |
| | 4M-HCl/MTHP |
| 1-1-266 | 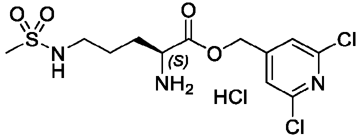 |
| | (2,6-Dichloropyridin-4-yl)methyl (S)-2-amino-5-(methylsulfonamido)pentanoate hydrochloride |
| | LC-MS (ESI) m/z [M+H]⁺: 369.94 |
| | tR 3.800 min (A) |
| | (S)-2-((tert-Butoxycarbonyl)amino)-5-(methylsulfonamido)pentanoic acid (R00826, Nanjing Peptide Biotechnology Co. Ltd.) |
| | 4-(Bromomethyl)-2,6-dichloropyridine (175204-45-2) |
| | 4M-HCl/MTHP |

**[Table 1-126]**

| | |
|---|---|
| 1-1-267 | 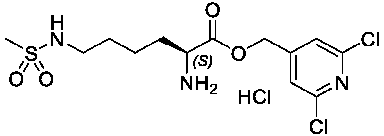 |
| | (2,6-Dichloropyridin-4-yl)methyl N⁶-(methylsulfonyl)-L-lysinate hydrochloride |
| | LC-MS (ESI) m/z [M+H]⁺: 384.00 |
| | tR 4.224 min (A) |
| | N²-(tert-Butoxycarbonyl)-N⁶-(methylsulfonyl)-L-lysine (J31160, GL Biochem (Shanghai) Ltd.) |
| | 4-(Bromomethyl)-2,6-dichloropyridine (175204-45-2) |
| | 4M-HCl/MTHP |
| 1-1-268 | 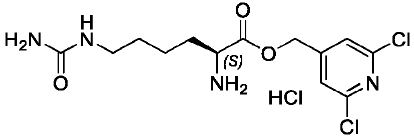 |
| | (2,6-Dichloropyridin-4-yl)methyl N⁶-carbamoyl-L-lysinate hydrochloride |
| | LC-MS (ESI) m/z [M+H]⁺: 349.01 |
| | tR 3.896 min (A) |
| | N²-(tert-Butoxycarbonyl)-N⁶-carbamoyl-L-lysine (201418-83-9) |
| | 4-(Bromomethyl)-2,6-dichloropyridine (175204-45-2) |
| | 4M-HCl/MTHP |

**[Table 1-127]**

| | |
|---|---|
| 1-1-269 | 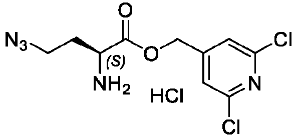 |
| | (2,6-Dichloropyridin-4-yl)methyl (S)-2-amino-4-azidobutanoate hydrochloride |
| | LC-MS (ESI) m/z [M+H]⁺: 303.98 |
| | tR 4.908 min (A) |
| | (S)-4-Azido-2-((tert-butoxycarbonyl)amino)butanoic acid (120042-08-2) |
| | 4-(Bromomethyl)-2,6-dichloropyridine (175204-45-2) |
| | 4M-HCl/MTHP |
| 1-1-270 | 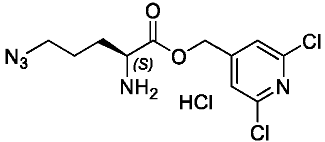 |
| | (2,6-Dichloropyridin-4-yl)methyl (S)-2-amino-5-azidopentanoate hydrochloride |
| | LC-MS (ESI) m/z [M+H]⁺: 317.98 |
| | tR 3.256 min (B) |
| | (S)-5-Azido-2-((tert-butoxycarbonyl)amino)pentanoic acid (763139-35-1) |
| | 4-(Bromomethyl)-2,6-dichloropyridine (175204-45-2) |
| | 4M-HCl/MTHP |

**[Table 1-128]**

| | |
|---|---|
| 1-1-271 | 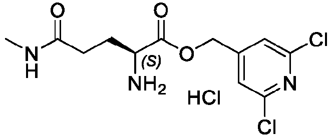 |
| | (2,6-Dichloropyridin-4-yl)methyl N⁵-methyl-L-glutamate hydrochloride |
| | LC-MS (ESI) m/z [M+H]⁺: 319.97 |
| | tR 3.544 min (A) |
| | N²-(tert-Butoxycarbonyl)-N⁵-methyl-L-glutamine (84575-51-9) |
| | 4-(Bromomethyl)-2,6-dichloropyridine (175204-45-2) |
| | 4M-HCl/MTHP |
| 1-1-272 | 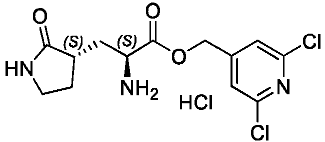 |
| | (2,6-Dichloropyridin-4-yl)methyl (S)-2-amino-3-((S)-2-oxopyrrolidin-3-yl)propanoate hydrochloride |
| | LC-MS (ESI) m/z [M+H]⁺: 332.02 |
| | tR 2.644 min (B) |
| | (S)-2-((tert-Butoxycarbonyl)amino)-3-((S)-2-oxopyrrolidin-3-yl)propanoic acid (741267-75-4) |
| | 4-(Bromomethyl)-2,6-dichloropyridine (175204-45-2) |
| | 4M-HCl/MTHP |

**[Table 1-129]**

| | |
|---|---|
| 1-1-273 | 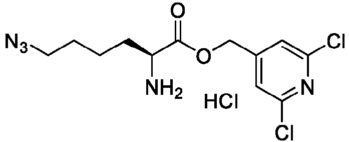 |
| | (2,6-Dichloropyridin-4-yl)methyl N⁶-diazo-L-lysinate hydrochloride |
| | LC-MS (ESI) m/z [M+H]⁺: 332.01 |
| | tR 3.296 min (B) |
| | N²-(tert-Butoxycarbonyl)-N⁶-diazo-L-lysine (846549-33-5) |
| | 4-(Bromomethyl)-2,6-dichloropyridine (175204-45-2) |
| | 4M-HCl/MTHP |
| 1-1-274 | 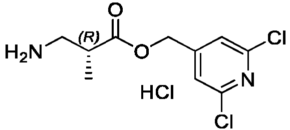 |
| | (2,6-Dichloropyridin-4-yl)methyl (R)-3-amino-2-methylpropanoate hydrochloride |
| | LC-MS (ESI) m/z [M+H]⁺: 262.98 |
| | tR 4.808 min (A) |
| | (R)-3-((tert-Butoxycarbonyl)amino)-2-methylpropanoic acid (132696-45-8) |
| | 4-(Bromomethyl)-2,6-dichloropyridine (175204-45-2) |
| | 4M-HCl/MTHP |

**[Table 1-130]**

| | |
|---|---|
| 1-1-275 | 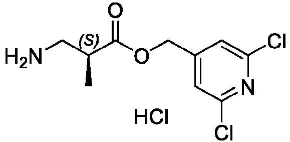 |
| | (2,6-Dichloropyridin-4-yl)methyl (S)-3-amino-2-methylpropanoate hydrochloride |
| | LC-MS (ESI) m/z [M+H]⁺: 262.99 |
| | tR 4.220 min (A) |
| | (S)-3-((tert-Butoxycarbonyl)amino)-2-methylpropanoic acid (190897-47-3) |
| | 4-(Bromomethyl)-2,6-dichloropyridine (175204-45-2) |
| | 4M-HCl/MTHP |
| 1-1-276 | 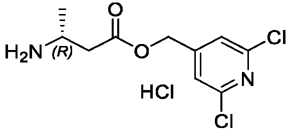 |
| | (2,6-Dichloropyridin-4-yl)methyl (R)-3-aminobutanoate hydrochloride |
| | LC-MS (ESI) m/z [M+H]⁺: 262.98 |
| | tR 4.020 min (A) |
| | (R)-3-((tert-Butoxycarbonyl)amino)butanoic acid (159991-23-8) |
| | 4-(Bromomethyl)-2,6-dichloropyridine (175204-45-2) |
| | 4M-HCl/MTHP |

**[Table 1-131]**

| | |
|---|---|
| 1-1-277 | 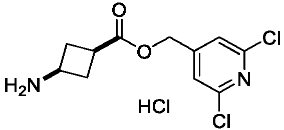 |
| | (2,6-Dichloropyridin-4-yl)methyl cis-3-aminocyclobutane-1-carboxylate hydrochloride |
| | LC-MS (ESI) m/z [M+H]⁺: 274.94 |
| | tR 4.196 min (A) |
| | cis-3-[[(1,1-Dimethylethoxy)carbonyl]amino]cyclobutanecarboxylic acid (1008773-79-2) |
| | 4-(Bromomethyl)-2,6-dichloropyridine (175204-45-2) |
| | 4M-HCl/MTHP |
| 1-1-278 | 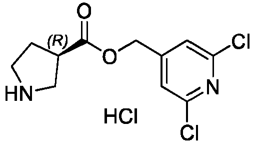 |
| | (2,6-Dichloropyridin-4-yl)methyl (R)-pyrrolidine-3-carboxylate hydrochloride |
| | LC-MS (ESI) m/z [M+H]⁺: 274.97 |
| | tR 2.516 min (B) |
| | (R)-1-(tert-Butoxycarbonyl)pyrrolidine-3-carboxylic acid (72925-16-7) |
| | 4-(Bromomethyl)-2,6-dichloropyridine (175204-45-2) |
| | 4M-HCl/MTHP |

**[Table 1-132]**

| | |
|---|---|
| 1-1-279 | 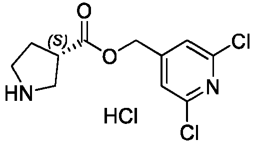 |
| | (2,6-Dichloropyridin-4-yl)methyl (S)-pyrrolidine-3-carboxylate hydrochloride |
| | LC-MS (ESI) m/z [M+H]⁺: 274.98 |
| | tR 3.880 min (A) |
| | (S)-1-(tert-Butoxycarbonyl)pyrrolidine-3-carboxylic acid (140148-70-5) |
| | 4-(Bromomethyl)-2,6-dichloropyridine (175204-45-2) |
| | 4M-HCl/MTHP |
| 1-1-280 | 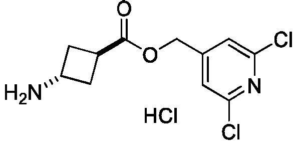 |
| | (2,6-Dichloropyridin-4-yl)methyl trans-3-aminocyclobutane-1-carboxylate hydrochloride |
| | LC-MS (ESI) m/z [M+H]⁺: 274.93 |
| | tR 4.476 min (A) |
| | trans-3-[[(1,1-Dimethylethoxy)carbonyl]amino]cyclobutanecarboxylic acid (939400-34-7) |
| | 4-(Bromomethyl)-2,6-dichloropyridine (175204-45-2) |
| | 4M-HCl/MTHP |

**[Table 1-133]**

| | |
|---|---|
| 1-1-281 | 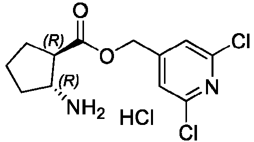 |
| | (2,6-Dichloropyridin-4-yl)methyl (1R,2R)-2-aminocyclopentane-1-carboxylate hydrochloride |
| | LC-MS (ESI) m/z [M+H]⁺: 288.98 |
| | tR 3.008 min (B) |
| | (1R,2R)-2-((tert-Butoxycarbonyl)amino)cyclopentane-1-carboxylic acid (245115-25-7) |
| | 4-(Bromomethyl)-2,6-dichloropyridine (175204-45-2) |
| | 4M-HCl/MTHP |
| 1-1-282 | 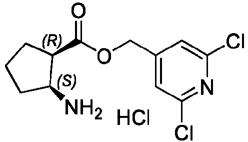 |
| | (2,6-Dichloropyridin-4-yl)methyl (1R,2S)-2-aminocyclopentane-1-carboxylate hydrochloride |
| | LC-MS (ESI) m/z [M+H]⁺: 288.98 |
| | tR 2.992 min (B) |
| | (1R,2S)-2-((tert-Butoxycarbonyl)amino)cyclopentane-1-carboxylic acid (130981-12-3) |
| | 4-(Bromomethyl)-2,6-dichloropyridine (175204-45-2) |
| | 4M-HCl/MTHP |

**[Table 1-134]**

| | |
|---|---|
| 1-1-283 | 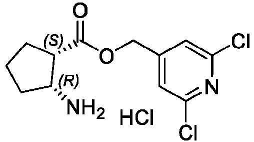 |
| | (2,6-Dichloropyridin-4-yl)methyl (1S,2R)-2-aminocyclopentane-1-carboxylate hydrochloride |
| | LC-MS (ESI) m/z [M+H]⁺: 288.98 |
| | tR 2.992 min (B) |
| | (1S,2R)-2-((tert-Butoxycarbonyl)amino)cyclopentane-1-carboxylic acid (137170-89-9) |
| | 4-(Bromomethyl)-2,6-dichloropyridine (175204-45-2) |
| | 4M-HCl/MTHP |
| 1-1-284 | 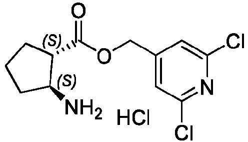 |
| | (2,6-Dichloropyridin-4-yl)methyl (1S,2S)-2-aminocyclopentane-1-carboxylate hydrochloride |
| | LC-MS (ESI) m/z [M+H]⁺: 288.99 |
| | tR 3.004 min (B) |
| | (1S,2S)-2-((tert-Butoxycarbonyl)amino)cyclopentane-1-carboxylic acid (143679-80-5) |
| | 4-(Bromomethyl)-2,6-dichloropyridine (175204-45-2) |
| | 4M-HCl/MTHP |

**[Table 1-135]**

| | |
|---|---|
| 1-1-285 | 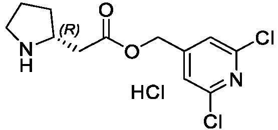 |
| | (2,6-Dichloropyridin-4-yl)methyl (R)-2-(pyrrolidin-2-yl)acetate hydrochloride |
| | LC-MS (ESI) m/z [M+H]⁺: 288.98 |
| | tR 3.024 min (B) |
| | (R)-2-(1-(tert-Butoxycarbonyl)pyrrolidin-2-yl)acetic acid (101555-60-6) |
| | 4-(Bromomethyl)-2,6-dichloropyridine (175204-45-2) |
| | 4M-HCl/MTHP |
| 1-1-286 | 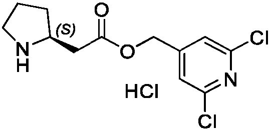 |
| | (2,6-Dichloropyridin-4-yl)methyl (S)-2-(pyrrolidin-2-yl)acetate hydrochloride |
| | LC-MS (ESI) m/z [M+H]⁺: 288.98 |
| | tR 4.284 min (A) |
| | (S)-2-(1-(tert-Butoxycarbonyl)pyrrolidin-2-yl)acetic acid (56502-01-3) |
| | 4-(Bromomethyl)-2,6-dichloropyridine (175204-45-2) |
| | 4M-HCl/MTHP |

**[Table 1-136]**

| | |
|---|---|
| 1-1-287 | 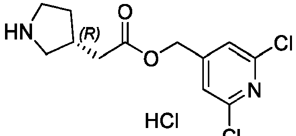 |
| | (2,6-Dichloropyridin-4-yl)methyl (R)-2-(pyrrolidin-3-yl)acetate hydrochloride |
| | LC-MS (ESI) m/z [M+H]⁺: 288.98 |
| | tR 4.368 min (A) |
| | (R)-2-(1-(tert-Butoxycarbonyl)pyrrolidin-3-yl)acetic acid (204688-60-8) |
| | 4-(Bromomethyl)-2,6-dichloropyridine (175204-45-2) |
| | 4M-HCl/MTHP |
| 1-1-288 | 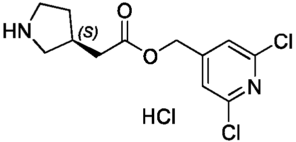 |
| | (2,6-Dichloropyridin-4-yl)methyl (S)-2-(pyrrolidin-3-yl)acetate hydrochloride |
| | LC-MS (ESI) m/z [M+H]⁺: 288.98 |
| | tR 4.376 min (A) |
| | (S)-2-(1-(tert-Butoxycarbonyl)pyrrolidin-3-yl)acetic acid (204688-61-9) |
| | 4-(Bromomethyl)-2,6-dichloropyridine (175204-45-2) |
| | 4M-HCl/MTHP |

**[Table 1-137]**

| | |
|---|---|
| 1-1-289 | 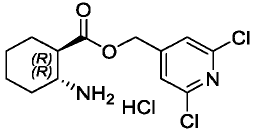 |
| | (2,6-Dichloropyridin-4-yl)methyl (1R,2R)-2-aminocyclohexane-1-carboxylate hydrochloride |
| | LC-MS (ESI) m/z [M+H]⁺: 303.04 |
| | tR 5.128 min (A) |
| | (1R,2R)-2-((tert-Butoxycarbonyl)amino)cyclohexane-1-carboxylic acid (233661-54-6) |
| | 4-(Bromomethyl)-2,6-dichloropyridine (175204-45-2) |
| | 4M-HCl/MTHP |
| 1-1-290 | 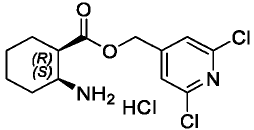 |
| | (2,6-Dichloropyridin-4-yl)methyl (1R,2S)-2-aminocyclohexane-1-carboxylate hydrochloride |
| | LC-MS (ESI) m/z [M+H]⁺: 302.98 |
| | tR 5.208 min (A) |
| | (1R,2S)-2-((tert-Butoxycarbonyl)amino)cyclohexane-1-carboxylic acid (352356-38-8) |
| | 4-(Bromomethyl)-2,6-dichloropyridine (175204-45-2) |
| | 4M-HCl/MTHP |

**[Table 1-138]**

| | |
|---|---|
| 1-1-291 | 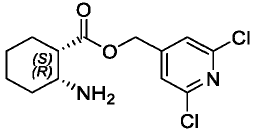 |
| | (2,6-Dichloropyridin-4-yl)methyl (1S,2R)-2-aminocyclohexane-1-carboxylate hydrochloride |
| | LC-MS (ESI) m/z [M+H]⁺: 303.00 |
| | tR 5.200 min (A) |
| | (1S,2R)-2-((tert-Butoxycarbonyl)amino)cyclohexane-1-carboxylic acid (865689-36-7) |
| | 4-(Bromomethyl)-2,6-dichloropyridine (175204-45-2) |
| | 4M-HCl/MTHP |
| 1-1-292 | 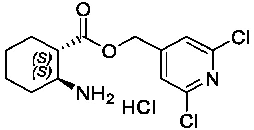 |
| | (2,6-Dichloropyridin-4-yl)methyl (1S,2S)-2-aminocyclohexane-1-carboxylate hydrochloride |
| | LC-MS (ESI) m/z [M+H]⁺: 303.01 |
| | tR 5.124 min (A) |
| | (1S,2S)-2-((tert-Butoxycarbonyl)amino)cyclohexane-1-carboxylic acid (488703-60-2) |
| | 4-(Bromomethyl)-2,6-dichloropyridine (175204-45-2) |
| | 4M-HCl/MTHP |

**[Table 1-139]**

| | |
|---|---|
| 1-1-293 | 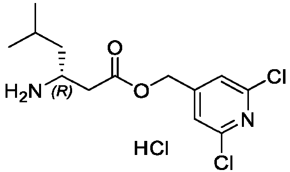 |
| | (2,6-Dichloropyridin-4-yl)methyl (R)-3-amino-5-methylhexanoate hydrochloride |
| | LC-MS (ESI) m/z [M+H]⁺: 304.99 |
| | tR 3.404 min (B) |
| | (R)-3-((tert-Butoxycarbonyl)amino)-5-methylhexanoic acid (146398-18-7) |
| | 4-(Bromomethyl)-2,6-dichloropyridine (175204-45-2) |
| | 4M-HCl/MTHP |
| 1-1-294 | 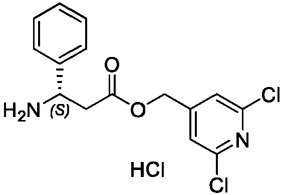 |
| | (2,6-Dichloropyridin-4-yl)methyl (S)-3-amino-3-phenylpropanoate hydrochloride |
| | LC-MS (ESI) m/z [M+H]⁺: 326.91 |
| | tR 3.236 min (B) |
| | (S)-3-((tert-Butoxycarbonyl)amino)-3-phenylpropanoic acid (103365-47-5) |
| | 4-(Bromomethyl)-2,6-dichloropyridine (175204-45-2) |
| | 4M-HCl/MTHP |

**[Table 1-140]**

| | |
|---|---|
| 1-1-295 | 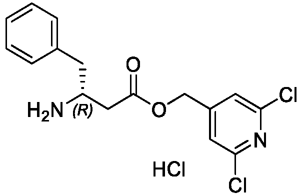 |
| | (2,6-Dichloropyridin-4-yl)methyl (R)-3-amino-4-phenylbutanoate hydrochloride |
| | LC-MS (ESI) m/z [M+H]⁺: 339.00 |
| | tR 5.632 min (B) |
| | (R)-3-((tert-Butoxycarbonyl)amino)-4-phenylbutanoic acid (101555-61-7) |
| | 4-(Bromomethyl)-2,6-dichloropyridine (175204-45-2) |
| | 4M-HCl/MTHP |
| 1-1-296 | 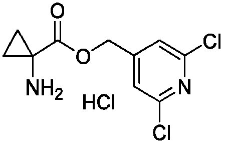 |
| | (2,6-Dichloropyridin-4-yl)methyl 1-aminocyclopropane-1-carboxylate hydrochloride |
| | LC-MS (ESI) m/z [M+H]⁺: 261.03 |
| | tR 2.476 min (B) |
| | 1-((tert-Butoxycarbonyl)amino)cyclopropane-1-carboxylic acid (88950-64-5) |
| | 4-(Bromomethyl)-2,6-dichloropyridine (175204-45-2) |
| | 4M-HCl/MTHP |

**[Table 1-141]**

| | |
|---|---|
| 1-1-297 | 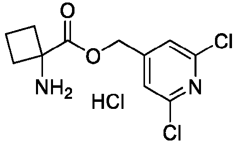 |
| | (2,6-Dichloropyridin-4-yl)methyl 1-aminocyclobutane-1-carboxylate hydrochloride |
| | LC-MS (ESI) m/z [M+H]⁺: 275.02 |
| | tR 2.752 min (B) |
| | 1-((tert-Butoxycarbonyl)amino)cyclobutane-1-carboxylic acid (120728-10-1) |
| | 4-(Bromomethyl)-2,6-dichloropyridine (175204-45-2) |
| | 4M-HCl/MTHP |
| 1-1-298 | 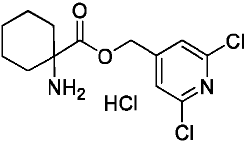 |
| | (2,6-Dichloropyridin-4-yl)methyl 1-aminocyclohexane-1-carboxylate hydrochloride |
| | LC-MS (ESI) m/z [M+H]⁺: 303.01 |
| | tR 3.020 min (B) |
| | 1-((tert-Butoxycarbonyl)amino)cyclohexane-1-carboxylic acid (115951-16-1) |
| | 4-(Bromomethyl)-2,6-dichloropyridine (175204-45-2) |
| | 4M-HCl/MTHP |

**[Table 1-142]**

| | |
|---|---|
| 1-1-299 | 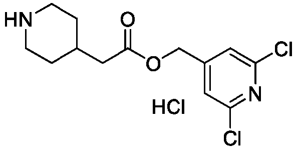 |
| | (2,6-Dichloropyridin-4-yl)methyl 2-(piperidin-4-yl)acetate hydrochloride |
| | LC-MS (ESI) m/z [M+H]⁺: 303.01 |
| | tR 4.668 min (A) |
| | 2-(1-(tert-Butoxycarbonyl)piperidin-4-yl)acetic acid (157688-46-5) |
| | 4-(Bromomethyl)-2,6-dichloropyridine (175204-45-2) |
| | 4M-HCl/MTHP |
| 1-1-300 | 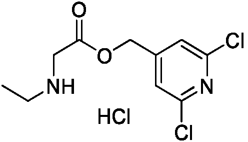 |
| | (2,6-Dichloropyridin-4-yl)methyl ethylglycinate hydrochloride |
| | LC-MS (ESI) m/z [M+H]⁺: 263.02 |
| | tR 3.576 min (A) |
| | N-(tert-Butoxycarbonyl)-N-ethylglycine (149794-10-5) |
| | 4-(Bromomethyl)-2,6-dichloropyridine (175204-45-2) |
| | 4M-HCl/MTHP |

**[Table 1-143]**

| | |
|---|---|
| 1-1-301 | 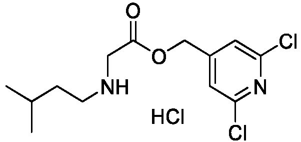 |
| | (2,6-Dichloropyridin-4-yl)methyl isopentylglycinate hydrochloride |
| | LC-MS (ESI) m/z [M+H]⁺: 305.04 |
| | tR 3.056 min (B) |
| | N-(tert-Butoxycarbonyl)-N-isopentylglycine (2383843-67-0) |
| | 4-(Bromomethyl)-2,6-dichloropyridine (175204-45-2) |
| | 4M-HCl/MTHP |
| 1-1-302 | 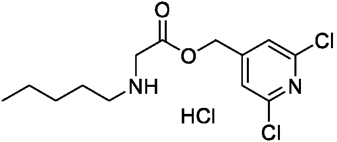 |
| | (2,6-Dichloropyridin-4-yl)methyl pentylglycinate hydrochloride |
| | LC-MS (ESI) m/z [M+H]⁺: 305.04 |
| | tR 3.288 min (B) |
| | N-(tert-Butoxycarbonyl)-N-pentylglycine (1259397-10-8) |
| | 4-(Bromomethyl)-2,6-dichloropyridine (175204-45-2) |
| | 4M-HCl/MTHP |

**[Table 1-144]**

| | |
|---|---|
| 1-1-303 | 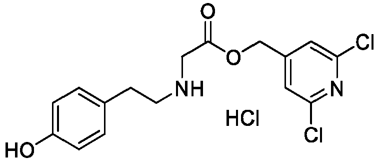 |
| | (2,6-Dichloropyridin-4-yl)methyl (4-hydroxyphenethyl)glycinate hydrochloride |
| | LC-MS (ESI) m/z [M+H]⁺: 354.93 |
| | tR 2.952 min (B) |
| | N-(4-(tert-Butoxy)phenethyl)-N-(tert-butoxycarbonyl)glycine (2680724-23-4) |
| | 4-(Bromomethyl)-2,6-dichloropyridine (175204-45-2) |
| | 2,2,2-Trifluoroacetic acid |
| 1-1-304 | 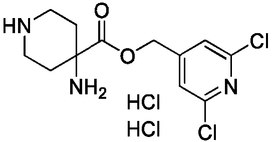 |
| | (2,6-Dichloropyridin-4-yl)methyl 4-aminopiperidine-4-carboxylate dihydrochloride |
| | LC-MS (ESI) m/z [M+H]⁺: 303.98 |
| | tR 3.800 min (C) |
| | 4-((tert-Butoxycarbonyl)amino)piperidine-4-carboxylic acid (189321-65-1) |
| | 4-(Bromomethyl)-2,6-dichloropyridine (175204-45-2) |
| | 4M-HCl/MTHP |

**[Table 1-145]**

| | |
|---|---|
| 1-1-305 | 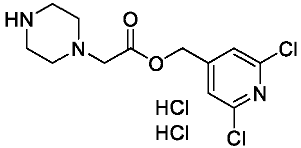 |
| | (2,6-Dichloropyridin-4-yl)methyl 2-(piperazin-1-yl)acetate dihydrochloride |
| | LC-MS (ESI) m/z [M+H]⁺: 303.98 |
| | tR 4.232 min (A) |
| | 2-(4-(tert-Butoxycarbonyl)piperazin-1-yl)acetic acid (156478-71-6) |
| | 4-(Bromomethyl)-2,6-dichloropyridine (175204-45-2) |
| | 4M-HCl/MTHP |
| 1-1-306 | 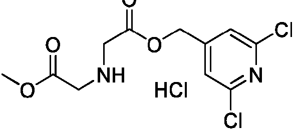 |
| | (2,6-Dichloropyridin-4-yl)methyl (2-methoxy-2-oxoethyl)glycinate hydrochloride |
| | LC-MS (ESI) m/z [M+H]⁺: 306.99 |
| | tR 2.636 min (B) |
| | N-(tert-Butoxycarbonyl)-N-(2-methoxy-2-oxoethyl)glycine (1013401-79-0) |
| | 4-(Bromomethyl)-2,6-dichloropyridine (175204-45-2) |
| | 4M-HCl/MTHP |

**[Table 1-146]**

| | |
|---|---|
| 1-1-307 | 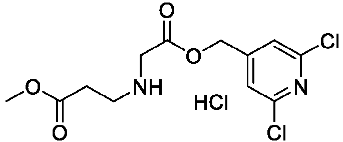 |
| | Methyl 3-((2-((2,6-dichloropyridin-4-yl)methoxy)-2-oxoethyl)amino)propanoate hydrochloride |
| | LC-MS (ESI) m/z [M+H]⁺: 321.02 |
| | tR 2.620 min (B) |
| | N-(tert-Butoxycarbonyl)-N-(3-methoxy-3-oxopropyl)glycine (2152548-00-8) |
| | 4-(Bromomethyl)-2,6-dichloropyridine (175204-45-2) |
| | 4M-HCl/MTHP |
| 1-1-308 | 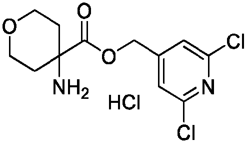 |
| | (2,6-Dichloropyridin-4-yl)methyl 4-aminotetrahydro-2H-pyran-4-carboxylate hydrochloride |
| | LC-MS (ESI) m/z [M+H]⁺: 304.97 |
| | tR 3.772 min (A) |
| | 4-((tert-Butoxycarbonyl)amino)tetrahydro-2H-pyran-4-carboxylic acid (172843-97-9) |
| | 4-(Bromomethyl)-2,6-dichloropyridine (175204-45-2) |
| | 4M-HCl/MTHP |

**[Table 1-147]**

| | |
|---|---|
| 1-1-309 | 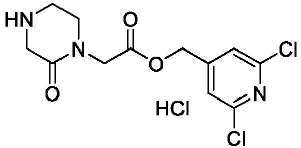 |
| | (2,6-Dichloropyridin-4-yl)methyl 2-(2-oxopiperazin-1-yl)acetate hydrochloride |
| | LC-MS (ESI) m/z [M+H]⁺: 317.96 |
| | tR 3.936 min (A) |
| | 2-(4-(tert-Butoxycarbonyl)-2-oxopiperazin-1-yl)acetic acid (549506-47-0) |
| | 4-(Bromomethyl)-2,6-dichloropyridine (175204-45-2) |
| | 4M-HCl/MTHP |

Each of the following compounds was synthesized using the corresponding starting amino acid in each of the following Tables according to the procedure of Example 1-2.

**[Table 2-1]**

| ExampleNo. | Structure |
|---|---|
| | Compound name |
| | ¹HNMR spectrum analysis results |
| | LC/MS analysis results |
| | tR (LC/MS analysis condition) |
| | Starting amino acid |
| 1-2-1 | 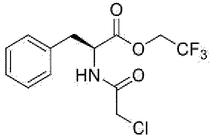 |
| | 2,2,2-Trifluoroethyl (2-chloroacetyl)-L-phenylalaninate |
| | ¹H NMR (500 MHz, *DMSO- d₆*) *δ* 3.00 (dd, 1H, *J* = 7.5, 14.0 Hz), 3.08 (dd, 1H, *J* = 5.5, 14.0 Hz), 4.06 (s, 2H), 4.57-4.61 (m, 1H), 4.74-4.79 (m, 2H), 7.23-7.31 (m, 5H), 8.83 (d, 1H, *J* = 7.5 Hz) |
| | LC-MS (ESI) *m*/*z* [M+H]⁺: 323.94 |
| | tR 4.996 min (B) |
| | 2,2,2-Trifluoroethyl L-phenylalaninate hydrochloride |
| 1-2-2 | 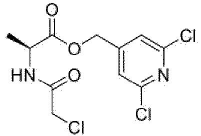 |
| | (2,6-Dichloropyridin-4-yl)methyl (2-chloroacetyl)-L-alaninate |
| | LC-MS (ESI) *m*/*z* [M+H]⁺: 324.92 |
| | tR 4.484 min (B) |
| | (2,6-Dichloropyridin-4-yl)methyl L-alaninate hydrochloride |

**[Table 2-2]**

| | |
|---|---|
| 1-2-3 | 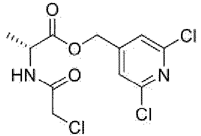 |
| | (2,6-Dichloropyridin-4-yl)methyl (2-chloroacetyl)-D-alaninate |
| | LC-MS (EST) *m*/*z* [M+H]⁺: 324.92 |
| | tR 4.480 min (B) |
| | (2,6-Dichloropyridin-4-yl)methyl D-alaninate hydrochloride |
| 1-2-4 | 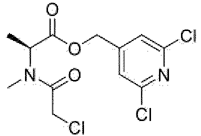 |
| | (2,6-Dichloropyridin-4-yl)methyl N-(2-chloroacetyl)-N-methyl-L-alaninate |
| | LC-MS (ESI) *m*/*z* [M+H]⁺: 339.01 |
| | tR 4.796 min (B) |
| | (2,6-Dichloropyridin-4-yl)methyl N-methyl-L-alaninate hydrochloride |
| 1-2-5 | 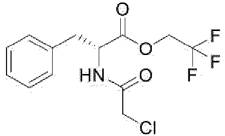 |
| | 2,2,2-Trifluoroethyl (2-chloroacetyl)-D-phenylalaninate |
| | LC-MS (ESI) *m*/*z* [M+H]⁺: 323.93 |
| | tR 5.000 min (B) |
| | 2,2,2-Trifluoroethyl D-phenylalaninate hydrochloride |

**[Table 2-3]**

| | |
|---|---|
| 1-2-6 | 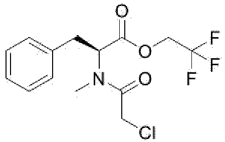 |
| | 2,2,2-Trifluoroethyl N-(2-chloroacetyl)-N-methyl-L-phenylalaninate |
| | LC-MS (ESI) *m*/*z* [M+H]⁺: 338.01 |
| | tR 5.116 min (B) |
| | 2,2,2-Trifluoroethyl methyl-L-phenylalaninate hydrochloride |
| 1-2-7 | 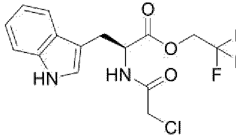 |
| | 2,2,2-Trifluoroethyl (2-chloroacetyl)-L-tryptophanate |
| | LC-MS (ESI) *m*/*z* [M+H]⁺: 363.02 |
| | tR 4.780 min (B) |
| | 2,2,2-Trifluoroethyl L-tryptophanate hydrochloride |
| 1-2-8 | 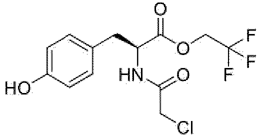 |
| | 2,2,2-Trifluoroethyl (2-chloroacetyl)-L-tyrosinate |
| | LC-MS (ESI) *m*/*z* [M+H]⁺: 340.04 |
| | tR 4.032 min (B) |
| | 2,2,2-Trifluoroethyl L-tyrosinate hydrochloride |

**[Table 2-4]**

| | |
|---|---|
| Example No. | Structure |
| | Compound name |
| | LC/MS analysis results |
| | tR (LC/MS analysis condition) |
| | Starting amino acid |
| 1-2-9 | 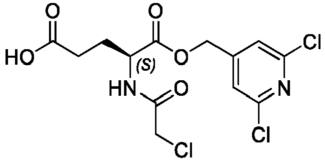 |
| | (S)-4-(2-Chloroacetamido)-5-((2,6-dichloropyridin-4-yl)methoxy)-5-oxopentanoic acid |
| | LC-MS (ESI) m/z [M+H]⁺: 382.89 |
| | tR 4.076 min (B) |
| | (S)-3-Amino-5-((2,6-dichloropyridin-4-yl)methoxy)-5-oxopentanoic acid hydrochloride |
| 1-2-10 | 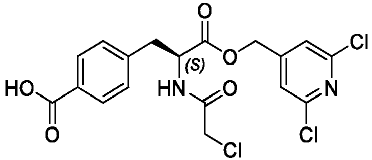 |
| | (S)-4-(2-(2-Chloroacetamido)-3-((2,6-dichloropyridin-4-yl)methoxy)-3-oxopropyl)benzoic acid |
| | LC-MS (ESI) m/z [M+H]⁺: 444.90 |
| | tR 4.604 min (B) |
| | (S)-4-(2-Amino-3-((2,6-dichloropyridin-4-yl)methoxy)-3-oxopropyl)benzoic acid hydrochloride |

**[Table 2-5]**

| | |
|---|---|
| 1-2-11 | 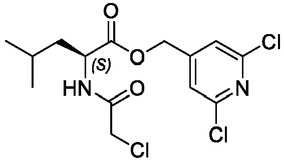 |
| | (2,6-Dichloropyridin-4-yl)methyl (2-chloroacetyl)-L-leucinate |
| | LC-MS (ESI) m/z [M+H]⁺: 366.97 |
| | tR 5.408 min (B) |
| | (2,6-Dichloropyridin-4-yl)methyl L-leucinate hydrochloride |
| 1-2-12 | 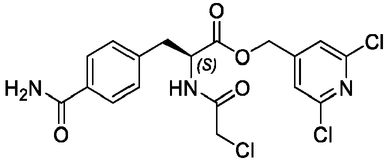 |
| | (2,6-Dichloropyridin-4-yl)methyl (S)-3-(4-carbamoylphenyl)-2-(2-chloroacetamido)propanoate |
| | LC-MS (ESI) m/z [M+H]⁺: 443.96 |
| | tR 4.232 min (B) |
| | (2,6-Dichloropyridin-4-yl)methyl (S)-2-amino-3-(4-carbamoylphenyl)propanoate hydrochloride |
| 1-2-13 | 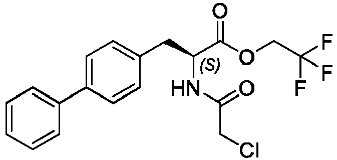 |
| | 2,2,2-Trifluoroethyl (S)-3-([1,1'-biphenyl]-4-yl)-2-(2-chloroacetamido)propanoate |
| | LC-MS (ESI) m/z [M+H]⁺: 400.02 |
| | tR 5.968 min (B) |
| | 2,2,2-Trifluoroethyl (S)-3-((1,1'-biphenyl)-4-yl)-2-aminopropanoate hydrochloride |

**[Table 2-6]**

| | |
|---|---|
| 1-2-14 | 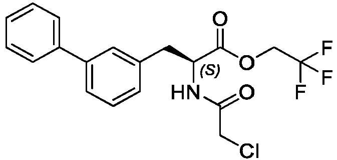 |
| | 2,2,2-Trifluoroethyl (S)-3-([1,1'-biphenyl]-3-yl)-2-(2-chloroacetamido)propanoate |
| | LC-MS (ESI) m/z [M+H]⁺: 400.04 |
| | tR 5.776 min (B) |
| | 2,2,2-Trifluoroethyl (S)-3-((1,1'-biphenyl)-3-yl)-2-aminopropanoate hydrochloride |
| 1-2-15 | 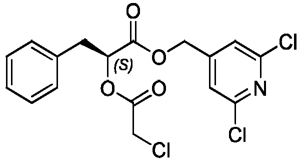 |
| | (2,6-Dichloropyridin-4-yl)methyl (S)-2-(2-chloroacetoxy)-3-phenylpropanoate |
| | LC-MS (ESI) m/z [M+H]⁺: 402.00 |
| | tR 5.920 min (B) |
| | (2,6-Dichloropyridin-4-yl)methyl (S)-2-hydroxy-3-phenylpropanoate |
| 1-2-16 | 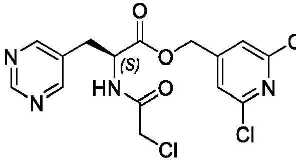 |
| | (2,6-Dichloropyridin-4-yl)methyl (S)-2-(2-chloroacetamido)-3-(pyrimidin-5-yl)propanoate |
| | LC-MS (ESI) m/z [M+H]⁺: 402.89 |
| | tR 4.032 min (B) |
| | (2,6-Dichloropyridin-4-yl)methyl (S)-2-amino-3-(pyrimidin-5-yl)propanoate dihydrochloride |

**[Table 2-7]**

| | |
|---|---|
| 1-2-17 | 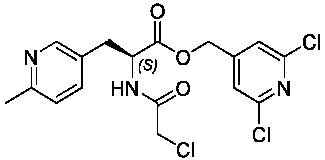 |
| | (2,6-Dichloropyridin-4-yl)methyl (S)-2-(2-chloroacetamido)-3-(6-methylpyridin-3-yl)propanoate |
| | LC-MS (ESI) m/z [M+H]⁺: 417.90 |
| | tR 3.116 min (B) |
| | (2,6-Dichloropyridin-4-yl)methyl (S)-2-amino-3-(6-methylpyridin-3-yl)propanoate dihydrochloride |
| 1-2-18 | 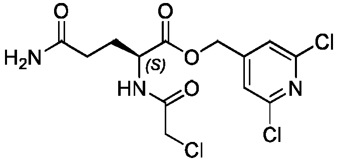 |
| | (2,6-Dichloropyridin-4-yl)methyl (2-chloroacetyl)-L-glutamate |
| | LC-MS (ESI) m/z [M+H]⁺: 383.93 |
| | tR 3.436 min (B) |
| | (2,6-Dichloropyridin-4-yl)methyl L-glutamate hydrochloride |
| 1-2-19 | 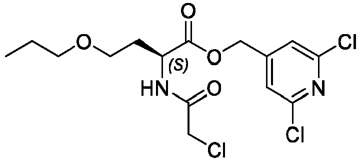 |
| | (2,6-Dichloropyridin-4-yl)methyl N-(2-chloroacetyl)-O-propyl-L-homoserinate |
| | LC-MS (ESI) m/z [M+H]⁺: 398.96 |
| | tR 5.524 min (B) |
| | (2,6-Dichloropyridin-4-yl)methyl O-propyl-L-homoserinate hydrochloride |

**[Table 2-8]**

| | |
|---|---|
| 1-2-20 | 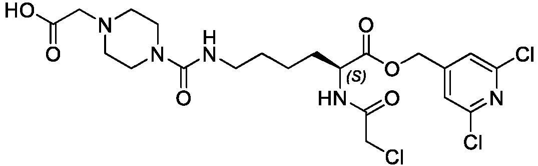 |
| | (S)-2-(4-((5-(2-Chloroacetamido)-6-((2,6-dichloropyridin-4-yl)methoxy)-6-oxohexyl)carbamoyl)piperazin-1-yl)acetic acid |
| | LC-MS (ESI) m/z [M+H]⁺: 554.06 |
| | tR 3.560 min (B) |
| | (S)-2-(4-((5-Amino-6-((2,6-dichloropyridin-4-yl)methoxy)-6-oxohexyl)carbamoyl)piperazin-1-yl)acetic acid dihydrochloride |

Each of the following compounds was synthesized using the corresponding starting amino acid and esterification reagent in each of the following Tables according to the procedure of Example 1-3.

**[Table 3-1]**

| | |
|---|---|
| ExampleNo. | Structure |
| | Compound name |
| | ¹HNMR spectrum analysis results |
| | LC/MS analysis results |
| | tR (LC/MS analysis condition) |
| | Starting amino acid (CAS Registry Number) |
| | Esterification reagent (CAS Registry Number) |
| 1-3-1 | 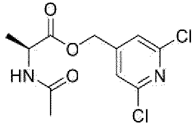 |
| | (2,6-Dichloropyridin-4-yl)methyl acetyl-L-alaninate |
| | ¹H NMR (500 MHz, DMSO*-d₆*) *δ* 1.32 (d, 3H, J = 7.0 Hz), 1.86 (s, 3H), 4.30-4.35 (m, 1H), 5.17-5.26 (m, 2H), 7.55 (s, 2H), 8.43 (d, 1H, J = 6.0 Hz) |
| | LC-MS (ESI) *m*/*z* [M+H]⁺: 291.04 |
| | tR 3.968 min (B) |
| | Acetyl-L-alanine (97-69-8) |
| | 4-(Bromomethyl)-2,6-dichloropyridine (175204-45-2) |
| 1-3-2 | 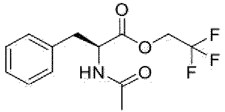 |
| | 2,2,2-Trifluoroethyl acetyl-L-phenylalaninate |
| | LC-MS (ESI) *m*/*z* [M+H]⁺: 290.11 |
| | tR 4.528 min (B) |
| | Acetyl-L-phenylalanine (2018-61-3) |
| | 2,2,2-Trifluoroethyl trifluoromethanesulfonate (6226-25-1) |

**[Table 3-2]**

| | |
|---|---|
| Example No. | Structure |
| | Compound name |
| | LC/MS analysis results |
| | tR (LC/MS analysis condition) |
| | Starting amino acid (CAS Registry Number) |
| | Esterification reagent (CAS Registry Number) |
| 1-3-3 | 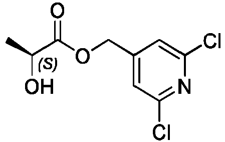 |
| | (2,6-Dichloropyridin-4-yl)methyl (S)-2-hydroxypropanoate |
| | LC-MS (ESI) m/z [M+H]⁺: 249.98 |
| | tR 3.928 min (B) |
| | L-Lactic Acid (79-33-4) |
| | 4-(Bromomethyl)-2,6-dichloropyridine (175204-45-2) |
| 1-3-4 | 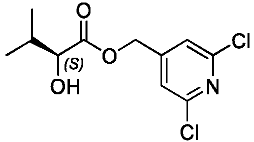 |
| | (2,6-Dichloropyridin-4-yl)methyl (S)-2-hydroxy-3-methylbutanoate |
| | LC-MS (ESI) m/z [M+H]⁺: 277.97 |
| | tR 4.856 min (B) |
| | (S)-2-Hydroxy-3-methylbutanoic acid (17407-55-5) |
| | 4-(Bromomethyl)-2,6-dichloropyridine (175204-45-2) |

**Table 3-3]**

| | |
|---|---|
| 1-3-5 | 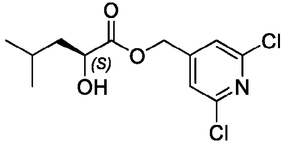 |
| | (2,6-Dichloropyridin-4-yl)methyl (S)-2-hydroxy-4-methylpentanoate |
| | LC-MS (ESI) m/z [M+H]⁺: 292.2 |
| | tR 2.89 min (D) |
| | L-Leucic acid (13748-90-8) |
| | 4-(Bromomethyl)-2,6-dichloropyridine (175204-45-2) |
| 1-3-6 | 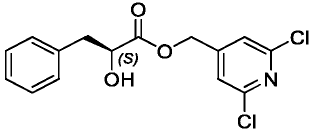 |
| | (2,6-Dichloropyridin-4-yl)methyl (S)-2-hydroxy-3-phenylpropanoate |
| | LC-MS (ESI) m/z [M+H]⁺: 326.01 |
| | tR 5.032 min (B) |
| | (S)-2-Hydroxy-3-phenylpropanoic acid (20312-36-1) |
| | 4-(Bromomethyl)-2,6-dichloropyridine (175204-45-2) |

Each of the following compounds was synthesized using the corresponding starting amino acid and esterification reagent in each of the following Tables according to the procedure of Example 1-4.

**[Table 4-1]**

| | |
|---|---|
| ExampleNo. | Structure |
| | Compound name |
| | 1HNMR spectrum analysis results |
| | LC/MS analysis results |
| | tR (LC/MS analysis condition) |
| | Starting amino acid (CAS Registry Number) |
| | Esterification reagent (CAS Registry Number) |
| | Deprotection condition (CAS Registry Number) |
| 1-4-1 | 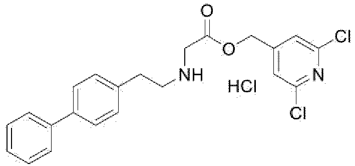 |
| | (2,6-Dichloropyridin-4-yl)methyl (2-((1,1'-biphenyl)-4-yl)ethyl)glycinate hydrochloride |
| | 1H NMR (500 MHz, DMSO-d6) *δ* ppm 2.87 - 3.14 (m, 2 H) 3.14 - 3.30 (m, 2 H) 4.23 (s, 2 H) 5.35 (s, 2 H) 7.36 (br d, 3 H, *J* = 7.5 Hz) 7.47 (br t, 2 H, *J* = 7.5 Hz) 7.56 - 7.80 (m, 6 H) 9.55 (brs, 2 H) |
| | LC-MS (ESI) *m*/*z* [M+H]⁺: 415.04 |
| | tR 4.280 min (B) |
| | (2-([1,1'-Biphenyl]-4-yl)ethyl)glycine (1906593-76-7) |
| | 4-(Bromomethyl)-2,6-dichloropyridine (175204-45-2) |
| | 4N-HCl/MTHP |

**[Table 4-2]**

| | |
|---|---|
| 1-4-2 | 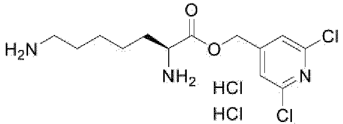 |
| | (2,6-Dichloropyridin-4-yl)methyl (S)-2,7-diaminoheptanoate dihydrochloride |
| | LC-MS (ESI) *m*/*z* [M+H]⁺: 320.01 |
| | tR 2.952 min (A) |
| | (S)-2-Amino-7-((tert-butoxycarbonyl)amino)heptanoic acid (1142814-17-2) |
| | 4-(Bromomethyl)-2,6-dichloropyridine (175204-45-2) |
| | 4N-HCl/MTHP |

Each of the following compounds was synthesized using the corresponding starting amino acid and esterification reagent in each of the following Tables according to the procedure of Example 1-5.

**[Table 5-1]**

| | |
|---|---|
| Example No. | Structure |
| | Compound name |
| | 1HNMR spectrum analysis results |
| | LC/MS analysis results |
| | tR (LC/MS analysis condition) |
| | Starting amino acid (CAS Registry Number) |
| | Esterification reagent (CAS Registry Number) |
| | Deprotection condition (CAS Registry Number) |
| 1-5-1 | 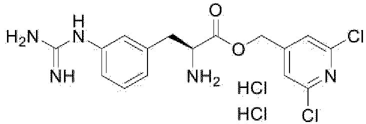 |
| | (2,6-Dichloropyridin-4-yl)methyl (S)-2-amino-3-(3-guanidinophenyl) propanoate dihydrochloride |
| | 1H NMR (500 MHz, DMSO-d6) *δ* ppm 3.17 - 3.31 (m, 2 H) 4.55 (br t, 1 H,*J* = 6.5 Hz) 5.12 - 5.42 (m, 2 H) 7.14 (d, 1 H, *J* = 8.0 Hz) 7.18 (d, , 1 H, *J* = 7.5 Hz) 7.27 (s, 1 H) 7.39 (t, 1 H, *J* = 8.0 Hz) 7.45 - 7.58 (m, 1 H) 7.56 (br s, 2 H) 7.61 (s, 2 H) 8.65 - 8.86 (m, 1 H) 8.76 (br s, 2 H) 10.04 - 10.12 (m, 1 H) 10.08 (s, 1 H) |
| | LC-MS (ESI) *m*/*z* [M+H]⁺: 381.98 |
| | tR 3.844 min (A) |
| | (S)-3-(3-Aminophenyl)-2-((tert-butoxycarbonyl)amino)propanoic acid (170157-55-8) |
| | 4-(Bromomethyl)-2,6-dichloropyridine (175204-45-2) |
| | 4N-HCl/MTHP |

**[Table 5-2]**

| | |
|---|---|
| 1-5-2 | 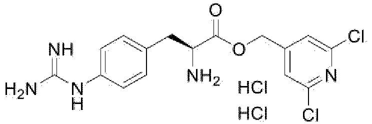 |
| | (2,6-Dichloropyridin-4-yl)methyl (S)-2-amino-3-(4-guanidinophenyl) propanoate dihydrochloride |
| | LC-MS (ESI) *m*/*z* [M+H]⁺: 381.95 |
| | tR 2.128 min (B) |
| | (S)-3-(4-Aminophenyl)-2-((tert-butoxycarbonyl)amino)propanoic acid (55533-24-9) |
| | 4-(Bromomethyl)-2,6-dichloropyridine (175204-45-2) |
| | 4N-HCl/MTHP |

**[Table 5-3]**

| | |
|---|---|
| Example No. | Structure |
| | Compound name |
| | LC/MS analysis results |
| | tR (LC/MS analysis condition) |
| | Starting amino acid (CAS Registry Number) |
| | Esterification reagent (CAS Registry Number) |
| 1-5-3 | 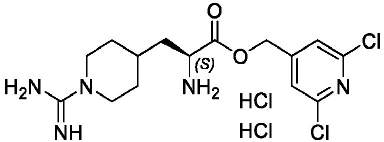 |
| | (2,6-Dichloropyridin-4-yl)methyl (S)-2-amino-3-(1-carbamimidoylpiperidin-4-yl)propanoate dihydrochloride |
| | LC-MS (ESI) m/z [M+H]⁺: 374.02 |
| | tR 3.256 min (B) |
| | (S)-2-((tert-Butoxycarbonyl)amino)-3-(piperidin-4-yl)propanoic acid hydrochloride (2204285-77-6) |
| | 4-(Bromomethyl)-2,6-dichloropyridine (175204-45-2) |

Each of the following compounds was synthesized using the corresponding starting material in each of the following Tables according to the procedure of Example 1-6.

**[Table 6-1]**

| | |
|---|---|
| Example No. | Structure |
| | Compound name |
| | ¹HNMR spectrum analysis results |
| | LC/MS analysis results |
| | tR (LC/MS analysis condition) |
| | Starting amino acid (CAS Registry Number) |
| | Esterification reagent (CAS Registry Number) |
| | Deprotection reagent |
| 1-6-1 | 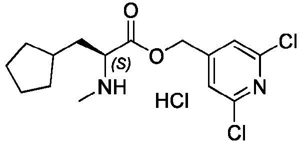 |
| | (2,6-Dichloropyridin-4-yl)methyl (S)-3-cyclopentyl-2-(methylamino)propanoate hydrochloride |
| | ¹H NMR (500 MHz, DMSO-d6) δ10.1-9.29 (m, 2H), 7.68 (s, 2H), 5.39-5.30 (m, 2H), 4.13 (dd, J = 8.0, 5.0 Hz, 1H), 2.60 (s, 3H), 2.00-1.68 (m, 5H), 1.68-1.36 (m, 4H), 1.16-1.03 (m, 2H) |
| | LC-MS (ESI) m/z [M+H]⁺: 331.03 |
| | tR 3.668 min (B) |
| | (2S)-2-[(tert- Butoxycarbonyl)amino]-3-cyclopentylpropionic acid (143415-31-0) |
| | 4-(Bromomethyl)-2,6-dichloropyridine (175204-45-2) |
| | 4M-HCl/MTHP |

**[Table 6-2]**

| | |
|---|---|
| 1-6-2 | 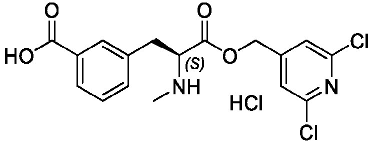 |
| | (S)-3-(3-((2,6-Dichloropyridin-4-yl)methoxy)-2-(methylamino)-3-oxopropyl)benzoic acid hydrochloride |
| | LC-MS (ESI) m/z [M+H]⁺: 383.00 |
| | tR 4.912 min (A) |
| | (S)-2-((tert-Butoxycarbonyl)amino)-3-(3-(tert-butoxycarbonyl)phenyl)propanoic acid (2245801-13-0) |
| | 4-(Bromomethyl)-2,6-dichloropyridine (175204-45-2) |
| | 4M-HCl/MTHP |
| 1-6-3 | 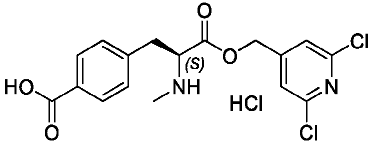 |
| | (S)-4-(3-((2,6-Dichloropyridin-4-yl)methoxy)-2-(methylamino)-3-oxopropyl)benzoic acid hydrochloride |
| | LC-MS (ESI) m/z [M+H]⁺: 383.00 |
| | tR 4.696 min (A) |
| | (S)-2-((tert-Butoxycarbonyl)amino)-3 -(4-(tert-butoxycarbonyl)phenyl)propanoic acid (214750-69-3) |
| | 4-(Bromomethyl)-2,6-dichloropyridine (175204-45-2) |
| | 4M-HCl/MTHP |

**[Table 6-3]**

| | |
|---|---|
| 1-6-4 | 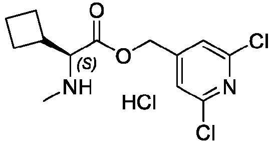 |
| | (2,6-Dichloropyridin-4-yl)methyl (S)-2-cyclobutyl-2-(methylamino)acetate hydrochloride |
| | LC-MS (ESI) m/z [M+H]⁺: 303.03 |
| | tR 4.964 min (A) |
| | (S)-2-((tert-Butoxycarbonyl)(methyl)amino)-2-cyclobutylacetic acid (155905-77-4) |
| | 4-(Bromomethyl)-2,6-dichloropyridine (175204-45-2) |
| | 4M-HCl/MTHP |
| 1-6-5 | 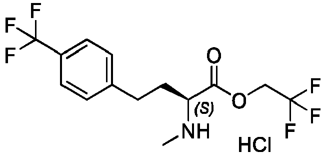 |
| | 2,2,2-Trifluoroethyl (S)-2-(methylamino)-4-(4-(trifluoromethyl)phenyl)butanoate hydrochloride |
| | LC-MS (ESI) m/z [M+H]⁺: 344.67 |
| | tR 3.868 min (B) |
| | (S)-2-((tert-Butoxycarbonyl)amino)-4-(4-(trifluoromethyl)phenyl)butanoic acid (1260592-66-2) |
| | 2,2,2-Trifluoroethyl trifluoromethanesulfonate (6226-25-1) |
| | 4M-HCl/MTHP |

**[Table 6-4]**

| | |
|---|---|
| 1-6-6 | 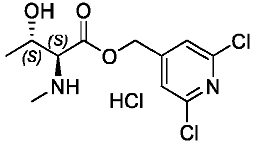 |
| | (2,6-Dichloropyridin-4-yl)methyl methyl-L-allothreoninate hydrochloride |
| | LC-MS (ESI) m/z [M+H]⁺: 292.97 |
| | tR 3.968 min (A) |
| | N-[(1,1-Dimethylethoxy)carbonyl]-O-(1,1-dimethylethyl)-L-allothreonine (474334-59-3) |
| | 4-(Bromomethyl)-2,6-dichloropyridine (175204-45-2) |
| | 4M-HCl/MTHP |
| 1-6-7 | 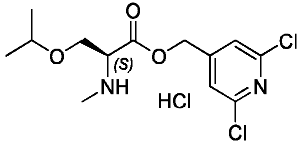 |
| | (2,6-Dichloropyridin-4-yl)methyl O-isopropyl-N-methyl-L-serinate hydrochloride |
| | LC-MS (ESI) m/z [M+H]⁺: 321.01 |
| | tR 5.168 min (A) |
| | N-(tert-Butoxycarbonyl)-O-isopropyl-L-serine (711018-10-9) |
| | 4-(Bromomethyl)-2,6-dichloropyridine (175204-45-2) |
| | 4M-HCl/MTHP |

**[Table 6-5]**

| | |
|---|---|
| 1-6-8 | 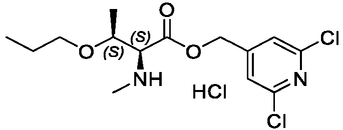 |
| | (2,6-Dichloropyridin-4-yl)methyl N-methyl-O-propyl-L-allothreoninate hydrochloride |
| | LC-MS (ESI) m/z [M+H]⁺: 335.00 |
| | tR 5.756 min (A) |
| | N-(tert-Butoxycarbonyl)-O-propyl-L-threonine (C127533, Kanglong Huacheng Chiral Pharmaceutical Technology (Ningbo) Co., Ltd.) |
| | 4-(Bromomethyl)-2,6-dichloropyridine (175204-45-2) |
| | 4M-HCl/MTHP |
| 1-6-9 | 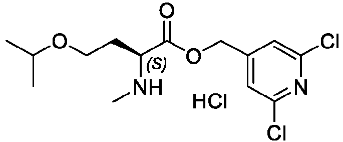 |
| | (2,6-Dichloropyridin-4-yl)methyl O-isopropyl-N-methyl-L-homoserinate hydrochloride |
| | LC-MS (ESI) m/z [M+H]⁺: 334.98 |
| | tR 3.356 min (B) |
| | N-(tert-Butoxycarbonyl)-O-isopropyl-L-homoserine (2349312-43-0) |
| | 4-(Bromomethyl)-2,6-dichloropyridine (175204-45-2) |
| | 4M-HCl/MTHP |

**[Table 6-6]**

| | |
|---|---|
| 1-6-10 | 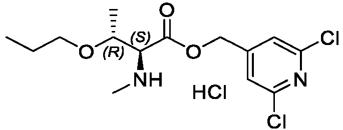 |
| | (2,6-Dichloropyridin-4-yl)methyl N-methyl-O-propyl-L-allothreoninate hydrochloride |
| | LC-MS (ESI) m/z [M+H]⁺: 334.97 |
| | tR 3.536 min (B) |
| | N-(tert-Butoxycarbonyl)-O-propyl-L-threonine (2883499-60-1) |
| | 4-(Bromomethyl)-2,6-dichloropyridine (175204-45-2) |
| | 4M-HCl/MTHP |

Each of the following compounds was synthesized using the corresponding starting compound in each of the following Tables according to the procedure of Example 1-7.

**[Table 7-1]**

| | |
|---|---|
| Example No. | Structure |
| | Compound name |
| | ¹HNMR spectrum analysis results |
| | LC/MS analysis results |
| | tR (LC/MS analysis condition) |
| | Starting compound (CAS Registry Number) |
| | Esterification reagent (CAS Registry Number) |
| | Deprotection reagent |
| 1-7-1 | 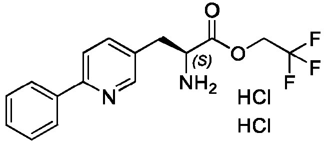 |
| | 2,2,2-Trifluoroethyl (S)-2-amino-3-(6-phenylpyridin-3-yl)propanoate dihydrochloride |
| | ¹H NMR (500 MHz, DMSO-d6) δ 8.88 (brs, 3H), 8.71 (s, 1H), 8.1-78.03 (m, 4H), 7.61-7.49 (m, 3H), 4.99-4.85 (m, 2H), 4.71-4.63 (m, 1H), 3.36-3.31 (m, 2H) |
| | LC-MS (ESI) m/z [M+H]⁺: 324.98 |
| | tR 2.852 min (B) |
| | 5-Bromo-2-phenylpyridine (27012-25-5) |
| | 2,2,2-Trifluoroethyl trifluoromethanesulfonate (6226-25-1) |
| | 4M-HCl/MTHP |
| 1-7-2 | 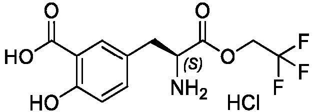 |
| | (S)-5-(2-Amino-3-oxo-3-(2,2,2-trifluoroethoxy)propyl)-2-hydroxybenzoic acid hydrochloride |
| | LC-MS (ESI) m/z [M+H]⁺: 307.92 |
| | tR 4.204 min (A) |
| | tert-Butyl 5-bromo-2-hydroxybenzoate (224775-76-2) |
| | 2,2,2-Trifluoroethyl trifluoromethanesulfonate (6226-25-1) |
| | 4M-HCl/MTHP |

**[Table 7-2]**

| | |
|---|---|
| 1-7-3 | 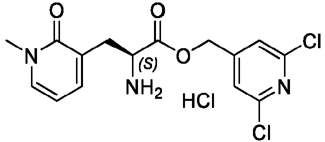 |
| | (2,6-Dichloropyridin-4-yl)methyl (S)-2-amino-3-(1-methyl-2-oxo-1,2-dihydropyridin-3-yl)propanoate hydrochloride |
| | LC-MS (ESI) m/z [M+H]⁺: 355.95 |
| | tR 4.408 min (A) |
| | 3-Bromo-1-methylpyridin-2(1H)-one (81971-38-2) |
| | 4-(Bromomethyl)-2,6-dichloropyridine (175204-45-2) |
| | 4M-HCl/MTHP |
| 1-7-4 | 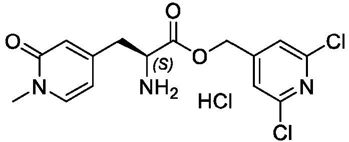 |
| | (2,6-Dichloropyridin-4-yl)methyl (S)-2-amino-3-(1-methyl-2-oxo-1,2-dihydropyridin-4-yl)propanoate hydrochloride |
| | LC-MS (ESI) m/z [M+H]⁺: 356.02 |
| | tR 3.652 min (A) |
| | 4-Bromo-1-methylpyridin-2(1H)-one (214342-63-9) |
| | 4-(Bromomethyl)-2,6-dichloropyridine (175204-45-2) |
| | 4M-HCl/MTHP |
| 1-7-5 | 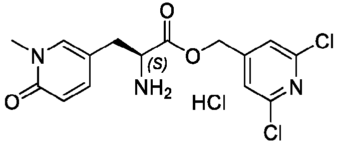 |
| | (2,6-Dichloropyridin-4-yl)methyl (S)-2-amino-3-(1-methyl-6-oxo-1,6-dihydropyridin-3-yl)propanoate hydrochloride |
| | LC-MS (ESI) m/z [M+H]⁺: 356.03 |
| | tR 4.016 min (A) |
| | 5-Bromo-1-methyl-1H-pyridin-2-one (81971-39-3) |
| | 4-(Bromomethyl)-2,6-dichloropyridine (175204-45-2) |
| | 4M-HCl/MTHP |

**[Table 7-3]**

| | |
|---|---|
| 1-7-6 | 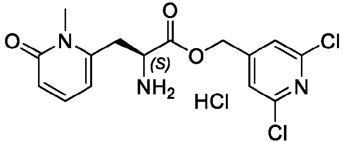 |
| | (2,6-Dichloropyridin-4-yl)methyl (S)-2-amino-3-(1-methyl-6-oxo-1,6-dihydropyridin-2-yl)propanoate hydrochloride |
| | LC-MS (ESI) m/z [M+H]⁺: 355.98 |
| | tR 3.576 min (A) |
| | 6-Bromo-1-methylpyridin-2(1H)-one (873383-11-0) |
| | 4-(Bromomethyl)-2,6-dichloropyridine (175204-45-2) |
| | 4M-HCl/MTHP |
| 1-7-7 | 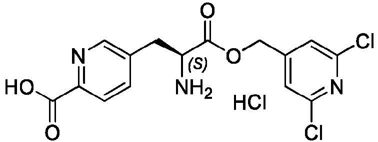 |
| | (S)-5-(2-Amino-3 -((2, 6-dichloropyridin-4-yl)methoxy)-3 -oxopropyl)picolinic acid hydrochloride |
| | LC-MS (ESI) m/z [M+H]⁺: 369.93 |
| | tR 3.296 min (A) |
| | tert-Butyl 5-bromo-2-pyridinecarboxylate (845306-08-3) |
| | 4-(Bromomethyl)-2,6-dichloropyridine (175204-45-2) |
| | 4M-HCl/MTHP and 2,2,2-Trifluoroacetic acid |
| 1-7-8 | 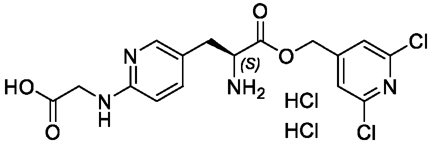 |
| | (S)-(5-(2-Amino-3-((2,6-dichloropyridin-4-yl)methoxy)-3-oxopropyl)pyridin-2-yl)glycine dihydrochloride |
| | LC-MS (ESI) m/z [M+H]⁺: 399.00 |
| | tR 3.076 min (A) |
| | tert-Butyl (5-bromopyridin-2-yl)glycinate (1344711-32-5) |
| | 4-(Bromomethyl)-2,6-dichloropyridine (175204-45-2) |
| | 4M-HCl/MTHP |

**[Table 7-4]**

| | |
|---|---|
| 1-7-9 | 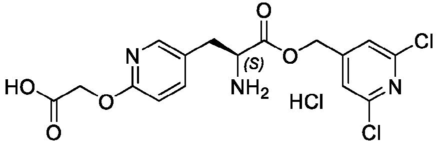 |
| | (S)-2-((5-(2-Amino-3-((2,6-dichloropyridin-4-yl)methoxy)-3-oxopropyl)pyridin-2-yl)oxy)acetic acid hydrochloride |
| | LC-MS (ESI) m/z [M+H]⁺: 399.98 |
| | tR 4.632 min (A) |
| | tert-Butyl 2-((5-bromopyridin-2-yl)oxy)acetate (2408336-10-5) |
| | 4-(Bromomethyl)-2,6-dichloropyridine (175204-45-2) |
| | 4M-HCl/MTHP |
| 1-7-10 | 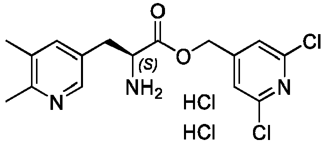 |
| | (2,6-Dichloropyridin-4-yl)methyl (S)-2-amino-3-(5,6-dimethylpyridin-3-yl)propanoate dihydrochloride |
| | LC-MS (ESI) m/z [M+H]⁺: 354.01 |
| | tR 3.352 min (A) |
| | 5-Bromo-2,3-dimethylpyridine (27063-90-7) |
| | 4-(Bromomethyl)-2,6-dichloropyridine (175204-45-2) |
| | 4M-HCl/MTHP |

**[Table 7-5]**

| | |
|---|---|
| 1-7-11 | 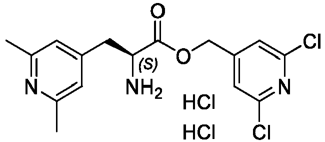 |
| | (2,6-Dichloropyridin-4-yl)methyl (S)-2-amino-3-(2,6-dimethylpyridin-4-yl)propanoate dihydrochloride |
| | LC-MS (ESI) m/z [M+H]⁺: 354.00 |
| | tR 2.868 min (A) |
| | 4-Bromo-2,6-dimethylpyridine (5093-70-9) |
| | 4-(Bromomethyl)-2,6-dichloropyridine (175204-45-2) |
| | 4M-HCl/MTHP |
| 1-7-12 | 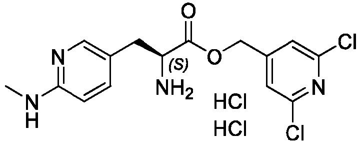 |
| | (2,6-Dichloropyridin-4-yl)methyl (S)-2-amino-3-(6-(methylamino)pyridin-3-yl)propanoate dihydrochloride |
| | LC-MS (ESI) m/z [M+H]⁺: 354.97 |
| | tR 4.452 min (C) |
| | 1,1-Dimethylethyl N-(5-bromo-2-pyridinyl)-N-methylcarbamate (227939-01-7) |
| | 4-(Bromomethyl)-2,6-dichloropyridine (175204-45-2) |
| | 4M-HCl/MTHP |
| 1-7-13 | 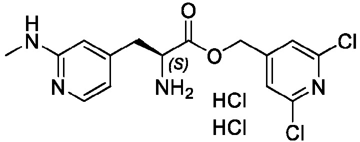 |
| | (2,6-Dichloropyridin-4-yl)methyl (S)-2-amino-3-(2-(methylamino)pyridin-4-yl)propanoate dihydrochloride |
| | LC-MS (ESI) m/z [M+H]⁺: 354.98 |
| | tR 4.152 min (C) |
| | 1, 1 -Dimethylethyl N-(4-bromo-2-pyridinyl)-N-methylcarbamate (946000-13-1) |
| | 4-(Bromomethyl)-2,6-dichloropyridine (175204-45-2) |
| | 4M-HCl/MTHP |

**[Table 7-6]**

| | |
|---|---|
| 1-7-14 | 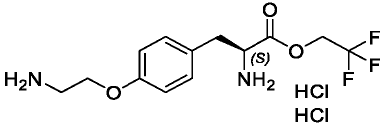 |
| | 2,2,2-Trifluoroethyl (S)-2-amino-3-(4-(2-aminoethoxy)phenyl)propanoate dihydrochloride |
| | LC-MS (ESI) m/z [M+H]⁺: 307.03 |
| | tR 3.908 min (C) |
| | tert-Butyl (2-(4-bromophenoxy)ethyl)carbamate (1593201-17-2) |
| | 2,2,2-Trifluoroethyl trifluoromethanesulfonate (6226-25-1) |
| | 4M-HCl/MTHP |
| 1-7-15 | 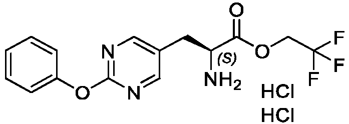 |
| | 2,2,2-Trifluoroethyl (S)-2-amino-3-(2-phenoxypyrimidin-5-yl)propanoate dihydrochloride |
| | LC-MS (ESI) m/z [M+H]⁺: 342.02 |
| | tR 3.164 min (A) |
| | 5-Bromo-2-phenoxypyrimidine (257280-25-4) |
| | 2,2,2-Trifluoroethyl trifluoromethanesulfonate (6226-25-1) |
| | 4M-HCl/MTHP |
| 1-7-16 | 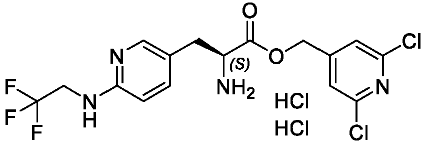 |
| | (2,6-Dichloropyridin-4-yl)methyl (S)-2-amino-3-(6-((2,2,2-trifluoroethyl)amino)pyridin-3-yl)propanoate dihydrochloride |
| | LC-MS (ESI) m/z [M+H]⁺: 422.97 |
| | tR 4.072 min (A) |
| | tert-Butyl (5-bromopyridin-2-yl)(2,2,2-trifluoroethyl)carbamate |
| | 4-(Bromomethyl)-2,6-dichloropyridine (175204-45-2) |
| | 4M-HCl/MTHP |

**[Table 7-7]**

| | |
|---|---|
| 1-7-17 | 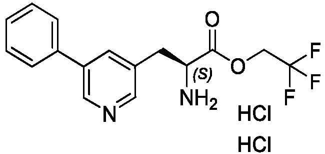 |
| | 2,2,2-Trifluoroethyl (S)-2-amino-3-(5-phenylpyridin-3-yl)propanoate dihydrochloride |
| | LC-MS (ESI) m/z [M+H]⁺: 324.96 |
| | tR 2.700 min (B) |
| | 3-Bromo-5-phenylpyridine (142137-17-5) |
| | 2,2,2-Trifluoroethyl trifluoromethanesulfonate (6226-25-1) |
| | 4M-HCl/MTHP |
| 1-7-18 | 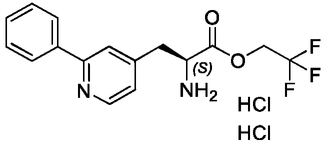 |
| | 2,2,2-Trifluoroethyl (S)-2-amino-3-(2-phenylpyridin-4-yl)propanoate dihydrochloride |
| | LC-MS (ESI) m/z [M+H]⁺: 324.94 |
| | tR 2.604 min (B) |
| | 4-Bromo-2-phenylpyridine (98420-98-5) |
| | 2,2,2-Trifluoroethyl trifluoromethanesulfonate (6226-25-1) |
| | 4M-HCl/MTHP |
| 1-7-19 | 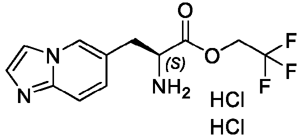 |
| | 2,2,2-Trifluoroethyl (S)-2-amino-3-(imidazo[1,2-a]pyridin-6-yl)propanoate dihydrochloride |
| | LC-MS (ESI) m/z [M+H]⁺: 288.03 |
| | tR 3.132 min (C) |
| | 6-Bromoimidazo[1,2-a]pyridine (6188-23-4) |
| | 2,2,2-Trifluoroethyl trifluoromethanesulfonate (6226-25-1) |
| | 4M-HCl/MTHP |

**[Table 7-8]**

| | |
|---|---|
| 1-7-20 | 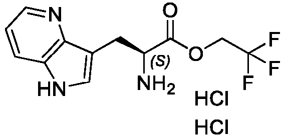 |
| | 2,2,2-Trifluoroethyl (S)-2-amino-3-(1H-pyrrolo[3,2-b]pyridin-3-yl)propanoate dihydrochloride |
| | LC-MS (ESI) m/z [M+H]⁺: 287.99 |
| | tR 3.388 min (C) |
| | tert-Butyl 3-iodo-1H-pyrrolo[3,2-b]pyridine-1-carboxylate (1316228-21-3) |
| | 2,2,2-Trifluoroethyl trifluoromethanesulfonate (6226-25-1) |
| | 4M-HCl/MTHP |
| 1-7-21 | 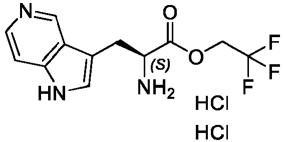 |
| | 2,2,2-Trifluoroethyl (S)-2-amino-3-(1H-pyrrolo[3,2-c]pyridin-3-yl)propanoate dihydrochloride |
| | LC-MS (ESI) m/z [M+H]⁺: 288.03 |
| | tR 3.384 min (C) |
| | tert-Butyl 3-iodo-1H-pyrrolo[3,2-c]pyridine-1-carboxylate (877060-48-5) |
| | 2,2,2-Trifluoroethyl trifluoromethanesulfonate (6226-25-1) |
| | 4M-HCl/MTHP |
| 1-7-22 | 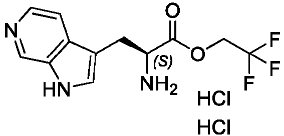 |
| | 2,2,2-Trifluoroethyl (S)-2-amino-3-(1H-pyrrolo[2,3-c]pyridin-3-yl)propanoate dihydrochloride |
| | LC-MS (ESI) m/z [M+H]⁺: 288.03 |
| | tR 3.484 min (C) |
| | tert-Butyl 3-iodo-1H-pyrrolo[2,3-c]pyridine-1-carboxylate (1174038-59-5) |
| | 2,2,2-Trifluoroethyl trifluoromethanesulfonate (6226-25-1) |
| | 4M-HCl/MTHP |

**[Table 7-9]**

| | |
|---|---|
| 1-7-23 | 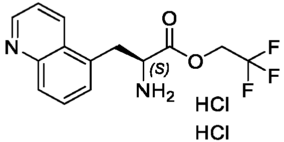 |
| | 2,2,2-Trifluoroethyl (S)-2-amino-3-(quinolin-5-yl)propanoate dihydrochloride |
| | LC-MS (ESI) m/z [M+H]⁺: 298.95 |
| | tR 3.172 min (A) |
| | 5-Bromoquinoline (4964-71-0) |
| | 2,2,2-Trifluoroethyl trifluoromethanesulfonate (6226-25-1) |
| | 4M-HCl/MTHP |

Each of the following compounds was synthesized using the corresponding starting compound in the following Table according to the procedure of Example 1-8.

**Table 8]**

| | |
|---|---|
| Example No. | Structure |
| | Compound name |
| | ¹HNMR spectrum analysis results |
| | LC/MS analysis results |
| | tR (LC/MS analysis condition) |
| | Starting compound (CAS Registry Number) |
| | Esterification reagent (CAS Registry Number) |
| | Deprotection reagent |
| 1-8-1 | 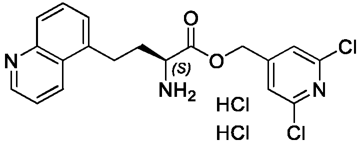 |
| | (2,6-Dichloropyridin-4-yl)methyl (S)-2-amino-4-(quinolin-5-yl)butanoate dihydrochloride |
| | ¹H NMR (500 MHz, DMSO-d6) δ 9.24-9.08 (m, 2H), 8.95 (brd, J = 3.5 Hz, 3H), 8.18 (d, J = 8.5 Hz, 1H), 8.02-7.88 (m, 2H), 7.75 (d, J = 7.0 Hz), 7.68 (s, 2H), 5.38-5.27 (m, 2H), 4.45-4.36 (m, 1H), 3.44-3.27 (m, 2H), 2.39-2.22 (m, 2H) |
| | LC-MS (ESI) m/z [M+H]⁺: 390.00 |
| | tR 3.908 min (A) |
| | 5-Bromoquinoline (4964-71-0) |
| | 4-(Bromomethyl)-2,6-dichloropyridine (175204-45-2) |
| | 4M-HCl/MTHP |
| 1-8-2 | 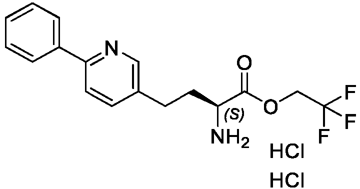 |
| | 2,2,2-Trifluoroethyl (S)-2-amino-4-(6-phenylpyridin-3-yl)butanoate dihydrochloride |
| | LC-MS (ESI) m/z [M+H]⁺: 339.02 |
| | tR 2.836 min (B) |
| | 5-Bromo-2-phenylpyridine (27012-25-5) |
| | 2,2,2-Trifluoroethyl trifluoromethanesulfonate (6226-25-1) |
| | 4M-HCl/MTHP |

Each of the following compounds was synthesized using the corresponding starting compound in each of the following Tables according to the procedure of Example 1-9.

**[Table 9-1]**

| | |
|---|---|
| Example No. | Structure |
| | Compound name |
| | ¹HNMR spectrum analysis results |
| | LC/MS analysis results |
| | tR (LC/MS analysis condition) |
| | Starting compound (CAS Registry Number) |
| | Esterification reagent (CAS Registry Number) |
| | Deprotection reagent |
| 1-9-1 | 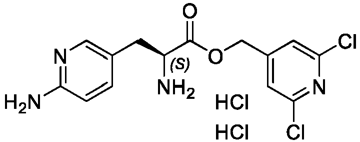 |
| | (2,6-Dichloropyridin-4-yl)methyl (S)-2-amino-3-(6-aminopyridin-3-yl)propanoate dihydrochloride |
| | ¹H NMR (500 MHz, DMSO-d6) δ 14.07 (brs, 1H), 8.82 (brs, 3H), 8.15 (brs, 2H), 7.92-7.83 (m, 2H), 7.64 (s, 2H), 6.98 (d, J = 9.5 Hz, 1H), 5.38-5.26 (m, 2H), 4.59-4.43 (m, 1H), 3.25-3.09 (m, 2H) |
| | LC-MS (ESI) m/z [M+H]⁺: 341.01 |
| | tR 2.816 min (A) |
| | 2-Amino-5-bromopyridine (1072-97-5) |
| | 4-(Bromomethyl)-2,6-dichloropyridine (175204-45-2) |
| | 4M-HCl/MTHP |

**[Table 9-2]**

| | |
|---|---|
| 1-9-2 | 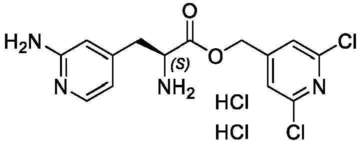 |
| | (2,6-Dichloropyridin-4-yl)methyl (S)-2-amino-3-(2-aminopyridin-4-yl)propanoate dihydrochloride |
| | LC-MS (ESI) m/z [M+H]⁺: 340.94 |
| | tR 4.036 min (C) |
| | 2-Amino-4-bromopyridine (84249-14-9) |
| | 4-(Bromomethyl)-2,6-dichloropyridine (175204-45-2) |
| | 2,2,2-Trifluoroacetic acid (76-05-1) and triisopropylsilane (6485-79-6) |
| 1-9-3 | 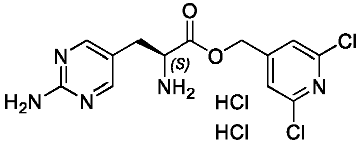 |
| | (2,6-Dichloropyridin-4-yl)methyl (S)-2-amino-3-(2-aminopyrimidin-5-yl)propanoate dihydrochloride |
| | LC-MS (ESI) m/z [M+H]⁺: 342.03 |
| | tR 3.640 min (A) |
| | 2-Amino-5-bromopyrimidine (7752-82-1) |
| | 4-(Bromomethyl)-2,6-dichloropyridine (175204-45-2) |
| | 4M-HCl/MTHP |

Each of the following compounds was synthesized using the corresponding starting compound in the following Tables according to the procedure of Example 1-10.

**[Table 10]**

| | |
|---|---|
| Example No. | Structure |
| | Compound name |
| | ¹HNMR spectrum analysis results |
| | LC/MS analysis results |
| | tR (LC/MS analysis condition) |
| | Starting compound (CAS Registry Number) |
| | Esterification reagent (CAS Registry Number) |
| | Deprotection reagent |
| 1-10-1 | 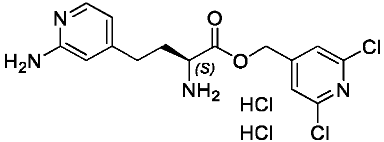 |
| | (2,6-Dichloropyridin-4-yl)methyl (S)-2-amino-4-(2-aminopyridin-3-yl)butanoate dihydrochloride |
| | ¹H NMR (500 MHz, DMSO-d6) δ 13.80 (brs, 1H), 8.93 (brs, 3H), 8.11 (brs, 2H), 7.90 (d, J = 7.0 Hz, 1H), 7.70 (s, 2H), 6.84 (s, 1H), 6.78 (dd, J = 7.0, 1.5 Hz, 1H), 5.39-5.26 (m, 2H), 4.31-4.21 (m, 1H), 2.92-2.71 (m, 2H), 2.29-2.11 (m, 2H) |
| | LC-MS (ESI) m/z [M+H]⁺: 354.96 |
| | tR 3.248 min (A) |
| | 2-Amino-4-bromopyridine (84249-14-9) |
| | 4-(Bromomethyl)-2,6-dichloropyridine (175204-45-2) |
| | 4M-HCl/MTHP |
| 1-10-2 | 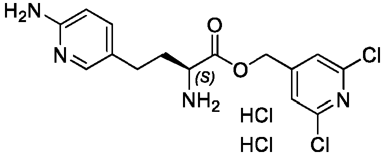 |
| | (2,6-Dichloropyridin-4-yl)methyl (S)-2-amino-4-(6-aminopyridin-3-yl)butanoate dihydrochloride |
| | LC-MS (ESI) m/z [M+H]⁺: 354.98 |
| | tR 4.448 min (A) |
| | 2-Amino-5-bromopyridine (1072-97-5) |
| | 4-(Bromomethyl)-2,6-dichloropyridine (175204-45-2) |
| | 4M-HCl/MTHP |

Each of the following compounds was synthesized using the corresponding starting compound in each of the following Tables according to the procedure of Example 1-11.

**[Table 11-1]**

| | |
|---|---|
| Example No. | Structure |
| | Compound name |
| | ¹HNMR spectrum analysis results |
| | LC/MS analysis results |
| | tR (LC/MS analysis condition) |
| | Starting compound (CAS Registry Number) |
| | Esterification reagent (CAS Registry Number) |
| | Deprotection reagent |
| 1-11-1 | 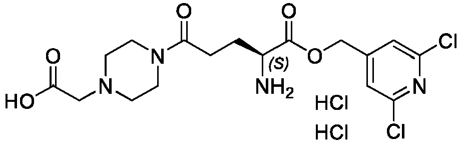 |
| | (S)-2-(4-(4-Amino-5-((2,6-dichloropyridin-4-yl)methoxy)-5-oxopentanoyl)piperazin-1-yl)acetic acid dihydrochloride |
| | ¹H NMR (500 MHz, MeOH-d₄) δ 7.53 (s, 2H), 5.41-5.29 (m, 2H), 4.31 (dd, J = 6.5 Hz, 1H), 4.17 (s, 2H), 4.10-3.60 (m, 4H), 3.60-3.38 (m, 4H), 2.86-2.70 (m, 2H), 2.43-2.19 (m, 2H) |
| | LC-MS (ESI) m/z [M+H]⁺: 432.98 |
| | tR 4.128 min (C) |
| | tert-Butyl 2-(piperazin-1-yl)acetate (112257-22-4) |
| | 4-(Bromomethyl)-2,6-dichloropyridine (175204-45-2) |
| | 4M-HCl/CPME |
| 1-11-2 | 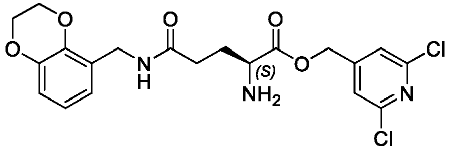 |
| | (2,6-Dichloropyridin-4-yl)methyl N⁵-((2,3-dihydrobenzo[b][1,4]dioxin-5-yl)methyl)-L-glutamate hydrochloride |
| | LC-MS (ESI) m/z [M+H]⁺: 454.08 |
| | tR 3.468 min (B) |
| | (2,3-Dihydrobenzo[b][1,4]dioxin-5-yl)methanamine (261633-71-0) |
| | 4-(Bromomethyl)-2,6-dichloropyridine (175204-45-2) |
| | 4M-HCl/1,4-dioxane |

**[Table 11-2]**

| | |
|---|---|
| 1-11-3 | 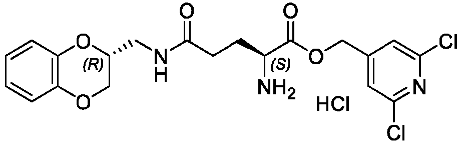 |
| | (2,6-Dichloropyridin-4-yl)methyl N⁵-((R)-2,3-dihydrobenzo[b][1,4]dioxin-2-yl)methyl)-L-glutamate hydrochloride |
| | LC-MS (ESI) m/z [M+H]⁺: 454.06 |
| | tR 3.620 min (B) |
| | (R)-(2,3-Dihydrobenzo[b][1,4]dioxin-2-yl)methanamine (46049-48-3) |
| | 4-(Bromomethyl)-2,6-dichloropyridine (175204-45-2) |
| | 4M-HCl/1,4-dioxane |

The following compound was synthesized according to the procedure of Example 1-12.

**[Table 12]**

| | |
|---|---|
| Example No. | Structure |
| | Compound name |
| | ¹HNMR spectrum analysis results |
| | LC/MS analysis results |
| | tR (LC/MS analysis condition) |
| 1-12-1 | 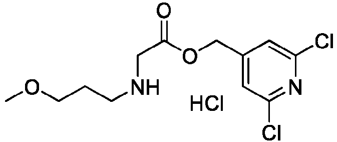 |
| | (2,6-Dichloropyridin-4-yl)methyl (3-methoxypropyl)glycinate hydrochloride |
| | ¹H NMR (500 MHz, DMSO-d6) δ 9.40 (brs, 2H), 7.67 (s, 2H), 5.34 (s, 2H), 4.17 (s, 2H), 3.40 (dd, J = 6.0 Hz, 2H), 3.24 (s, 3H), 3.05-2.96 (m, 2H), 1.95-1.85 (m, 2H) |
| | LC-MS (ESI) m/z [M+H]⁺: 307.01 |
| | tR 3.900 min (A) |

The following compound was synthesized according to the procedure of Example 1-13.

**Table 13]**

| | |
|---|---|
| Example No. | Structure |
| | Compound name |
| | ¹HNMR spectrum analysis results |
| | LC/MS analysis results |
| | tR (LC/MS analysis condition) |
| 1-13-1 | 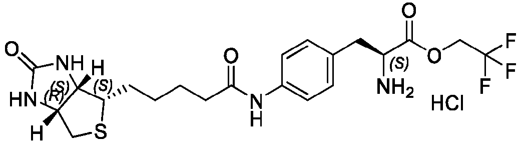 |
| | 2,2,2-Trifluoroethyl (S)-2-amino-3-(4-(5-((3aS,4S,6aR)-2-oxohexahydro-1H-thieno[3,4-d]imidazol-4-yl)pentanamido)phenyl)propanoate hydrochloride |
| | ¹H NMR (500 MHz, DMSO-d6) δ 9.99 (brs, 1H), 8.69 (brs, 3H), 7.56 (d, J = 8.0 Hz, 2H), 7.16 (dd, J = 8.0Hz, 2H), 6.46 (brs, 2H), 4.94-4.81 (m, 2H), 4.35-4.27 (m, 1H), 4.18-4.10 (m, 1H), 3.20-3.02 (m, 3H), 2.88-2.78 (m, 1H), 2.62-2.55 (m, 1H), 2.36-2.25 (m, 2H), 1.72-1.26 (m, 6H) |
| | LC-MS (ESI) m/z [M+H]⁺: 489.09 |
| | tR 5.068 min (A) |

The following compound was synthesized according to the procedure of Example 1-14.

**[Table 14]**

| | |
|---|---|
| Example No. | Structure |
| | Compound name |
| | ¹HNMR spectrum analysis results |
| | LC/MS analysis results |
| | tR (LC/MS analysis condition) |
| 1-14-1 | 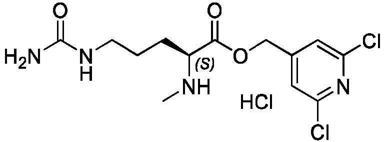 |
| | (2,6-Dichloropyridin-4-yl)methyl (S)-2-(methylamino)-5-ureidopentanoate hydrochloride |
| | ¹H NMR (500 MHz, DMSO-d6) δ 9.77-9.42 (m ,2H), 7.70 (s, 2H), 5.42-5.27 (m, 2H), 4.31-4.18 (m, 1H), 3.05-2.94 (m, 2H), 2.54-2.46 (s, 3H), 2.00-1.82 (m, 2H), 1.58-1.32 (m, 2H) |
| | LC-MS (ESI) m/z [M+H]⁺: 348.94 |
| | tR 3.500 min (A) |

The following compound was synthesized according to the procedure of Example 1-15.

**[Table 15]**

| | |
|---|---|
| Example No. | Structure |
| | Compound name |
| | ¹HNMR spectrum analysis results |
| | LC/MS analysis results |
| | tR (LC/MS analysis condition) |
| 1-15-1 | 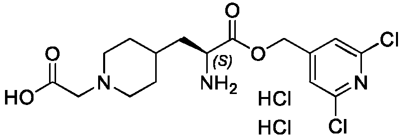 |
| | (S)-2-(4-(2-Amino-3-((2,6-dichloropyridin-4-yl)methoxy)-3-oxopropyl)piperidin-1-yl)acetic acid dihydrochloride |
| | ¹H NMR (500 MHz, DMSO-d6) δ 13.99 (brs, 1H), 10.08 (brs, 1H), 8.84 (brs, 3H), 7.71 (s, 2H), 5.39-5.26 (m, 2H), 4.32-4.19 (m, 1H), 4.19-4.03 (s, 2H), 3.60-3.47 (m, 2H), 3.42-3.36 (m, 2H), 3.10-2.91 (m, 2H), 2.04-1.74 (m, 5H) |
| | LC-MS (ESI) m/z [M+H]⁺: 390.06 |
| | tR 4.152 min (C) |

The following compound was synthesized according to the procedure of Example 1-16.

**Table 16]**

| | |
|---|---|
| Example No. | Structure |
| | Compound name |
| | ¹HNMR spectrum analysis results |
| | LC/MS analysis results |
| | tR (LC/MS analysis condition) |
| 1-16-1 | 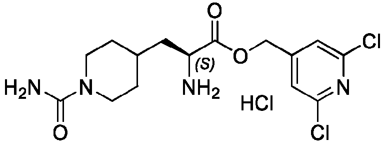 |
| | (2,6-Dichloropyridin-4-yl)methyl (S)-2-amino-3-(1-carbamoylpiperidin-4-yl)propanoate hydrochloride |
| | ¹H NMR (500 MHz, DMSO-d6) δ 8.72 (brd, J = 4.0 Hz, 3H), 7.69 (s, 2H), 5.37-5.27 (m, 2H), 4.28-4.15 (m, 1H), 3.99-3.86 (m, 2H), 2.68-2.53 (m, 2H), 1.84-1.58 (m, 5H), 1.06-0.92 (m, 2H) |
| | LC-MS (ESI) m/z [M+H]⁺: 375.06 |
| | tR 4.136 min (A) |

The following compound was synthesized according to the procedure of Example 1-17.

**[Table 17]**

| | |
|---|---|
| Example No. | Structure |
| | Compound name |
| | ¹HNMR spectrum analysis results |
| | LC/MS analysis results |
| | tR (LC/MS analysis condition) |
| 1-17-1 | 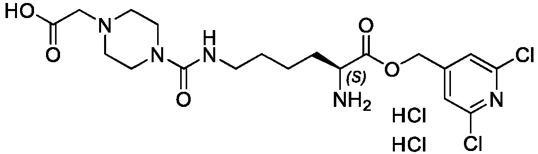 |
| | (S)-2-(4-((5-Amino-6-((2,6-dichloropyridin-4-yl)methoxy)-6-oxohexyl)carbamoyl)piperazin-1-yl)acetic acid dihydrochloride |
| | ¹H NMR (500 MHz, D₂O) δ 7.44 (s, 2H), 5.32-5.24 (m, 2H), 4.21 (dd, J = 6.0 Hz, 1H), 4.17-2.81 (m, 12H), 2.01-1.86 (m, 2H), 1.51-1.19 (m, 4H) |
| | LC-MS (ESI) m/z [M+H]⁺: 476.02 |
| | tR 3.708 min (A) |

The following compound was synthesized according to the procedure of Example 1-18.

**[Table 18]**

| | |
|---|---|
| Example No. | Structure |
| | Compound name |
| | ¹HNMR spectrum analysis results |
| | LC/MS analysis results |
| | tR (LC/MS analysis condition) |
| 1-18-1 | 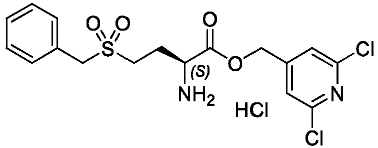 |
| | (2,6-Dichloropyridin-4-yl)methyl (S)-2-amino-4-(benzylsulfonyl)butanoate hydrochloride |
| | ¹H NMR (500 MHz, DMSO-d6) δ 8.73 (brs, 3H), 7.70 (s, 2H), 7.46-7.33 (m, 5H), 5.39-5.26 (m, 2H), 4.56 (s, 2H), 4.44-4.32 (m, 1H), 3.40-3.22 (m, 2H), 2.39-2.24 (m, 2H) |
| | LC-MS (ESI) m/z [M+H]⁺: 416.98 |
| | tR 3.168 min (B) |

The following compound was synthesized according to the procedure of Example 1-19.

**[Table 19]**

| | |
|---|---|
| Example No. | Structure |
| | Compound name |
| | ¹HNMR spectrum analysis results |
| | LC/MS analysis results |
| | tR (LC/MS analysis condition) |
| 1-19-1 | 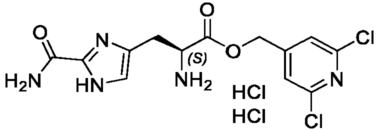 |
| | (2,6-Dichloropyridin-4-yl)methyl (S)-2-amino-3-(2-carbamoyl-1H-imidazol-4-yl)propanoate dihydrochloride |
| | ¹H NMR (500 MHz, DMSO-d6) δ 8.83 (brs, 3H), 8.24 (brs, 1H), 7.81 (brs, 1H), 7.54 (s, 2H), 7.30 (s, 1H), 5.35-5.22 (m, 2H), 4.65-4.53 (s, 1H), 3.32-3.21 (m, 1H) |
| | LC-MS (ESI) m/z [M+H]⁺: 357.98 |
| | tR 3.824 min (A) |

Each of the following compounds was synthesized using the corresponding starting amino acid in each of the following Tables according to the procedure of Example 1-20.

**[Table 20-11**

| | |
|---|---|
| Example No. | Structure |
| | Compound name |
| | ¹HNMR spectrum analysis results |
| | LC/MS analysis results |
| | tR (LC/MS analysis condition) |
| | Starting amino acid (CAS Registry Number) |
| | Esterification reagent (CAS Registry Number) |
| | Deprotection reagent |
| 1-20-1 | 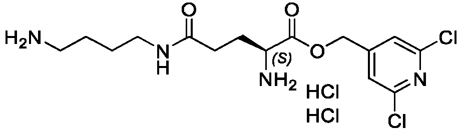 |
| | (2,6-Dichloropyridin-4-yl)methyl N⁵-(4-aminobutyl)-L-glutamate dihydrochloride |
| | ¹H NMR (500 MHz, DMSO-d6) δ8.74 (brs, 3H), 8.12 (t, J = 5.5 Hz), 1H), 7.94 (brs, 3H), 7.69 (s, 3H), 5.37-5.24 (m, 2H), 4.26-4.16 (m, 1H), 3.09-2.99 (m, 2H), 2.81-2.71 (m, 2H), 2.45-2.24 (m, 2H), 2.17-2.03 (m, 2H), 1.59-1.37 (m, 4H) |
| | LC-MS (ESI) m/z [M+H]⁺: 377.02 |
| | tR 4.192 min (C) |
| | N²-(((9H-Fluoren-9-yl)methoxy)carbonyl)-N⁵-(4-((tert-butoxycarbonyl)amino)butyl)-L-glutamine (710326-52-6) |
| | 4-(Bromomethyl)-2,6-dichloropyridine (175204-45-2) |
| | 4M-HCl/MTHP |

**[Table 20-2]**

| | |
|---|---|
| 1-20-2 | 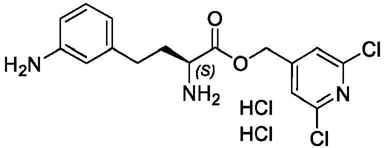 |
| | (2,6-Dichloropyridin-4-yl)methyl (S)-2-amino-4-(3-aminophenyl)butanoate dihydrochloride |
| | LC-MS (ESI) m/z [M+H]⁺: 354.07 |
| | tR 2.556 min (B) |
| | (S)-2-((tert-Butoxycarbonyl)amino)-4-(3-((tert-butoxycarbonyl)amino)phenyl)butanoic acid (K25791, Mimotopes) |
| | 4-(Bromomethyl)-2,6-dichloropyridine (175204-45-2) |
| | 4M-HCl/MTHP |
| 1-20-3 | 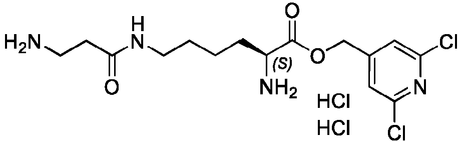 |
| | (2,6-Dichloropyridin-4-yl)methyl N⁶-(3-aminopropanoyl)-L-lysinate dihydrochloride |
| | LC-MS (ESI) m/z [M+H]⁺: 376.97 |
| | tR 3.352 min (A) |
| | N²-(tert-Butoxycarbonyl)-N⁶-(3-((tert-butoxycarbonyl)amino)propanoyl)-L-lysine (1433718-38-7) |
| | 4-(Bromomethyl)-2,6-dichloropyridine (175204-45-2) |
| | 4M-HCl/MTHP |

The following compound was synthesized according to the procedure of Example 1-21.

**[Table 21]**

| | |
|---|---|
| Example No. | Structure |
| | Compound name |
| | ¹HNMR spectrum analysis results |
| | LC/MS analysis results |
| | tR (LC/MS analysis condition) |
| 1-21-1 | 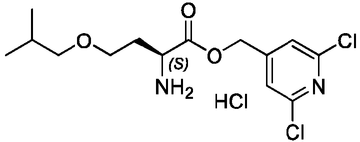 |
| | (2,6-Dichloropyridin-4-yl)methyl O-isobutyl-L-homoserinate hydrochloride |
| | ¹H NMR (500 MHz, DMSO-d6) δ 8.60 (brs, 3H), 7.68 (s, 2H), 5.35-5.25 (m, 2H), 4.25-4.23 (m, 1H), 3.56-3.47 (m, 2H), 3.15-3.06 (m, 2H), 2.20-2.06 (m, 2H), 1.78-1.69 (m, 1H), 0.8-40.75 (m, 6H) |
| | LC-MS (ESI) m/z [M+H]⁺: 336.94 |
| | tR 3.460 min (B) |

Each of the following compounds was synthesized using the corresponding starting compound in each of the following Tables according to the procedure of Example 1-22.

**[Table 22-1]**

| | |
|---|---|
| Example No. | Structure |
| | Compound name |
| | ¹HNMR spectrum analysis results |
| | LC/MS analysis results |
| | tR (LC/MS analysis condition) |
| | Starting compound (CAS Registry Number) |
| 1-22-1 | 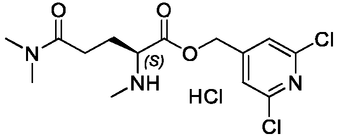 |
| | (2,6-Dichloropyridin-4-yl)methyl N²,N⁵,N⁵-trimethyl-L-glutamate hydrochloride |
| | ¹H NMR (500 MHz, DMSO-d6) δ 9.74 (brs, 1H), 9.43 (brs, 1H), 7.69 (s, 2H), 5.39-5.21 (m, 2H), 4.28-4.16 (m, 1H), 2.94 (s, 3H), 2.85 (s, 3H), 2.67-2.54 (m, 4H), 2.94-2.42 (m, 1H), 2.24-2.05 (m, 2H) |
| | LC-MS (ESI) m/z [M+H]⁺: 347.98 |
| | tR 4.588 min (A) |
| | Dimethylamine hydrochloride (506-59-2) |
| 1-22-2 | 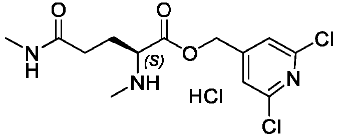 |
| | (2,6-Dichloropyridin-4-yl)methyl N²,N³-dimethyl-L-glutamate hydrochloride |
| | LC-MS (ESI) m/z [M+H]⁺: 334.02 |
| | tR 3.720 min (A) |
| | Methylamine (74-89-5) |

**[Table 22-2]**

| | |
|---|---|
| 1-22-3 | 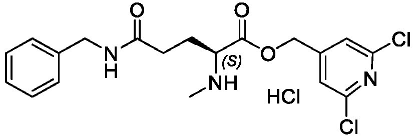 |
| | (2,6-Dichloropyridin-4-yl)methyl N⁵-benzyl-N²-methyl-L-glutamate hydrochloride |
| | LC-MS (ESI) m/z [M+H]⁺: 410.06 |
| | tR 5.704 min (A) |
| | Benzylamine (100-46-9) |

Each of the following compounds was synthesized using the corresponding starting compound in each of the following Tables according to the procedure of Example 1-23.

**[Table 23-1]**

| | |
|---|---|
| Example No. | Structure |
| | Compound name |
| | ¹HNMR spectrum analysis results |
| | LC/MS analysis results |
| | tR (LC/MS analysis conditions) |
| | Starting compound (CAS Registry Number) |
| | Esterification reagent (CAS Registry Number) |
| 1-23-1 | 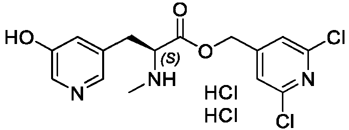 |
| | (2,6-Dichloropyridin-4-yl)methyl (S)-3-(5-hydroxypyridin-3-yl)-2-(methylamino)propanoate dihydrochloride |
| | ¹H NMR (500 MHz, DMSO-d6) δ 11.82 (brs, 1H), 10.46 (brs, 1H), 9.38 (brs, 1H), 8.44 (s, 1H), 8.33 (s, 1H), 7.88 (s, 1H), 7.56 (s, 1H), 5.40-5.25 (m, 2H), 4.74-4.65 (m, 1H), 3.54 (dd, J = 14.5, 5.0 Hz, 1H), 3.42 (dd, J = 14.5, 8.0 Hz, 1H), 2.66 (s, 3H) |
| | LC-MS (ESI) m/z [M+H]⁺: 356.01 |
| | tR 4.436 min (C) |
| | 5-Bromopyridin-3-ol (74115-13-2) |
| | 4-(Bromomethyl)-2,6-dichloropyridine (175204-45-2) |
| 1-23-2 | 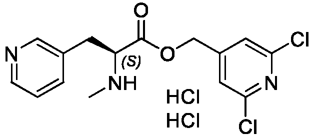 |
| | (2,6-Dichloropyridin-4-yl)methyl (S)-2-(methylamino)-3-(pyridin-3-yl)propanoate dihydrochloride |
| | LC-MS (ESI) m/z [M+H]⁺: 339.97 |
| | tR 3.132 min (A) |
| | 3-Bromopyridine (626-55-1) |
| | 4-(Bromomethyl)-2,6-dichloropyridine (175204-45-2) |

**[Table 23-2]**

| | |
|---|---|
| 1-23-3 | 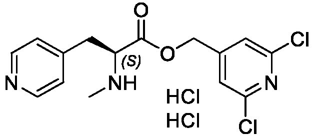 |
| | (2,6-Dichloropyridin-4-yl)methyl (S)-2-(methylamino)-3-(pyridin-4-yl)propanoate dihydrochloride |
| | LC-MS (ESI) m/z [M+H]⁺: 339.96 |
| | tR 4.176 min (C) |
| | 4-Bromopyridine (1120-87-2) |
| | 4-(Bromomethyl)-2,6-dichloropyridine (175204-45-2) |
| 1-23-4 | 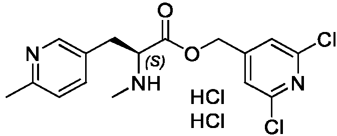 |
| | (2,6-Dichloropyridin-4-yl)methyl (S)-2-(methylamino)-3-(6-methylpyridin-3-yl)propanoate dihydrochloride |
| | LC-MS (ESI) m/z [M+H]⁺: 354.01 |
| | tR 3.436 min (A) |
| | 5-Bromo-2-methylpyridine (3430-13-5) |
| | 4-(Bromomethyl)-2,6-dichloropyridine (175204-45-2) |
| 1-23-5 | 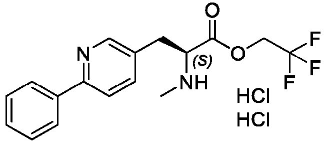 |
| | (2,6-Dichloropyridin-4-yl)methyl (S)-2-(methylamino)-3-(6-phenylpyridin-3-yl)propanoate dihydrochloride |
| | LC-MS (ESI) m/z [M+H]⁺: 339.01 |
| | tR 4656 min (A) |
| | 5-Bromo-2-phenylpyridine (27012-25-5) |
| | 2,2,2-Trifluoroethyl trifluoromethanesulfonate (6226-25-1) |

The following compound was synthesized according to the procedure of Example 1-24.

**[Table 24]**

| | |
|---|---|
| Example No. | Structure |
| | Compound name |
| | ¹HNMR spectrum analysis results |
| | LC/MS analysis results |
| | tR (LC/MS analysis condition) |
| 1-24-1 | 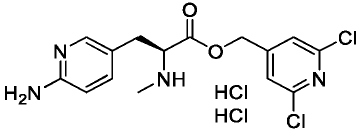 |
| | (2,6-Dichloropyridin-4-yl)methyl (S)-3-(5-hydroxypyridin-3-yl)-2-(methylamino)propanoate dihydrochloride |
| | ¹H NMR (500 MHz, DMSO-d6) δ 14.08 (brs, 1H), 10.14 (brs, 1H), 9.56 (brs, 1H), 8.15 (brs, 2H), 7.96 (d, J = 2.0 Hz, 1H), 7.81 (dd, J = 9.0, 2.0 Hz, 1H), 7.59 (s, 2H), 6.95 (d, J = 9.0 Hz, 1H), 5.40-5.22 (m, 2H), 4.59-4.50 (m, 1H), 3.34-3.26 (m, 1H), 3.26-3.16 (m, 1H), 2.64 (s, 3H) |
| | LC-MS (ESI) m/z [M+H]⁺: 354.99 |
| | tR 4.156 min (C) |

The following compound was synthesized according to the procedure of Example 1-25.

**[Table 25]**

| | |
|---|---|
| Example No. | Structure |
| | Compound name |
| | ¹HNMR spectrum analysis results |
| | LC/MS analysis results |
| | tR (LC/MS analysis condition) |
| 1-25-1 | 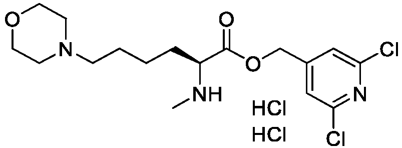 |
| | (2,6-Dichloropyridin-4-yl)methyl (S)-2-(methylamino)-6-morpholinohexanoate dihydrochloride |
| | ¹H NMR (500 MHz, DMSO-d6) δ 11.32 (brs, 1H), 9.91 (brs, 1H), 9.57 (brs, 1H), 7.72 (s, 2H), 5.45-5.25 (m, 2H), 4.30-4.21 (m, 1H), 3.97-3.79 (m, 4H), 3.43-3.29 (m, 2H), 3.11-2.93 (m, 4H), 2.61 (s, 3H), 2.08-1.83 (m, 2H), 1.83-1.66 (m, 2H), 1.54-1.26 (m, 2H) |
| | LC-MS (ESI) m/z [M+H]⁺: 390.07 |
| | tR 4.380 min (C) |

The following compound was synthesized according to the procedure of Example 1-26.

**[Table 26]**

| | |
|---|---|
| Example No. | Structure |
| | Compound name |
| | ¹HNMR spectrum analysis results |
| | LC/MS analysis results |
| | tR (LC/MS analysis condition) |
| 1-26-1 | 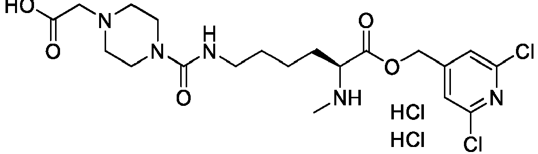 |
| | (S)-2-(4-((6-((2,6-Dichloropyridin-4-yl)methyl)-5-(methylamino)-6-oxohexyl)carbamoyl)piperazin-1-yl)acetic acid dihydrochloride |
| | ¹H NMR (500 MHz, D₂O) δ 7.41 (s, 2H), 5.32-5.20 (m, 2H), 4.17-4.09 (m, 1H), 4.05-2.92 (m, 12H), 2.67 (s, 3H), 2.06-1.89 (m, 2H), 1.44-1.04 (m, 4H) |
| | LC-MS (ESI) m/z [M+H]⁺: 490.03 |
| | tR 3.752 min (A) |

Each of the following compounds was synthesized using the corresponding starting compound in each of the following Tables according to the procedure of Example 1-27.

**[Table 27-1]**

| | |
|---|---|
| Example No. | Structure |
| | Compound name |
| | ¹HNMR spectrum analysis results |
| | LC/MS analysis results |
| | tR (LC/MS analysis condition) |
| | Starting compound (CAS Registry Number) |
| | Esterification reagent (CAS Registry Number) |
| | Deprotection reagent |
| 1-27-1 | 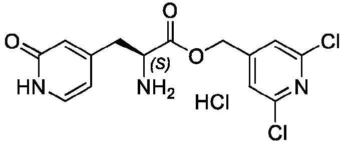 |
| | (2,6-Dichloropyridin-4-yl)methyl (S)-2-amino-3-(2-oxo-1,2-dihydropyridin-4-yl)propanoate hydrochloride |
| | ¹H NMR (500 MHz, DMSO-d6) δ 8.80 (brs, 3H), 7.58 (s, 2H), 7.42 (d, J = 7.0 Hz, 1H), 6.38 (s, 1H), 6.26 (dd, J = 2.0, 7.0 Hz, 1H), 5.35-5.24 (m, 2H), 4.58-4.49 (m, 1H), 3.12-3.00 (m, 2H) |
| | LC-MS (ESI) m/z [M+H]⁺: 341.93 |
| | tR 4.972 min (C) |
| | 4-Bromo-2-methoxypyridine (100367-39-3) |
| | 4-(Bromomethyl)-2,6-dichloropyridine (175204-45-2) |
| | 4M-HCl/MTHP |

**[Table 27-2]**

| | |
|---|---|
| 1-27-2 | 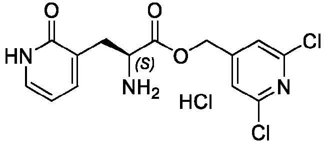 |
| | (2,6-Dichloropyridin-4-yl)methyl (S)-2-amino-3-(2-oxo-1,2-dihydropyridin-3-yl)propanoate hydrochloride |
| | LC-MS (ESI) m/z [M+H]⁺: 341.93 |
| | tR 4.028 min (A) |
| | 3-Bromo-2-methoxypyridine (13472-59-8) |
| | 4-(Bromomethyl)-2,6-dichloropyridine (175204-45-2) |
| | 4M-HCl/MTHP |
| 1-27-3 | 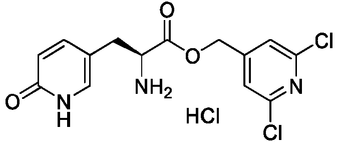 |
| | (2,6-Dichloropyridin-4-yl)methyl (S)-2-amino-3-(6-oxo-1,6-dihydropyridin-3-yl)propanoate hydrochloride |
| | LC-MS (ESI) m/z [M+H]⁺: 342.02 |
| | tR 3.772 min (A) |
| | 2-Bromo-2-methoxypyridine (13472-85-0) |
| | 4-(Bromomethyl)-2,6-dichloropyridine (13472-85-0) |
| | 4M-HCl/MTHP |

**[Table 27-3]**

| | |
|---|---|
| 1-27-4 | 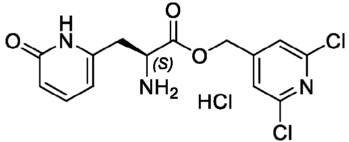 |
| | (2,6-Dichloropyridin-4-yl)methyl (S)-2-amino-3-(6-oxo-1,6-dihydropyridin-2-yl)propanoate hydrochloride |
| | LC-MS (ESI) m/z [M+H]⁺: 341.96 |
| | tR 3.608 min (A) |
| | 2-Bromo-6-methoxypyridine (40473-07-2) |
| | 4-(Bromomethyl)-2,6-dichloropyridine (175204-45-2) |
| | 4M-HCl/MTHP |
| 1-27-5 | 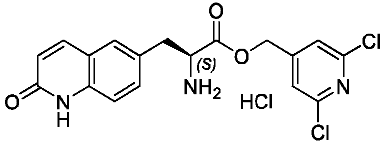 |
| | (2,6-Dichloropyridin-4-yl)methyl (S)-2-amino-3-(2-oxo-1,2-dihydroquinolin-6-yl)propanoate hydrochloride |
| | LC-MS (ESI) m/z [M+H]⁺: 391.99 |
| | tR 4.120 min (A) |
| | 6-Bromo-2-methoxyquinoline (99455-05-7) |
| | 4-(Bromomethyl)-2,6-dichloropyridine (175204-45-2) |
| | 4M-HCl/MTHP |

The following compound was synthesized according to the procedure of Example 1-28.

**[Table 28]**

| | |
|---|---|
| Example No. | Structure |
| | Compound name |
| | ¹HNMR spectrum analysis results |
| | LC/MS analysis results |
| | tR (LC/MS analysis condition) |
| | Starting compound (CAS Registry Number) |
| 1-28-1 | 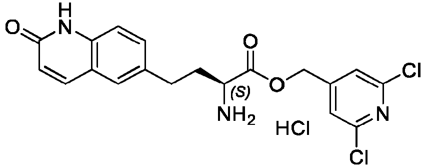 |
| | (2,6-Dichloropyridin-4-yl)methyl (S)-2-amino-4-(2-oxo-1,2-dihydroquinolin-6-yl)butanoate hydrochloride |
| | ¹H NMR (500 MHz, DMSO-d6) δ11.8 (brs, 1H), 8.82 (brs, 3H), 7.83 (d, J = 9.0 Hz, 1H), 7.68 (s, 2H), 7.49 (d, J = 2.0 Hz, 1H), 7.38 (dd, J = 2.0, 8.5 Hz, 1H), 7.26 (d, J = 8.5 Hz, 1H), 6.49 (d, J = 9.0 Hz, 1H), 5.36-5.24 (m, 2H), 4.28-4.15 (m, 1H), 2.91-2.65 (m, 2H), 2,26-2.21 (m, 2H) |
| | LC-MS (ESI) m/z [M+H]⁺: 406.00 |
| | tR 4.792 min (A) |
| | 6-Bromo-2-methoxyquinoline (99455-05-7) |

The following compound was synthesized according to the procedure of Example 1-29.

**[Table 29]**

| | |
|---|---|
| Example No. | Structure |
| | Compound name |
| | ¹HNMR spectrum analysis results |
| | LC/MS analysis results |
| | tR (LC/MS analysis condition) |
| 1-29-1 | 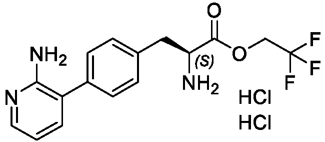 |
| | 2,2,2-Trifluoroethyl (S)-2-amino-3-(4-(2-aminopyridin-3-yl)phenyl)propanoate dihydrochloride |
| | ¹H NMR (500 MHz, DMSO-d6) δ 8.77 (brs, 3H), 8.05 (dd, J = 1.5, 6.5 H, 1H), 7.78 (dd, J = 1.5, 7.0 Hz, 1H), 7.62 (brs, 2H), 7.47-7.40 (m, 4H), 7.02-6.99 (m, 1H), 4.94-4.85 (m, 2H), 4.58-4.48 (m, 1H), 3.29-3.17 (m, 2H) |
| | LC-MS (ESI) m/z [M+H]⁺: 340.00 |
| | tR 4.704 min (C) |

The following compound was synthesized according to the procedure of Example 1-30.

**[Table 30]**

| | |
|---|---|
| Example No. | Structure |
| | Compound name |
| | ¹HNMR spectrum analysis results |
| | LC/MS analysis results |
| | tR (LC/MS analysis condition) |
| 1-30-1 | 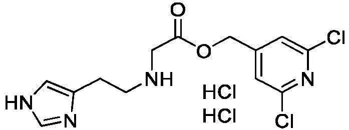 |
| | (2,6-Dichloropyridin-4-yl)methyl (S)-2-amino-3-(2-carbamoyl-1H-imidazol-4-yl)propanoate dihydrochloride |
| | ¹H NMR (500 MHz, DMSO-d6) δ 14.8 (brs, 1H), 9.83 (brs, 2H), 9.06 (s, 1H), 7.68 (s, 2H), 7.54 (s, 1H), 5.35 (s, 2H), 4.42 (s, 2H), 3.33 (t, J = 7.5 H, 2H), 3.15 (t, J = 7.5 Hz, 2H) |
| | LC-MS (ESI) m/z [M+H]⁺: 328.98 |
| | tR 4.128 min (C) |

Each of the following compounds was synthesized using the corresponding starting amino acid in the following Table according to the procedure of Example 1-31.

**[Table 31]**

| | |
|---|---|
| Example No. | Structure |
| | Compound name |
| | ¹HNMR spectrum analysis results |
| | LC/MS analysis results |
| | tR (LC/MS analysis condition) |
| | Starting compound (CAS Registry Number) |
| 1-31-1 | 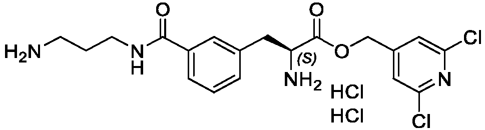 |
| | (2,6-Dichloropyridin-4-yl)methyl (S)-2-amino-3-(3-((3-aminopropyl)carbamoyl)phenyl)propanoate dihydrochloride |
| | ¹H NMR (500 MHz, DMSO-d6) δ 8.96-8.70 (m, 4H), 7.96 (brs, 3H), 7.82 (s, 1H), 7.76 (s, 1H), 7.47-7.34 (m, 4H), 5.28-5.17 (m, 2H), 4.61-4.46 (m, 1H), 3.37-3.24 (m, 2H), 3.24-3.16 (m, 2H), 2.90-2.76 (m, 2H), 1.90-1.77 (m, 2H) |
| | LC-MS (ESI) m/z [M+H]⁺: 425.05 |
| | tR 3.860 min (A) |
| | tert-Butyl (3-aminopropyl)carbamate (75178-96-0) |
| 1-31-2 | 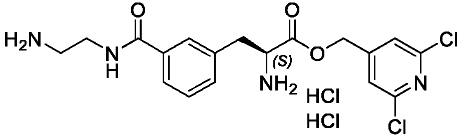 |
| | (2,6-Dichloropyridin-4-yl)methyl (S)-2-amino-3-(3-((2-aminoethyl)carbamoyl)phenyl)propanoate dihydrochloride |
| | LC-MS (ESI) m/z [M+H]⁺: 411.04 |
| | tR 3.756 min (A) |
| | tert-Butyl (3-aminoethyl)carbamate (57260-73-8) |

The following compound was synthesized according to the procedure of Example 1-32.

**[Table 32]**

| | |
|---|---|
| Example No. | Structure |
| | Compound name |
| | ¹HNMR spectrum analysis results |
| | LC/MS analysis results |
| | tR (LC/MS analysis condition) |
| 1-32-1 | 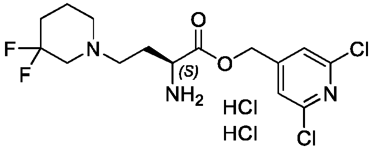 |
| | (2,6-Dichloropyridin-4-yl)methyl (S)-2-amino-4-(3,3-difluoropiperidin-1-yl)butanoate dihydrochloride |
| | ¹H NMR (500 MHz, DMSO-d6) δ 11.3 (brs, 1H), 8.91 (brs, 3H), 7.74 (s, 2H), 5.39-5.27 'm, 2H), 4.53 (m, 1H), 4.17-2.84 (m, 6H), 2.47-1.62 (m, 6H) |
| | LC-MS (ESI) m/z [M+H]⁺: 381.99 |
| | tR 3.912 min (A) |

### Example 2: Confirmation of aminoacylation of tRNA and measurement of aminoacylation efficiency thereof

In the present embodiment, a tRNA was aminoacylated with the active ester synthesized in Example 1 as a substrate using a flexizyme. In order to conveniently confirm acylation with various amino acids, the acylation reaction was performed using, instead of an initiating tRNA (tRNA^{Met}), a microhelix corresponding to a short analog of the initiating tRNA and the solution after the reaction was subjected to polyacrylamide gel electrophoresis analysis under acidic conditions in order to confirm the aminoacylation efficiency. The mobility of the band derived from the microhelix is slow when it has been aminoacylated. The aminoacylation efficiency can be determined by comparing the intensity of the microhelix band to that of the acylated microhelix band.

### Example 2-1: Measurement of tRNA aminoacylation efficiency of (2,6-dichloropyridin-4-yl)methyl-L-isoleucinate (Compound No. 21) and (2,6-dichloropyridin-4-yl)methyl glycinate (Compound No. 99)

The acylation reaction was carried out overnight on ice in a 50 mM Hepes-K buffer (pH 7.5) and 50 mM MgCl₂, with a 25 M flexizyme (dFx), a 25 µM tRNA analog (microhelix) and a 5 mM substrate added to 20% DMSO. The nucleotide sequence of dFx is set forth in SEQ ID NO: 3.

The detailed procedure was as follows: 1 µL of a 500 mM Hepes-K buffer (pH 7.5), 1 µL of a 250 µM flexizyme (dFx) and 1 µL of a 250 µM tRNA analog were added to 3 µL of ultrapure water, and the mixture was heated at 95°C for 2 minutes, and allowed to cool at room temperature for 5 minutes. 2 µL of 250 mM MgCl₂ was added thereto, and the mixture was allowed to stand on ice for 1 minute. 1.5 µL of DMSO and a 0.5 µL of 100 mM substrate were added thereto to initiate the acylation reaction of the tRNA analog, and the mixture was allowed to stand on ice overnight. 1 µL of 3 M sodium acetate, pH 5.2 was added thereto to quench the reaction. This solution was analyzed with 20% modified PAGE (50 mM sodium acetate, 6 M urea) under acidic conditions.

The results of acylation using (2,6-dichloropyridin-4-yl)methyl L-isoleucinate (Compound No. 21) or (2,6-dichloropyridin-4-yl)methyl glycinate (Compound No. 99) as a substrate are shown in Fig. 1-1 and Fig. 1-2. From these results, it was confirmed that the amino acid derivatives were efficiently acylated by the flexizyme.

Example 2-2: Measurement of tRNA aminoacylation efficiency of 2,2,2-trifluoroethyl (S)-2-amino-4-phenylbutanoate (Compound No. 47) and 2,2,2-trifluoroethyl (S)-2-amino-3-(1H-pyrrolo[2,3-b]pyridin-3-yl)propanoate (Compound No. 81)

The acylation reaction was carried out overnight on ice in a 50 mM Hepes-K buffer (pH 7.5) and 600 mM MgCl₂, with a 25 µM flexizyme (eFx), a 25 µM tRNA analog (microhelix) and 5 mM substrate added to 20% DMSO. The nucleic acid sequence of eFx is set forth in SEQ ID NO: 2.

The detailed procedure was as follows: 4 µL of a 500 mM Hepes-K buffer (pH 7.5), 4 µL of a 250 µM flexizyme (eFx) and 4 µL of a 250 µM tRNA analog were added to 12 µL of ultrapure water, and the mixture was heated at 95°C for 3 minutes, and allowed to cool at room temperature for 5 minutes. 8 µL of 3 M MgCl₂ was added thereto, and the mixture was allowed to stand at room temperature for 5 minutes and then allowed to stand on ice for 5 minutes. 6 µL of DMSO and 2 µL of a 100 mM substrate were added thereto to initiate the acylation reaction of the tRNA analog, and the mixture was allowed to stand on ice overnight. 4 µL of 3 M sodium acetate, pH 5.2 was added thereto to quench the reaction. This solution was analyzed with 20% modified PAGE (50 mM sodium acetate, 6 M urea) under acidic conditions.

The results of acylation using 2,2,2-trifluoroethyl (S)-2-amino-4-phenylbutanoate (Compound No. 47) and 2,2,2-trifluoroethyl (S)-2-amino-3-(1H-pyrrolo[2,3-b]pyridin-3-yl)propanoate (Compound No. 81) as substrates are shown in Fig. 1-3 and Fig. 1-4. From these results, it was confirmed that the amino acid derivatives were efficiently acylated by the flexizyme.

### Test Example:

Each of Compounds 21, 47, 81 and 99 prepared in Example 1-1 was subjected to differential scanning calorimetry (DSC) to calculate the reaction starting temperature (T_{DSC}) and reaction heat (Q_{DSC}). The Q_{DSC} and T_{DSC} values of 2,4-dinitrotoluene (Compound No. 115) and benzoyl peroxide (Compound No. 116) were taken from the following literature: DSC Data Collection for Reactive Substances (2) (RIIS-SD-89), https://www.jniosh.johas.go.jp/publication/houkoku/houkoku_2007_03_list.html.

The detailed procedure for differential scanning calorimetry (DSC) is as follows:
The amino acid active ester (about 2 mg), which had been sealed in a SUS pressure-resistant cell, was set in a heat flux type differential scanning calorimeter, and heated to 500°C at 10°C /min in an atmosphere of nitrogen 50 mL/min. The sealing into the cell was carried out in atmospheric air, and a blank cell was used as a reference material. The value of indium (In) measured under the same conditions was used as a standard for correction.

The results are shown in Fig. 2. Fig. 2 shows the results of confirming the presence or absence of hazards for each amino acid active ester, by using 2,4-dinitrotoluene (Compound No. 115) and benzoyl peroxide (Compound No. 116) as reference compounds, plotting the reference points of Q_{DSC} and T_{DSC} for each of the compounds and drawing a straight line connecting the two points as the hazard determination line. In Fig. 2, the dotted line indicates the hazard determination line. If the chemical substance is on or above the determination line, it is considered "hazardous" (it falls under the Category V Hazardous materials), and if it is below the determination line, it is considered "not hazardous" (it does not fall under the Category V Hazardous materials). The abscissa axis indicates the log(T_{DSC}-25) value, and the ordinate axis indicates the logQ_{DSC} value. In Fig. 2, -L-Hph-DBE_HCl indicates the hydrochloride of 3,5-dinitrobenzyl (S)-2-amino-4-phenylbutanoate (Compound No. 114), Gly-DBE_HCl indicates the hydrochloride of 3,5-dinitrobenzyl glycinate (Compound No. 112), -L-Ile-DBE_HCl indicates the hydrochloride of 3,5-dinitrobenzyl L-isoleucinate (Compound No. 111), -L-W7N-DBE_HCl indicates the hydrochloride of 3,5-dinitrobenzyl (S)-2-amino-3-(1H-pyrrolo[2,3-b]pyridin-3-yl)propanoate (Compound No. 113), -L-Ile-DCPE_HCl indicates (2,6-dichloropyridin-4-yl)methyl L-isoleucinate (Compound No. 21), -L-W7N-TEE_HCl indicates 2,2,2-trifluoroethyl (S)-2-amino-3-(1H-pyrrolo[2,3-b]pyridin-3-yl)propanoate (Compound No. 81), and Gly-DCPE_HCl indicates (2,6-dichloropyridin-4-yl)methyl glycinate (Compound No. 99). L-Hph-DBE_HCl, Gly-DBE_HCl, L-Ile-DBE_HCl and L-W7N-DBE_HCl were plotted above the hazard determination line, and L-Ile-DCPE_HCl, L-W7N-TEE_HCl and Gly-DCPE_HCl were plotted below the hazard determination line (Fig. 2). The above indicated that it has been determined that DCPE and TEE do not fall under the Category V Hazardous materials.

## Claims

1. A compound represented by formula (Ia): 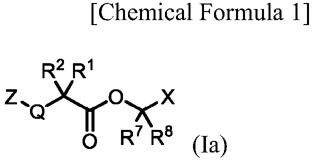 wherein:
X is pyridyl substituted with two or more halogen atoms, or a C₁₋₃ alkyl substituted with three or more fluorine atoms;
Z is -NR⁵R⁶ or -OR^{d};
Q is a group represented by 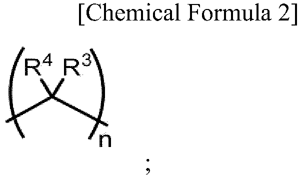
n is an integer from 0 to 3, and preferably an integer from 0 to 2;
R⁷ and R⁸ are each independently selected from a hydrogen atom or a C₁₋₃ alkyl;
R¹ and R² are each independently selected from a hydrogen atom, a halogen atom, cyano, a C₁₋₁₀ alkyl optionally substituted with one or more substituents selected from Y¹, a C₂₋₁₀ alkenyl optionally substituted with one or more substituents selected from Y¹, a C₂₋₁₀ alkynyl optionally substituted with one or more substituents selected from Y¹, a C₃₋₁₀ cycloalkyl optionally substituted with one or more substituents selected from Y², a C₃₋₁₀ cycloalkenyl optionally substituted with one or more substituents selected from Y², a 3- to 14-membered non-aromatic heterocyclyl optionally substituted with one or more substituents selected from Y², a C₆₋₁₄ aryl optionally substituted with one or more substituents selected from Y³, a 5- to 14-membered heteroaryl optionally substituted with one or more substituents selected from Y³, -COOR¹¹, and -CONR¹²R¹³; or
R¹ and R⁵, together with the carbon atom and the nitrogen atom, respectively, to which they are attached, form a 3- to 10-membered non-aromatic heterocyclic ring optionally substituted with one or more substituents selected from Y²;
when n is an integer from 1 to 3, R¹ and any one of R³, together with the carbon atoms to which they are attached, form a C₃₋₁₀ carbocyclic ring optionally substituted with one or more substituents selected from Y², or a 3- to 10-membered non-aromatic heterocyclic ring optionally substituted with one or more substituents selected from Y²;
R¹ and R², together with the carbon atom to which they are attached, form a C₃₋₁₀ carbocyclic ring optionally substituted with one or more substituents selected from Y², or a 3- to 10-membered non-aromatic heterocyclic ring optionally substituted with one or more substituents selected from Y²;
R³ and R⁴ are each independently selected from a hydrogen atom, a halogen atom, cyano, a C₁₋₁₀ alkyl optionally substituted with one or more substituents selected from Y¹, a C₂₋₁₀ alkenyl optionally substituted with one or more substituents selected from Y¹, a C₂₋₁₀ alkynyl optionally substituted with one or more substituents selected from Y¹, a C₃₋₁₀ cycloalkyl optionally substituted with one or more substituents selected from Y², a C₃₋₁₀ cycloalkenyl optionally substituted with one or more substituents selected from Y², a 3- to 14-membered non-aromatic heterocyclyl optionally substituted with one or more substituents selected from Y², a C₆₋₁₄ aryl optionally substituted with one or more substituents selected from Y³, and a 5- to 14-membered heteroaryl optionally substituted with one or more substituents selected from Y³, -COOR¹¹, and -CONR¹²R¹³; or
R³ and R⁴, together with the carbon atom to which they are attached, form a C₃₋₁₀ carbocyclic ring optionally substituted with one or more substituents selected from Y², or a 3- to 10-membered non-aromatic heterocyclic ring optionally substituted with one or more substituents selected from Y²;
when n is an integer from 1 to 3, R⁵ and any one of R³, together with the carbon atom to which they are attached, form a 3- to 10-membered nitrogen-containing non-aromatic heterocyclic ring optionally substituted with one or more substituents selected from Y²;
R¹¹, R¹² and R¹³ are each independently selected from a C₁₋₁₀ alkyl optionally substituted with one or more substituents selected from Y¹, a C₂₋₁₀ alkenyl optionally substituted with one or more substituents selected from Y¹, a C₂₋₁₀ alkynyl optionally substituted with one or more substituents selected from Y¹, a C₃₋₁₀ cycloalkyl optionally substituted with one or more substituents selected from Y², a C₃₋₁₀ cycloalkenyl optionally substituted with one or more substituents selected from Y², a 3- to 14-membered non-aromatic heterocyclyl optionally substituted with one or more substituents selected from Y², a C₆₋₁₄ aryl optionally substituted with one or more substituents selected from Y³, and a 5- to 14-membered heteroaryl optionally substituted with one or more substituents selected from Y³;
each Y¹ is independently selected from a halogen atom, nitro, cyano, a C₃₋₁₀ cycloalkyl optionally substituted with one or more substituents selected from Y⁵, a C₃₋₁₀ cycloalkenyl optionally substituted with one or more substituents selected from Y⁵, a 3- to 14-membered non-aromatic heterocyclyl optionally substituted with one or more substituents selected from Y⁵, a C₆₋₁₄ aryl optionally substituted with one or more substituents selected from Y⁶, a 5- to 14-membered heteroaryl optionally substituted with one or more substituents selected from Y⁶, -SH, -COOR²¹, -NR²²R²³, -N₃, -S(O)ₚR²⁴, -OR²⁵, -SO₂NR²⁶R²⁷ and - CONR²⁸R²⁹, and oxo;
each Y² is independently selected from a halogen atom, nitro, cyano, a C₁₋₁₀ alkyl optionally substituted with one or more substituents selected from Y⁴, a C₂₋₁₀ alkenyl optionally substituted with one or more substituents selected from Y⁴, a C₂₋₁₀ alkynyl optionally substituted with one or more substituents selected from Y⁴, a C₃₋₁₀ cycloalkyl optionally substituted with one or more substituents selected from Y⁵, a C₃₋₁₀ cycloalkenyl optionally substituted with one or more substituents selected from Y⁵, a 3- to 14-membered non-aromatic heterocyclyl optionally substituted with one or more substituents selected from Y⁵, a C₆₋₁₄ aryl optionally substituted with one or more substituents selected from Y⁶, a 5- to 14-membered heteroaryl optionally substituted with one or more substituents selected from Y⁶, -SH, -COOR²¹, -NR²²R²³, -S(O)ₚR²⁴, -OR²⁵, -SO₂NR²⁶R²⁷ and -CONR²⁸R²⁹, and oxo;
each Y³ is independently selected from a halogen atom, nitro, cyano, a C₁₋₁₀ alkyl optionally substituted with one or more substituents selected from Y⁴, a C₂₋₁₀ alkenyl optionally substituted with one or more substituents selected from Y⁴, a C₂₋₁₀ alkynyl optionally substituted with one or more substituents selected from Y⁴, a C₃₋₁₀ cycloalkyl optionally substituted with one or more substituents selected from Y⁵, a C₃₋₁₀ cycloalkenyl optionally substituted with one or more substituents selected from Y⁵, a 3- to 14-membered non-aromatic heterocyclyl optionally substituted with one or more substituents selected from Y⁵, a C₆₋₁₄ aryl optionally substituted with one or more substituents selected from Y⁶, a 5- to 14-membered heteroaryl optionally substituted with one or more substituents selected from Y⁶, -SH, -COOR²¹, -NR²²R²³, -S(O)ₚR²⁴, -OR²⁵, -SO₂NR²⁶R²⁷ and -CONR²⁸R²⁹;
R²¹, R²⁶, R²⁷, R²⁸ and R²⁹ are each independently selected from a hydrogen atom, a C₁₋₁₀ alkyl optionally substituted with one or more substituents selected from Y⁴, a C₂₋₁₀ alkenyl optionally substituted with one or more substituents selected from Y⁴, a C₂₋₁₀ alkynyl optionally substituted with one or more substituents selected from Y⁴, a C₃₋₁₀ cycloalkyl optionally substituted with one or more substituents selected from Y⁵, a C₃₋₁₀ cycloalkenyl optionally substituted with one or more substituents selected from Y⁵, a 3- to 14-membered non-aromatic heterocyclyl optionally substituted with one or more substituents selected from Y⁵, a C₆₋₁₄ aryl optionally substituted with one or more substituents selected from Y⁶, and a 5- to 14-membered heteroaryl optionally substituted with one or more substituents selected from Y⁶;
R²² and R²⁵ are each independently selected from a hydrogen atom, a C₁₋₁₀ alkyl optionally substituted with one or more substituents selected from Y⁴, a C₂₋₁₀ alkenyl optionally substituted with one or more substituents selected from Y⁴, a C₂₋₁₀ alkynyl optionally substituted with one or more substituents selected from Y⁴, a C₃₋₁₀ cycloalkyl optionally substituted with one or more substituents selected from Y⁵, a C₃₋₁₀ cycloalkenyl optionally substituted with one or more substituents selected from Y⁵, a 3- to 14-membered non-aromatic heterocyclyl optionally substituted with one or more substituents selected from Y⁵, a C₆₋₁₄ aryl optionally substituted with one or more substituents selected from Y⁶, a 5- to 14-membered heteroaryl optionally substituted with one or more substituents selected from Y⁶, -COR^{a1}, -CONR^{a2}R^{a3}, -COOR^{a4}, -C(=NR^{a5})NR^{a6}R^{a7}, -S(O)ₚR²⁴ and -SO₂NR^{a8}R^{a9};
R²³ is selected from a hydrogen atom, a C₁₋₁₀ alkyl optionally substituted with one or more substituents selected from Y⁴, a C₂₋₁₀ alkenyl optionally substituted with one or more substituents selected from Y⁴, a C₂₋₁₀ alkynyl optionally substituted with one or more substituents selected from Y⁴, a C₃₋₁₀ cycloalkyl optionally substituted with one or more substituents selected from Y⁵, a C₃₋₁₀ cycloalkenyl optionally substituted with one or more substituents selected from Y⁵, a 3- to 14-membered non-aromatic heterocyclyl optionally substituted with one or more substituents selected from Y⁵, a C₆₋₁₄ aryl optionally substituted with one or more substituents selected from Y⁶, and a 5- to 14-membered heteroaryl optionally substituted with one or more substituents selected from Y⁶;
R²⁴ is selected from a C₁₋₁₀ alkyl optionally substituted with one or more substituents selected from Y⁴, a C₂₋₁₀ alkenyl optionally substituted with one or more substituents selected from Y⁴, a C₂₋₁₀ alkynyl optionally substituted with one or more substituents selected from Y⁴, a C₃₋₁₀ cycloalkyl optionally substituted with one or more substituents selected from Y⁵, a C₃₋₁₀ cycloalkenyl optionally substituted with one or more substituents selected from Y⁵, a 3-to 14-membered non-aromatic heterocyclyl optionally substituted with one or more substituents selected from Y⁵, a C₆₋₁₄ aryl optionally substituted with one or more substituents selected from Y⁶, and a 5- to 14-membered heteroaryl optionally substituted with one or more substituents selected from Y⁶;
R^{a1}, R^{a2}, R^{a3}, R^{a4}, R^{a5}, R^{a6}, R^{a7}, R^{a8} and R^{a9} are each independently selected from a hydrogen atom, a C₁₋₁₀ alkyl optionally substituted with one or more substituents selected from Y⁴, a C₂₋₁₀ alkenyl optionally substituted with one or more substituents selected from Y⁴, a C₂₋₁₀ alkynyl optionally substituted with one or more substituents selected from Y⁴, a C₃₋₁₀ cycloalkyl optionally substituted with one or more substituents selected from Y⁵, a C₃₋₁₀ cycloalkenyl optionally substituted with one or more substituents selected from Y⁵, a 3- to 14-membered non-aromatic heterocyclyl optionally substituted with one or more substituents selected from Y⁵, a C₆₋₁₄ aryl optionally substituted with one or more substituents selected from Y⁶, and a 5- to 14-membered heteroaryl optionally substituted with one or more substituents selected from Y⁶;
each Y⁴ is independently selected from a halogen atom, nitro, cyano, a C₃₋₁₀ cycloalkyl optionally substituted with one or more substituents selected from Y⁵, a C₃₋₁₀ cycloalkenyl optionally substituted with one or more substituents selected from Y⁵, a 3- to 14-membered non-aromatic heterocyclyl optionally substituted with one or more substituents selected from Y⁵, a C₆₋₁₄ aryl optionally substituted with one or more substituents selected from Y⁶, a 5- to 14-membered heteroaryl optionally substituted with one or more substituents selected from Y⁶, -SH, -COOR³¹, -NR³²R³³, -S(O)ₚR³⁴, -OR³⁵, -SO₂NR³⁶R³⁷ and - CONR³⁸R³⁹, and oxo;
each Y⁵ is independently selected from a halogen atom, nitro, cyano, a C₁₋₁₀ alkyl optionally substituted with one or more substituents selected from Y⁷, a C₂₋₁₀ alkenyl optionally substituted with one or more substituents selected from Y⁷, a C₂₋₁₀ alkynyl optionally substituted with one or more substituents selected from Y⁷, a C₃₋₁₀ cycloalkyl optionally substituted with one or more substituents selected from Y⁸, a C₃₋₁₀ cycloalkenyl optionally substituted with one or more substituents selected from Y⁸, a 3- to 14-membered non-aromatic heterocyclyl optionally substituted with one or more substituents selected from Y⁸, a C₆₋₁₄ aryl optionally substituted with one or more substituents selected from Y⁹, a 5- to 14-membered heteroaryl optionally substituted with one or more substituents selected from Y⁹, -SH, -COOR³¹, -NR³²R³³, -S(O)ₚR³⁴, -OR³⁵, -SO₂NR³⁶R³⁷, -CONR³⁸R³⁹, and oxo;
each Y⁶ is independently selected from a halogen atom, nitro, cyano, a C₁₋₁₀ alkyl optionally substituted with one or more substituents selected from Y⁷, a C₂₋₁₀ alkenyl optionally substituted with one or more substituents selected from Y⁷, a C₂₋₁₀ alkynyl optionally substituted with one or more substituents selected from Y⁷, a C₃₋₁₀ cycloalkyl optionally substituted with one or more substituents selected from Y⁸, a C₃₋₁₀ cycloalkenyl optionally substituted with one or more substituents selected from Y⁸, a 3- to 14-membered non-aromatic heterocyclyl optionally substituted with one or more substituents selected from Y⁸, a C₆₋₁₄ aryl optionally substituted with one or more substituents selected from Y⁹, a 5- to 14-membered heteroaryl optionally substituted with one or more substituents selected from Y⁹, -SH, -COOR³¹, -NR³²R³³, -S(O)ₚR³⁴, -OR³⁵, -SO₂NR³⁶R³⁷, and -CONR³⁸R³⁹;
R³¹, R³⁶, R³⁷, R³⁸ and R³⁹ are each independently selected from a hydrogen atom, a C₁₋₁₀ alkyl optionally substituted with one or more substituents selected from Y⁷, a C₂₋₁₀ alkenyl optionally substituted with one or more substituents selected from Y⁷, a C₂₋₁₀ alkynyl optionally substituted with one or more substituents selected from Y⁷, a C₃₋₁₀ cycloalkyl optionally substituted with one or more substituents selected from Y⁸, a C₃₋₁₀ cycloalkenyl optionally substituted with one or more substituents selected from Y⁸, a 3- to 14-membered non-aromatic heterocyclyl optionally substituted with one or more substituents selected from Y⁸, a C₆₋₁₄ aryl optionally substituted with one or more substituents selected from Y⁹, and a 5- to 14-membered heteroaryl optionally substituted with one or more substituents selected from Y⁹;
R³² and R³⁵ are each independently selected from a hydrogen atom, a C₁₋₁₀ alkyl optionally substituted with one or more substituents selected from Y⁷, a C₂₋₁₀ alkenyl optionally substituted with one or more substituents selected from Y⁷, a C₂₋₁₀ alkynyl optionally substituted with one or more substituents selected from Y⁷, a C₃₋₁₀ cycloalkyl optionally substituted with one or more substituents selected from Y⁸, a C₃₋₁₀ cycloalkenyl optionally substituted with one or more substituents selected from Y⁸, a 3- to 14-membered non-aromatic heterocyclyl optionally substituted with one or more substituents selected from Y⁸, a C₆₋₁₄ aryl optionally substituted with one or more substituents selected from Y⁹, a 5- to 14-membered heteroaryl optionally substituted with one or more substituents selected from Y⁹, -COR^{b1}, -CONR^{b2}R^{b3}, -CO₂R^{b4}, -C(=NR^{b5})NR^{b6}R^{b7} and -SO₂NR^{b8}R^{b9};
R³³ is selected from a hydrogen atom, a C₁₋₁₀ alkyl optionally substituted with one or more substituents selected from Y⁷, a C₂₋₁₀ alkenyl optionally substituted with one or more substituents selected from Y⁷, a C₂₋₁₀ alkynyl optionally substituted with one or more substituents selected from Y⁷, a C₃₋₁₀ cycloalkyl optionally substituted with one or more substituents selected from Y⁸, a C₃₋₁₀ cycloalkenyl optionally substituted with one or more substituents selected from Y⁸, a 3- to 14-membered non-aromatic heterocyclyl optionally substituted with one or more substituents selected from Y⁸, a C₆₋₁₄ aryl optionally substituted with one or more substituents selected from Y⁹, and a 5- to 14-membered heteroaryl optionally substituted with one or more substituents selected from Y⁹;
R³⁴ is selected from a C₁₋₁₀ alkyl optionally substituted with one or more substituents selected from Y⁷, a C₂₋₁₀ alkenyl optionally substituted with one or more substituents selected from Y⁷, a C₂₋₁₀ alkynyl optionally substituted with one or more substituents selected from Y⁷, a C₃₋₁₀ cycloalkyl optionally substituted with one or more substituents selected from Y⁸, a C₃₋₁₀ cycloalkenyl optionally substituted with one or more substituents selected from Y⁸, a 3-to 14-membered non-aromatic heterocyclyl optionally substituted with one or more substituents selected from Y⁸, a C₆₋₁₄ aryl optionally substituted with one or more substituents selected from Y⁹, and a 5- to 14-membered heteroaryl optionally substituted with one or more substituents selected from Y⁹;
R^{b1}, R^{b2}, R^{b3}, R^{b4}, R^{b5}, R^{b6}, R^{b7}, R^{b8} and R^{b9} are each independently selected from a hydrogen atom, a C₁₋₁₀ alkyl optionally substituted with one or more substituents selected from Y⁷, a C₂₋₁₀ alkenyl optionally substituted with one or more substituents selected from Y⁷, a C₂₋₁₀ alkynyl optionally substituted with one or more substituents selected from Y⁷, a C₃₋₁₀ cycloalkyl optionally substituted with one or more substituents selected from Y⁸, a C₃₋₁₀ cycloalkenyl optionally substituted with one or more substituents selected from Y⁸, a 3- to 14-membered non-aromatic heterocyclyl optionally substituted with one or more substituents selected from Y⁸, a C₆₋₁₄ aryl optionally substituted with one or more substituents selected from Y⁹, and a 5- to 14-membered heteroaryl optionally substituted with one or more substituents selected from Y⁹;
each Y⁷ is independently selected from a halogen atom, nitro, cyano, a C₁₋₁₀ alkyl optionally substituted with one or more substituents selected from Y¹⁰, a C₂₋₁₀ alkenyl optionally substituted with one or more substituents selected from Y¹⁰, a C₂₋₁₀ alkynyl optionally substituted with one or more substituents selected from Y¹⁰, a C₃₋₁₀ cycloalkyl optionally substituted with one or more substituents selected from Y¹¹, a C₃₋₁₀ cycloalkenyl optionally substituted with one or more substituents selected from Y¹¹, a 3- to 14-membered non-aromatic heterocyclyl optionally substituted with one or more substituents selected from Y¹¹, a C₆₋₁₄ aryl optionally substituted with one or more substituents selected from Y¹², a 5- to 14-membered heteroaryl optionally substituted with one or more substituents selected from Y¹², -SH, -COOR⁴⁰, -NR⁴¹R⁴², -S(O)ₚR⁴³, -OR⁴⁴, -SO₂NR⁴⁵R⁴⁶ and -CONR⁴⁷R⁴⁸, and oxo;
each Y⁸ is independently selected from a halogen atom, nitro, cyano, a C₁₋₁₀ alkyl optionally substituted with one or more substituents selected from Y¹⁰, a C₂₋₁₀ alkenyl optionally substituted with one or more substituents selected from Y¹⁰, a C₂₋₁₀ alkynyl optionally substituted with one or more substituents selected from Y¹⁰, a C₃₋₁₀ cycloalkyl optionally substituted with one or more substituents selected from Y¹¹, a C₃₋₁₀ cycloalkenyl optionally substituted with one or more substituents selected from Y¹¹, a 3- to 14-membered non-aromatic heterocyclyl optionally substituted with one or more substituents selected from Y¹¹, a C₆₋₁₄ aryl optionally substituted with one or more substituents selected from Y¹², a 5- to 14-membered heteroaryl optionally substituted with one or more substituents selected from Y¹², -SH, -COOR⁴⁰, -NR⁴¹R⁴², -S(O)ₚR⁴³, -OR⁴⁴, -SO₂NR⁴⁵R⁴⁶, -CONR⁴⁷R⁴⁸, and oxo;
each Y⁹ is independently selected from a halogen atom, nitro, cyano, a C₁₋₁₀ alkyl optionally substituted with one or more substituents selected from Y¹⁰, a C₂₋₁₀ alkenyl optionally substituted with one or more substituents selected from Y¹⁰, a C₂₋₁₀ alkynyl optionally substituted with one or more substituents selected from Y¹⁰, a C₃₋₁₀ cycloalkyl optionally substituted with one or more substituents selected from Y¹¹, a C₃₋₁₀ cycloalkenyl optionally substituted with one or more substituents selected from Y¹¹, a 3- to 14-membered non-aromatic heterocyclyl optionally substituted with one or more substituents selected from Y¹¹, a C₆₋₁₄ aryl optionally substituted with one or more substituents selected from Y¹², a 5- to 14-membered heteroaryl optionally substituted with one or more substituents selected from Y¹², -SH, -COOR⁴⁰, -NR⁴¹R⁴², -S(O)ₚR⁴³, -OR⁴⁴, -SO₂NR⁴⁵R⁴⁶ and -CONR⁴⁷R⁴⁸;
R⁴⁰, R⁴⁵, R⁴⁶, R⁴⁷ and R⁴⁸ are each independently selected from a hydrogen atom, a C₁₋₁₀ alkyl optionally substituted with one or more substituents selected from Y¹⁰, a C₂₋₁₀ alkenyl optionally substituted with one or more substituents selected from Y¹⁰, a C₂₋₁₀ alkynyl optionally substituted with one or more substituents selected from Y¹⁰, a C₃₋₁₀ cycloalkyl optionally substituted with one or more substituents selected from Y¹¹, a C₃₋₁₀ cycloalkenyl optionally substituted with one or more substituents selected from Y¹¹, a 3- to 14-membered non-aromatic heterocyclyl optionally substituted with one or more substituents selected from Y¹¹, a C₆₋₁₄ aryl optionally substituted with one or more substituents selected from Y¹², and a 5- to 14-membered heteroaryl optionally substituted with one or more substituents selected from Y¹²,
R⁴¹ and R⁴⁴ are each independently selected from a hydrogen atom, a C₁₋₁₀ alkyl optionally substituted with one or more substituents selected from Y¹⁰, a C₂₋₁₀ alkenyl optionally substituted with one or more substituents selected from Y¹⁰, a C₂₋₁₀ alkynyl optionally substituted with one or more substituents selected from Y¹⁰, a C₃₋₁₀ cycloalkyl optionally substituted with one or more substituents selected from Y¹¹, a C₃₋₁₀ cycloalkenyl optionally substituted with one or more substituents selected from Y¹¹, a 3- to 14-membered non-aromatic heterocyclyl optionally substituted with one or more substituents selected from Y¹¹, a C₆₋₁₄ aryl optionally substituted with one or more substituents selected from Y¹², a 5- to 14-membered heteroaryl optionally substituted with one or more substituents selected from Y¹², -COR^{c1}, -CONR^{c2}R^{c3}, -COOR^{c4}, -C(=NR^{c5})NR^{c6}R^{c7} , and -SO₂NR^{c8}R^{c9};
R⁴² is selected from a hydrogen atom, a C₁₋₁₀ alkyl optionally substituted with one or more substituents selected from Y¹⁰, a C₂₋₁₀ alkenyl optionally substituted with one or more substituents selected from Y¹⁰, a C₂₋₁₀ alkynyl optionally substituted with one or more substituents selected from Y¹⁰, a C₃₋₁₀ cycloalkyl optionally substituted with one or more substituents selected from Y¹¹, a C₃₋₁₀ cycloalkenyl optionally substituted with one or more substituents selected from Y¹¹, a 3- to 14-membered non-aromatic heterocyclyl optionally substituted with one or more substituents selected from Y¹¹, a C₆₋₁₄ aryl optionally substituted with one or more substituents selected from Y¹², and a 5- to 14-membered heteroaryl optionally substituted with one or more substituents selected from Y¹²,
R⁴³ is selected from a C₁₋₁₀ alkyl optionally substituted with one or more substituents selected from Y¹⁰, a C₂₋₁₀ alkenyl optionally substituted with one or more substituents selected from Y¹⁰, a C₂₋₁₀ alkynyl optionally substituted with one or more substituents selected from Y¹⁰, a C₃₋₁₀ cycloalkyl optionally substituted with one or more substituents selected from Y¹¹, a C₃₋₁₀ cycloalkenyl optionally substituted with one or more substituents selected from Y¹¹, a 3- to 14-membered non-aromatic heterocyclyl optionally substituted with one or more substituents selected from Y¹¹, a C₆₋₁₄ aryl optionally substituted with one or more substituents selected from Y¹², and a 5- to 14-membered heteroaryl optionally substituted with one or more substituents selected from Y¹²,
R^{c1}, R^{c2}, R^{c3}, R^{c4}, R^{c5} , R^{c6}, R^{c7}, R^{c8} and R^{c9} are each independently selected from a hydrogen atom, a C₁₋₁₀ alkyl optionally substituted with one or more substituents selected from Y¹⁰, a C₂₋₁₀ alkenyl optionally substituted with one or more substituents selected from Y¹⁰, a C₂₋₁₀ alkynyl optionally substituted with one or more substituents selected from Y¹⁰, a C₃₋₁₀ cycloalkyl optionally substituted with one or more substituents selected from Y¹¹, a C₃₋₁₀ cycloalkenyl optionally substituted with one or more substituents selected from Y¹¹, a 3- to 14-membered non-aromatic heterocyclyl optionally substituted with one or more substituents selected from Y¹¹, a C₆₋₁₄ aryl optionally substituted with one or more substituents selected from Y¹², and a 5- to 14-membered heteroaryl optionally substituted with one or more substituents selected from Y¹²,
each Y¹⁰ is independently selected from a halogen atom, nitro, cyano, a C₁₋₁₀ alkoxy selected from a halogen atom and an alkoxy, a C₃₋₁₀ cycloalkyl optionally substituted with one or more substituents selected from a halogen atom and a C₁₋₆ alkoxy, a C₃₋₁₀ cycloalkenyl optionally substituted with one or more substituents selected from a halogen atom and a C₁₋₆ alkoxy, a 3- to 14-membered non-aromatic heterocyclyl optionally substituted with one or more substituents selected from a halogen atom and a C₁₋₆ alkoxy, a C₆₋₁₄ aryl optionally substituted with one or more substituents selected from a halogen atom and a C₁₋₆ alkoxy, a 5-to 14-membered heteroaryl optionally substituted with one or more substituents selected from a halogen atom and a C₁₋₆ alkoxy, a (C₁₋₁₀ alkoxy)carbonyl optionally substituted with one or more substituents selected from a halogen atom and a C₁₋₆ alkoxy, a di(C₁₋₆ alkyl)aminocarbonyl optionally substituted with one or more substituents selected from a halogen atom and a C₁₋₆ alkoxy, a C₁₋₆ alkoxy optionally substituted with one or more substituents selected from a halogen atom and a C₁₋₆ alkoxy, a C₁₋₆ alkylthio optionally substituted with one or more substituents selected from a halogen atom and a C₁₋₆ alkoxy, a C₁₋₆ alkylsulfanyl optionally substituted with one or more substituents selected from a halogen atom and a C₁₋₆ alkoxy, a C₁₋₆ alkylsulfinyl optionally substituted with one or more substituents selected from a halogen atom and a C₁₋₆ alkoxy, a C₁₋₆ alkylsulfonyl optionally substituted with one or more substituents selected from a halogen atom and a C₁₋₆ alkoxy, and oxo;
each Y¹¹ is independently selected from a halogen atom, nitro, cyano, a C₁₋₁₀ alkyl optionally substituted with one or more substituents selected from a halogen atom and an alkoxy, a C₂₋₁₀ alkenyl optionally substituted with one or more substituents selected from a halogen atom and an alkoxy, a C₂₋₁₀ alkynyl optionally substituted with one or more substituents selected from a halogen atom and an alkoxy, a C₁₋₁₀ alkoxy optionally substituted with one or more substituents selected from a halogen atom and an alkoxy, a C₃₋₁₀ cycloalkyl optionally substituted with one or more substituents selected from a halogen atom and a C₁₋₆ alkoxy, a C₃₋₁₀ cycloalkenyl optionally substituted with one or more substituents selected from a halogen atom and a C₁₋₆ alkoxy, a 3- to 14-membered non-aromatic heterocyclyl optionally substituted with one or more substituents selected from a halogen atom and a C₁₋₆ alkoxy, a C₆₋₁₄ aryl optionally substituted with one or more substituents selected from a halogen atom and a C₁₋₆ alkoxy, a 5- to 14-membered heteroaryl optionally substituted with one or more substituents selected from a halogen atom and a C₁₋₆ alkoxy, a (C₁₋₁₀ alkoxy)carbonyl optionally substituted with one or more substituents selected from a halogen atom and a C₁₋₆ alkoxy, a di(C₁₋₆ alkyl)aminocarbonyl optionally substituted with one or more substituents selected from a halogen atom and a C₁₋₆ alkoxy, a C₁₋₆ alkoxy optionally substituted with one or more substituents selected from a halogen atom and a C₁₋₆ alkoxy, a C₁₋₆ alkylthio optionally substituted with one or more substituents selected from a halogen atom and a C₁₋₆ alkoxy, a C₁₋₆ alkylsulfanyl optionally substituted with one or more substituents selected from a halogen atom and a C₁₋₆ alkoxy, a C₁₋₆ alkylsulfinyl optionally substituted with one or more substituents selected from a halogen atom and a C₁₋₆ alkoxy, a C₁₋₆ alkylsulfonyl optionally substituted with one or more substituents selected from a halogen atom and a C₁₋₆ alkoxy, and oxo;
each Y¹² is independently selected from a halogen atom, nitro, cyano, a C₁₋₁₀ alkyl optionally substituted with one or more substituents selected from a halogen atom and an alkoxy, a C₂₋₁₀ alkenyl optionally substituted with one or more substituents selected from a halogen atom and an alkoxy, a C₂₋₁₀ alkynyl optionally substituted with one or more substituents selected from a halogen atom and an alkoxy, a C₁₋₁₀ alkoxy optionally substituted with one or more substituents selected from a halogen atom and an alkoxy, a C₃₋₁₀ cycloalkyl optionally substituted with one or more substituents selected from a halogen atom and a C₁₋₆ alkoxy, a C₃₋₁₀ cycloalkenyl optionally substituted with one or more substituents selected from a halogen atom and a C₁₋₆ alkoxy, a 3- to 14-membered non-aromatic heterocyclyl optionally substituted with one or more substituents selected from a halogen atom and a C₁₋₆ alkoxy, a C₆₋₁₄ aryl optionally substituted with one or more substituents selected from a halogen atom and a C₁₋₆ alkoxy, a 5- to 14-membered heteroaryl optionally substituted with one or more substituents selected from a halogen atom and a C₁₋₆ alkoxy, a (C₁₋₁₀ alkoxy)carbonyl optionally substituted with one or more substituents selected from a halogen atom and a C₁₋₆ alkoxy, a di(C₁₋₆ alkyl)aminocarbonyl optionally substituted with one or more substituents selected from a halogen atom and a C₁₋₆ alkoxy, a C₁₋₆ alkoxy optionally substituted with one or more substituents selected from a halogen atom and a C₁₋₆ alkoxy, a C₁₋₆ alkylthio optionally substituted with one or more substituents selected from a halogen atom and a C₁₋₆ alkoxy, a C₁₋₆ alkylsulfanyl optionally substituted with one or more substituents selected from a halogen atom and a C₁₋₆ alkoxy, a C₁₋₆ alkylsulfinyl optionally substituted with one or more substituents selected from a halogen atom and a C₁₋₆ alkoxy, and a C₁₋₆ alkylsulfonyl optionally substituted with one or more substituents selected from a halogen atom and a C₁₋₆ alkoxy;
each p is independently an integer from 0 to 2;
R⁵ is selected from a hydrogen atom, benzyl optionally substituted on the benzene ring with one or more substituents selected from Y¹³, benzyloxycarbonyl optionally substituted on the benzene ring with one or more substituents selected from Y¹³, phenylcarbonyl and (C₁₋₁₀ alkoxy)carbonyl optionally substituted with one or more substituents selected from Y¹³, (C₁₋₁₀ alkyl)carbonyl optionally substituted with a halogen atom, a C₁₋₁₀ alkyl optionally substituted with one or more substituents selected from Y¹, a C₂₋₁₀ alkenyl optionally substituted with one or more substituents selected from Y¹, a C₂₋₁₀ alkynyl optionally substituted with one or more substituents selected from Y¹, a C₃₋₁₀ cycloalkyl optionally substituted with one or more substituents selected from Y², a C₃₋₁₀ cycloalkenyl optionally substituted with one or more substituents selected from Y², a 3- to 14-membered non-aromatic heterocyclyl optionally substituted with one or more substituents selected from Y², a C₆₋₁₄ aryl optionally substituted with one or more substituents selected from Y³, and a 5- to 14-membered heteroaryl optionally substituted with one or more substituents selected from Y³;
R⁶ is selected from a hydrogen atom, benzyl optionally substituted on the benzene ring with one or more substituents selected from Y¹³, benzyloxycarbonyl optionally substituted on the benzene ring with one or more substituents selected from Y¹³, and phenylcarbonyl, (C₁₋₁₀ alkoxy)carbonyl and (C₁₋₁₀ alkyl)carbonyl optionally substituted with one or more substituents selected from Y¹³; or
R⁵ and R⁶, together with the nitrogen atom to which they are attached, form a 5- to 14-membered nitrogen-containing heterocyclyl having an imide structure, or a 3- to 14-membered non-aromatic heterocyclyl containing, as ring atoms, two or more nitrogen atoms optionally substituted with one or more substituents selected from Y²;
R^{d} is selected from a hydrogen atom, benzyl optionally substituted on the benzene ring with one or more substituents selected from Y¹³, benzyloxycarbonyl optionally substituted on the benzene ring with one or more substituents selected from Y¹³, and phenylcarbonyl, (C₁₋₁₀ alkoxy)carbonyl and (C₁₋₁₀ alkyl)carbonyl optionally substituted with one or more substituents selected from Y¹³;
each Y¹³ is independently selected from a halogen atom, C₁₋₁₀ alkoxy, and C₁₋₁₀ alkyl;
when the 3- to 14-membered non-aromatic heterocyclyl is a monocyclic heterocyclyl, the heterocyclyl is optionally fused with a benzene ring; and
when the C₆₋₁₄ aryl is phenyl, the phenyl is optionally fused with a 5- to 7-membered non-aromatic heterocyclic ring;
or a salt thereof, except for the compound of formula (Ia) wherein Z is hydroxy, n is 0, R⁷ and
R⁸ are each hydrogen atom, X is 2,6-dichloropyridin-4-yl, and;
R¹ and R² are each a hydrogen atom; or
R¹ is a hydrogen atom and R² is methyl, ethyl, isopropyl, n-butyl, tert-butyl or isobutyl; or
R¹ is ethyl and R¹ is methyl or ethyl.

2. The compound or a salt thereof according to claim 1, wherein Z is -NR⁵R⁶.

3. The compound or a salt thereof according to claim 1 or 2, wherein n is 0 or 1.

4. The compound or a salt thereof according to any one of claims 1 to 3, wherein R¹ is hydrogen atom.

5. The compound or a salt thereof according to any one of claims 1 to 4, wherein
R⁷ and R⁸ are each a hydrogen atom.

6. The compound or a salt thereof according to any one of claims 1 to 5, wherein X is 2,6-dihalo-4-pyridyl.

7. The compound or a salt thereof according to any one of claims 1 to 5, wherein X is 2,6-dichloro-4-pyridyl.

8. The compound or a salt thereof according to any one of claims 1 to 5, wherein X is a perfluoro-C₁₋₃ alkyl.

9. The compound or a salt thereof according to any one of claims 1 to 5, wherein X is trifluoromethyl.

10. A composition for use in the acylation of a tRNA, comprising the compound or a salt thereof according to claims 1 to 9.

11. The composition according to claim 10 for use in the acylation of a tRNA in the presence of a flexizyme.

12. A method for producing a compound represented by formula (Ia): 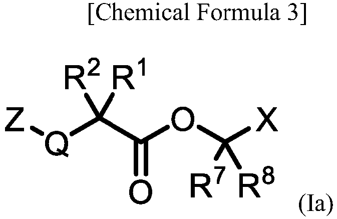
wherein R¹, R², R⁷, R⁸, Q, X and Z are as defined in any one of claims 1 to 9;
the method comprising reacting a compound represented by formula (IIa): 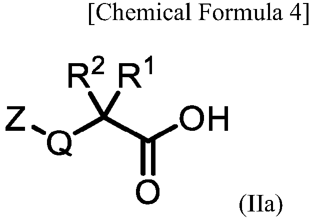 with a compound represented by formula (III): 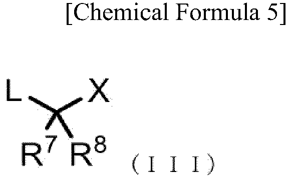
wherein L is a leaving group.

13. The method according to claim 12, wherein the reaction is carried out in the presence of a base.

14. The method according to claim 13, wherein the base is diisopropylethylamine.

15. A method for producing a tRNA acylated at the 3'-terminus, wherein:
the acyl group at the 3'-terminus is a group represented by: 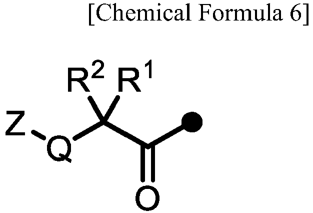
wherein R¹, R², R⁵, R⁶, Q and Z are as defined in any one of claims 1 to 9;
the method comprising reacting a compound represented by formula (Ia): 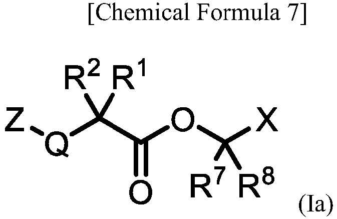
wherein R¹, R², R⁵, R⁶, R⁷, R⁸, Q, X and Z are as defined in any one of claims 1 to 9 with the tRNA in the presence of a flexizyme.

16. A method for preparing a peptide library, comprising:
producing a tRNA acylated at the 3'-terminus by the method according to claim 14;
preparing an mRNA library; and
synthesizing a peptide corresponding to each mRNA from the mRNA library with a cell-free translation system to prepare a peptide library.
